# EUROPEAN PATENT APPLICATION

(11) **EP 4 803 540 A1**
(43) Date of publication of application: **09.09.2026**
(21) Application number: 24886232.8
(22) Date of filing: 30.10.2024
(51) Int. Cl.: C07K 16/24, A61K 39/00

(54) **NOVEL FUNCTION OF CSF3 INHIBITOR AND USE THEREOF**

(30) Priority: 30.10.2023 KR 20230146774
(71) Applicant: FNCTBiotech Inc., Seoul 04626 (KR)
(72) Inventor: JIN, Young Woo, Seoul 04354 (KR); LEE, Su Jae, Seoul 03168 (KR); KIM, Min Jung, Seoul 01040 (KR)
(74) Representative: dompatent
(86) International application number: PCT/KR2024/016743
(87) International publication number: WO 2025/095559

(57) **Abstract**

Disclosed herein is a therapeutic agent for fibrosis comprising a CSF3 inhibitor. Also disclosed herein is a therapeutic agent for fibrosis comprising a CSF3R inhibitor. Also disclosed herein is a novel anti-CSF3 antibody.

## Description

### Technical Field

The present disclosure relates to: the mechanism of action underlying the effect of CSF3 inhibitors in restoring fibrotic tissue to normal tissue; the mechanism of action underlying the effect of CSF3R inhibitors in restoring fibrotic tissue to normal tissue; therapeutic agents and methods for treating fibrosis and related diseases; and technologies related to anti-CSF3 antibodies.

### Background Art

Fibrosis is a condition in which extracellular matrix, most notably collagen, accumulates excessively in tissue. When fibrosis occurs, the tissue loses its original function and becomes hardened due to extracellular matrix deposition. Fibrosis can develop in nearly all tissues of the body, and it is known that fibrosis in many tissues can cause a wide variety of diseases, disorders, and/or conditions. Fibrosis may be caused by sustained stimulation or severe damage to tissue, and is also the final, common pathological outcome of many chronic inflammatory diseases.

CSF3 (Colony-Stimulating Factor 3), also referred to as granulocyte colony-stimulating factor (G-CSF), is known to stimulate the proliferation, differentiation, adhesion, and terminal functional activation of granulocytes.

### Detailed Description of the Invention

### Technical Problem

The present specification aims to elucidate the mechanism of fibrosis treatment associated with CSF3 or CSF3R inhibition, and further to provide methods of treating fibrosis, therapeutic agents for fibrosis, uses of related substances, and related antibodies utilizing the same.

### Solution to Problem

To solve the above technical problem, the present specification provides: the mechanism of action underlying the effect of CSF3 inhibitors in restoring fibrotic tissue to normal tissue; and the mechanism of action underlying the effect of CSF3R inhibitors in restoring fibrotic tissue to normal tissue. Also, the present invention provides: the methods of treating fibrosis using said CSF3 inhibitors or said CSF3R inhibitors. The inventors of the present specification confirmed that the therapeutic effects of CSF3 inhibitors and CSF3R inhibitors on fibrosis occur regardless of the type of fibrotic tissue, and therefore may constitute an effective mechanism of treatment for all types of fibrosis. In the present specification, anti-CSF3 antibodies and siRNAs targeting the CSF3 gene are presented as representative means for inhibiting CSF3, and their efficacy has been demonstrated experimentally. In addition, anti-CSF3R antibodies and siRNAs targeting the CSF3R gene are presented as representative means for inhibiting CSF3R, and their efficacy has been demonstrated experimentally.

### Advantageous Effects of the Invention

By using the method of treating fibrosis with CSF3 inhibitors or CSF3R inhibitors provided herein, fibrosis can be prevented or treated regardless of the type of tissue in which it occurs.

### Brief Description of Drawings

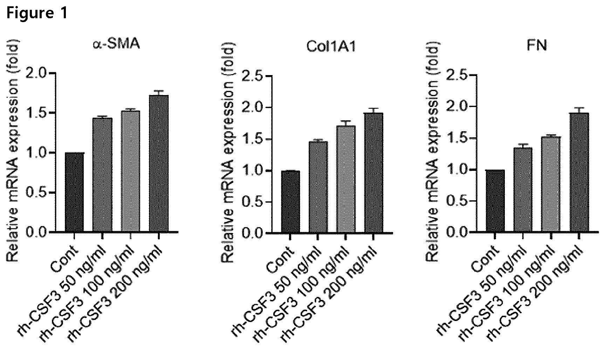

FIG. 1 shows the results of qPCR analysis of mRNA expression levels of α-SMA, Col1A, and Fibronectin in normal lung fibroblasts (MRC5) treated with recombinant CSF3 protein and analyzed 24 hours later, according to Experimental Example 2.1. Here, Cont refers to the control group treated with PBS, and rh-CSF3 50 ng/ml, rh-CSF3 100 ng/ml, and rh-CSF3 200 ng/ml refer to groups treated with recombinant CSF3 protein at concentrations of 50 ng/ml, 100 ng/ml, and 200 ng/ml, respectively.

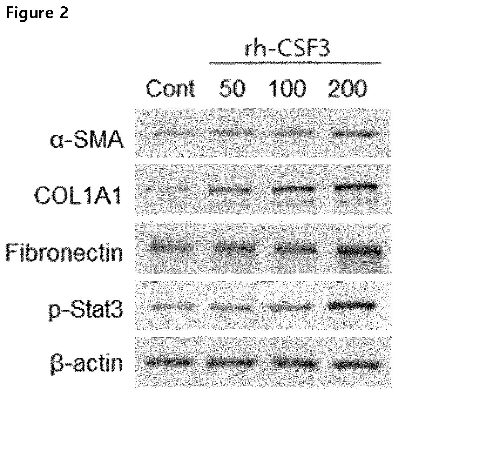

FIG. 2 shows the results of Western blot analysis of α-SMA, Col1A, and Fibronectin protein expression and Stat3 activation (p-Stat3) in normal lung fibroblasts (MRC5) treated with recombinant CSF3 protein and analyzed 24 hours later, according to Experimental Example 2.1. Here, Cont refers to the control group treated with PBS, and 50, 100, and 200 under rh-CSF3 refer to groups treated with recombinant CSF3 protein at concentrations of 50 ng/ml, 100 ng/ml, and 200 ng/ml, respectively.

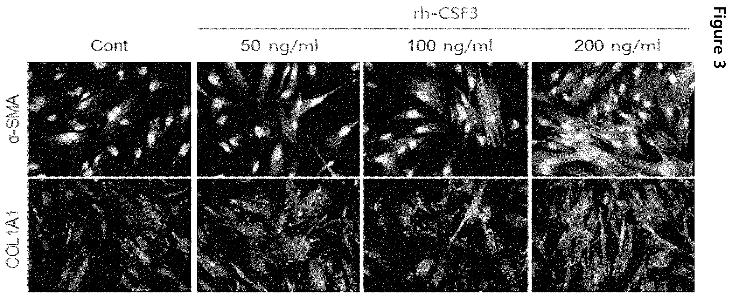

FIG. 3 shows the results of immunofluorescence staining analysis of α-SMA and Col1A proteins in normal lung fibroblasts (MRC5) treated with recombinant CSF3 protein and analyzed 24 hours later, according to Experimental Example 2.1. Here, Cont refers to the control group treated with PBS, and 50 ng/ml, 100 ng/ml, and 200 ng/ml under rh-CSF3 refer to groups treated with recombinant CSF3 protein at concentrations of 50 ng/ml, 100 ng/ml, and 200 ng/ml, respectively.

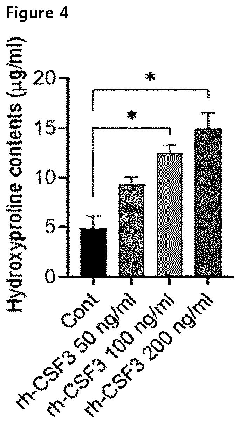

FIG. 4 shows the results of hydroxyproline assay analysis of collagen secretion and accumulation in normal lung fibroblasts (MRC5) treated with recombinant CSF3 protein and analyzed 24 hours later, according to Experimental Example 2.1. Here, Cont refers to the control group treated with PBS, and rh-CSF3 50 ng/ml, rh-CSF3 100 ng/ml, and rh-CSF3 200 ng/ml refer to groups treated with recombinant CSF3 protein at concentrations of 50 ng/ml, 100 ng/ml, and 200 ng/ml, respectively.

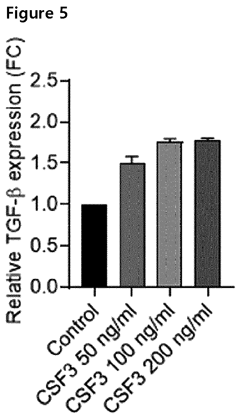

FIG. 5 shows the results of qPCR analysis of TGF-β mRNA expression levels in normal lung fibroblasts (MRC5) treated with recombinant CSF3 protein and analyzed 24 hours later, according to Experimental Example 2.2. Here, Control refers to the control group treated with PBS, and rh-CSF3 50 ng/ml, rh-CSF3 100 ng/ml, and rh-CSF3 200 ng/ml refer to groups treated with recombinant CSF3 protein at concentrations of 50 ng/ml, 100 ng/ml, and 200 ng/ml, respectively.

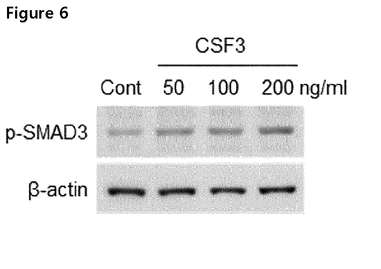

FIG. 6 shows the results of Western blot analysis of Smad3 activation (p-Smad3) in normal lung fibroblasts (MRC5) treated with recombinant CSF3 protein and analyzed 24 hours later, according to Experimental Example 2.2. Here, Cont refers to the control group treated with PBS, and 50, 100, and 200 under rh-CSF3 refer to groups treated with recombinant CSF3 protein at concentrations of 50 ng/ml, 100 ng/ml, and 200 ng/ml, respectively.

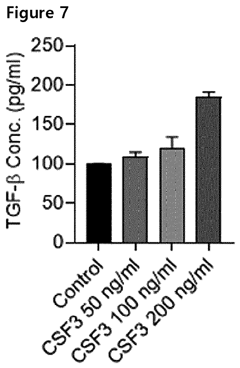

FIG. 7 shows the results of TGF-β ELISA assay analysis of TGF-β protein concentration in normal lung fibroblasts (MRC5) treated with recombinant CSF3 protein and analyzed 24 hours later, according to Experimental Example 2.2. Here, Control refers to the control group treated with PBS, and rh-CSF3 50 ng/ml, rh-CSF3 100 ng/ml, and rh-CSF3 200 ng/ml refer to groups treated with recombinant CSF3 protein at concentrations of 50 ng/ml, 100 ng/ml, and 200 ng/ml, respectively.

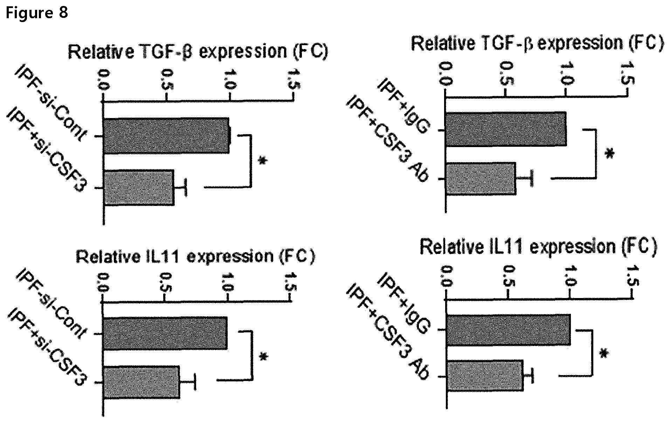

FIG. 8 shows the results of qPCR analysis of TGF-β and IL-11 mRNA expression levels in lung fibroblasts (DHLF-IPF) treated with a non-CSF3-targeting antibody, an anti-CSF3 antibody, a non-CSF3/CSF3R-targeting siRNA (hereinafter "si-cont"), and a CSF3 mRNA-targeting siRNA (hereinafter "si-CSF3"), respectively, according to Experimental Example 2.3. Here, IPF+IgG refers to the group treated with the non-CSF3-targeting antibody; IPF+CSF3 Ab refers to the group treated with the anti-CSF3 antibody; IPF+si-Cont refers to the group treated with si-cont; and IPF+si-CSF3 refers to the group treated with si-CSF3.

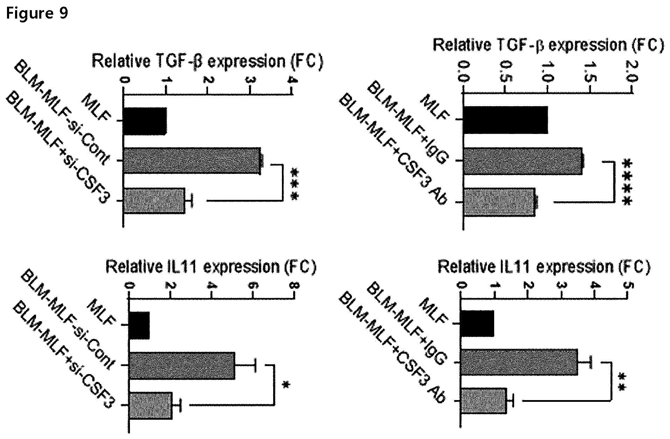

FIG. 9 shows the results of qPCR analysis of TGF-β and IL-11 mRNA expression levels in lung fibroblasts isolated from bleomycin-induced pulmonary fibrosis mouse lung tissue (BLM-MLF) treated with a non-CSF3-targeting antibody, an anti-CSF3 antibody, si-cont, and si-CSF3, respectively, according to Experimental Example 2.3. Here, MLF refers to the group of lung fibroblasts (MLF) isolated from normal mouse lung tissue; BLM-MLF+IgG refers to the group treated with the non-CSF3-targeting antibody; BLM-MLF+CSF3 Ab refers to the group treated with the anti-CSF3 antibody; BLM-MLF+si-Cont refers to the group treated with si-cont; and BLM-MLF+si-CSF3 refers to the group treated with si-CSF3.

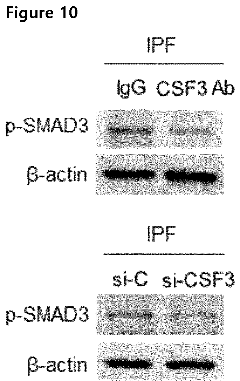

FIG. 10 shows the results of Western Blot analysis of SMAD3 activation (p-SMAD3) in lung fibroblasts (DHLF-IPF) treated with a non-CSF3-targeting antibody, an anti-CSF3 antibody, si-cont, and si-CSF3, respectively, according to Experimental Example 2.3. Here, IgG refers to the group treated with the non-CSF3-targeting antibody; CSF3 Ab refers to the group treated with the anti-CSF3 antibody; si-C refers to the group treated with si-cont; and si-CSF3 refers to the group treated with si-CSF3.

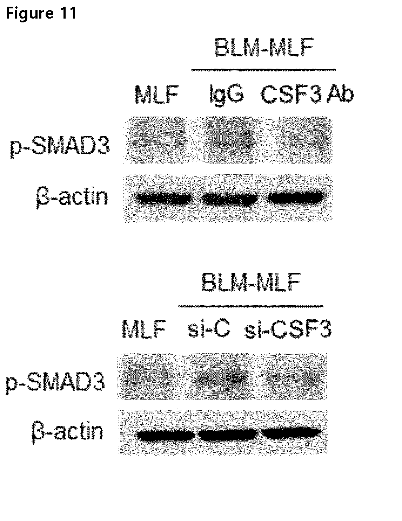

FIG. 11 shows the results of Western Blot analysis of SMAD3 activation (p-SMAD3) in lung fibroblasts isolated from bleomycin-induced pulmonary fibrosis mouse lung tissue (BLM-MLF) treated with a non-CSF3-targeting antibody, si-cont, and si-CSF3, respectively, according to Experimental Example 2.3. Here, MLF refers to the group of lung fibroblasts (MLF) isolated from normal mouse lung tissue; IgG refers to the group treated with the non-CSF3-targeting antibody; CSF3 Ab refers to the group treated with the anti-CSF3 antibody; si-C refers to the group treated with si-cont; and si-CSF3 refers to the group treated with si-CSF3.

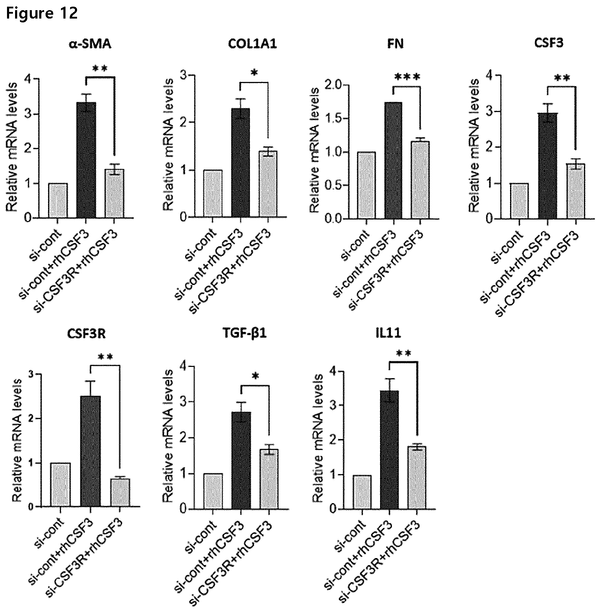

FIG. 12 shows the results of qPCR analysis of mRNA expression levels of α-SMA, Col1A1, FN, CSF3, CSF3R, TGF-β, and IL11 in normal lung fibroblasts (MRC5) treated with si-cont, recombinant CSF3 protein, and a CSF3R mRNA-targeting siRNA (hereinafter "si-CSF3R"), respectively, according to Experimental Example 2.4. Here, si-cont refers to the group treated with si-cont; si-cont+rhCSF3 refers to the group treated with si-cont and recombinant CSF3 protein; and si-CSF3R+rhCSF3 refers to the group treated with si-CSF3R and recombinant CSF3 protein.

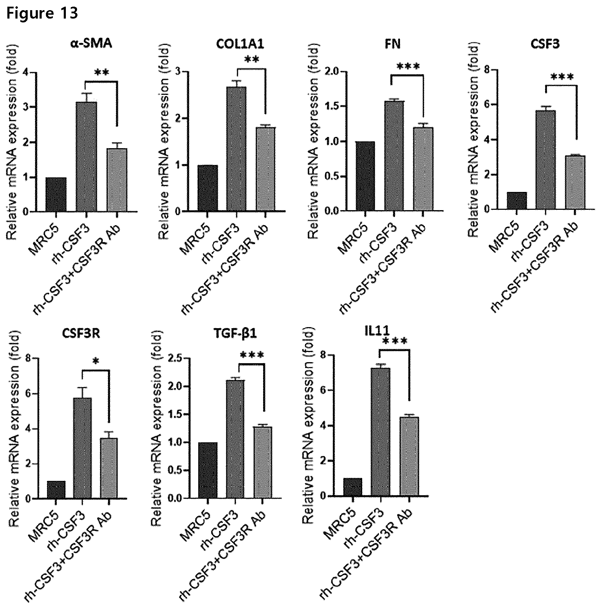

FIG. 13 shows the results of qPCR analysis of mRNA expression levels of α-SMA, Col1A1, FN, CSF3, CSF3R, TGF-β, and IL11 in normal lung fibroblasts (MRC5) treated with recombinant CSF3 protein and an anti-CSF3R antibody, respectively, according to Experimental Example 2.4. Here, MRC5 refers to the control group; rh-CSF3 refers to the group treated with recombinant CSF3 protein; and rh-CSF3+CSF3R Ab refers to the group treated with recombinant CSF3 protein and the anti-CSF3R antibody.

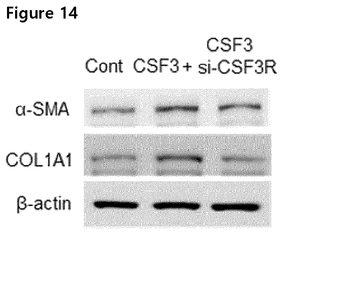

FIG. 14 shows the results of Western Blot analysis of α-SMA and Col1A1 protein expression in normal lung fibroblasts (MRC5) treated with recombinant CSF3 protein and si-CSF3R, respectively, according to Experimental Example 2.4. Here, Cont refers to the control group; CSF3 refers to the group treated with recombinant CSF3 protein; and CSF3+si-CSF3R refers to the group treated with recombinant CSF3 protein and si-CSF3R.

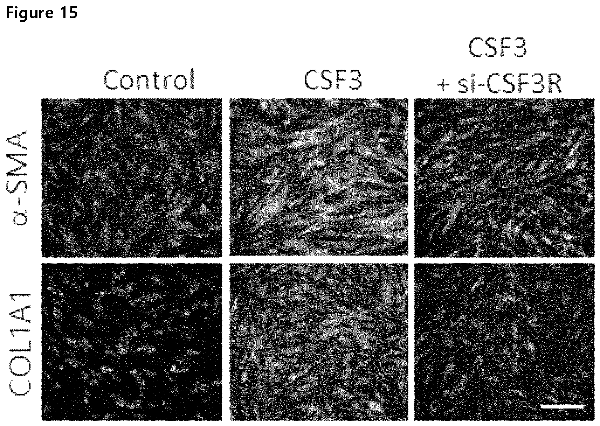

FIG. 15 shows the results of immunofluorescence staining analysis of α-SMA and Col1A1 protein expression in normal lung fibroblasts (MRC5) treated with recombinant CSF3 protein and si-CSF3R, respectively, according to Experimental Example 2.4. Here, Control refers to the control group; CSF3 refers to the group treated with recombinant CSF3 protein; and CSF3+si-CSF3R refers to the group treated with recombinant CSF3 protein and si-CSF3R.

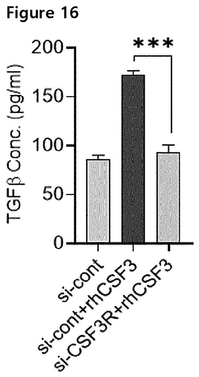

FIG. 16 shows the results of TGF-β ELISA assay analysis of TGF-β protein concentration in normal lung fibroblasts (MRC5) treated with si-cont, recombinant CSF3 protein, and si-CSF3R, respectively, according to Experimental Example 2.4. Here, si-cont refers to the group treated with si-cont; si-cont+rhCSF3 refers to the group treated with si-cont and recombinant CSF3 protein; and si-CSF3R+rhCSF3 refers to the group treated with recombinant CSF3 protein and si-CSF3R.

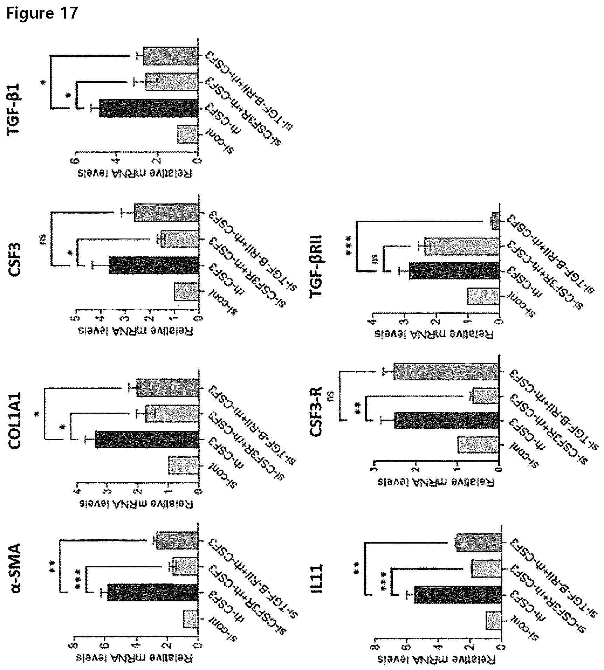

FIG. 17 shows the results of qPCR analysis of mRNA expression levels of α-SMA, Col1A1, CSF3, TGF-β, IL11, CSF3R, and TGF-βRII in normal lung fibroblasts (MRC5) treated with si-cont, recombinant CSF3 protein, si-CSF3R, and a TGF-β receptor 2 (TGF-βRII) mRNA-targeting siRNA (hereinafter "si-TGF-βRII"), respectively, and analyzed 24 hours later, according to Experimental Example 2.5. Here, si-cont refers to the group treated with si-cont; rh-CSF3 refers to the group treated with recombinant CSF3 protein; si-CSF3R+rh-CSF3 refers to the group treated with si-CSF3R and recombinant CSF3 protein; and si-TGF-B-RII+rhCSF3 refers to the group treated with si-TGF-βRII and recombinant CSF3 protein.

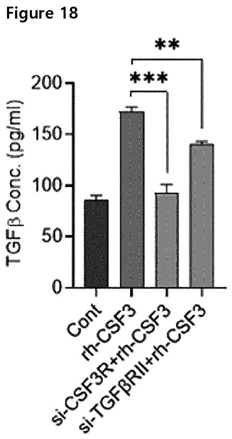

FIG. 18 shows the results of TGF-β ELISA assay analysis of TGF-β protein concentration in normal lung fibroblasts (MRC5) treated with si-cont, recombinant CSF3 protein, si-CSF3R, and si-TGF-βRII, respectively, and analyzed 24 hours later, according to Experimental Example 2.5. Here, Cont refers to the control group; rh-CSF3 refers to the group treated with recombinant CSF3 protein; si-CSF3R+rh-CSF3 refers to the group treated with si-CSF3R and recombinant CSF3 protein; and si-TGF-B-RII+rhCSF3 refers to the group treated with si-TGF-βRII and recombinant CSF3 protein.

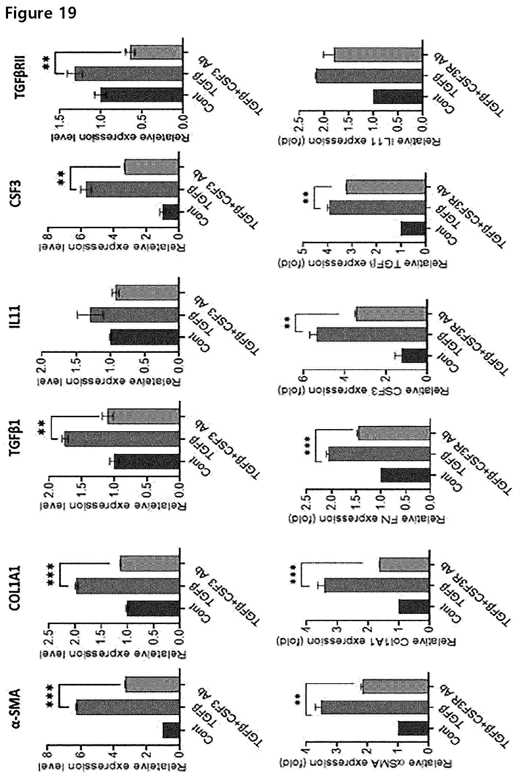

FIG. 19 shows the results of qPCR analysis of mRNA expression levels of α-SMA, Col1A1, FN, CSF3, TGF-β, IL11, and CSF3R in normal lung fibroblasts (MRC5) treated with TGF-β, an anti-CSF3 antibody, and an anti-CSF3R antibody, respectively, and analyzed 24 hours later, according to Experimental Example 2.6. Here, Cont refers to the control group; TGFβ refers to the group treated with TGF-β; TGFβ+CSF3 Ab refers to the group treated with TGF-β and the anti-CSF3 antibody; and TGFβ+CSF3R Ab refers to the group treated with TGF-β and the anti-CSF3R antibody.

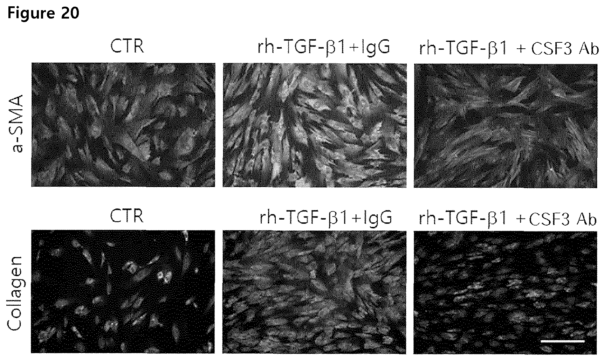

FIG. 20 shows the results of immunofluorescence staining analysis of α-SMA and Col1A1 protein expression in normal lung fibroblasts (MRC5) treated with TGF-β and an anti-CSF3 antibody, respectively, and analyzed 24 hours later, according to Experimental Example 2.6. Here, rh-TGF-β1+IgG refers to the group treated with TGF-β and a non-CSF3-binding antibody; and rh-TGF-β1+CSF3 Ab refers to the group treated with TGF-β and the anti-CSF3 antibody.

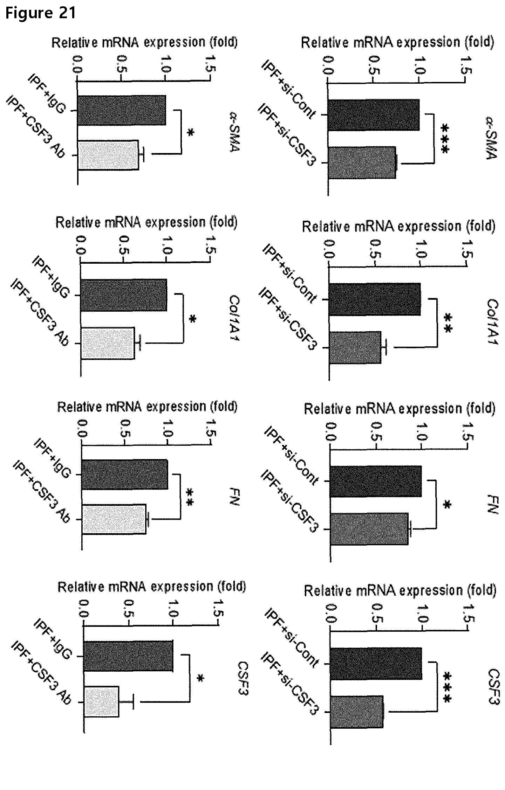

FIG. 21 shows the results of qPCR analysis of mRNA expression levels of α-SMA, Col1A1, FN, and CSF3 in DHLF-IPF cells treated with si-cont, si-CSF3, a non-CSF3-targeting antibody, and an anti-CSF3 antibody, respectively, according to Experimental Examples 3.1 and 3.1.1. Here, IPF+si-Cont refers to the group treated with si-cont; IPF+si-CSF3 refers to the group treated with si-CSF3; IPF+IgG refers to the group treated with the non-CSF3-targeting antibody; and IPF+CSF3 Ab refers to the group treated with the anti-CSF3 antibody.

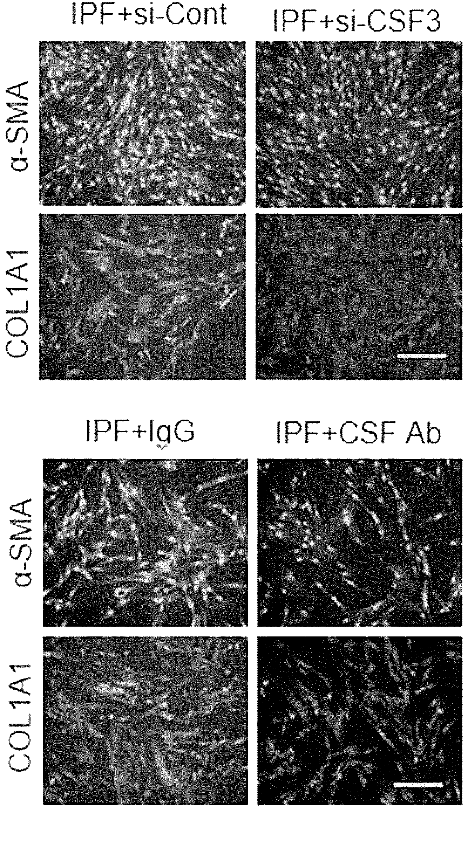

FIG. 22 shows the results of immunofluorescence staining analysis of α-SMA and Col1A1 protein expression in DHLF-IPF cells treated with si-cont, si-CSF3, a non-CSF3-targeting antibody, and an anti-CSF3 antibody, respectively, according to Experimental Example 3.1.1. Here, IPF+si-Cont refers to the group treated with si-cont; IPF+si-CSF3 refers to the group treated with si-CSF3; IPF+IgG refers to the group treated with the non-CSF3-targeting antibody; and IPF+CSF3 Ab refers to the group treated with the anti-CSF3 antibody.

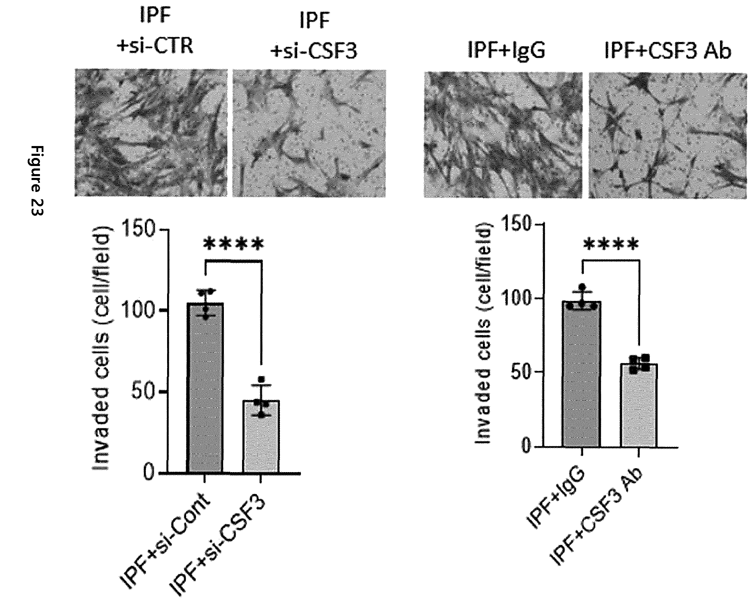

FIG. 23 shows the results of transwell cell culture chamber analysis of changes in the invasive capacity of lung fibroblasts treated with si-cont, si-CSF3, a non-CSF3-targeting antibody, and an anti-CSF3 antibody, respectively, in DHLF-IPF cells, according to Experimental Example 3.1.1. Here, IPF+si-Cont refers to the group treated with si-cont; IPF+si-CSF3 refers to the group treated with si-CSF3; IPF+IgG refers to the group treated with the non-CSF3-targeting antibody; and IPF+CSF3 Ab refers to the group treated with the anti-CSF3 antibody.

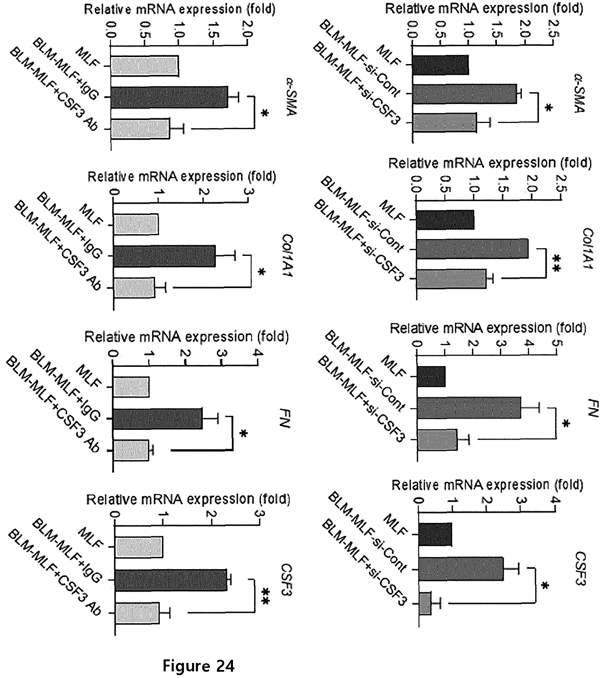

FIG. 24 shows the results of qPCR analysis of mRNA expression levels of α-SMA, Col1A1, FN, and CSF3 in BLM-MLF cells treated with si-cont, si-CSF3, a non-CSF3-targeting antibody, and an anti-CSF3 antibody, respectively, according to Experimental Example 3.1.2. Here, BLM+si-Cont refers to the group treated with si-cont; BLM+si-CSF3 refers to the group treated with si-CSF3; BLM+IgG refers to the group treated with the non-CSF3-targeting antibody; and BLM+CSF3 Ab refers to the group treated with the anti-CSF3 antibody.

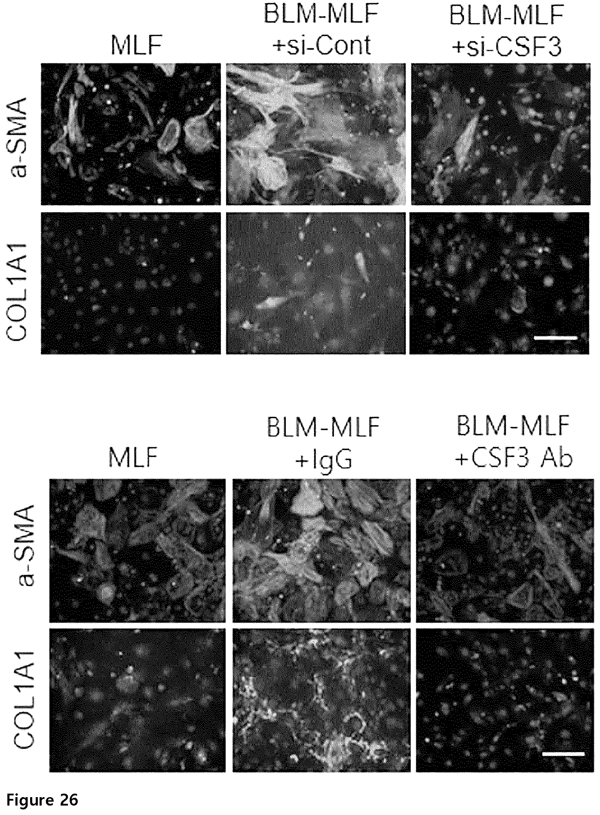

FIG. 25 shows the results of immunofluorescence staining analysis of α-SMA and Col1A1 protein expression in BLM-MLF cells treated with si-cont, si-CSF3, a non-CSF3-targeting antibody, and an anti-CSF3 antibody, respectively, according to Experimental Example 3.1.2. Here, BLM+si-Cont refers to the group treated with si-cont; BLM+si-CSF3 refers to the group treated with si-CSF3; BLM+IgG refers to the group treated with the non-CSF3-targeting antibody; and BLM+CSF3 Ab refers to the group treated with the anti-CSF3 antibody.

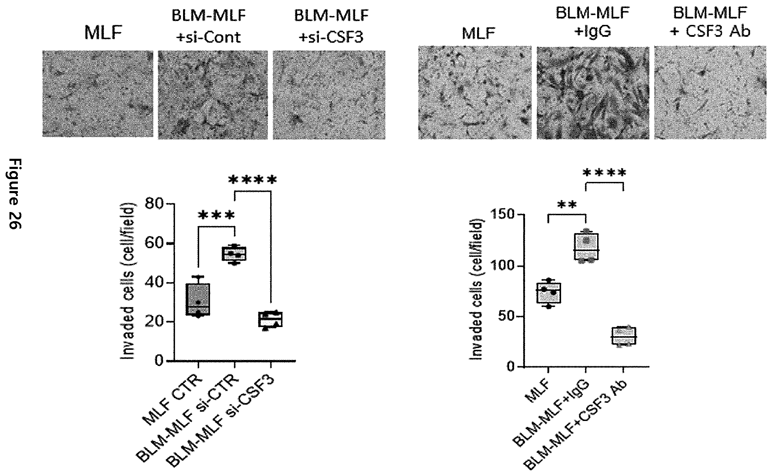

FIG. 26 shows the results of transwell cell culture chamber analysis of changes in the invasive capacity of lung fibroblasts in BLM-MLF cells treated with si-cont, si-CSF3, a non-CSF3-targeting antibody, and an anti-CSF3 antibody, respectively, according to Experimental Example 3.1.2. Here, BLM+si-Cont refers to the group treated with si-cont; BLM+si-CSF3 refers to the group treated with si-CSF3; BLM+IgG refers to the group treated with the non-CSF3-targeting antibody; and BLM+CSF3 Ab refers to the group treated with the anti-CSF3 antibody.

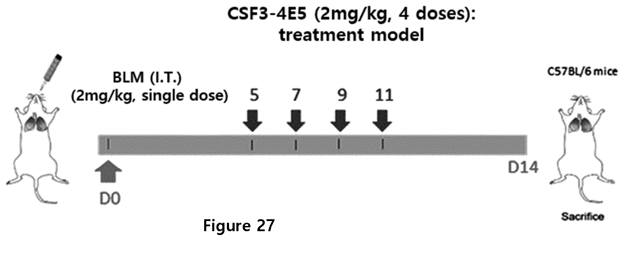

FIG. 27 is a schematic diagram of the experimental model used to conduct the animal study of Experimental Example 3.2.

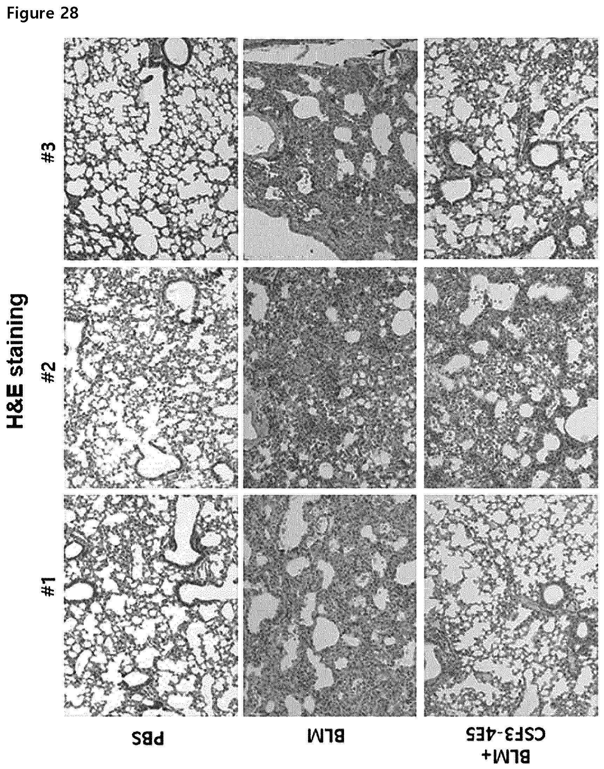

FIG. 28 shows the results of H&E staining analysis of morphological changes in lung tissue obtained from mice treated with PBS, bleomycin (BLM), and the anti-CSF3 antibody, respectively, according to Experimental Example 3.2.2.

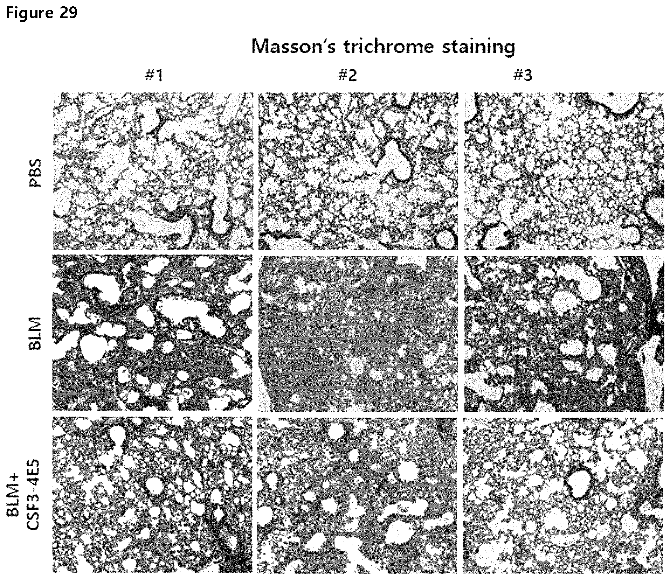

FIG. 29 shows the results of Masson's trichrome staining analysis of collagen accumulation in lung tissue obtained from mice treated with PBS, bleomycin, and the anti-CSF3 antibody, respectively, according to Experimental Example 3.2.2.
In FIGS. 28 and 29, #1, #2, and #3 represent lung tissues obtained from different individual mice; PBS refers to the group treated with PBS; BLM refers to the group treated with bleomycin; and BLM+CSF3-4E5 refers to the group treated with bleomycin and the anti-CSF3 antibody.
FIG. 30 shows the results of hydroxyproline content measurement analysis of collagen content in lung tissue obtained from mice treated with PBS, bleomycin, and the anti-CSF3 antibody, respectively, according to Experimental Example 3.2.3.
FIG. 31 shows the results of qPCR analysis of α-SMA and Col1A1 mRNA expression levels in lung tissue obtained from mice treated with PBS, bleomycin, and the anti-CSF3 antibody, respectively, according to Experimental Example 3.2.4.
FIG. 32 shows the results of TGF-β ELISA assay analysis of TGF-β protein secretion in lung tissue obtained from mice treated with PBS, bleomycin, and the anti-CSF3 antibody, respectively, according to Experimental Example 3.2.4.
FIG. 33 shows the results of qPCR analysis of TGF-β mRNA expression levels (FIG. 33A) and Western Blot analysis of SMAD3 protein phosphorylation (p-SMAD3) (FIG. 33B) in lung tissue obtained from mice treated with PBS, bleomycin, and the anti-CSF3 antibody, respectively, according to Experimental Example 3.2.4.

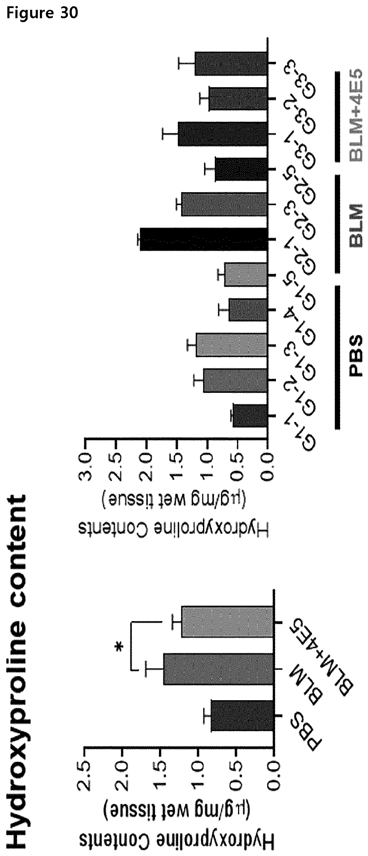

In FIGS. 30 to 33, PBS refers to the group treated with PBS; BLM refers to the group treated with bleomycin; and BLM+CSF3-4E5 or BLM+4E5 refers to the group treated with bleomycin and the anti-CSF3 antibody. Here, G1-1, G1-2, G1-3, G1-4, and G1-5 each refer to different individual mice in the PBS-treated group; G2-1, G2-3, and G2-5 each refer to different individual mice in the bleomycin-treated group; and G3-1, G3-2, and G3-3 each refer to different individual mice in the bleomycin- and the anti-CSF3 antibody-treated group.

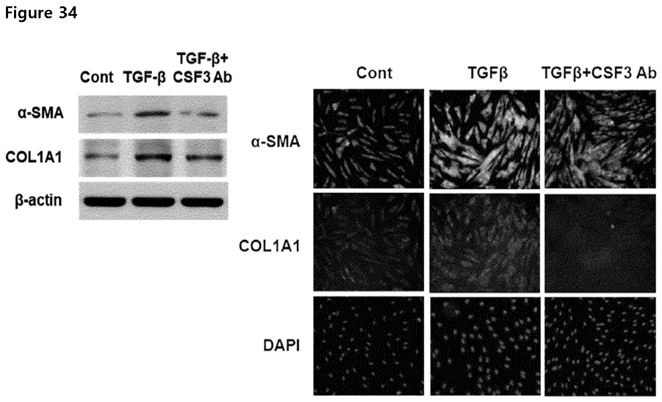

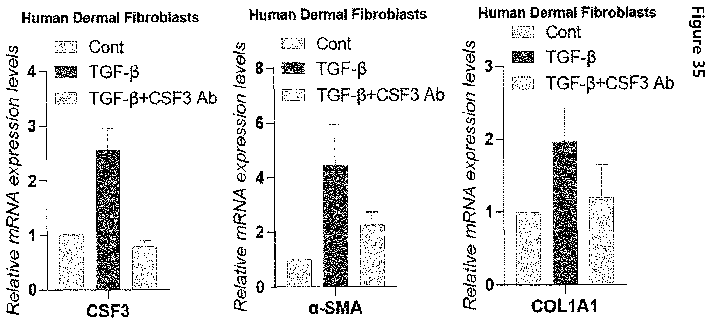

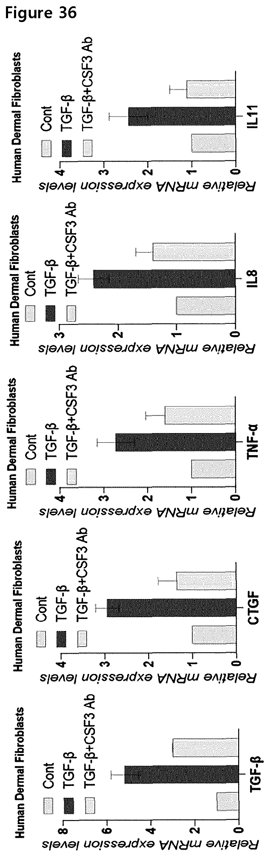

FIGS. 34, 35, and 36 relate to expression levels of CSF3, α-SMA, Col1A1, TGF-β, CTGF, TNF-α, IL8, and IL11 in skin fibroblasts treated with PBS, TGF-β, and/or CSF3 antibody, according to Experimental Examples 3.3.1 and 3.3.2. The left panel of FIG. 34 shows expression levels of α-SMA and Col1A1 proteins as determined by Western blot. The right panel of FIG. 34 shows expression levels of α-SMA and Col1A1 proteins as determined by fluorescence staining. FIG. 35 shows mRNA expression levels of CSF3, α-SMA, and Col1A1 as determined by qPCR. FIG. 36 shows mRNA expression levels of TGF-β, CTGF, TNF-α, IL8, and IL11 as determined by qPCR. In FIGS. 34, 35, and 36, Cont refers to the group treated with PBS only; TGF-β refers to the group treated with TGF-β only; and TGF-β+CSF3 Ab refers to the group treated with TGF-β and the anti-CSF3 antibody.

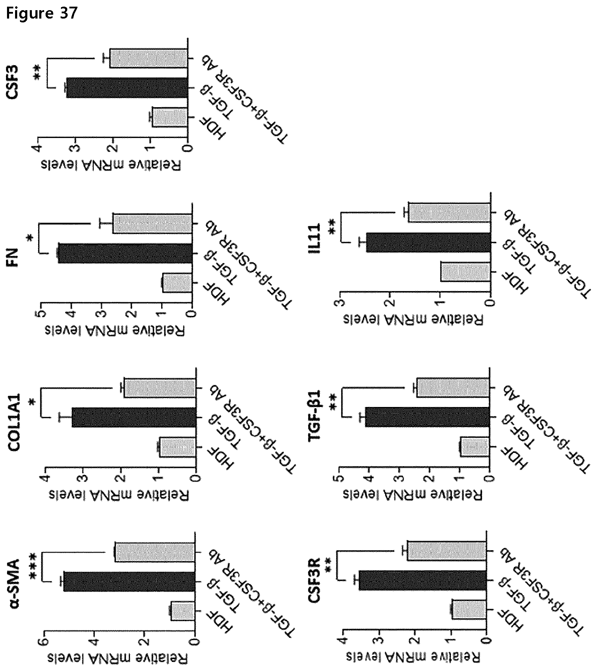

FIG. 37 shows the results of qPCR analysis of mRNA expression levels of α-SMA, Col1A1, TGF-β, IL11, CSF3, and CSF3R in skin fibroblasts treated with TGF-β and anti-CSF3R antibody, respectively, according to Experimental Example 3.3.3. Here, TGF-β refers to the group treated with TGF-β; and TGF-β+CSF3R Ab refers to the group treated with TGF-β and anti-CSF3R antibody.

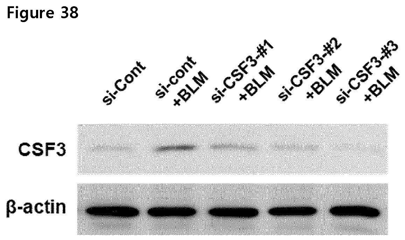

FIG. 38 shows the results of Western Blot analysis of CSF3 protein expression in skin fibroblasts treated with bleomycin, si-cont, and si-CSF3, respectively, according to Experimental Example 3.3.4.

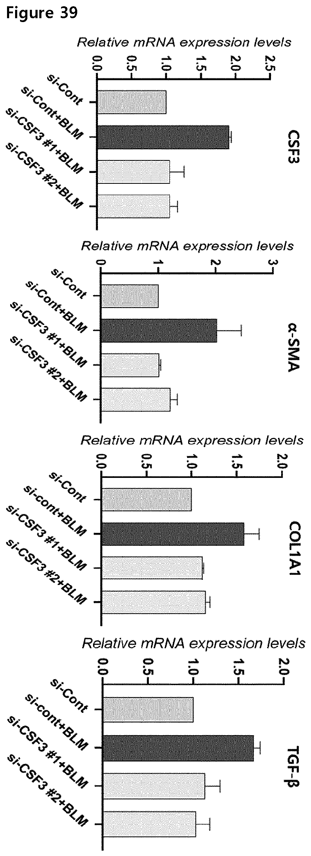

FIG. 39 shows the results of qPCR analysis of mRNA expression levels of CSF3, α-SMA, Col1A1, and TGF-β in skin fibroblasts treated with bleomycin, si-cont, and si-CSF3, respectively, according to Experimental Example 3.3.4.
In FIGS. 38 and 39, si-Cont refers to the group treated with si-cont; si-cont_BLM refers to the group treated with bleomycin and si-cont; and si-CSF3-#1+BLM, si-CSF3-#2+BLM, and si-CSF3-#3+BLM refer to groups treated with si-CSF3 and bleomycin.

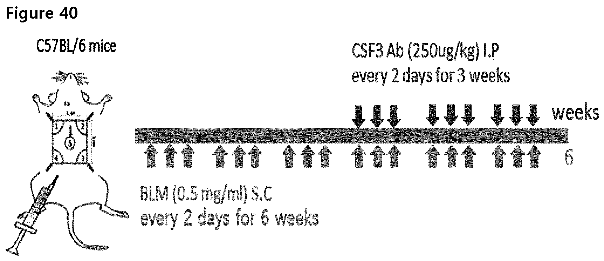

FIG. 40 is a schematic diagram of the experimental model used to conduct the animal study of Experimental Example 3.4.

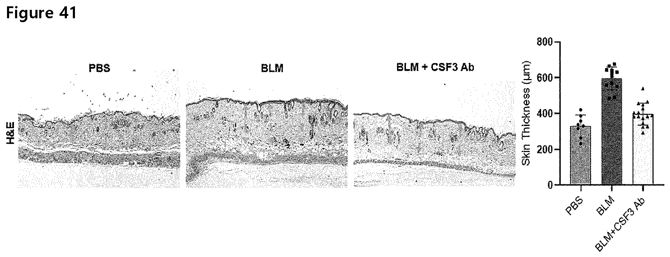

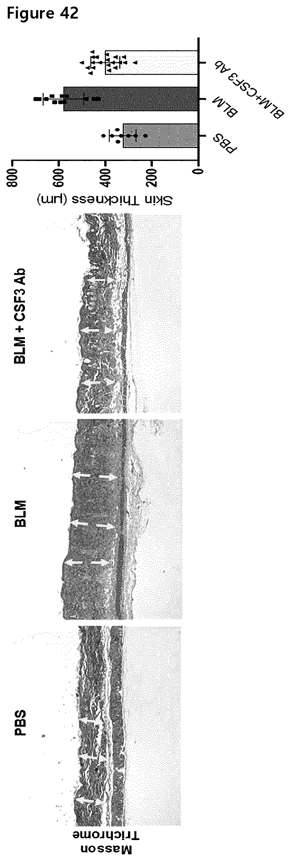

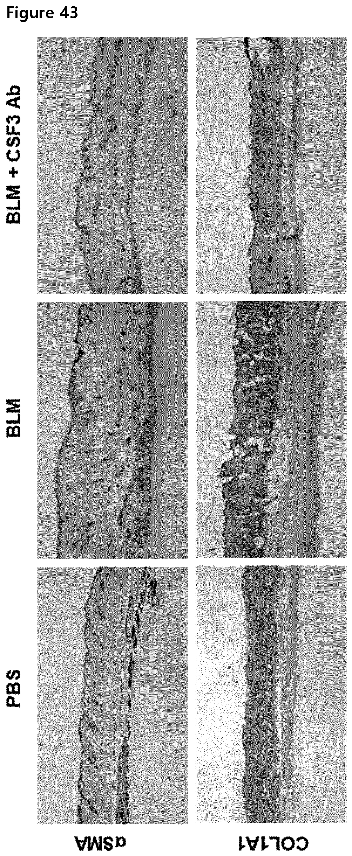

FIGS. 41, 42, and 43 relate to skin tissue observed in C57BL/6 mice in a control group treated with PBS only, an experimental group treated with bleomycin only, and an experimental group treated with bleomycin in addition to the anti-CSF3 antibody, according to Experimental Example 3.4.2. FIG. 41 shows H&E staining results of mouse skin tissue. FIG. 42 shows Masson's trichrome staining results of collagen in mouse skin tissue. FIG. 43 shows immunostaining results of α-SMA and Col1A1 proteins in mouse skin tissue. In FIGS. 41 to 43, Cont refers to the group treated with PBS only; BLM refers to the group treated with bleomycin only; and BLM+CSF3 Ab refers to the group treated with bleomycin and the anti-CSF3 antibody.

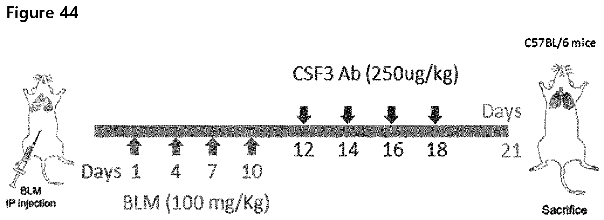

FIG. 44 is a schematic diagram of the experimental model used to conduct the animal study of Experimental Example 3.4.

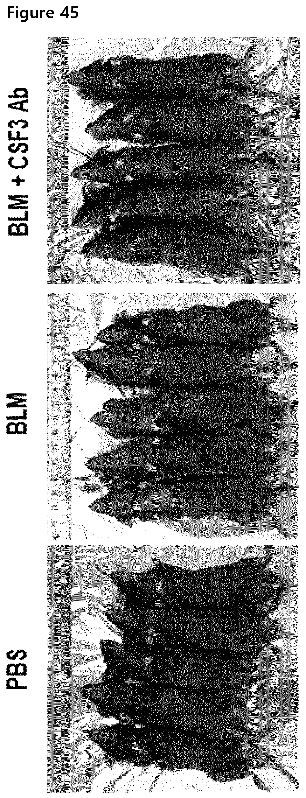

FIG. 45 relates to hair loss and hair loss recovery observed in a control group of mice administered PBS only, an experimental group administered bleomycin only, and an experimental group administered bleomycin in addition to the anti-CSF3 antibody, according to Experimental Example 3.4.3. In FIG. 45, PBS refers to the group treated with PBS only; BLM refers to the group treated with bleomycin only; and BLM+CSF3 Ab refers to the group treated with bleomycin and the anti-CSF3 antibody.

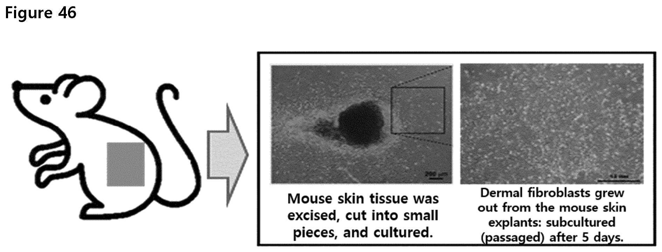

FIG. 46 is a schematic diagram of the experimental model used to conduct the study of Experimental Example 3.4.4.

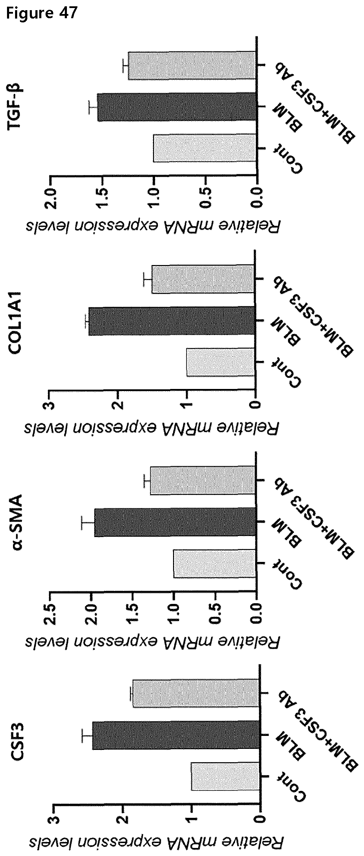

FIG. 47 relates to expression levels of CSF3, α-SMA, Col1A1, and TGF-β in mouse fibroblasts from a control group treated with PBS only, an experimental group treated with bleomycin only, and an experimental group treated with bleomycin in addition to the anti-CSF3 antibody, according to Experimental Example 3.4.4. FIG. 47 shows gene expression levels of CSF3, α-SMA, Col1A1, and TGF-β. In FIG. 47, Cont refers to the group treated with PBS only; BLM refers to the group treated with bleomycin only; and BLM+CSF3 Ab refers to the group treated with bleomycin and the anti-CSF3 antibody.

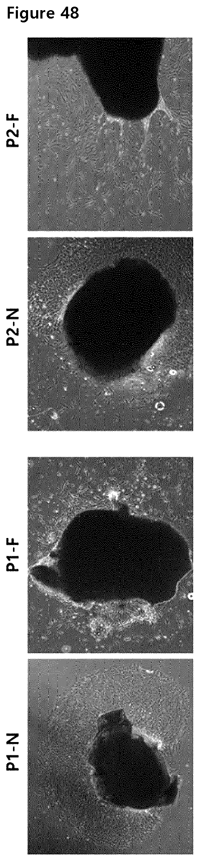

FIGS. 48 to 52 relate to expression levels of CSF3, α-SMA, Col1A1, and TGF-β in tissue from two burn patients, according to Experimental Examples 3.4.5. P1-N refers to normal tissue (or fibroblasts from normal tissue) from the first patient. P1-F refers to hypertrophic scar tissue (or fibroblasts from hypertrophic scar tissue) from the first patient. P2-N refers to normal tissue (or fibroblasts from normal tissue) from the second patient. P2-F refers to hypertrophic scar tissue (or fibroblasts from hypertrophic scar tissue) from the second patient.

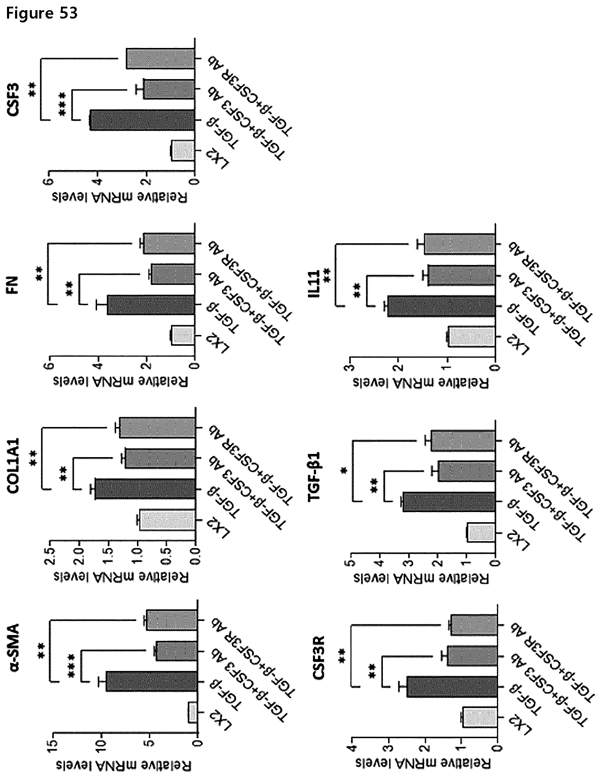

FIG. 53 shows the results of qPCR analysis of mRNA expression levels of α-SMA, Col1A1, FN, CSF3, CSF3R, TGF-β, and IL11 in human hepatic stellate cells (LX-2 cells) treated with PBS, TGF-β, the anti-CSF3 antibody, and the anti-CSF3R antibody, respectively, according to Experimental Example 3.5. Here, LX2 refers to the group treated with PBS; TGF-β refers to the group treated with TGF-β; TGF-β+CSF3 Ab refers to the group treated with TGF-β and the anti-CSF3 antibody; and TGF-β+CSF3R Ab refers to the group treated with TGF-β and the anti-CSF3R antibody.

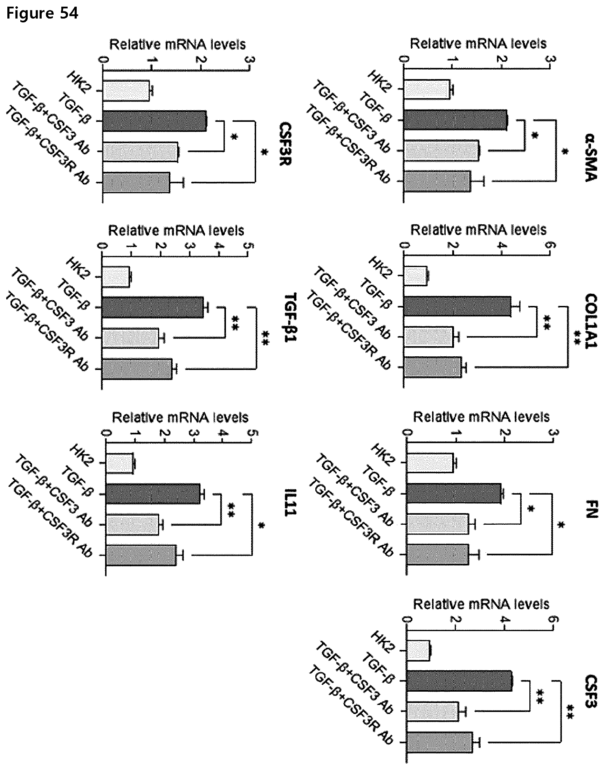

FIG. 54 shows the results of qPCR analysis of mRNA expression levels of α-SMA, Col1A1, FN, CSF3, CSF3R, TGF-β, and IL11 in human normal renal tubular epithelial cells (HK-2 cells) treated with PBS, TGF-β, the anti-CSF3 antibody, and the anti-CSF3R antibody, respectively, according to Experimental Example 3.6. Here, HK2 refers to the group treated with PBS; TGF-β refers to the group treated with TGF-β; TGF-β+CSF3 Ab refers to the group treated with TGF-β and the anti-CSF3 antibody; and TGF-β+CSF3R Ab refers to the group treated with TGF-β and the anti-CSF3R antibody.

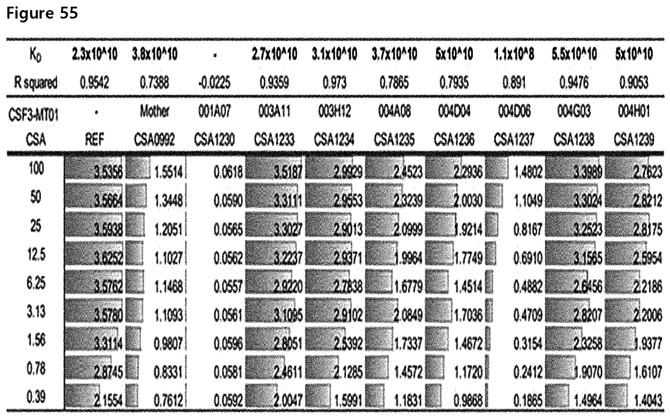

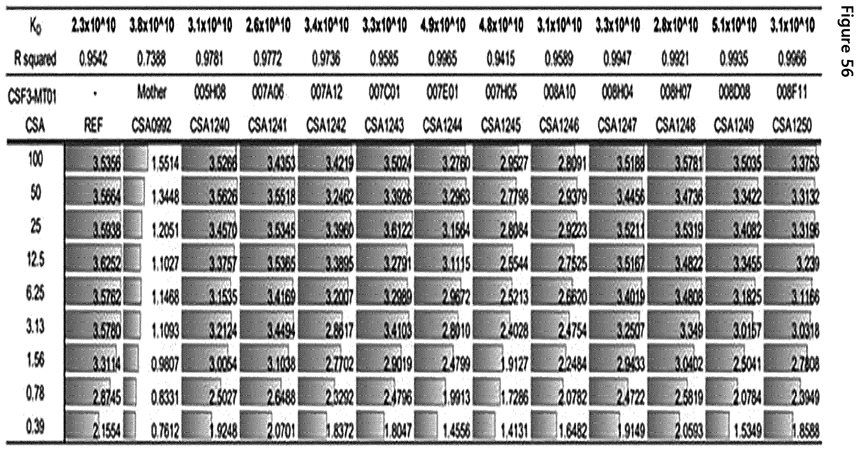

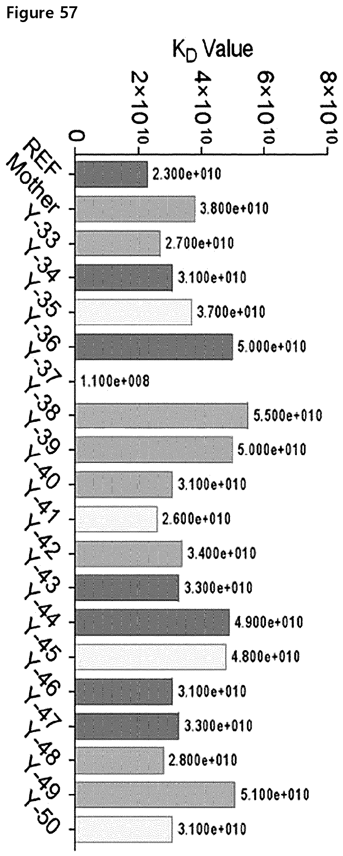

FIGS. 55 to 57 show the results of ELISA analysis of binding of candidate antibodies to CSF3 protein, according to Experimental Example 5.2.1. FIG. 57 presents the results from FIGS. 55 and 56 in graphical form.

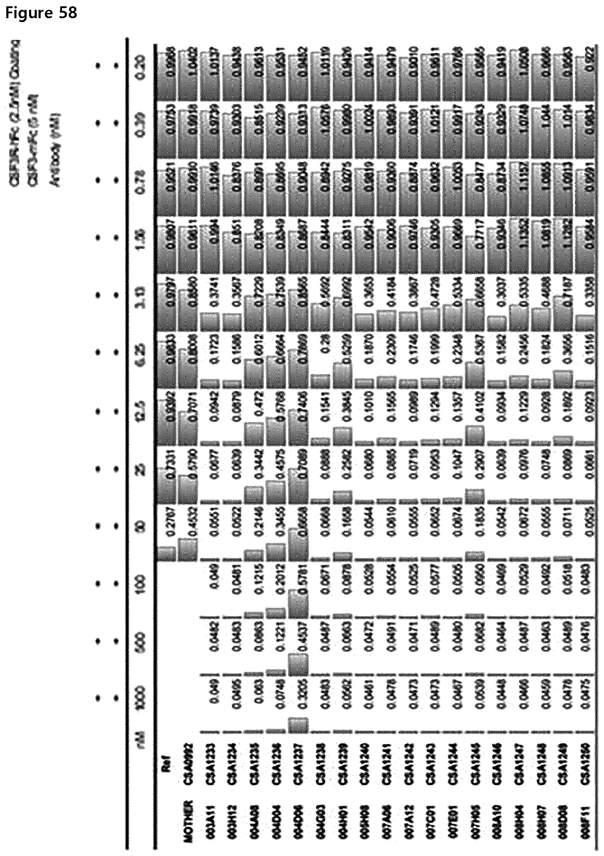

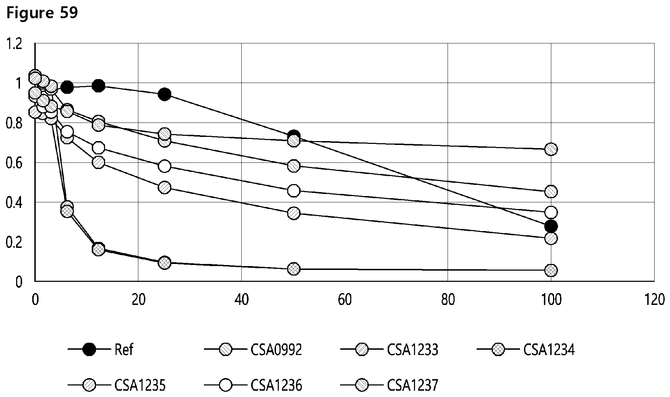

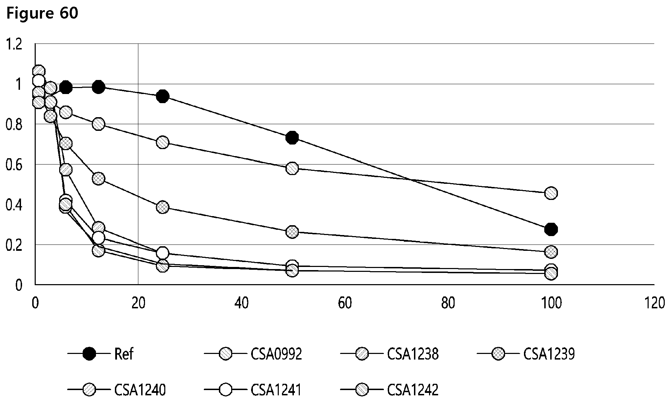

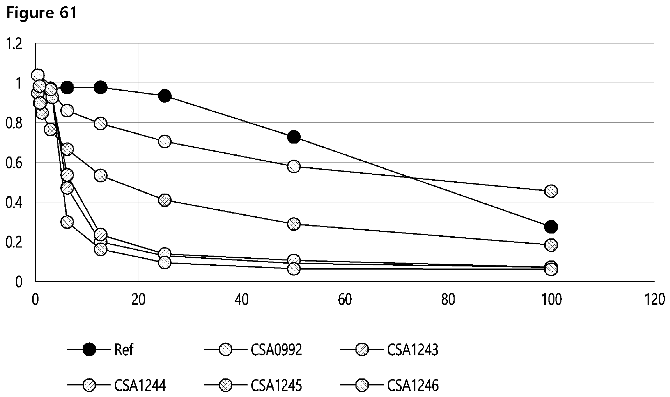

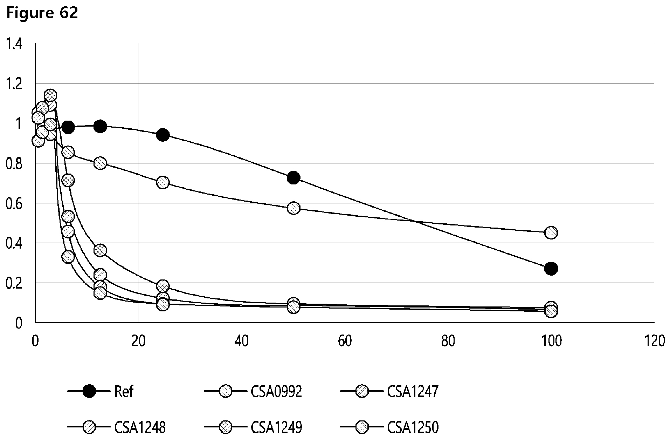

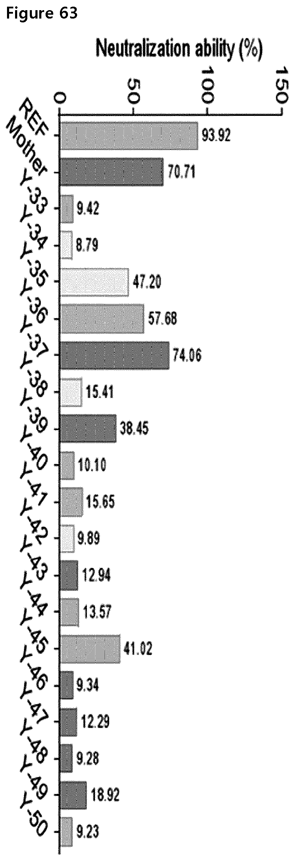

FIGS. 58 to 63 show the results of ELISA analysis of the neutralization ability of candidate antibodies against CSF3, according to Experimental Example 5.2.2. FIGS. 59 to 63 present the results from FIG. 58 in graphical form. FIG. 63 is a graph extracted from FIG. 58 showing only the data at a candidate antibody concentration of 25 nM.

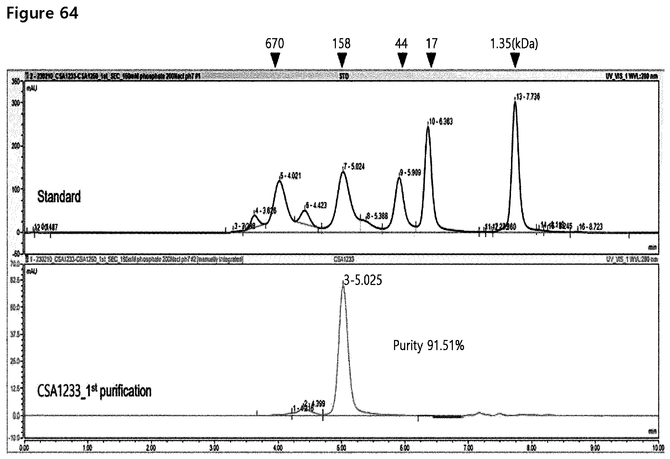

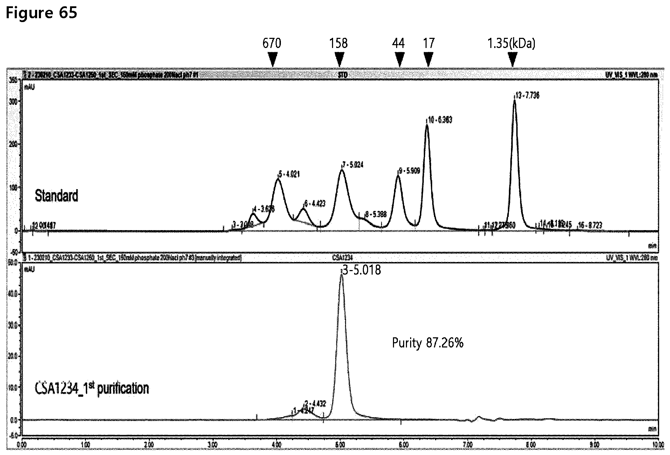

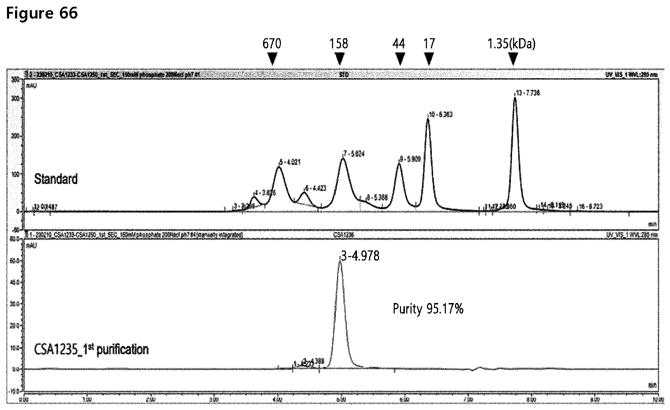

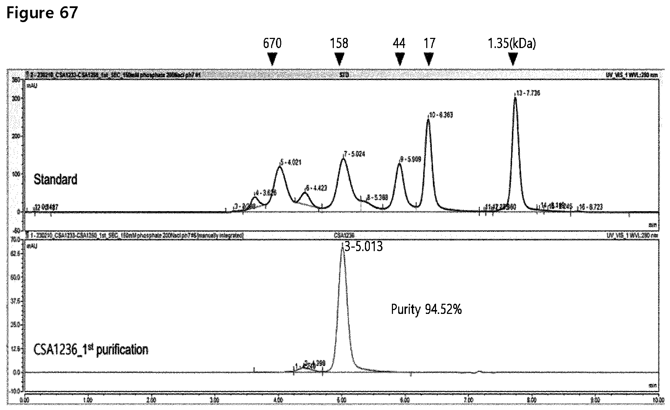

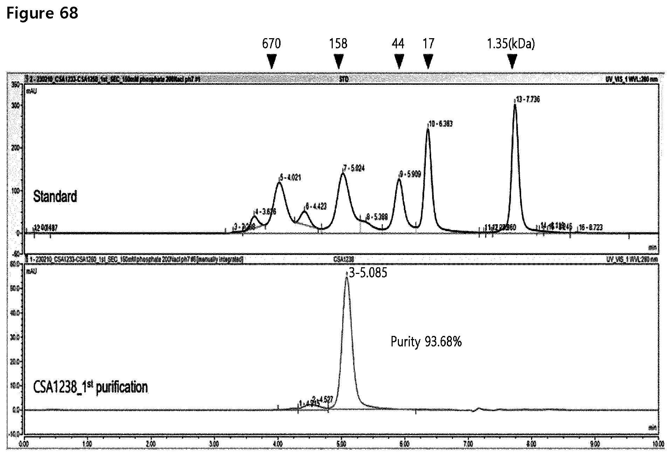

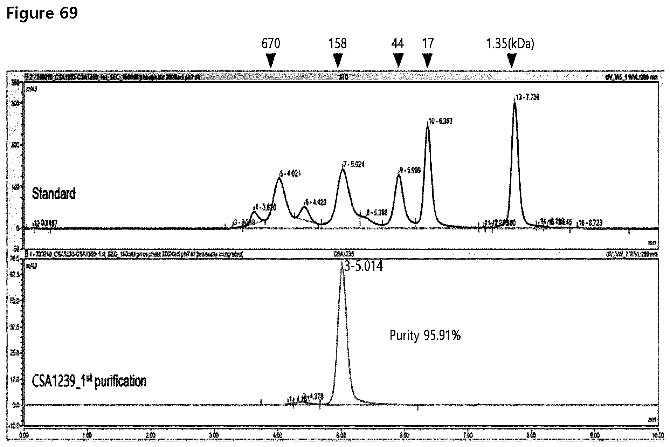

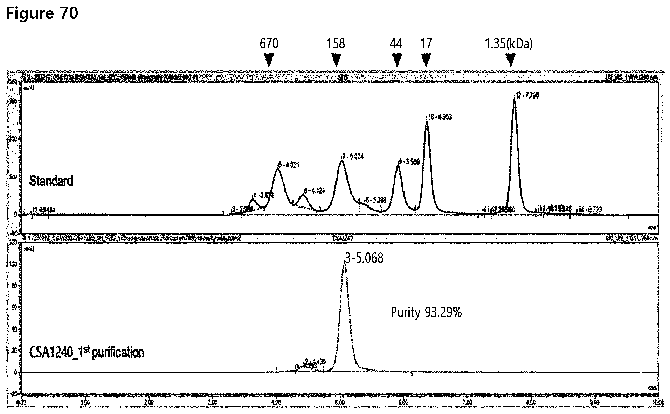

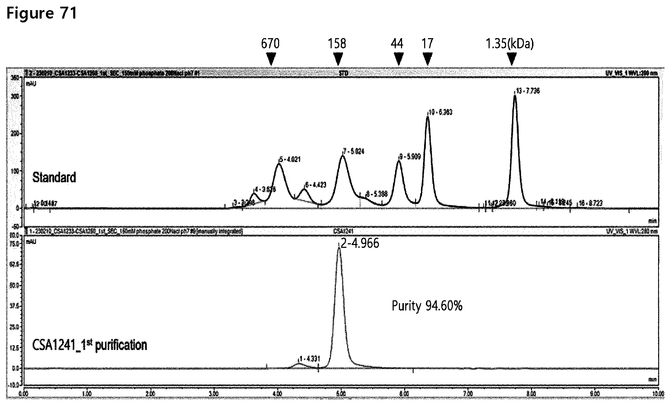

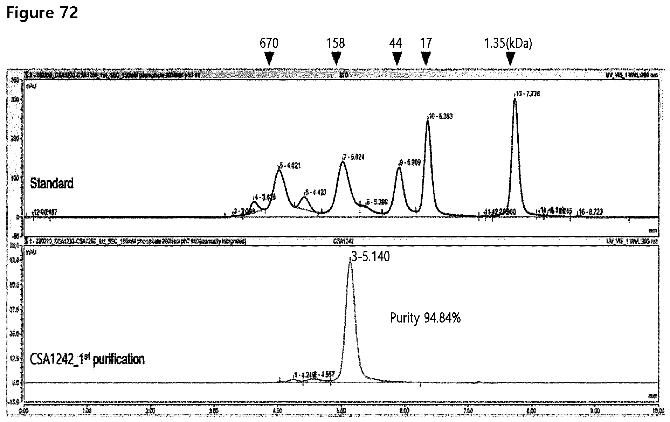

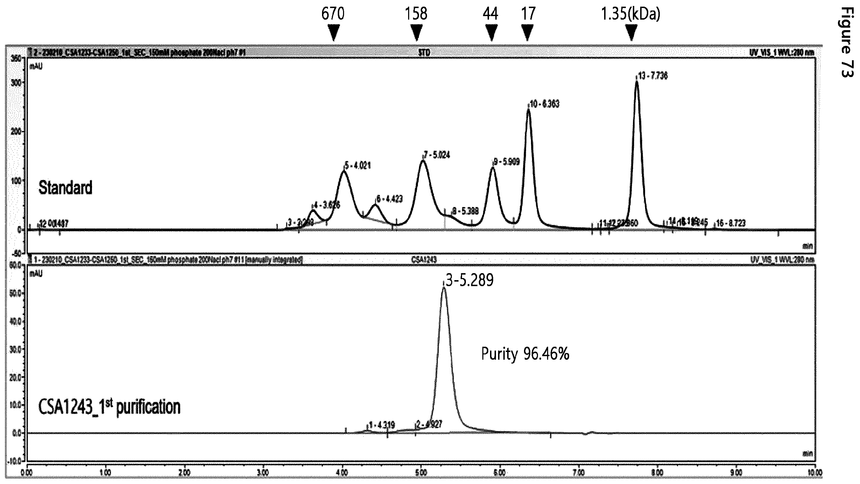

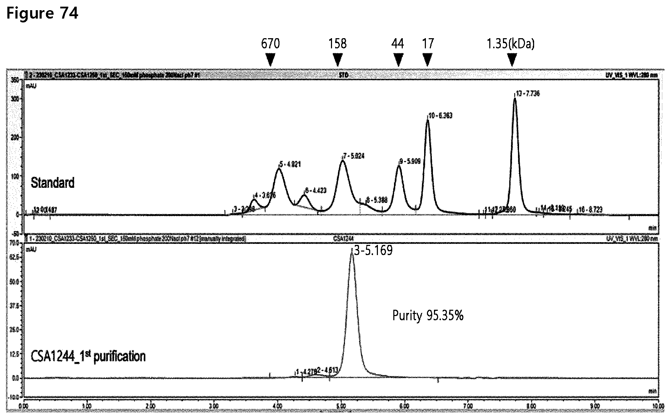

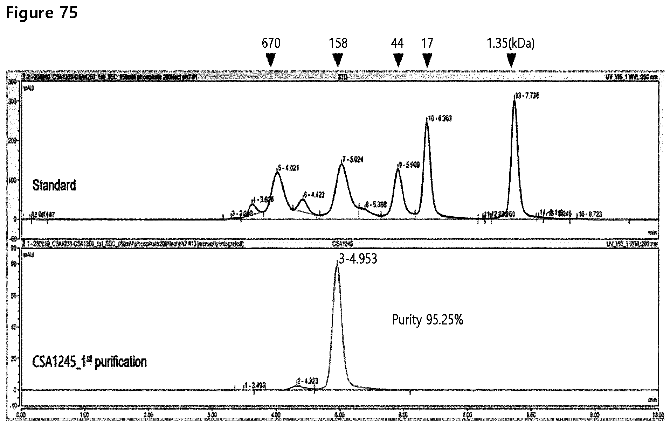

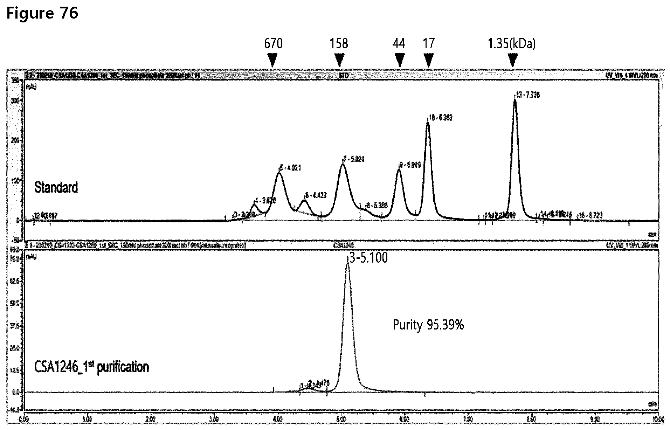

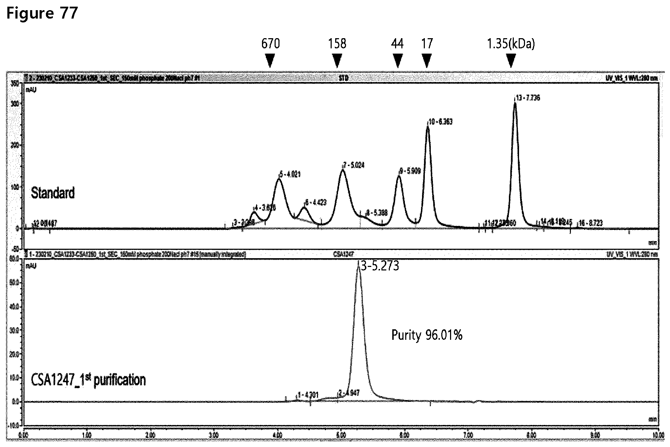

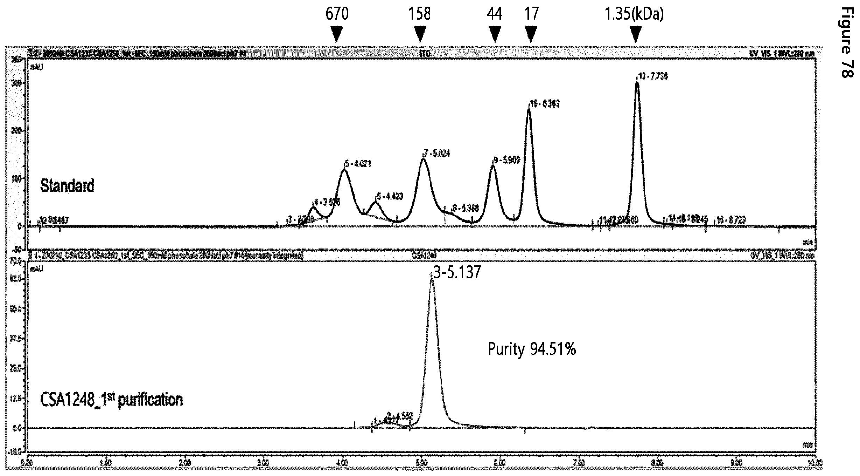

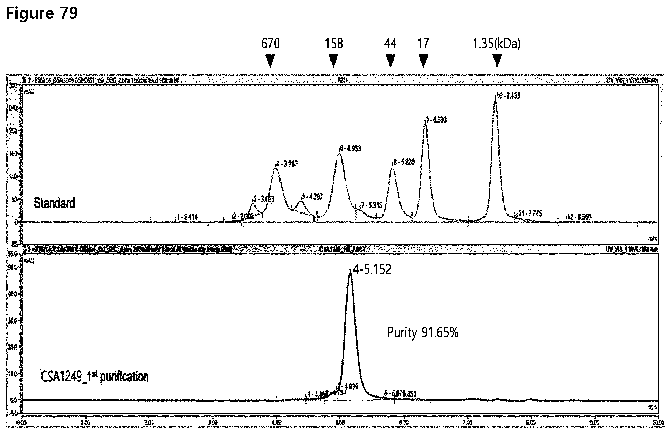

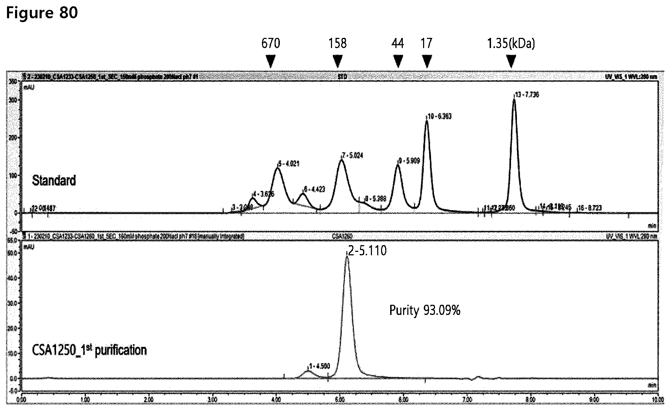

FIGS. 64 to 80 show the results of SEC-HPLC analysis of the purity of candidate antibodies, according to Experimental Example 5.2.3. The molecular weight of the candidate antibodies is approximately 158 kDa.

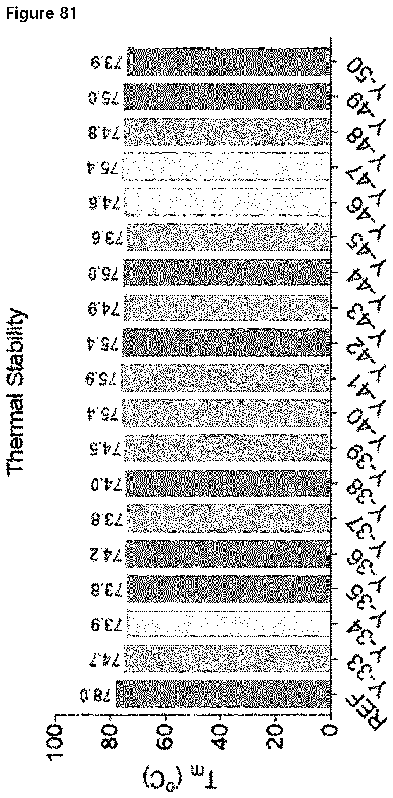

FIG. 81 shows the results of Protein Thermal Shift Dye Kit analysis of the melting points of candidate antibodies, according to Experimental Example 5.2.4.

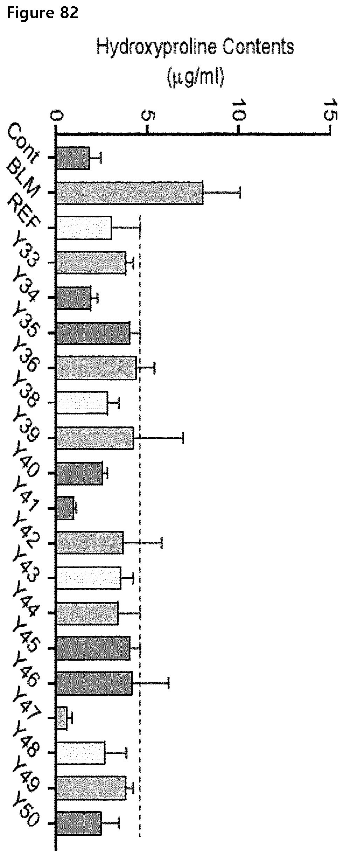

FIG. 82 shows the results of hydroxyproline assay analysis of collagen content in human lung cells treated with bleomycin and candidate antibodies, respectively, according to Experimental Example 5.2.5. Here, BLM refers to the group treated with bleomycin only; and Y33 to Y50 refer to groups treated with bleomycin in addition to each respective candidate antibody.

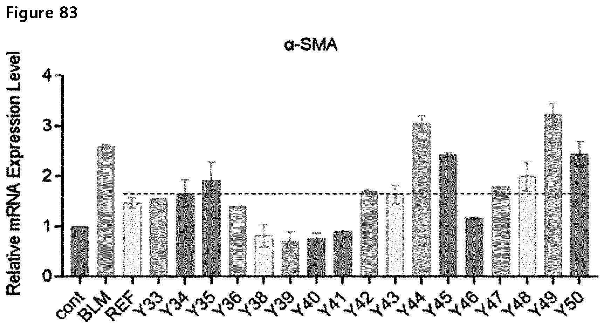

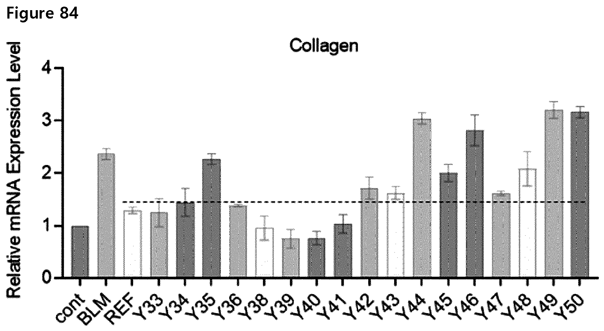

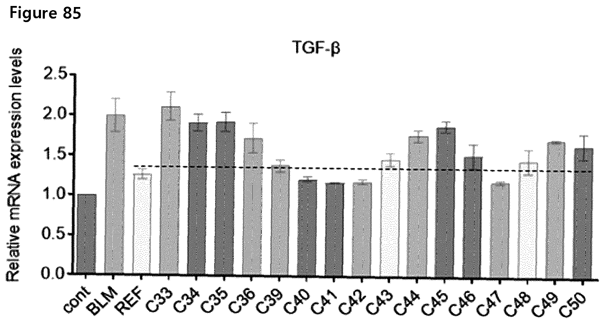

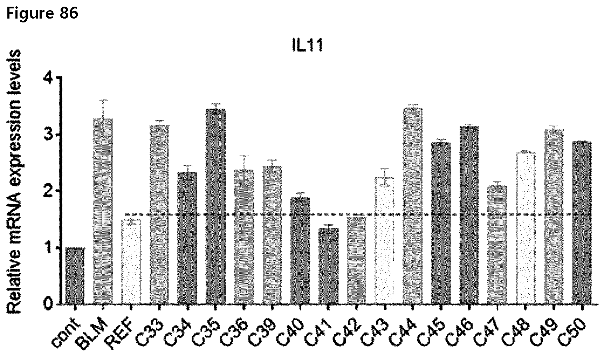

FIGS. 83 to 86 show the results of qPCR analysis of mRNA expression levels of α-SMA, Col1A1, TGF-β, and IL-11 in human lung cells treated with bleomycin and candidate antibodies, respectively, according to Experimental Example 5.2.6. Here, BLM refers to the group treated with bleomycin only; and Y33 to Y50 refer to groups treated with bleomycin in addition to each respective candidate antibody.

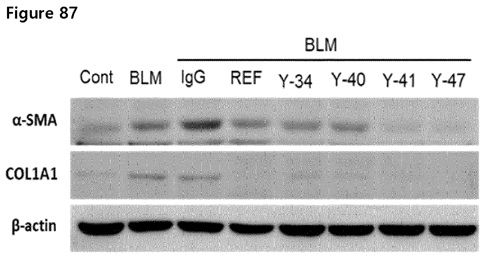

FIG. 87 shows the results of Western Blot analysis of α-SMA and Col1A1 protein expression in human lung cells treated with bleomycin and candidate antibodies, respectively, according to Experimental Example 5.2.7. Here, BLM refers to the group treated with bleomycin only; IgG refers to the group treated with bleomycin in addition to a non-CSF3-binding antibody; REF refers to the group treated with bleomycin in additition to an existing commercially available antibody; and Y-34, Y-40, Y-41, and Y-47 refer to groups treated with bleomycin in addition to each respective candidate antibody.

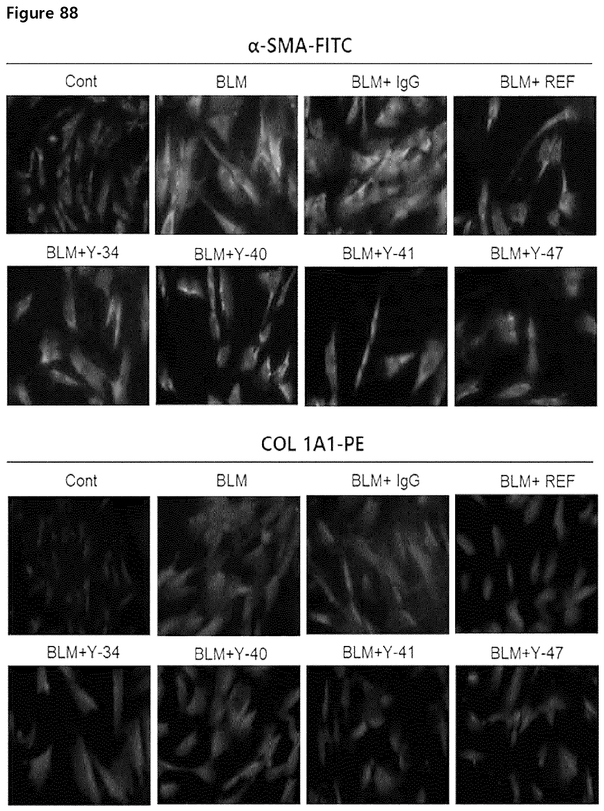

FIG. 88 shows the results of immunofluorescence staining analysis of α-SMA and Col1A1 protein expression in human lung cells treated with bleomycin and candidate antibodies, respectively, according to Experimental Example 5.2.7. Here, BLM refers to the group treated with bleomycin only; BLM+IgG refers to the group treated with bleomycin in addition to a non-CSF3-binding antibody (control antibody); BLM+REF refers to the group treated with bleomycin in addition to an existing commercially available antibody; and BLM+Y-34, BLM+Y-40, BLM+Y-41, and BLM+Y-47 refer to groups treated with bleomycin in addition to each respective candidate antibody.

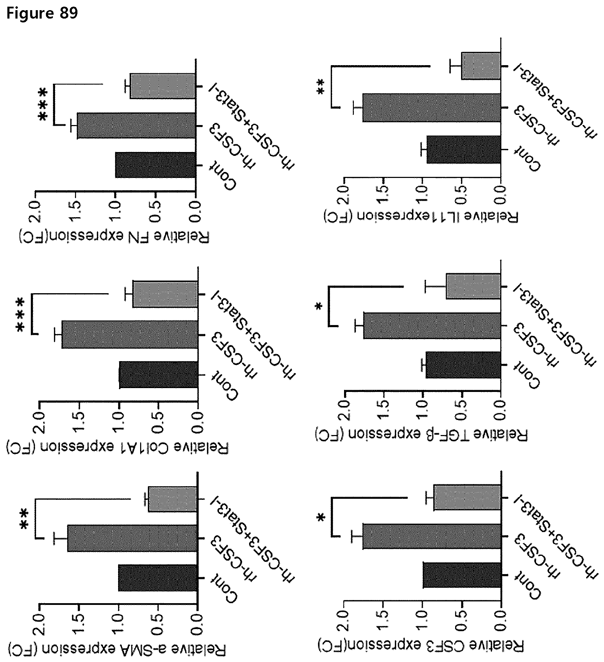

FIG. 89 shows the results of qPCR analysis of mRNA expression levels of α-SMA, Col1A1, FN, CSF3, TGF-β, and IL11 in lung fibroblasts treated with recombinant CSF3 protein and a STAT3 mRNA-targeting siRNA (hereinafter "si-STAT3"), respectively, and analyzed 24 hours later, according to Experimental Example 2.7. Here, rh-CSF3 refers to the group treated with recombinant CSF3 protein; and rh-CSF3+STAT3-I refers to the group treated with recombinant CSF3 protein and si-STAT3.

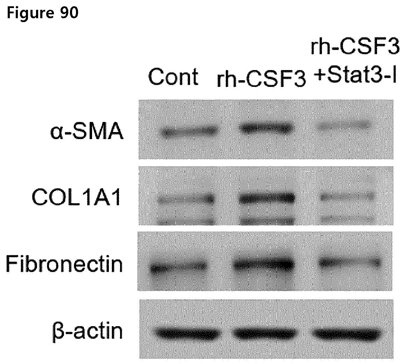

FIG. 90 shows the results of Western Blot analysis of α-SMA, Col1A1, and FN protein expression in lung fibroblasts treated with recombinant CSF3 protein and si-STAT3, respectively, and analyzed 24 hours later, according to Experimental Example 2.7. Here, rh-CSF3 refers to the group treated with recombinant CSF3 protein; and rh-CSF3+STAT3-I refers to the group treated with recombinant CSF3 protein and si-STAT3.

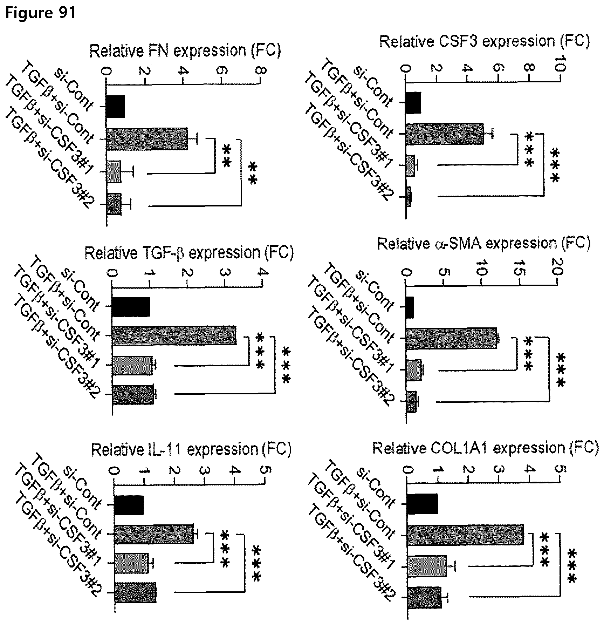

FIG. 91 shows the results of qPCR analysis of mRNA expression levels of α-SMA, Col1A1, FN, CSF3, TGF-β, IL11, and CSF3R in lung fibroblasts treated with TGF-β, si-cont, and si-CSF3, respectively, and analyzed 24 hours later, according to Experimental Example 2.6. Here, si-cont refers to the group treated with si-cont only; TGF-β+si-Cont refers to the group treated with TGF-β and si-cont; TGF-β+si-CSF3#1 refers to the group treated with TGF-β and si-CSF3; and TGF-β+si-CSF3#2 refers to the group treated with TGF-β and si-CSF3.

### Best Mode for Carrying Out the Invention

Hereinafter, the best mode for carrying out the invention is disclosed by way of example. This includes some, but not all, embodiments of the invention disclosed in the present specification. The examples described in this section are merely illustrative, and should not be construed as the only "best mode for carrying out the invention." A person skilled in the art will be able to conceive of many variations and more preferred embodiments based on the examples described in this section, and such content should also be considered included in the best mode for carrying out the invention.

The present specification provides a CSF3 inhibitor for use in treating fibrosis.

In one embodiment, the fibrosis may be selected from the following:

Hypertrophic scar; systemic sclerosis; multiple cancers; pulmonary arterial hypertension; glial scar; Alzheimer's disease; cardiac fibrosis; hypertrophic cardiomyopathy; cardiac dysfunction; valvular disease; arrhythmia; myelofibrosis; myelodysplastic syndrome; chronic myelogenous leukemia; cirrhosis; portal hypertension; hepatocellular carcinoma; nonalcoholic steatohepatitis (NASH); intestinal fibrosis; enteropathies; inflammatory bowel disease; arthrofibrosis; subretinal fibrosis; epiretinal fibrosis; vision loss; idiopathic pulmonary fibrosis; cystic fibrosis; pulmonary hypertension; thromboembolic disease; emphysema; renal fibrosis; cystic fibrosis; nephrogenic systemic fibrosis; chronic kidney disease; renal anemia; retroperitoneal fibrosis; mediastinal fibrosis; pancreatic fibrosis; cystic fibrosis; chronic pancreatitis; duct obstruction; autoimmune-interstitial lung disease such as ankylosing spondylitis and rheumatoid arthritis; connective tissue disease-interstitial lung disease such as rheumatoid arthritis-interstitial lung disease; and non-idiopathic pulmonary fibrosis-interstitial lung disease.

In one embodiment, the CSF3 inhibitor may induce degradation of extracellular matrix accumulated in the tissue in which the fibrosis occurs.

In one embodiment, the extracellular matrix accumulated in the tissue in which the fibrosis occurs may be collagen.

In one embodiment, the CSF3 inhibitor may inactivate myofibroblasts in the tissue in which the fibrosis occurs.

In one embodiment, inactivating myofibroblasts in the tissue in which the fibrosis occurs may mean at least one selected from the following:
(a) degrading extracellular matrix secreted by the myofibroblasts;
(b) inhibiting extracellular matrix secretion by the myofibroblasts; and
(c) reducing the number of myofibroblasts in the tissue in which the fibrosis occurs.

In one embodiment, the extracellular matrix in (a) and (b) may be collagen.

In one embodiment, (b) may involve inducing a reduction in the expression level of the COL1A1 gene in the tissue in which the fibrosis occurs.

In one embodiment, (c) may involve inducing a reduction in the expression level of the α-SMA gene in the tissue in which the fibrosis occurs.

In one embodiment, (c) may occur by reversion of the myofibroblasts to epithelial cells.

The present specification provides a method of preventing or treating fibrosis, comprising:
administering a therapeutically effective amount of the CSF3 inhibitor of any one of the above embodiments to a subject.

The present specification provides a pharmaceutical composition for treating fibrosis, comprising:
a therapeutically effective amount of the CSF3 inhibitor of any one of the above embodiments; and
a pharmaceutically acceptable carrier.

In one embodiment, the pharmaceutically acceptable carrier may be at least one selected from the following:
binders such as lactose, sucrose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol wax; sweeteners; flavoring agents; syrups; liquid carriers such as fatty oils; sterilized aqueous solutions; propylene glycol; polyethylene glycol; injectable esters such as ethyl oleate; suspending agents; emulsifiers; lyophilized preparations; topical preparations; stabilizers; buffers; animal oils; vegetable oils; waxes; paraffin; starch; tragacanth; cellulose derivatives; polyethylene glycol; silicone; bentonite; silica; talc; zinc oxide; and suitable combinations thereof.

The present specification provides use of the CSF3 inhibitor of any one of the above embodiments for the manufacture of a medicament for treating fibrosis.

The present specification provides sequences of novel anti-CSF3 antibodies.

The present specification provides a CSF3R inhibitor for use in treating fibrosis.

In one embodiment, the fibrosis may be selected from the following:
Hypertrophic scar; systemic sclerosis; multiple cancers; pulmonary arterial hypertension; glial scar; Alzheimer's disease; cardiac fibrosis; hypertrophic cardiomyopathy; cardiac dysfunction; valvular disease; arrhythmia; myelofibrosis; myelodysplastic syndrome; chronic myelogenous leukemia; cirrhosis; portal hypertension; hepatocellular carcinoma; nonalcoholic steatohepatitis (NASH); intestinal fibrosis; enteropathies; inflammatory bowel disease; arthrofibrosis; subretinal fibrosis; epiretinal fibrosis; vision loss; idiopathic pulmonary fibrosis; cystic fibrosis; pulmonary hypertension; thromboembolic disease; emphysema; renal fibrosis; cystic fibrosis; nephrogenic systemic fibrosis; chronic kidney disease; renal anemia; retroperitoneal fibrosis; mediastinal fibrosis; pancreatic fibrosis; cystic fibrosis; chronic pancreatitis; duct obstruction; autoimmune-interstitial lung disease such as ankylosing spondylitis and rheumatoid arthritis; connective tissue disease-interstitial lung disease such as rheumatoid arthritis-interstitial lung disease; and non-idiopathic pulmonary fibrosis-interstitial lung disease.

In one embodiment, the CSF3R inhibitor may induce degradation of extracellular matrix accumulated in the tissue in which the fibrosis occurs.

In one embodiment, the extracellular matrix accumulated in the tissue in which the fibrosis occurs may be collagen.

In one embodiment, the CSF3R inhibitor may inactivate myofibroblasts in the tissue in which the fibrosis occurs.

In one embodiment, inactivating myofibroblasts in the tissue in which the fibrosis occurs may mean at least one selected from the following:
(a) degrading extracellular matrix secreted by the myofibroblasts;
(b) inhibiting extracellular matrix secretion by the myofibroblasts; and
(c) reducing the number of myofibroblasts in the tissue in which the fibrosis occurs.

In one embodiment, the extracellular matrix in (a) and (b) may be collagen.

In one embodiment, (b) may involve inducing a reduction in the expression level of the COL1A1 gene in the tissue in which the fibrosis occurs.

In one embodiment, (c) may involve inducing a reduction in the expression level of the α-SMA gene in the tissue in which the fibrosis occurs.

In one embodiment, (c) may occur by reversion of the myofibroblasts to epithelial cells.

The present specification provides a method of preventing or treating fibrosis, comprising:
administering a therapeutically effective amount of the CSF3R inhibitor of any one of the above embodiments to a subject.

The present specification provides a pharmaceutical composition for treating fibrosis, comprising:
a therapeutically effective amount of the CSF3R inhibitor of any one of the above embodiments; and
a pharmaceutically acceptable carrier.

In one embodiment, the pharmaceutically acceptable carrier may be at least one selected from the following:
binders such as lactose, sucrose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol wax; sweeteners; flavoring agents; syrups; liquid carriers such as fatty oils; sterilized aqueous solutions; propylene glycol; polyethylene glycol; injectable esters such as ethyl oleate; suspending agents; emulsifiers; lyophilized preparations; topical preparations; stabilizers; buffers; animal oils; vegetable oils; waxes; paraffin; starch; tragacanth; cellulose derivatives; polyethylene glycol; silicone; bentonite; silica; talc; zinc oxide; and suitable combinations thereof.

The present specification provides use of the CSF3R inhibitor of any one of the above embodiments for the manufacture of a medicament for treating fibrosis.

### Mode for Carrying Out the Invention

Hereinafter, the content of the invention is described in further detail with reference to the accompanying drawings, through specific embodiments and examples. It should be noted that the accompanying drawings include some, but not all, embodiments of the invention. The content of the invention disclosed in the present specification may be implemented in various forms and is not limited to the specific embodiments described herein. These embodiments should be understood as being provided to satisfy the legal requirements applicable to the present specification. A person skilled in the art to which the invention disclosed in the present specification pertains will be able to conceive of many modifications and other embodiments of the content of the invention disclosed herein. Accordingly, the content of the invention disclosed herein is not limited to the specific embodiments described, and modifications and other embodiments thereof should be understood as being encompassed within the scope of the claims.

### Definition of Terms

The definitions of terms used in the present disclosure are as follows.

### About

As used in the present disclosure, the term "about" refers to an amount, level, value, number, frequency, percentage, dimension, size, quantity, weight, or length that varies by as much as 30, 25, 20, 15, 10, 9, 8, 7, 6, 5, 4, 3, 2, or 1% relative to a reference amount, level, value, number, frequency, percentage, dimension, size, quantity, weight, or length.

### Amino Acid Sequence Notation

Unless otherwise stated, amino acid sequences in the present disclosure are written using single-letter or three-letter amino acid codes, in the direction from N-terminus to C-terminus. For example, RNVP denotes a peptide in which arginine, asparagine, valine, and proline are linked in sequence from N-terminus to C-terminus. As another example, Thr-Leu-Lys denotes a peptide in which threonine, leucine, and lysine are linked in sequence from N-terminus to C-terminus. Amino acids that cannot be represented by the single-letter code are denoted using other characters and are supplemented with additional explanation.

The notation for each amino acid is as follows: Alanine (Ala, A); Arginine (Arg, R); Asparagine (Asn, N); Aspartic acid (Asp, D); Cysteine (Cys, C); Glutamic acid (Glu, E); Glutamine (Gln, Q); Glycine (Gly, G); Histidine (His, H); Isoleucine (Ile, I); Leucine (Leu, L); Lysine (Lys, K); Methionine (Met, M); Phenylalanine (Phe, F); Proline (Pro, P); Serine (Ser, S); Threonine (Thr, T); Tryptophan (Trp, W); Tyrosine (Tyr, Y); and Valine (Val, V).

### Nucleic Acid Sequence Notation

The symbols A, T, C, G, and U as used in the present disclosure are interpreted in the manner understood by those skilled in the art. Depending on context and description, they may be appropriately interpreted as bases, nucleosides, or nucleotides on DNA or RNA. For example, when referring to bases, they may be interpreted as adenine (A), thymine (T), cytosine (C), guanine (G), or uracil (U) themselves, respectively; when referring to nucleosides, they may be interpreted as adenosine (A), thymidine (T), cytidine (C), guanosine (G), or uridine (U), respectively; and when referring to nucleotides in a sequence, they should be interpreted as nucleotides comprising each of the respective nucleosides.

The symbol N as used in the present disclosure may be appropriately interpreted as a base, nucleoside, or nucleotide on DNA or RNA depending on context and description. For example, when referring to a base, it may be interpreted as any one of adenine (A), thymine (T), cytosine (C), guanine (G), and uracil (U); when referring to a nucleoside, it may be interpreted as any one of adenosine (A), thymidine (T), cytidine (C), guanosine (G), and uridine (U); and when referring to a nucleotide in a sequence, it should be interpreted as a nucleotide comprising each of the respective nucleosides.

### Vector

As used in the present disclosure, "vector," unless otherwise specified, refers collectively to any substance capable of delivering genetic material into cells. For example, a vector may contain a target genetic material in the form of a nucleic acid sequence. For example, it may be, but is not limited to, a DNA molecule containing a nucleic acid encoding a protein (e.g., an antibody). The term encompasses all meanings recognizable by a person skilled in the art and may be appropriately interpreted depending on context.

### Antibody

### Overview

The antibodies disclosed in the present disclosure are described below.

The structure of an antibody is divided, based on chain type, into a heavy chain region and a light chain region. The structure of an antibody is divided, based on antigen-binding function, into a fragment antigen-binding region (Fab region) and a fragment crystallizable region (Fc region). The structure of an antibody is divided, based on amino acid sequence variability, into a variable region and a constant region. Other structural components of an antibody include a hinge portion and a tail portion.

### Antibody Structure 1 - Heavy Chain and Light Chain Regions

An antibody is structurally divided largely into a heavy chain region and a light chain region. The heavy chain region has a polypeptide chain structure and comprises four or five immunoglobulin domains. The light chain region has a polypeptide chain structure and comprises two immunoglobulin domains. Each immunoglobulin domain comprises approximately 100 to 120 amino acids.

### Antibody Structure 2 - Fragment Antigen-Binding and Crystallizable Regions

The heavy chain region and the light chain region are functionally divided largely into a fragment antigen-binding region (Fab region) and a fragment crystallizable region (Fc region). The Fab region is the portion that contains the antigen-binding site. The Fc region is the portion capable of binding to Fc receptors. Both the Fab region and the Fc region are present in the heavy chain region. The Fab region is present in the light chain region.

### Antibody Structure 3 - Variable and Constant Regions

The Fab region of the heavy chain region comprises a heavy chain variable region (VH) and a heavy chain constant region 1 (CH1). The Fc region of the heavy chain region comprises a heavy chain constant region 2 (CH2) and a heavy chain constant region 3 (CH3), or comprises CH2, CH3, and a heavy chain constant region 4 (CH4). The entirety of the constant region of the heavy chain is referred to as CH. For example, the entire region combining CH1, CH2, and CH3 of IgG1 can be expressed as CH.

The Fab region of the light chain region comprises a light chain variable region (VL) and a light chain constant region (CL). There is no Fc region in the light chain region. VH, CH1, CH2, CH3, CH4, VL, and CL are each one immunoglobulin domain, and each immunoglobulin domain comprises approximately 100 to 120 amino acids.

### Antibody Structure 4 - Connectivity

The immunoglobulin domains contained in the heavy chain region are positioned in the order of VH, CH1, CH2, and CH3, or VH, CH1, CH2, CH3, and CH4, in the direction from N-terminus to C-terminus. The immunoglobulin domains contained in the light chain region are positioned in the order of VL and CL in the direction from N-terminus to C-terminus.

The heavy chain region and the light chain region are connected by disulfide bonds. Specifically, they are connected by a disulfide bond between CH1 of the heavy chain region and CL of the light chain region. The Fab region and the Fc region are connected by the hinge portion. Specifically, the C-terminal portion of CH1 and the N-terminal portion of CH2 of the heavy chain region are connected by the hinge portion. The hinge portion consists of approximately 10 to 70 amino acids.

### Characteristics of the Variable Region

The variable region (VH and VL) is the region that includes the antigen-binding site. Therefore, the variable region may differ in sequence depending on the type of antigen bound and/or the binding site (epitope), unlike the constant regions (CH1, CH2, CH3, CH4, and CL).

Within the variable region, there is a hypervariable region with the greatest variability, which is called the complementarity-determining region (CDR). Each CDR comprises approximately 3 to 15 amino acids.

VH comprises three CDRs, which are referred to individually as CDRH1, CDRH2, or CDRH3. The CDRs in VH are positioned in the order of CDRH1, CDRH2, and CDRH3 in the direction from N-terminus to C-terminus.

VL comprises three CDRs, which are referred to individually as CDRL1, CDRL2, or CDRL3. The CDRs in VL are positioned in the order of CDRL1, CDRL2, and CDRL3 in the direction from N-terminus to C-terminus.

The region of the variable region excluding the CDRs is called the framework region (FR). VH comprises four FRs, which are referred to individually as FRH1, FRH2, FRH3, or FRH4. The FRs in VH are positioned in the order of FRH1, FRH2, FRH3, and FRH4 in the direction from N-terminus to C-terminus.

VL comprises four FRs, which are referred to individually as FRL1, FRL2, FRL3, or FRL4. The FRs in VL are positioned in the order of FRL1, FRL2, FRL3, and FRL4 in the direction from N-terminus to C-terminus.

VH is positioned in the order of FRH1, CDRH1, FRH2, CDRH2, FRH3, CDRH3, and FRH4 in the direction from N-terminus to C-terminus. VL is positioned in the order of FRL1, CDRL1, FRL2, CDRL2, FRL3, CDRL3, and FRL4 in the direction from N-terminus to C-terminus.

### Characteristics of the Constant Region

The constant region of an antibody is a region separate from the antigen-binding site. The constant region may interact with cells or molecules of the immune system. In one embodiment, the constant region may interact with (bind to or associate with) the cell membrane of immune cells (e.g., lymphocytes, neutrophils, dendritic cells, and/or macrophages). Specifically, the hinge region and/or CH2 portion of the constant region may bind to receptors (such as FcεRIII) on the cell membrane of immune cells. In another embodiment, the constant region may bind to FcRn.

The constant region of the heavy chain region (hereinafter "heavy chain constant region") is broadly classified into five types (classes or isotypes): alpha (α), gamma (γ), delta (δ), epsilon (ε), and mu (µ). The type of heavy chain constant region is not determined individually for CH1, CH2, CH3, and CH4, but is determined by considering all heavy chain constant regions (CH1, CH2, and CH3; or CH1, CH2, CH3, and CH4) contained in the antibody.

The constant region of the light chain region (hereinafter "light chain constant region") has two types: lambda (λ) and kappa (κ).

### Antibody Types - Overview

Antibodies are broadly classified into five types (classes or isotypes), which are determined by the type of heavy chain constant region.

The five types of antibodies are immunoglobulin M (IgM), immunoglobulin D (IgD), immunoglobulin G (IgG), immunoglobulin A (IgA), and immunoglobulin E (IgE). When the type of heavy chain constant region is alpha, the antibody type is IgA. When the type of heavy chain constant region is gamma, the antibody type is IgG. When the type of heavy chain constant region is delta, the antibody type is IgD. When the type of heavy chain constant region is epsilon, the antibody type is IgE. When the type of heavy chain constant region is mu, the antibody type is IgM.

Among the five antibody types, IgG and IgA are further divided into more specific subclasses. When the antibody is a human antibody, the antibody type is IgG1 if the type of heavy chain constant region is gamma 1 (γ1); IgG2 if gamma 2 (γ2); IgG3 if gamma 3 (γ3); and IgG4 if gamma 4 (γ4). The antibody type is IgA1 if the heavy chain constant region of a human antibody is alpha 1 (α1); and IgA2 if alpha 2 (α2).

### Antibody Type 1 - IgG

Immunoglobulin G (IgG), one of the five antibody types, is a monomer comprising two heavy chains and two light chains. In one embodiment, the two heavy chains comprised in IgG may be identical heavy chains. Specifically, the two heavy chains comprised in IgG may consist of identical amino acid sequences. In another embodiment, the two heavy chains comprised in IgG may be different heavy chains. Specifically, the amino acid sequences of the two heavy chains comprised in IgG may differ from each other. Each heavy chain of IgG comprises four immunoglobulin domains (VH, CH1, CH2, and CH3) and does not include CH4.

In one embodiment, the two light chains comprised in IgG may be identical light chains. Specifically, the two light chains comprised in IgG may consist of identical amino acid sequences. In another embodiment, the two light chains comprised in IgG may be different light chains. Specifically, the amino acid sequences of the two light chains comprised in IgG may differ from each other.

For convenience of describing the structure of IgG, within the heading "Antibody Type 1 - IgG," the two heavy chains and two light chains comprised in IgG are referred to as the first heavy chain, second heavy chain, first light chain, and second light chain, respectively. In IgG, one light chain is connected to one heavy chain by a disulfide bond. That is, the first heavy chain is connected to the first light chain by a disulfide bond, and the second heavy chain is connected to the second light chain by a disulfide bond. IgG as a whole forms a Y-shaped quaternary structure.

When the IgG subclass is IgG1, the sequences of CH1, CH2, CH3, and the hinge portion may each be known amino acid sequences known in the art.

When the IgG subclass is IgG2, the sequences of CH1, CH2, CH3, and the hinge portion may each be known amino acid sequences known in the art.

When the IgG subclass is IgG3, the sequences of CH1, CH2, CH3, and the hinge portion may each be known amino acid sequences known in the art.

When the IgG subclass is IgG4, the sequences of CH1, CH2, CH3, and the hinge portion may each be known amino acid sequences known in the art.

### Antibody Type 2 - IgA

Immunoglobulin A (IgA), one of the five antibody types, has two subclasses: IgA1 and IgA2.

IgA1 is a monomer comprising two heavy chains and two light chains. In one embodiment, the two heavy chains comprised in IgA1 may be identical heavy chains. Specifically, the two heavy chains comprised in IgA1 may consist of identical amino acid sequences. In another embodiment, the two heavy chains comprised in IgA1 may be different heavy chains. Specifically, the amino acid sequences of the two heavy chains comprised in IgA1 may differ from each other. Each heavy chain of IgA1 comprises four immunoglobulin domains (VH, CH1, CH2, and CH3) and does not include CH4.

In one embodiment, the two light chains comprised in IgA1 may be identical light chains. Specifically, the two light chains comprised in IgA1 may consist of identical amino acid sequences. In another embodiment, the two light chains comprised in IgA1 may be different light chains. Specifically, the amino acid sequences of the two light chains comprised in IgA1 may differ from each other.

For convenience of describing the structure of IgA1, within the heading "Antibody Type 2 - IgA," the two heavy chains and two light chains comprised in IgA1 are referred to as the first heavy chain, second heavy chain, first light chain, and second light chain, respectively. In IgA1, one light chain is connected to one heavy chain by a disulfide bond. That is, the first heavy chain is connected to the first light chain by a disulfide bond, and the second heavy chain is connected to the second light chain by a disulfide bond. The first heavy chain and second heavy chain are connected by disulfide bonds at the hinge portion of each heavy chain. IgA1 as a whole forms a Y-shaped quaternary structure.

IgA2 is a dimer comprising four heavy chains and four light chains. In one embodiment, the four heavy chains comprised in IgA2 may be identical heavy chains. Specifically, the four heavy chains comprised in IgA2 may consist of identical amino acid sequences. In another embodiment, the four heavy chains comprised in IgA2 may be different heavy chains. Specifically, the amino acid sequences of the four heavy chains comprised in IgA2 may differ from each other. Each heavy chain of IgA2 comprises four immunoglobulin domains (VH, CH1, CH2, and CH3) and does not include CH4.

In one embodiment, the four light chains comprised in IgA2 may be identical light chains. Specifically, the four light chains comprised in IgA2 may consist of identical amino acid sequences. In another embodiment, the four light chains comprised in IgA2 may be different light chains. Specifically, the amino acid sequences of the four light chains comprised in IgA2 may differ from each other.

For convenience of describing the structure of IgA2, within the heading "Antibody Type 2 - IgA," the four heavy chains and four light chains comprised in IgA2 are referred to as the first, second, third, and fourth heavy chains, and the first, second, third, and fourth light chains, respectively. In IgA2, one light chain is connected to one heavy chain by a disulfide bond. That is, the first heavy chain is connected to the first light chain by a disulfide bond; the second heavy chain is connected to the second light chain by a disulfide bond; the third heavy chain is connected to the third light chain by a disulfide bond; and the fourth heavy chain is connected to the fourth light chain by a disulfide bond. The first heavy chain and second heavy chain are connected by disulfide bonds at the hinge portion of each heavy chain. The third heavy chain and fourth heavy chain are connected by disulfide bonds at the hinge portion of each heavy chain. The portion comprising the first heavy chain, second heavy chain, first light chain, and second light chain forms a Y-shaped quaternary structure as a whole. Similarly, the portion comprising the third heavy chain, fourth heavy chain, third light chain, and fourth light chain forms a Y-shaped quaternary structure as a whole.

The C-terminal portions of the first and second heavy chains are connected to the C-terminal portions of the third and fourth heavy chains by a joining chain (J chain).

When the IgA subclass is IgA1, the sequences of CH1, CH2, CH3, and the hinge portion may each be known amino acid sequences known in the art.

When the IgA subclass is IgA2, the sequences of CH1, CH2, CH3, and the hinge portion may each be known amino acid sequences known in the art.

### Antibody Type 3 - IgE

Immunoglobulin E (IgE), one of the five antibody types, is a monomer comprising two heavy chains and two light chains. In one embodiment, the two heavy chains comprised in IgE may be identical heavy chains. Specifically, the two heavy chains comprised in IgE may consist of identical amino acid sequences. In another embodiment, the two heavy chains comprised in IgE may be different heavy chains. Specifically, the amino acid sequences of the two heavy chains comprised in IgE may differ from each other. Each heavy chain of IgE comprises five immunoglobulin domains (VH, CH1, CH2, CH3, and CH4).

In one embodiment, the two light chains comprised in IgE may be identical light chains. Specifically, the two light chains comprised in IgE may consist of identical amino acid sequences. In another embodiment, the two light chains comprised in IgE may be different light chains. Specifically, the amino acid sequences of the two light chains comprised in IgE may differ from each other.

For convenience of describing the structure of IgE, within the heading "Antibody Type 3 - IgE," the two heavy chains and two light chains comprised in IgE are referred to as the first heavy chain, second heavy chain, first light chain, and second light chain, respectively. In IgE, one light chain is connected to one heavy chain by a disulfide bond. That is, the first heavy chain is connected to the first light chain by a disulfide bond, and the second heavy chain is connected to the second light chain by a disulfide bond. The first heavy chain and second heavy chain are connected by disulfide bonds at the hinge portion of each heavy chain. IgE as a whole forms a Y-shaped quaternary structure.

The sequences of CH1, CH2, CH3, CH4, and the hinge portion of IgE may each be known amino acid sequences known in the art.

### Antibody Type 4 - IgM

Immunoglobulin M (IgM), one of the five antibody types, is a pentamer comprising ten heavy chains and ten light chains. In one embodiment, the ten heavy chains comprised in IgM may be identical heavy chains. Specifically, the ten heavy chains comprised in IgM may consist of identical amino acid sequences. In another embodiment, the ten heavy chains comprised in IgM may be different heavy chains. Specifically, the amino acid sequences of the ten heavy chains comprised in IgM may differ from each other. Each heavy chain of IgM comprises five immunoglobulin domains (VH, CH1, CH2, CH3, and CH4).

In one embodiment, the ten light chains comprised in IgM may be identical light chains. Specifically, the ten light chains comprised in IgM may consist of identical amino acid sequences. In another embodiment, the ten light chains comprised in IgM may be different light chains. Specifically, the amino acid sequences of the ten light chains comprised in IgM may differ from each other.

For convenience of describing the structure of IgM, within the heading "Antibody Type 4 - IgM," the ten heavy chains and ten light chains comprised in IgM are referred to as the first, second, third, fourth, fifth, sixth, seventh, eighth, ninth, and tenth heavy chains, and the first, second, third, fourth, fifth, sixth, seventh, eighth, ninth, and tenth light chains, respectively. In IgM, one light chain is connected to one heavy chain by a disulfide bond. That is, the first heavy chain is connected to the first light chain; the second heavy chain is connected to the second light chain; the third heavy chain is connected to the third light chain; the fourth heavy chain is connected to the fourth light chain; the fifth heavy chain is connected to the fifth light chain; the sixth heavy chain is connected to the sixth light chain; the seventh heavy chain is connected to the seventh light chain; the eighth heavy chain is connected to the eighth light chain; the ninth heavy chain is connected to the ninth light chain; and the tenth heavy chain is connected to the tenth light chain, each by a disulfide bond.

The first and second heavy chains are connected by disulfide bonds at the hinge portion of each heavy chain. The third and fourth heavy chains are connected by disulfide bonds at the hinge portion of each heavy chain. The fifth and sixth heavy chains are connected by disulfide bonds at the hinge portion of each heavy chain. The seventh and eighth heavy chains are connected by disulfide bonds at the hinge portion of each heavy chain. The ninth and tenth heavy chains are connected by disulfide bonds at the hinge portion of each heavy chain. The first and second heavy chains, the third and fourth heavy chains, the fifth and sixth heavy chains, the seventh and eighth heavy chains, and the ninth and tenth heavy chains each form a Y-shaped quaternary structure as a whole.

The C-terminal portions of the first, second, third, fourth, fifth, sixth, seventh, eighth, ninth, and tenth heavy chains are connected by a joining chain (J chain).

The sequences of CH1, CH2, CH3, CH4, and the hinge portion of IgM may each be known amino acid sequences known in the art.

### Antibody Type 5 - IgD

Immunoglobulin D (IgD), one of the five antibody types, is a monomer comprising two heavy chains and two light chains. In one embodiment, the two heavy chains comprised in IgD may be identical heavy chains. Specifically, the two heavy chains comprised in IgD may consist of identical amino acid sequences. In another embodiment, the two heavy chains comprised in IgD may be different heavy chains. Specifically, the amino acid sequences of the two heavy chains comprised in IgD may differ from each other. Each heavy chain of IgD comprises four immunoglobulin domains (VH, CH1, CH2, and CH3) and does not include CH4.

In one embodiment, the two light chains comprised in IgD may be identical light chains. Specifically, the two light chains comprised in IgD may consist of identical amino acid sequences. In another embodiment, the two light chains comprised in IgD may be different light chains. Specifically, the amino acid sequences of the two light chains comprised in IgD may differ from each other.

For convenience of describing the structure of IgD, within the heading "Antibody Type 5 - IgD," the two heavy chains and two light chains comprised in IgD are referred to as the first heavy chain, second heavy chain, first light chain, and second light chain, respectively. In IgD, one light chain is connected to one heavy chain by a disulfide bond. That is, the first heavy chain is connected to the first light chain by a disulfide bond, and the second heavy chain is connected to the second light chain by a disulfide bond. The first heavy chain and second heavy chain are connected by disulfide bonds at the hinge portion of each heavy chain. IgD as a whole forms a Y-shaped quaternary structure.

The sequences of CH1, CH2, CH3, and the hinge portion of IgD may each be known amino acid sequences known in the art.

### Fibrosis

As used in the present disclosure, "fibrosis," unless otherwise specified, refers collectively to fibrosis-related diseases, fibrosis-related disorders, and fibrosis-related conditions. For example, such fibrosis-related diseases, disorders, and conditions may be selected from, but are not limited to: hypertrophic scar; systemic sclerosis; multiple cancers; pulmonary arterial hypertension; glial scar; Alzheimer's disease; cardiac fibrosis; hypertrophic cardiomyopathy; cardiac dysfunction; valvular disease; arrhythmia; myelofibrosis; myelodysplastic syndrome; chronic myelogenous leukemia; cirrhosis; portal hypertension; hepatocellular carcinoma; nonalcoholic steatohepatitis (NASH); intestinal fibrosis; enteropathies; inflammatory bowel disease; arthrofibrosis; subretinal fibrosis; epiretinal fibrosis; vision loss; idiopathic pulmonary fibrosis; cystic fibrosis; pulmonary hypertension; thromboembolic disease; emphysema; renal fibrosis; cystic fibrosis; nephrogenic systemic fibrosis; chronic kidney disease; renal anemia; retroperitoneal fibrosis; mediastinal fibrosis; pancreatic fibrosis; cystic fibrosis; chronic pancreatitis; duct obstruction; autoimmune-interstitial lung disease such as ankylosing spondylitis and rheumatoid arthritis; connective tissue disease-interstitial lung disease such as rheumatoid arthritis-interstitial lung disease; and non-idiopathic pulmonary fibrosis-interstitial lung disease. Fibrosis may also include fibrosis occurring in tissues such as skin, brain, nervous system, heart, bone marrow, liver, gut, joint, eye, lung, kidney, retroperitoneum, mediastinum, and/or pancreas, but is not limited thereto. The term encompasses all meanings recognizable by a person skilled in the art and may be appropriately interpreted depending on context.

### Background Art

Fibrosis occurs in various tissues and has been reported as the pathological outcome of various chronic inflammatory diseases. Although therapeutic agents have been developed for fibrosis occurring in some tissues, it is difficult to predict that a therapeutic agent for fibrosis in one tissue type would also be effective against fibrosis in skin. For example, nintedanib and pirfenidone are marketed as treatments for idiopathic pulmonary fibrosis, a type of fibrosis, but nintedanib and pirfenidone are not currently used as therapeutic agents for fibrosis in tissues other than the lung.

The inventors of the present disclosure have previously discovered the use of CSF3 inhibitors for the treatment of pulmonary fibrosis. Details regarding the use of CSF3 inhibitors for the treatment of pulmonary fibrosis discovered by the inventors of the present disclosure are publicly available in the literature (WO 2020/159243). However, as described above, since it is difficult to predict that a therapeutic agent for fibrosis in lung tissue would also have therapeutic effects on fibrosis in other tissues, the content described in the published literature (WO 2020/159243) alone makes it difficult to predict that CSF3 inhibitors may have therapeutic effects on fibrosis in a tissue-agnostic manner. For example, the published literature (WO 2020/159243) only describes that CSF3 inhibitors exhibit therapeutic effects on pulmonary fibrosis, and does not describe that CSF3 inhibitors may exhibit therapeutic effects on fibrosis in other tissues.

Therefore, there is an unmet need for therapeutic agents capable of treating fibrosis in a tissue-agnostic manner, and related research is required.

### 1. Mechanism of Action Underlying the Effect of CSF3 Inhibitors in Restoring Fibrotic Tissue to Normal Tissue

### 1.1. Background Art - Effect of CSF3 Inhibitors in Restoring Fibrotic Tissue to Normal Tissue

As described in this background art section, the inventors of the present disclosure have previously discovered the use of CSF3 inhibitors for the treatment of pulmonary fibrosis. Specifically, the inventors of the present disclosure discovered that when the function of CSF3 in fibrotic lung tissue is inhibited using an antibody, the fibrotic lung tissue is restored to normal lung tissue and fibrosis is treated (WO 2020/159243). The therapeutic effect of restoring fibrotic lung tissue to normal lung tissue is an effect that has not been confirmed with treatments using commercially available pulmonary fibrosis therapeutic agents. Accordingly, the inventors conducted research to elucidate the mechanism of action underlying the effect of CSF3 inhibitors in restoring fibrotic tissue to normal tissue, in order to specifically understand the cause of the therapeutic effect wherein fibrotic lung tissue is restored to normal lung tissue, and the results are disclosed in the present disclosure.

### 1.2. Overview of the Mechanism of Action of CSF3 Inhibitors

One aspect of the present disclosure provides a method of restoring fibrotic tissue (or cells) to normal tissue (or cells). Another aspect of the present disclosure provides a method of inhibiting extracellular matrix deposition and epithelial to mesenchymal transition (EMT). Here, the method of restoring fibrotic tissue to normal tissue may include the method of inhibiting extracellular matrix deposition and EMT. In one embodiment, the method of inhibiting extracellular matrix deposition and EMT may be a method of inhibiting extracellular matrix deposition and EMT in a tissue of a subject. In this case, the tissue of the subject may be a tissue at risk of or in which extracellular matrix deposition and/or EMT has occurred.

The method of restoring fibrotic tissue to normal tissue comprises administering a CSF3 inhibitor to a subject or to a tissue of the subject. The method of inhibiting extracellular matrix deposition and EMT comprises administering a CSF3 inhibitor to a subject or to a tissue of the subject.

In one embodiment, the CSF3 inhibitor may inhibit the TGF-β-mediated signaling pathway. The TGF-β-mediated signaling pathway is a signaling pathway in which fibrosis may be induced by the interaction between TGF-β and its receptor (TGF-βR).

In one embodiment, the CSF3 inhibitor may inhibit the non-TGF-β-mediated signaling pathway. The non-TGF-β-mediated signaling pathway is a signaling pathway that induces fibrosis through the interaction between CSF3 and CSF3R, and is a pathway that induces fibrosis independently of the TGF-β-mediated signaling pathway. Therefore, the non-TGF-β-mediated signaling pathway may also be referred to as the CSF3-associated and non-TGF-β-mediated signaling pathway.

In one embodiment, the CSF3 inhibitor may inhibit the positive-feedback loop of CSF3. In one embodiment, the CSF3 inhibitor may inhibit the positive-feedback loop of TGF-β. In one embodiment, the CSF3 inhibitor may restore fibrotic tissue (or cells) to normal tissue (or cells). In one embodiment, the CSF3 inhibitor may inhibit extracellular matrix deposition and EMT.

In one embodiment, the subject may be a human or a non-human animal.

In one embodiment, the tissue may be skin, brain, nervous system, heart, bone marrow, liver, gut, joint, eye, lung, kidney, retroperitoneum, mediastinum, and/or pancreas.

In one embodiment, the cell may be a cell of the skin, brain, nervous system, heart, bone marrow, liver, gut, joint, eye, lung, kidney, retroperitoneum, mediastinum, and/or pancreas.

### 1.3. Definition of CSF3 Inhibitor

Colony-Stimulating Factor 3 (CSF3) of the present disclosure is also referred to as granulocyte colony-stimulating factor (G-CSF). CSF3 is known to stimulate the proliferation, differentiation, adhesion, and terminal functional activation of granulocytes in animals, including human subjects. The term "CSF3 inhibitor" as used in the present disclosure refers to a substance that ultimately inhibits the function of CSF3. The CSF3 inhibitor is not otherwise limited in its type, form, or composition, as long as it is capable of inhibiting the function of CSF3. Specifically, inhibiting the function of CSF3 means: 1) binding to the CSF3 protein to inhibit the intrinsic function of the CSF3 protein itself; 2) inhibiting the interaction of the CSF3 protein with other molecules; 3) inhibiting the expression of CSF3 to thereby block the opportunity for CSF3 to function; or 4) inhibiting the function of CSF3 through other direct or indirect pathways.

In one embodiment, the CSF3 inhibitor may be an anti-CSF3 antibody. In another embodiment, the CSF3 inhibitor may be a nucleic acid molecule capable of silencing the mRNA of the CSF3 gene or inducing RNA interference. Specifically, the nucleic acid molecule may include antisense oligonucleotides; iRNA (interfering RNA), such as miRNA, siRNA (small interfering RNA), shRNA (short hairpin RNA), and the like. In another embodiment, the CSF3 inhibitor may be a compound that inhibits the function of CSF3.

### 1.4. Mechanism of Action of CSF3 Inhibitors 1: Inhibition of the TGF-β-Mediated Signaling Pathway

In one embodiment, the CSF3 inhibitor may inhibit the TGF-β-mediated signaling pathway. In another embodiment, the CSF3 inhibitor may inhibit the TGF-β-mediated signaling pathway in a tissue (or cells) of a subject. In yet another embodiment, when CSF3 is inhibited, the TGF-β-mediated signaling pathway may be inhibited in a tissue (or cells) of a subject. That is, the activity or activation of the TGF-β-mediated signaling pathway involved in fibrosis may be reduced.

### 1.4.1. Definition the TGF-β-Mediated Signaling Pathway

The term "TGF-β-mediated signaling pathway" as used in the present disclosure refers to a signaling pathway by which fibrosis, epithelial to mesenchymal transition (EMT), fibroblast-to-myofibroblast transdifferentiation (FMT), and/or extracellular matrix deposition is induced by transforming growth factor beta (TGF-β). Specifically, the TGF-β-mediated signaling pathway refers to a signaling pathway by which fibrosis, EMT, FMT, and/or extracellular matrix deposition is induced through the interaction between TGF-β and its receptor (TGF-βR).

In one embodiment, the TGF-β-mediated signaling pathway may induce fibrosis, epithelial to mesenchymal transition, fibroblast-to-myofibroblast transdifferentiation, and/or extracellular matrix deposition.

In one embodiment, fibrosis, epithelial to mesenchymal transition, fibroblast-to-myofibroblast transdifferentiation, and/or extracellular matrix deposition may be induced by activation of the TGF-β-mediated signaling pathway.

In one embodiment, the TGF-β-mediated signaling pathway may be a signaling pathway in which fibrosis, epithelial to mesenchymal transition, fibroblast-to-myofibroblast transdifferentiation, and/or extracellular matrix deposition is induced as the expression level of TGF-β increases.

In one embodiment, the TGF-β-mediated signaling pathway may be a signaling pathway in which fibrosis, epithelial to mesenchymal transition, fibroblast-to-myofibroblast transdifferentiation, and/or extracellular matrix deposition is induced by the interaction (or binding) between TGF-β and its receptor.

In one embodiment, the TGF-β-mediated signaling pathway may be a signaling pathway in which fibrosis, epithelial to mesenchymal transition, fibroblast-to-myofibroblast transdifferentiation, and/or extracellular matrix deposition is induced as the level of interaction between TGF-β and its receptor increases.

In one embodiment, TGF-β may be TGF-β1 (transforming growth factor beta-1), TGF-β2 (transforming growth factor beta-2), or TGF-β3 (transforming growth factor beta-3). In one embodiment, the receptor for TGF-β may be TGF-βRII.

In one embodiment, the TGF-β-mediated signaling pathway may be expressed as TGF-β1-mediated signaling pathway, TGF-β2-mediated signaling pathway, or TGF-β3-mediated signaling pathway.

### 1.4.2. Relationship Between the TGF-β-Mediated Signaling Pathway and Fibrosis

The TGF-β-mediated signaling pathway may induce fibrosis or fibrosis-related diseases, disorders, and/or conditions (hereinafter "fibrosis"). Specifically, activation of the TGF-β-mediated signaling pathway may induce fibrosis.

In this case, activation of the TGF-β-mediated signaling pathway may induce fibrosis through any one of the following actions:

In one example, activation of the TGF-β-mediated signaling pathway may induce TGF-β-related immune responses and/or inflammatory responses.

In one embodiment, activation of the TGF-β-mediated signaling pathway may induce activation of fibroblasts and myofibroblasts.

In one embodiment, activation of the TGF-β-mediated signaling pathway may promote extracellular matrix production and/or secretion by myofibroblasts.

In one embodiment, myofibroblasts activated by activation of the TGF-β-mediated signaling pathway may actively secrete extracellular matrix, particularly fibrous connective tissue such as collagen.

In one embodiment, activation of the TGF-β-mediated signaling pathway may sustain the activity of myofibroblasts, causing extracellular matrix such as collagen to continuously accumulate within the tissue.

In one embodiment, activation of the TGF-β-mediated signaling pathway may induce epithelial to mesenchymal transition (EMT) of tissue epithelial cells.

Through such activation of the TGF-β-mediated signaling pathway, fibrous connective tissue may be formed excessively in the tissue, thereby inducing fibrosis.

### 1.4.3. Biomarkers Associated with the TGF-β-Mediated Signaling Pathway

Accordingly, activation of the TGF-β-mediated signaling pathway affects the expression levels of factors associated with fibrosis. For example, activation of the TGF-β-mediated signaling pathway may increase the expression levels of factors that influence the induction/aggravation of fibrosis in or around the tissue in which fibrosis has occurred. For example, activation of the TGF-β-mediated signaling pathway may decrease the expression levels of factors that influence the alleviation/inhibition of fibrosis.

In one embodiment, activation of the TGF-β-mediated signaling pathway may increase the expression level of one or more of CSF3, TGF-β, α-SMA, COL1A1, and IL-11.

In one embodiment, activation of the TGF-β-mediated signaling pathway may induce the expression of extracellular matrix. Specifically, activation of the TGF-β-mediated signaling pathway may increase the expression level of one or more of collagen, fibronectin, hyaluronic acid, versican, and osteopontin (OPN).

In one embodiment, activation of the TGF-β-mediated signaling pathway may increase the expression level of hyaluronan synthase (HAS). Here, HAS may be HAS1, HAS2, and/or HAS3.

In one embodiment, activation of the TGF-β-mediated signaling pathway may suppress the expression of enzymes that promote extracellular matrix degradation. Specifically, activation of the TGF-β-mediated signaling pathway may decrease the expression level of matrix metalloproteinases (MMPs). Here, the MMP may be one or more selected from MMP2, MMP9, and MMP13.

In one embodiment, activation of the TGF-β-mediated signaling pathway may increase the expression level of tissue inhibitors of metalloproteinase (TIMPs).

In one embodiment, activation of the TGF-β-mediated signaling pathway may decrease the expression level of E-cadherin.

In one embodiment, activation of the TGF-β-mediated signaling pathway may increase the expression level of one or more of Snail1, Snail2, Zeb1, Zeb2, Vimentin, N-cadherin, and Fibronectin.

In one embodiment, when the TGF-β-mediated signaling pathway is inhibited, the expression of one or more of CSF3, TGF-β, α-SMA, COL1A1, IL-11, collagen, fibronectin, hyaluronic acid, versican, osteopontin (OPN), hyaluronan synthase (HAS), tissue inhibitors of metalloproteinase (TIMPs), Snail1, Snail2, Zeb1, Zeb2, Vimentin, N-cadherin, and Fibronectin may be suppressed or the expression level thereof may be reduced.

In another embodiment, when the TGF-β-mediated signaling pathway is inhibited, the expression of one or more of matrix metalloproteinases (MMPs) and E-cadherin may be induced or the expression level thereof may be increased.

### 1.4.4. Mechanism by Which CSF3 Inhibitors Inhibit the TGF-β-Mediated Signaling Pathway

The CSF3 inhibitor of the present disclosure inhibits the TGF-β-mediated signaling pathway. That is, the CSF3 inhibitor may reduce the activity or activation of the TGF-β-mediated signaling pathway involved in fibrosis.

In one example, the CSF3 inhibitor may inhibit the TGF-β-mediated signaling pathway by inhibiting the interaction (or binding) between CSF3 and CSF3R.

In one embodiment, the CSF3 inhibitor may inhibit the interaction (or binding) between TGF-β and its receptor (TGF-βR). In one embodiment, the CSF3 inhibitor may inhibit the interaction between TGF-β and its receptor by inhibiting the interaction between CSF3 and CSF3R. Accordingly, the CSF3 inhibitor of the present disclosure may inhibit the interaction (or binding) between TGF-β and its receptor.

In another example, the TGF-β-mediated signaling pathway may also be inhibited through a reduction in the activity of the non-TGF-β-mediated signaling pathway by the CSF3 inhibitor.

In one embodiment, the non-TGF-β-mediated signaling pathway may also be inhibited by the CSF3 inhibitor, and inhibition of the non-TGF-β-mediated signaling pathway may in turn inhibit the interaction between TGF-β and its receptor.

In yet another example, the CSF3 inhibitor may inhibit epithelial to mesenchymal transition (EMT), fibroblast-to-myofibroblast transdifferentiation (FMT), and/or extracellular matrix deposition by inhibiting the TGF-β-mediated signaling pathway. In yet another embodiment, the CSF3 inhibitor may inhibit EMT, FMT, and/or extracellular matrix deposition by inhibiting the interaction between CSF3 and CSF3R. In yet another embodiment, the non-TGF-β-mediated signaling pathway may also be inhibited by the CSF3 inhibitor, and such inhibition of the non-TGF-β-mediated signaling pathway may in turn inhibit EMT, FMT, and/or extracellular matrix deposition.

In one embodiment, the CSF3 inhibitor may suppress or reduce the expression level of one or more of CSF3, TGF-β, α-SMA, COL1A1, IL-11, collagen, fibronectin, hyaluronic acid, versican, osteopontin (OPN), hyaluronan synthase (HAS), tissue inhibitors of metalloproteinase (TIMPs), Snail1, Snail2, Zeb1, Zeb2, Vimentin, N-cadherin, and Fibronectin, whose expression levels have been increased by activation of the TGF-β-mediated signaling pathway.

In another embodiment, the CSF3 inhibitor may induce or increase the expression level of one or more of matrix metalloproteinases (MMPs) and E-cadherin, whose expression levels have been decreased by activation of the TGF-β-mediated signaling pathway.

### 1.5. Mechanism of Action of CSF3 Inhibitors 2: Inhibition of the Non-TGF-β-Mediated Signaling Pathway

The CSF3 inhibitor of the present disclosure may inhibit the non-TGF-β-mediated signaling pathway.

In one embodiment, the CSF3 inhibitor may inhibit the non-TGF-β-mediated signaling pathway in a tissue (or cells) of a subject. In another embodiment, when CSF3 is inhibited, the non-TGF-β-mediated signaling pathway may be inhibited in a tissue (or cells) of a subject. That is, the CSF3 inhibitor may reduce the activity or activation of the non-TGF-β-mediated signaling pathway involved in fibrosis.

### 1.5.1. Definition the Non-TGF-β-Mediated Signaling Pathway

The term "non-TGF-β-mediated signaling pathway" as used in the present disclosure refers to a signaling pathway that is separate from the TGF-β-mediated signaling pathway activated by the interaction between TGF-β and its receptor, by which fibrosis, epithelial to mesenchymal transition (EMT), fibroblast-to-myofibroblast transdifferentiation (FMT), and/or extracellular matrix deposition is induced by CSF3. Specifically, the non-TGF-β-mediated signaling pathway refers to a signaling pathway by which fibrosis, EMT, FMT, and/or extracellular matrix deposition is induced through the interaction between CSF3 and CSF3R. Therefore, the "non-TGF-β-mediated signaling pathway" as used herein may also be referred to as the "CSF3-associated and non-TGF-β-mediated signaling pathway."

In one embodiment, the CSF3-associated and non-TGF-β-mediated signaling pathway may induce fibrosis, epithelial to mesenchymal transition, fibroblast-to-myofibroblast transdifferentiation, and/or extracellular matrix deposition in tissue.

In one embodiment, fibrosis, epithelial to mesenchymal transition, fibroblast-to-myofibroblast transdifferentiation, and/or extracellular matrix deposition may be induced by activation of the CSF3-associated and non-TGF-β-mediated signaling pathway.

In one embodiment, the CSF3-associated and non-TGF-β-mediated signaling pathway may be a signaling pathway in which fibrosis, epithelial to mesenchymal transition, fibroblast-to-myofibroblast transdifferentiation, and/or extracellular matrix deposition is induced as the expression level of CSF3 increases.

In one embodiment, the CSF3-associated and non-TGF-β-mediated signaling pathway may be a signaling pathway in which fibrosis, epithelial to mesenchymal transition, fibroblast-to-myofibroblast transdifferentiation, and/or extracellular matrix deposition is induced by the interaction (or binding) between CSF3 and CSF3R.

In one embodiment, the CSF3-associated and non-TGF-β-mediated signaling pathway may be a signaling pathway in which fibrosis, epithelial to mesenchymal transition, fibroblast-to-myofibroblast transdifferentiation, and/or extracellular matrix deposition is induced as the level of interaction between CSF3 and CSF3R increases.

### 1.5.2. Relationship Between the Non-TGF-β-Mediated Signaling Pathway and Fibrosis

The CSF3-associated and non-TGF-β-mediated signaling pathway may induce fibrosis. Specifically, activation of the CSF3-associated and non-TGF-β-mediated signaling pathway may induce fibrosis.

In this case, activation of the CSF3-associated and non-TGF-β-mediated signaling pathway may induce fibrosis through any one of the following actions:

In one embodiment, activation of the CSF3-associated and non-TGF-β-mediated signaling pathway may induce immune responses and/or inflammatory responses in which TGF-β is not involved.

In one embodiment, activation of the CSF3-associated and non-TGF-β-mediated signaling pathway may induce activation of fibroblasts and myofibroblasts.

In one embodiment, activation of the CSF3-associated and non-TGF-β-mediated signaling pathway may promote extracellular matrix production and/or secretion by myofibroblasts.

In one embodiment, myofibroblasts activated by activation of the CSF3-associated and non-TGF-β-mediated signaling pathway may actively secrete extracellular matrix, particularly fibrous connective tissue such as collagen.

In one embodiment, activation of the CSF3-associated and non-TGF-β-mediated signaling pathway may sustain the activity of myofibroblasts, causing extracellular matrix such as collagen to continuously accumulate within the tissue.

In one embodiment, activation of the CSF3-associated and non-TGF-β-mediated signaling pathway may induce epithelial to mesenchymal transition (EMT) of tissue epithelial cells.

As such, activation of the CSF3-associated and non-TGF-β-mediated signaling pathway may also result in excessive formation of fibrous connective tissue in the tissue, thereby inducing fibrosis.

### 1.5.3. Biomarkers Associated with the Non-TGF-β-Mediated Signaling Pathway

Accordingly, activation of the CSF3-associated and non-TGF-β-mediated signaling pathway affects the expression levels of factors associated with fibrosis. For example, activation of the CSF3-associated and non-TGF-β-mediated signaling pathway may increase the expression levels of factors that influence the induction/aggravation of fibrosis. For example, activation of the CSF3-associated and non-TGF-β-mediated signaling pathway may decrease the expression levels of factors that influence the alleviation/inhibition of fibrosis.

In one embodiment, the expression level of one or more of CSF3, CSF3R, TGF-β, α-SMA, COL1A1, and IL-11 may be increased by the CSF3-associated and non-TGF-β-mediated signaling pathway. In one embodiment, activation of the CSF3-associated and non-TGF-β-mediated signaling pathway may increase the expression level of one or more of CSF3, CSF3R, TGF-β, α-SMA, COL1A1, and IL-11.

In one embodiment, the expression level of one or more of CSF3, CSF3R, TGF-β, α-SMA, COL1A1, and IL-11 may be increased by CSF3. In this case, the increase in the expression level of one or more of CSF3, TGF-β, α-SMA, COL1A1, and IL-11 by CSF3 may occur without involvement of the TGF-β-mediated signaling pathway activated by the interaction between TGF-β and its receptor.

In one embodiment, the expression of extracellular matrix may be induced. Specifically, the expression level of one or more of collagen, fibronectin, hyaluronic acid, versican, and osteopontin (OPN) may be increased.

In one embodiment, activation of the CSF3-associated and non-TGF-β-mediated signaling pathway may increase the expression level of hyaluronan synthase (HAS). Here, HAS may be HAS1, HAS2, and/or HAS3.

In one embodiment, activation of the CSF3-associated and non-TGF-β-mediated signaling pathway may suppress the expression of enzymes that promote extracellular matrix degradation. Specifically, activation of the non-TGF-β-mediated signaling pathway may decrease the expression level of matrix metalloproteinases (MMPs). Here, the MMP may be one or more selected from MMP2, MMP9, and MMP13.

In one embodiment, activation of the CSF3-associated and non-TGF-β-mediated signaling pathway may increase the expression level of tissue inhibitors of metalloproteinase (TIMPs).

In one embodiment, activation of the CSF3-associated and non-TGF-β-mediated signaling pathway may decrease the expression level of E-cadherin.

In one embodiment, activation of the CSF3-associated and non-TGF-β-mediated signaling pathway may increase the expression level of one or more of Snail1, Snail2, Zeb1, Zeb2, Vimentin, N-cadherin, and Fibronectin.

In one embodiment, when the CSF3-associated and non-TGF-β-mediated signaling pathway is inhibited, the expression of one or more of CSF3, TGF-β, α-SMA, COL1A1, IL-11, collagen, fibronectin, hyaluronic acid, versican, osteopontin (OPN), hyaluronan synthase (HAS), tissue inhibitors of metalloproteinase (TIMPs), Snail1, Snail2, Zeb1, Zeb2, Vimentin, N-cadherin, and Fibronectin may be suppressed or the expression level thereof may be reduced.

In one embodiment, when the CSF3-associated and non-TGF-β-mediated signaling pathway is inhibited, the expression of one or more of matrix metalloproteinases (MMPs) and E-cadherin may be induced or the expression level thereof may be increased.

### 1.5.4. Mechanism by Which CSF3 Inhibitors Inhibit the Non-TGF-β-Mediated Signaling Pathway

The CSF3 inhibitor of the present disclosure inhibits the CSF3-associated and non-TGF-β-mediated signaling pathway. That is, the CSF3 inhibitor may reduce the operation or activation of the signaling pathway involved in fibrosis, in which CSF3 acts but TGF-β does not.

In one embodiment, during the process of the CSF3-associated and non-TGF-β-mediated signaling pathway, the TGF-β-mediated signaling pathway may be triggered (or activated) by the resulting signaling. That is, the CSF3 inhibitor inhibits the CSF3-associated and non-TGF-β-mediated signaling pathway, thereby also inhibiting the TGF-β-mediated signaling pathway.

In one embodiment, the CSF3 inhibitor may inhibit the CSF3-associated and non-TGF-β-mediated signaling pathway by inhibiting the interaction (or binding) between CSF3 and CSF3R.

In one embodiment, the CSF3 inhibitor may inhibit the CSF3-associated and non-TGF-β-mediated signaling pathway by inhibiting the expression or function of CSF3, thereby inhibiting epithelial to mesenchymal transition (EMT), fibroblast-to-myofibroblast transdifferentiation (FMT), and/or extracellular matrix deposition. In yet another embodiment, the CSF3 inhibitor may inhibit EMT, FMT, and/or extracellular matrix deposition by inhibiting the interaction between CSF3 and CSF3R.

In one embodiment, the CSF3 inhibitor may suppress or reduce the expression level of one or more of CSF3, TGF-β, α-SMA, COL1A1, IL-11, collagen, fibronectin, hyaluronic acid, versican, osteopontin (OPN), hyaluronan synthase (HAS), tissue inhibitors of metalloproteinase (TIMPs), Snail1, Snail2, Zeb1, Zeb2, Vimentin, N-cadherin, and Fibronectin, whose expression levels have been increased by activation of the CSF3-associated and non-TGF-β-mediated signaling pathway.

In one embodiment, the CSF3 inhibitor may induce or increase the expression level of one or more of matrix metalloproteinases (MMPs) and E-cadherin, whose expression levels have been decreased by activation of the CSF3-associated and non-TGF-β-mediated signaling pathway.

### 1.6. Mechanism of Action of CSF3 Inhibitors 3: Inhibition of Positive-Feedback Loops

In one embodiment, the CSF3 inhibitor may inhibit one or more of the following positive-feedback loops:
the positive-feedback loop of CSF3,
the positive-feedback loop of TGF-β, and
the reciprocal positive-feedback loop between CSF3 and TGF-β.

In another embodiment, the CSF3 inhibitor may inhibit the positive-feedback loop of CSF3, the positive-feedback loop of TGF-β, and/or the reciprocal positive-feedback loop between CSF3 and TGF-β in a tissue (or cells) of a subject. That is, the activity of the positive-feedback loops may be reduced.

### 1.6.1. Definition the Positive-Feedback Loop of CSF3

The term "positive-feedback loop of CSF3" as used in the present disclosure refers to a signaling pathway in which an increase in CSF3 further induces the expression of CSF3. That is, the positive-feedback loop of CSF3 refers to the process in which CSF3 leads to the production of more CSF3, and the produced CSF3 in turn leads to the production of even more CSF3. In one embodiment, activation of the positive-feedback loop of CSF3 may further induce or increase the expression level of CSF3. Therefore, the "positive-feedback loop of CSF3" as used herein may also be referred to as the "autocrine positive-feedback loop of CSF3."

In one embodiment, the positive-feedback loop of CSF3 may be activated through one or more of the following processes:
activation of the TGF-β-mediated signaling pathway and/or the non-TGF-β-mediated signaling pathway by CSF3;
an increase in the expression level of CSF3 and/or TGF-β by the activated TGF-β-mediated signaling pathway and/or activated non-TGF-β-mediated signaling pathway;
further activation of the TGF-β-mediated signaling pathway and/or the non-TGF-β-mediated signaling pathway by the expressed CSF3;
further activation of the TGF-β-mediated signaling pathway by the expressed TGF-β; and
a further increase in the expression level of CSF3 and/or TGF-β by the activated TGF-β-mediated signaling pathway and/or activated non-TGF-β-mediated signaling pathway.

Here, the processes of "an increase in the expression level of CSF3 and/or TGF-β by the activated TGF-β-mediated signaling pathway and/or activated non-TGF-β-mediated signaling pathway" and "further activation of the TGF-β-mediated signaling pathway and/or the non-TGF-β-mediated signaling pathway by the expressed CSF3; and further activation of the TGF-β-mediated signaling pathway by the expressed TGF-β" may be repeated, and through this repetitive process (or loop structure), the positive-feedback loop that increases the expression level of CSF3 may be activated.

### 1.6.2. Definition the Positive-Feedback Loop of TGF-β

The term "positive-feedback loop of TGF-β" as used in the present disclosure refers to a signaling pathway in which an increase in TGF-β further induces the expression of TGF-β. That is, the positive-feedback loop of TGF-β refers to the process in which TGF-β leads to the production of more TGF-β, and the produced TGF-β in turn leads to the production of even more TGF-β. In one embodiment, activation of the positive-feedback loop of TGF-β may induce or increase the expression level of TGF-β. Therefore, the "positive-feedback loop of TGF-β" as used herein may also be referred to as the "autocrine positive-feedback loop of TGF-β."

In one embodiment, the positive-feedback loop of TGF-β may be activated through one or more of the following processes:
activation of the TGF-β-mediated signaling pathway by TGF-β;
an increase in the expression level of CSF3 and/or TGF-β by the activated TGF-β-mediated signaling pathway;
activation of the TGF-β-mediated signaling pathway and/or the non-TGF-β-mediated signaling pathway by the expressed CSF3;
further activation of the TGF-β-mediated signaling pathway by the expressed TGF-β; and
a further increase in the expression level of CSF3 and/or TGF-β by the activated TGF-β-mediated signaling pathway and/or activated non-TGF-β-mediated signaling pathway.

Here, the processes of "an increase in the expression level of CSF3 and/or TGF-β by the activated TGF-β-mediated signaling pathway and/or activated non-TGF-β-mediated signaling pathway" and "activation of the TGF-β-mediated signaling pathway and/or the non-TGF-β-mediated signaling pathway by the expressed CSF3; and further activation of the TGF-β-mediated signaling pathway by the expressed TGF-β" may be repeated, and through this repetitive process (or loop structure), the positive-feedback loop that increases the expression level of TGF-β may be activated.

### 1.6.3. Definition the Reciprocal Positive-Feedback Loop Between CSF3 and TGF-β

The term "reciprocal positive-feedback loop between CSF3 and TGF-β" as used in the present disclosure is a term that describes the reciprocal relationship between CSF3 and TGF-β in which they mutually influence each other's expression increase, referring to a signaling pathway in which: i) an increase in CSF3 induces the expression of TGF-β, and ii) an increase in TGF-β induces the expression of CSF3. That is, the reciprocal positive-feedback loop between CSF3 and TGF-β refers to the process in which CSF3 leads to the production of more TGF-β, and the produced TGF-β in turn leads to the production of more CSF3. Alternatively, the reciprocal positive-feedback loop between CSF3 and TGF-β refers to the process in which TGF-β leads to the production of more CSF3, and the produced CSF3 in turn leads to the production of more TGF-β. In one embodiment, activation of the reciprocal positive-feedback loop between CSF3 and TGF-β may induce or increase the expression levels of CSF3 and TGF-β.

In one embodiment, the reciprocal positive-feedback loop between CSF3 and TGF-β may be activated through one or more of the following processes:
activation of the TGF-β-mediated signaling pathway and/or the CSF3-associated and non-TGF-β-mediated signaling pathway by CSF3;
an increase in the expression level of CSF3 and/or TGF-β by the activated TGF-β-mediated signaling pathway and/or activated CSF3-associated and non-TGF-β-mediated signaling pathway;
activation of the TGF-β-mediated signaling pathway and/or the CSF3-associated and non-TGF-β-mediated signaling pathway by the expressed CSF3;
further activation of the TGF-β-mediated signaling pathway by the expressed TGF-β; and
an increase in the expression levels of CSF3 and TGF-β by the activated TGF-β-mediated signaling pathway and/or activated CSF3-associated and non-TGF-β-mediated signaling pathway.

Here, the processes of "an increase in the expression levels of CSF3 and TGF-β by the activated TGF-β-mediated signaling pathway and/or activated non-TGF-β-mediated signaling pathway" and "activation of the TGF-β-mediated signaling pathway and/or the non-TGF-β-mediated signaling pathway by the expressed CSF3; and further activation of the TGF-β-mediated signaling pathway by the expressed TGF-β" may be repeated, and through this repetitive process (or loop structure), the positive-feedback loop that increases the expression levels of CSF3 and TGF-β may be activated.

In yet another embodiment, the reciprocal positive-feedback loop between CSF3 and TGF-β may be activated through one or more of the following processes:
activation of the TGF-β-mediated signaling pathway by TGF-β;
an increase in the expression level of CSF3 and/or TGF-β by the activated TGF-β-mediated signaling pathway;
activation of the TGF-β-mediated signaling pathway and/or the non-TGF-β-mediated signaling pathway by the expressed CSF3;
further activation of the TGF-β-mediated signaling pathway by the expressed TGF-β; and
an increase in the expression levels of CSF3 and TGF-β by the activated TGF-β-mediated signaling pathway and/or activated non-TGF-β-mediated signaling pathway.

Here, the processes of "an increase in the expression levels of CSF3 and TGF-β by the activated TGF-β-mediated signaling pathway and/or activated non-TGF-β-mediated signaling pathway" and "activation of the TGF-β-mediated signaling pathway and/or the non-TGF-β-mediated signaling pathway by the expressed CSF3; and further activation of the TGF-β-mediated signaling pathway by the expressed TGF-β" may be repeated, and through this repetitive process (or loop structure), the positive-feedback loop that increases the expression levels of CSF3 and TGF-β may be activated.

### 1.6.4. Mechanism by Which CSF3 Inhibitors Inhibit the Positive-Feedback Loops

The CSF3 inhibitor of the present disclosure may inhibit one or more of the aforementioned positive-feedback loops.

For example, it may inhibit both the positive-feedback loop of CSF3 and the positive-feedback loop of TGF-β expression. Likewise, the CSF3 inhibitor may inhibit the reciprocal positive-feedback loop between CSF3 and TGF-β.

In one embodiment, the CSF3 inhibitor may inhibit both the expression of CSF3 and the expression of TGF-β. In another embodiment, the CSF3 inhibitor may reduce both the expression level of CSF3 and the expression level of TGF-β. That is, by inhibiting CSF3, both the expression of CSF3 and the expression of TGF-β may be suppressed.

In the positive-feedback loop of CSF3, the positive-feedback loop of TGF-β expression, and the reciprocal positive-feedback loop between CSF3 and TGF-β, CSF3 and TGF-β are closely interrelated. Within this close interrelationship, (i) inhibiting CSF3 can suppress the positive-feedback loop of CSF3, the positive-feedback loop of TGF-β expression, and the reciprocal positive-feedback loop between CSF3 and TGF-β to a meaningful degree, whereas (ii) inhibiting TGF-β alone makes it difficult to suppress the positive-feedback loop of CSF3, the positive-feedback loop of TGF-β expression, and the reciprocal positive-feedback loop between CSF3 and TGF-β to a meaningful degree. That is, compared to inhibiting TGF-β, inhibiting CSF3 results in stronger suppression of the positive-feedback loop of CSF3, the positive-feedback loop of TGF-β expression, and the reciprocal positive-feedback loop between CSF3 and TGF-β.

In one embodiment, when TGF-β is inhibited, the expression level of CSF3 and the expression level of TGF-β are reduced, and when CSF3 is inhibited, the expression level of CSF3 and the expression level of TGF-β are also reduced. In this case, compared to inhibiting TGF-β, inhibiting CSF3 results in a greater reduction in the expression levels of both CSF3 and TGF-β.

The reason is that inhibiting CSF3 suppresses both the TGF-β-mediated signaling pathway and the non-TGF-β-mediated signaling pathway within the loop structures of the positive-feedback loop of CSF3, the positive-feedback loop of TGF-β expression, and the reciprocal positive-feedback loop between CSF3 and TGF-β, whereas inhibiting TGF-β only suppresses the TGF-β-mediated signaling pathway.

### 1.7. Key Regulator of Fibrosis-Inducing Signaling Pathways: CSF3

The inventors of the present invention were the first to demonstrate that when CSF3 is inhibited, the TGF-β-mediated signaling pathway and the non-TGF-β-mediated signaling pathway are both inhibited, and when CSF3 increases, both the TGF-β-mediated signaling pathway and the non-TGF-β-mediated signaling pathway are activated. That is, it has been demonstrated for the first time that CSF3 is the key regulator of all signaling pathways that induce fibrosis or tissue fibrosis. Based on experimental data demonstrating that CSF3 is the key regulator in fibrosis induction, as described above, the inventors hypothesize that CSF3 is an upstream regulator of TGF-β.

For example, referring to Experimental Examples 2.3 to 2.7, when comparing (i) the reduction in CSF3 expression level resulting from inhibition of the TGF-β-mediated signaling pathway alone, with (ii) the reduction in CSF3 expression level resulting from inhibition of both the TGF-β-mediated signaling pathway and the non-TGF-β-mediated signaling pathway, the magnitude of reduction is greater in the latter (ii).

For example, referring to Experimental Examples 2.3 to 2.7, when comparing (i) the expression levels of α-SMA, Col1A1, CSF3, IL11, CSF3R, and the like resulting from inhibition of the TGF-β-mediated signaling pathway alone, with (ii) the expression levels of the same markers resulting from inhibition of both the TGF-β-mediated signaling pathway and the non-TGF-β-mediated signaling pathway, the magnitude of reduction is greater in the latter (ii).

For example, referring to Experimental Example 2.5, FIG. 17, and FIG. 18, it can be confirmed that when the interaction between TGF-β and its receptor (TGF-βR) is inhibited, the expression levels of fibrosis-related markers are reduced to a modest degree. In contrast, referring to Experimental Examples 2.5 and 2.6, FIG. 17, FIG. 18, and FIG. 19, it can be confirmed that when the interaction between CSF3 and its receptor (CSF3R) is inhibited, the expression levels of fibrosis-related markers are markedly reduced. That is, compared to inhibiting the interaction between TGF-β and its receptor, inhibiting the interaction between CSF3 and its receptor (CSF3R) results in expression levels of fibrosis-related markers that are closer to those of the control group (normal group without fibrosis induction).

### 1.8. Effect of CSF3 Inhibitors in Restoring Fibrotic Tissue to Normal Tissue

The CSF3 inhibitor of the present disclosure restores fibrotic tissue (or cells) to normal tissue (or cells). In another embodiment, the CSF3 inhibitor causes fibrotic tissue (or cells) to return to normal tissue (or cells).

Referring to the experimental examples and FIGS. 15, 20, 26, 28, 29, 41, 42, 43, and 45 of the present specification, it can be seen that fibrotic tissue (or cells) returns to normal tissue (or cells) due to CSF3 inhibition.

On the other hand, the method of inhibiting the TGF-β-mediated signaling pathway using commercially available pulmonary fibrosis therapeutic agents (e.g., nintedanib or pirfenidone) does not result in fibrotic tissue (or cells) returning to normal tissue (or cells). That is, it is widely recognized in the art that these therapeutic agents only provide symptomatic relief of fibrosis, without achieving the therapeutic effect of restoring the tissue to a normal state.

However, the CSF3 inhibitor disclosed in the present specification has the therapeutic effect of restoring fibrotic tissue to normal tissue.

The reason for the difference in efficacy between commercially available pulmonary fibrosis therapeutic agents and the CSF3 inhibitor may be that the CSF3 inhibitor not only inhibits the TGF-β-mediated signaling pathway, but also additionally possesses the effect of inhibiting the non-TGF-β-mediated signaling pathway, compared to commercially available pulmonary fibrosis therapeutic agents. From another perspective, the difference in efficacy may be because the CSF3 inhibitor more potently inhibits the TGF-β-mediated signaling pathway compared to commercially available pulmonary fibrosis therapeutic agents.

### 1.9. Examples of CSF3 Inhibitors

The CSF3 inhibitors described in the present disclosure are not otherwise limited, as long as they are capable of inhibiting the function of CSF3. The CSF3 inhibitor may: 1) interfere with the interaction between the CSF3 protein and other molecules; or 2) inhibit the expression of the CSF3 protein to reduce the absolute amount of CSF3 protein available to interact with other molecules, thereby ultimately interfering with the function of the CSF3 protein. Examples of CSF3 inhibitors capable of such actions include, but are not limited to, nucleic acids that silence the mRNA of the CSF3 gene, CRISPR/Cas systems that knock out or knock down the CSF3 gene, compounds that inhibit the CSF3 protein, and aptamers that inhibit the CSF3 protein.

### 1.9.1. Nucleic Acids That Silence the mRNA of the CSF3 Gene

The CSF3 inhibitor may silence the mRNA of the CSF3 gene to interfere with the translation of the CSF3 protein. In one embodiment, the CSF3 inhibitor may be a nucleic acid capable of specifically binding to the mRNA transcribed from the CSF3 gene, selected from siRNA (small interfering RNA), shRNA (small hairpin RNA), miRNA (microRNA), and antisense nucleic acids.

### 1.9.2. CRISPR/Cas System Targeting the CSF3 Gene 1 - Nuclease

The CSF3 inhibitor may knock out the CSF3 gene in the intracellular genome to prevent production of the CSF3 protein. For example, a CRISPR/Cas system capable of acting as a target-specific nuclease can cleave a specific gene in the cellular genome and induce errors in intracellular DNA repair mechanisms (e.g., non-homologous end joining (NHEJ) and/or homology-directed repair (HDR)) to generate indels within the specific gene and thereby knock out the specific gene. In one embodiment, the CSF3 inhibitor may be a composition comprising a CRISPR/Cas system comprising: a Cas protein or a nucleic acid encoding the same; and a guide RNA or a nucleic acid encoding the same, wherein the guide RNA is capable of targeting the CSF3 gene in the cellular genome and forming a complex by interacting with the Cas protein.

### 1.9.3. CRISPR/Cas System Targeting the CSF3 Gene 2 - Regulator

The CSF3 inhibitor may suppress the expression of the CSF3 gene in the intracellular genome to produce a knockdown effect, thereby inhibiting production of the CSF3 protein. In one embodiment, the CSF3 inhibitor may be a composition comprising a CRISPRi system comprising: a fusion protein in which a Cas protein and at least one gene expression regulatory domain are combined, or a nucleic acid encoding the fusion protein; and a guide RNA and a nucleic acid encoding the same, wherein the guide RNA is capable of targeting the CSF3 gene in the cellular genome and forming a complex by interacting with the fusion protein. Here, the gene expression regulatory domain may be a polypeptide or protein that suppresses the expression of the CSF3 gene in the intracellular genome.

### 1.9.4. Substances That Inhibit the CSF3 Protein 1 - Compound

In one embodiment, the CSF3 inhibitor may be a compound and/or small molecule that functions to prevent the CSF3 protein from interacting with other molecules.

### 1.9.5. Substances That Inhibit the CSF3 Protein 2 - Aptamer

In one embodiment, the CSF3 inhibitor may be a DNA and/or RNA aptamer that functions to prevent the CSF3 protein from interacting with other molecules.

### 1.9.6. Substances That Inhibit the CSF3 Protein 3 - Antibody

In one embodiment, the CSF3 inhibitor may be an anti-CSF3 antibody or a fragment thereof. The anti-CSF3 antibody refers to an antibody (or immunoglobulin) that recognizes the CSF3 protein as an antigen. The anti-CSF3 antibody refers to an antibody having the function of recognizing all or part of the CSF3 protein and/or the function of binding to all or part of the CSF3 protein. A fragment of the anti-CSF3 antibody is a molecule comprising a portion of the anti-CSF3 antibody and having the function of the anti-CSF3 antibody (i.e., recognizing and binding to all or part of the CSF3 protein). That is, the anti-CSF3 antibody or fragment thereof may recognize and/or bind to all or part of the CSF3 protein. Accordingly, the anti-CSF3 antibody or fragment thereof may bind to the CSF3 protein expressed in fibrotic tissue, interfere with the interaction of the CSF3 protein with other molecules (e.g., CSF3R), and ultimately inhibit the function of the CSF3 protein. The anti-CSF3 antibody or fragment thereof is not otherwise limited, as long as it is capable of inhibiting CSF3.

In one embodiment, the anti-CSF3 antibody may be immunoglobulin G (IgG). Here, the IgG may be selected from IgG1, IgG2, IgG3, and IgG4.

In one embodiment, the anti-CSF3 antibody may be any one of a human-derived antibody, a humanized antibody, and a chimeric antibody.

In one embodiment, the anti-CSF3 antibody may be selected from a monoclonal antibody, a polyclonal antibody, a hybridoma monoclonal antibody, and a recombinant monoclonal antibody.

In one embodiment, the anti-CSF3 antibody may be a human IgG.

In one embodiment, the anti-CSF3 antibody may be a CSF3-neutralizing antibody.

In one embodiment, the fragment of the anti-CSF3 antibody may be a fragment of a CSF3-neutralizing antibody.

In one embodiment, the fragment of the anti-CSF3 antibody may be selected from F(ab), F(ab'), F(ab')2, monospecific F(ab')2, bispecific F(ab')2, scFv, ScFv-Fc, and sdAb.

In one embodiment, the anti-CSF3 antibody may be as described in the heading "5. Anti-CSF3 Antibody."

### 2. Mechanism of Action of CSF3R Inhibitor in Restoring Fibrotic Tissue to Normal Tissue

### 2.1. Background Art - Effect of CSF3R Inhibitor in Restoring Fibrotic Tissue to Normal Tissue

As described in this background art, the inventors of the present disclosure have discovered a therapeutic use of a colony stimulating factor 3 receptor (CSF3R) inhibitor for pulmonary fibrosis. Specifically, the inventors discovered that when the function of CSF3R in fibrotic lung tissue is inhibited using an antibody, the fibrotic lung tissue is restored to normal lung tissue and the fibrosis is treated (WO 2023/038416). The therapeutic effect of restoring fibrotic lung tissue to normal lung tissue is an effect that has not been confirmed in treatments using commercialized pulmonary fibrosis therapeutics.

Based on the fact that CSF3 and CSF3R interact with each other, the present inventors conducted further research to confirm whether the effect and mechanism of action upon inhibiting CSF3R are the same as or similar to those upon inhibiting CSF3 as described above.

That is, to understand the cause of the therapeutic effect of restoring fibrotic lung tissue to normal lung tissue, the present inventors further studied the mechanism of action of the CSF3R inhibitor's effect in returning fibrotic tissue to normal tissue, and the results are disclosed herein.

Through this, it can be seen that even when CSF3R is inhibited, it has the same mechanism of action as when CSF3 is inhibited with respect to the "TGF-β mediated signaling pathway" and the "non-TGF-β mediated and CSF3-associated signaling pathway."

### 2.2. Overview of the Mechanism of Action of CSF3R Inhibitor

In one aspect of the present disclosure, a method for restoring fibrotic tissue (or cells) to normal tissue (or cells) using a CSF3R inhibitor is disclosed. In another aspect, a method for inhibiting extracellular matrix (ECM) deposition and epithelial to mesenchymal transition (EMT) using a CSF3R inhibitor is disclosed. At this time, the method for restoring fibrotic tissue to normal tissue may include the method for inhibiting ECM deposition and EMT. In one embodiment, the method for inhibiting ECM deposition and EMT may be a method for inhibiting ECM deposition and EMT in a tissue of a subject. At this time, the tissue of the subject may be a tissue at risk of occurring or a tissue in which ECM deposition and/or EMT has occurred.

The method for restoring fibrotic tissue to normal tissue includes administering a CSF3R inhibitor to a subject or a tissue of a subject. The method for inhibiting ECM deposition and EMT includes administering a CSF3R inhibitor to a subject or a tissue of a subject.

In one embodiment, the CSF3R inhibitor can inhibit a TGF-β mediated signaling pathway. The TGF-β mediated signaling pathway is a signaling pathway capable of inducing fibrosis through the interaction between TGF-β and a TGF-β receptor.

In one embodiment, the CSF3R inhibitor can inhibit a non-TGF-β mediated signaling pathway. The non-TGF-β mediated signaling pathway is a signaling pathway that induces fibrosis through the interaction between CSF3 and CSF3R, and is a signaling pathway that induces fibrosis through a pathway separate from the TGF-β mediated signaling pathway. Therefore, the non-TGF-β mediated signaling pathway may also be denoted as a CSF3-associated and non-TGF-β mediated signaling pathway, or a CSF3R-associated and non-TGF-β mediated signaling pathway.

In one embodiment, the CSF3R inhibitor can inhibit the positive-feedback of CSF3R. In one embodiment, the CSF3R inhibitor can inhibit the positive-feedback of TGF-β. In one embodiment, the CSF3R inhibitor can restore fibrotic tissue (or cells) to normal tissue (or cells). In one embodiment, the CSF3R inhibitor can inhibit ECM deposition and EMT.

In one embodiment, the subject may be a human or a non-human animal.

In one embodiment, the tissue may be skin, brain, nervous system, heart, bone marrow, liver, gut, joint, eye, lung, kidney, retroperitoneum, mediastinum, and/or pancreas.

In one embodiment, the cells may be cells derived from the skin, brain, nervous system, heart, bone marrow, liver, gut, joint, eye, lung, kidney, retroperitoneum, mediastinum, and/or pancreas.

### 2.3. Definition of CSF3R Inhibitor

Colony-stimulating factor 3 receptor (CSF3R) in the present disclosure is also referred to as granulocyte colony-stimulating factor (G-CSF) receptor. CSF3R is known to stimulate proliferation, differentiation, adhesion, and end-cell functional activation of granulocytes in animals, including human subjects. The term "CSF3R inhibitor" in the present disclosure refers to a substance that ultimately inhibits the function of CSF3R. As long as it can inhibit the function of CSF3R, its type, form, and composition are not particularly limited. Specifically, inhibiting the function of CSF3R means: 1) binding to the CSF3R protein to inhibit the function of the CSF3R protein itself; 2) inhibiting the interaction of the CSF3R protein with other molecules; 3) inhibiting the expression of CSF3R to consequently block the opportunity for CSF3R to function; or 4) inhibiting the function of CSF3R through other direct or indirect pathways.

In one embodiment, the CSF3R inhibitor may be an anti-CSF3R antibody. In another embodiment, the CSF3R inhibitor may be a nucleic acid molecule capable of silencing the mRNA of the CSF3R gene or causing RNA interference. Specifically, the nucleic acid molecule may include an antisense oligonucleotide or iRNA (interfering RNA), for example, miRNA, siRNA (small interfering RNA), shRNA (short hairpin RNA), and the like. In another embodiment, the CSF3R inhibitor may be a compound that inhibits the function of CSF3R.

### 2.4. Mechanism of Action 1 of CSF3R Inhibitor: Inhibition of TGF-β Mediated Signaling Pathway

In one embodiment, the CSF3R inhibitor can inhibit a TGF-β mediated signaling pathway. In another embodiment, the CSF3R inhibitor can inhibit the TGF-β mediated signaling pathway in a tissue (or cells) of a subject. In another embodiment, when CSF3R is inhibited, the TGF-β mediated signaling pathway may be inhibited in a tissue (or cells) of a subject. That is, the action or activation of the TGF-β mediated signaling pathway involved in fibrosis can be reduced.

### 2.4.1. Definition of TGF-β Mediated Signaling Pathway

The term "TGF-β mediated signaling pathway" in the present disclosure refers to a signaling pathway by which transforming growth factor beta (TGF-β) induces fibrosis, epithelial to mesenchymal transition (EMT), fibroblast-to-myofibroblast transdifferentiation (FMT), and/or extracellular matrix (ECM) deposition. Specifically, the TGF-β mediated signaling pathway refers to a signaling pathway by which fibrosis, EMT, FMT, and/or ECM deposition are induced through the interaction between TGF-β and its receptor (TGF-βR).

In one embodiment, the TGF-β mediated signaling pathway can induce fibrosis, epithelial to mesenchymal transition, fibroblast-to-myofibroblast transdifferentiation, and/or ECM deposition.

In one embodiment, fibrosis, epithelial to mesenchymal transition, fibroblast-to-myofibroblast transdifferentiation, and/or ECM deposition may be induced due to the activation of the TGF-β mediated signaling pathway.

In one embodiment, the TGF-β mediated signaling pathway may be a signaling pathway by which fibrosis, epithelial to mesenchymal transition, fibroblast-to-myofibroblast transdifferentiation, and/or ECM deposition are induced as the expression level of TGF-β increases.

In one embodiment, the TGF-β mediated signaling pathway may be a signaling pathway by which fibrosis, epithelial to mesenchymal transition, fibroblast-to-myofibroblast transdifferentiation, and/or ECM deposition are induced through the interaction (or binding) between TGF-β and its receptor.

In one embodiment, the TGF-β mediated signaling pathway may be a signaling pathway by which fibrosis, epithelial to mesenchymal transition, fibroblast-to-myofibroblast transdifferentiation, and/or ECM deposition are induced as the level of interaction between TGF-β and its receptor increases.

### 2.4.2. Relationship Between TGF-β Mediated Signaling Pathway and Fibrosis

The TGF-β mediated signaling pathway can induce fibrosis. Specifically, activation of the TGF-β mediated signaling pathway can induce fibrosis.

At this time, the activation of the TGF-β mediated signaling pathway can induce fibrosis through any one of the following actions:

In one example, the activation of the TGF-β mediated signaling pathway can induce immune reactions and/or inflammatory reactions related to TGF-β.

In one embodiment, the activation of the TGF-β mediated signaling pathway can induce the activation of fibroblasts and myofibroblasts.

In one embodiment, the activation of the TGF-β mediated signaling pathway can promote the production and/or secretion of extracellular matrix by myofibroblasts.

In one embodiment, myofibroblasts activated by the activation of the TGF-β mediated signaling pathway can actively secrete extracellular matrix, particularly fibrous connective tissue such as collagen.

In one embodiment, the activation of the TGF-β mediated signaling pathway can continuously maintain the activity of myofibroblasts, causing extracellular matrix such as collagen to continuously accumulate within the tissue.

In one embodiment, the activation of the TGF-β mediated signaling pathway can induce epithelial to mesenchymal transition (EMT) of tissue epithelial cells.

Through such activation of the TGF-β mediated signaling pathway, fibrous connective tissue can be excessively formed in a tissue, thereby inducing fibrosis.

### 2.4.3. Biomarkers Related to TGF-β Mediated Signaling Pathway

Therefore, the activation of the TGF-β mediated signaling pathway affects the expression levels of factors related to fibrosis. For example, the activation of the TGF-β mediated signaling pathway can increase the expression levels of factors that influence the induction/exacerbation of fibrosis in or around the tissue where fibrosis has occurred. For example, the activation of the TGF-β mediated signaling pathway can decrease the expression levels of factors that influence the alleviation/suppression of fibrosis.

In one embodiment, the activation of the TGF-β mediated signaling pathway can increase the expression level of at least one of CSF3R, TGF-β, α-SMA, COL1A1, and IL-11.

In one embodiment, the activation of the TGF-β mediated signaling pathway can induce the expression of extracellular matrix. Specifically, the activation of the TGF-β mediated signaling pathway can increase the expression level of at least one of collagen, fibronectin, hyaluronan, Versican, and Osteopontin (OPN).

In one embodiment, the activation of the TGF-β mediated signaling pathway can increase the expression level of hyaluronan synthase (HAS). At this time, the HAS may be HAS1, HAS2, and/or HAS3.

In one embodiment, the activation of the TGF-β mediated signaling pathway can suppress the expression of enzymes that promote extracellular matrix degradation. Specifically, the activation of the TGF-β mediated signaling pathway can decrease the expression level of matrix metalloproteinase (MMP). At this time, the MMP may be at least one selected from MMP2, MMP9, and MMP13.

In one embodiment, the activation of the TGF-β mediated signaling pathway can increase the expression level of tissue inhibitor of metalloproteinase (TIMP).

In one embodiment, the activation of the TGF-β mediated signaling pathway can decrease the expression level of E-cadherin.

In one embodiment, the activation of the TGF-β mediated signaling pathway can increase the expression level of at least one of Snail1, Snail2, Zeb1, Zeb2, Vimentin, N-cadherin, and Fibronectin.

In one embodiment, when the TGF-β mediated signaling pathway is inhibited, the expression of at least one of CSF3R, TGF-β, α-SMA, COL1A1, IL-11, collagen, fibronectin, hyaluronan, Versican, Osteopontin (OPN), hyaluronan synthase (HAS), tissue inhibitor of metalloproteinase (TIMP), Snail1, Snail2, Zeb1, Zeb2, Vimentin, N-cadherin, and Fibronectin may be suppressed or the expression level thereof may be decreased.

In one embodiment, when the TGF-β mediated signaling pathway is inhibited, the expression of at least one of matrix metalloproteinase (MMP) and E-cadherin may be induced or the expression level thereof may be increased.

### 2.4.4. Mechanism of Action by Which CSF3R Inhibitor Inhibits TGF-β Mediated Signaling Pathway

The CSF3R inhibitor of the present disclosure inhibits the TGF-β mediated signaling pathway.

That is, the CSF3R inhibitor can reduce the action or activation of the TGF-β mediated signaling pathway involved in fibrosis.

In one example, the CSF3R inhibitor can inhibit the TGF-β mediated signaling pathway by inhibiting the interaction (or binding) between CSF3R and CSF3.

In one embodiment, the CSF3R inhibitor can inhibit the interaction (or binding) between TGF-β and its TGF-β receptor. In another embodiment, the CSF3R inhibitor can inhibit the interaction between TGF-β and its TGF-β receptor by inhibiting the interaction between CSF3R and CSF3. Therefore, the CSF3R inhibitor of the present disclosure can inhibit the interaction (or binding) between TGF-β and its TGF-β receptor.

In another example, the TGF-β mediated signaling pathway can also be inhibited through a reduction in the activity of the non-TGF-β mediated signaling pathway by the CSF3R inhibitor.

In one embodiment, the non-TGF-β mediated signaling pathway may also be inhibited by the CSF3R inhibitor, and by the inhibition of the non-TGF-β mediated signaling pathway, the interaction between TGF-β and its TGF-β receptor may be inhibited.

In another example, the CSF3R inhibitor can inhibit epithelial to mesenchymal transition (EMT), fibroblast-to-myofibroblast transdifferentiation (FMT), and/or extracellular matrix (ECM) deposition by inhibiting the TGF-β mediated signaling pathway. In another embodiment, the CSF3R inhibitor can inhibit EMT, FMT, and/or ECM deposition by inhibiting the interaction between CSF3R and CSF3. In another embodiment, the non-TGF-β mediated signaling pathway may also be inhibited by the CSF3R inhibitor, and EMT, FMT, and/or ECM deposition may be inhibited by the inhibition of the non-TGF-β mediated signaling pathway.

In one embodiment, the CSF3R inhibitor can suppress or decrease the expression level of at least one of CSF3R, TGF-β, α-SMA, COL1A1, IL-11, collagen, fibronectin, hyaluronan, Versican, Osteopontin (OPN), hyaluronan synthase (HAS), tissue inhibitor of metalloproteinase (TIMP), Snail1, Snail2, Zeb1, Zeb2, Vimentin, N-cadherin, and Fibronectin, the expression level of which has been increased due to activation of the TGF-β mediated signaling pathway.

In another embodiment, the CSF3R inhibitor can induce or increase the expression level of at least one of matrix metalloproteinase (MMP) and E-cadherin, the expression level of which has been decreased due to activation of the TGF-β mediated signaling pathway.

### 2.5. Mechanism of Action 2 of CSF3R Inhibitor: Inhibition of Non-TGF-β Mediated Signaling Pathway

The CSF3R inhibitor of the present disclosure can inhibit a non-TGF-β mediated signaling pathway.

In one embodiment, the CSF3R inhibitor can inhibit the non-TGF-β mediated signaling pathway in a tissue (or cells) of a subject. In another embodiment, when CSF3R is inhibited, the non-TGF-β mediated signaling pathway may be inhibited in a tissue (or cells) of a subject. That is, the CSF3R inhibitor can reduce the action or activation of the non-TGF-β mediated signaling pathway involved in fibrosis.

### 2.5.1. Definition of Non-TGF-β Mediated Signaling Pathway

The term "non-TGF-β mediated signaling pathway" in the present disclosure refers to a signaling pathway that is separate from the TGF-β mediated signaling pathway activated by the interaction between TGF-β and its TGF-β receptor, and by which CSF3R induces fibrosis, epithelial to mesenchymal transition (EMT), fibroblast-to-myofibroblast transdifferentiation (FMT), and/or extracellular matrix (ECM) deposition. Specifically, the non-TGF-β mediated signaling pathway refers to a signaling pathway by which fibrosis, EMT, FMT, and/or ECM deposition are induced through the interaction between CSF3 and CSF3R. Therefore, in the present specification, the "non-TGF-β mediated signaling pathway" may also be denoted as "CSF3-associated and non-TGF-β-mediated signaling pathways." Alternatively, the non-TGF-β mediated signaling pathway may be denoted as CSF3R-associated and non-TGF-β mediated signaling pathway.

In one embodiment, the non-TGF-β mediated signaling pathway can induce fibrosis, epithelial to mesenchymal transition, fibroblast-to-myofibroblast transdifferentiation, and/or ECM deposition within a tissue.

In one embodiment, fibrosis, epithelial to mesenchymal transition, fibroblast-to-myofibroblast transdifferentiation, and/or ECM deposition may be induced due to the activation of the non-TGF-β mediated signaling pathway.

In one embodiment, the non-TGF-β mediated signaling pathway may be a signaling pathway by which fibrosis, epithelial to mesenchymal transition, fibroblast-to-myofibroblast transdifferentiation, and/or ECM deposition are induced as the expression level of CSF3R increases.

In one embodiment, the non-TGF-β mediated signaling pathway may be a signaling pathway by which fibrosis, epithelial to mesenchymal transition, fibroblast-to-myofibroblast transdifferentiation, and/or ECM deposition are induced through the interaction (or binding) between CSF3R and CSF3.

In one embodiment, the non-TGF-β mediated signaling pathway may be a signaling pathway by which fibrosis, epithelial to mesenchymal transition, fibroblast-to-myofibroblast transdifferentiation, and/or ECM deposition are induced as the level of interaction between CSF3R and CSF3 increases.

### 2.5.2. Relationship Between Non-TGF-β Mediated Signaling Pathway and Fibrosis

The non-TGF-β mediated signaling pathway can induce fibrosis. Specifically, activation of the non-TGF-β mediated signaling pathway can induce fibrosis.

At this time, the activation of the CSF3-associated and non-TGF-β mediated signaling pathways (Non-TGF-β-mediated and CSF3-associated signaling pathways) can induce fibrosis through any one of the following actions:

In one embodiment, the activation of the non-TGF-β mediated signaling pathway can induce immune reactions and/or inflammatory reactions that do not involve TGF-β.

In one embodiment, the activation of the non-TGF-β mediated signaling pathway can induce the activation of fibroblasts and myofibroblasts.

In one embodiment, the activation of the non-TGF-β mediated signaling pathway can promote the production and/or secretion of extracellular matrix by myofibroblasts.

In one embodiment, myofibroblasts activated by the activation of the non-TGF-β mediated signaling pathway can actively secrete extracellular matrix, particularly fibrous connective tissue such as collagen.

In one embodiment, the activation of the non-TGF-β mediated signaling pathway can continuously maintain the activity of myofibroblasts, causing extracellular matrix such as collagen to continuously accumulate within the tissue.

In one embodiment, the activation of the non-TGF-β mediated signaling pathway can induce epithelial to mesenchymal transition (EMT) of tissue epithelial cells.

In this way, the activation of the CSF3-associated and non-TGF-β mediated signaling pathways (Non-TGF-β-mediated and CSF3-associated signaling pathways) can also cause fibrous connective tissue to be excessively formed in a tissue, thereby inducing fibrosis.

### 2.5.3. Biomarkers Related to Non-TGF-β Mediated Signaling Pathway

Therefore, the activation of the CSF3-associated and non-TGF-β mediated signaling pathways affects the expression levels of factors related to fibrosis. For example, the activation of the CSF3-associated and non-TGF-β mediated signaling pathways can increase the expression levels of factors that influence the induction/exacerbation of fibrosis. For example, the activation of the CSF3-associated and non-TGF-β mediated signaling pathways can decrease the expression levels of factors that influence the alleviation/suppression of fibrosis.

In one embodiment, the expression level of at least one of CSF3, CSF3R, TGF-β, α-SMA, COL1A1, and IL-11 may be increased by the non-TGF-β mediated signaling pathway. In one embodiment, the activation of the non-TGF-β mediated signaling pathway can increase the expression level of at least one of CSF3, CSF3R, TGF-β, α-SMA, COL1A1, and IL-11.

In one embodiment, the expression level of at least one of CSF3, CSF3R, TGF-β, α-SMA, COL1A1, and IL-11 may be increased by CSF3R. At this time, the increase in the expression level of at least one of CSF3, TGF-β, α-SMA, COL1A1, and IL-11 by CSF3R may occur without involvement of the TGF-β mediated signaling pathway activated by the interaction between TGF-β and its TGF-β receptor.

In one embodiment, the activation of the non-TGF-β mediated signaling pathway can induce the expression of extracellular matrix. Specifically, the activation of the non-TGF-β mediated signaling pathway can increase the expression level of at least one of collagen, fibronectin, hyaluronan, Versican, and Osteopontin (OPN).

In one embodiment, the activation of the non-TGF-β mediated signaling pathway can increase the expression level of hyaluronan synthase (HAS). At this time, the HAS may be HAS1, HAS2, and/or HAS3.

In one embodiment, the activation of the non-TGF-β mediated signaling pathway can suppress the expression of enzymes that promote extracellular matrix degradation. Specifically, the activation of the non-TGF-β mediated signaling pathway can decrease the expression level of matrix metalloproteinase (MMP). At this time, the MMP may be at least one selected from MMP2, MMP9, and MMP13.

In one embodiment, the activation of the non-TGF-β mediated signaling pathway can increase the expression level of tissue inhibitor of metalloproteinase (TIMP).

In one embodiment, the activation of the non-TGF-β mediated signaling pathway can decrease the expression level of E-cadherin.

In one embodiment, the activation of the non-TGF-β mediated signaling pathway can increase the expression level of at least one of Snail1, Snail2, Zeb1, Zeb2, Vimentin, N-cadherin, and Fibronectin.

In one embodiment, when the non-TGF-β mediated signaling pathway is inhibited, the expression of at least one of CSF3R, TGF-β, α-SMA, COL1A1, IL-11, collagen, fibronectin, hyaluronan, Versican, Osteopontin (OPN), hyaluronan synthase (HAS), tissue inhibitor of metalloproteinase (TIMP), Snail1, Snail2, Zeb1, Zeb2, Vimentin, N-cadherin, and Fibronectin may be suppressed or the expression level thereof may be decreased.

In one embodiment, when the non-TGF-β mediated signaling pathway is inhibited, the expression of at least one of matrix metalloproteinase (MMP) and E-cadherin may be induced or the expression level thereof may be increased.

### 2.5.4. Mechanism of Action by Which CSF3R Inhibitor Inhibits Non-TGF-β Mediated Signaling Pathway

The CSF3R inhibitor of the present disclosure inhibits the CSF3-associated and non-TGF-β mediated signaling pathways (Non-TGF-β-mediated and CSF3-associated signaling pathways). That is, the CSF3R inhibitor can reduce the operation or activation of a signaling pathway involved in fibrosis in which CSF3R acts but TGF-β does not act.

In one embodiment, during the course of the CSF3-associated and non-TGF-β mediated signaling pathways, the TGF-β mediated signaling pathway may be triggered (or activated). That is, the CSF3R inhibitor inhibits the CSF3-associated and non-TGF-β mediated signaling pathways, whereby the TGF-β mediated signaling pathway may also be inhibited.

In one embodiment, the CSF3R inhibitor can inhibit the CSF3-associated and non-TGF-β mediated signaling pathways by inhibiting the interaction (or binding) between CSF3R and CSF3.

In one embodiment, the CSF3R inhibitor can inhibit the CSF3-associated and non-TGF-β mediated signaling pathways by inhibiting the expression or function of CSF3R, thereby inhibiting epithelial to mesenchymal transition (EMT), fibroblast-to-myofibroblast transdifferentiation (FMT), and/or extracellular matrix (ECM) deposition. In another embodiment, the CSF3R inhibitor can inhibit EMT, FMT, and/or ECM deposition by inhibiting the interaction between CSF3R and CSF3.

In one embodiment, the CSF3R inhibitor can suppress or decrease the expression level of at least one of CSF3R, TGF-β, α-SMA, COL1A1, IL-11, collagen, fibronectin, hyaluronan, Versican, Osteopontin (OPN), hyaluronan synthase (HAS), tissue inhibitor of metalloproteinase (TIMP), Snail1, Snail2, Zeb1, Zeb2, Vimentin, N-cadherin, and Fibronectin, the expression level of which has been increased due to activation of the non-TGF-β mediated signaling pathway.

In one embodiment, the CSF3R inhibitor can induce or increase the expression level of at least one of matrix metalloproteinase (MMP) and E-cadherin, the expression level of which has been decreased due to activation of the non-TGF-β mediated signaling pathway.

### 2.6. Mechanism of Action 3 of CSF3R Inhibitor: Inhibition of Positive-Feedback Loops

In one embodiment, the CSF3R inhibitor can inhibit at least one of the following positive-feedback loops:
The positive-feedback loop of CSF3R,
The positive-feedback loop of TGF-β, and
The reciprocal positive-feedback loop between CSF3R and TGF-β.

In another embodiment, the CSF3R inhibitor can inhibit the positive-feedback loop of CSF3R, the positive-feedback loop of TGF-β, and/or the reciprocal positive-feedback loop between CSF3R and TGF-β in a tissue (or cells) of a subject. That is, the action of the positive-feedback loops can be reduced.

### 2.6.1. Definition of Positive-Feedback Loop of CSF3R

The term "positive-feedback loop of CSF3R" in the present disclosure refers to a signaling pathway by which the expression of CSF3R is further induced by an increase in CSF3R. That is, the positive-feedback loop of CSF3R refers to a process in which more CSF3R is generated due to CSF3R, and more CSF3R is again generated due to the generated CSF3R. In one embodiment, the activation of the positive-feedback loop of CSF3R can induce the expression of CSF3R or increase the expression level of CSF3R. Therefore, in the present specification, the "positive-feedback loop of CSF3R" may also be denoted as the "self positive-feedback loop of CSF3R."

In one embodiment, the positive-feedback loop of CSF3R may be activated through at least one of the following processes:
The TGF-β mediated signaling pathway is activated and/or the non-TGF-β mediated signaling pathway is activated by CSF3R;
The expression level of CSF3R and/or TGF-β is increased by the activated TGF-β mediated signaling pathway and/or the activated non-TGF-β mediated signaling pathway;
The TGF-β mediated signaling pathway and/or the non-TGF-β mediated signaling pathway is further activated by the expressed CSF3R;
The TGF-β mediated signaling pathway is further activated by the expressed TGF-β; and
The expression level of CSF3R and/or TGF-β is further increased by the activated TGF-β mediated signaling pathway and/or the activated non-TGF-β mediated signaling pathway.

At this time, the processes of "the expression level of CSF3R and/or TGF-β is increased by the activated TGF-β mediated signaling pathway and/or the activated non-TGF-β mediated signaling pathway" and "the TGF-β mediated signaling pathway and/or the non-TGF-β mediated signaling pathway is activated by the expressed CSF3R; and the TGF-β mediated signaling pathway is activated by the expressed TGF-β" may be repeated, and through this repetitive process (or loop structure), the positive-feedback loop in which the expression level of CSF3R increases may be activated.

### 2.6.2. Definition of Positive-Feedback Loop of TGF-β

The term "positive-feedback loop of TGF-β" in the present disclosure refers to a signaling pathway by which the expression of TGF-β is further induced by an increase in TGF-β. That is, the positive-feedback loop of TGF-β refers to a process in which more TGF-β is generated due to TGF-β, and more TGF-β is again generated due to the generated TGF-β. In one embodiment, the activation of the positive-feedback loop of TGF-β can induce the expression of TGF-β or increase the expression level of TGF-β. Therefore, in the present specification, the "positive-feedback loop of TGF-β" may also be denoted as the "self positive-feedback loop of TGF-β."

In one embodiment, the positive-feedback loop of TGF-β may be activated through at least one of the following processes:

The TGF-β mediated signaling pathway is activated by TGF-β;

The expression level of CSF3R and/or TGF-β is increased by the activated TGF-β mediated signaling pathway;

The TGF-β mediated signaling pathway is activated and/or the non-TGF-β mediated signaling pathway is activated by the expressed CSF3R;

The TGF-β mediated signaling pathway is activated by the expressed TGF-β; and

The expression level of CSF3R and/or TGF-β is further increased by the activated TGF-β mediated signaling pathway and/or the activated non-TGF-β mediated signaling pathway.

At this time, the processes of "the expression level of CSF3R and/or TGF-β is increased by the activated TGF-β mediated signaling pathway and/or the activated non-TGF-β mediated signaling pathway" and "the TGF-β mediated signaling pathway and/or the non-TGF-β mediated signaling pathway is activated by the expressed CSF3R; and the TGF-β mediated signaling pathway is activated by the expressed TGF-β" may be repeated, and through this repetitive process (or loop structure), the positive-feedback loop in which the expression level of TGF-β increases may be activated.

### 2.6.3. Definition of Reciprocal Positive-Feedback Loop Between CSF3R and TGF-β

The term "reciprocal positive-feedback loop between CSF3R and TGF-β" in the present disclosure is a term representing the reciprocal relationship between CSF3R and TGF-β that mutually influences the increase in each other's expression, and refers to a signaling pathway in which i) the expression of TGF-β is induced by an increase in CSF3R, and ii) the expression of CSF3R is induced by an increase in TGF-β. That is, the reciprocal positive-feedback loop between CSF3R and TGF-β refers to a process in which more TGF-β is generated due to CSF3R, and more CSF3R is again generated due to the generated TGF-β. Alternatively, the reciprocal positive-feedback loop between CSF3R and TGF-β refers to a process in which more CSF3R is generated due to TGF-β, and more TGF-β is again generated due to the generated CSF3R.

The present inventors have revealed through experiments the reciprocal positive-feedback loop mechanism between CSF3R and TGF-β. Specifically, through Experimental Example 2.5, it was confirmed that when cells are treated with CSF3 protein, the expression levels of CSF3, CSF3R, and TGF-β increase, and that the expression levels of CSF3, CSF3R, and TGF-β increased by the CSF3R inhibitor are decreased. These results mean that the expression of CSF3, CSF3R, and TGF-β is increased by the interaction between CSF3 and CSF3R.

In one embodiment, the activation of the reciprocal positive-feedback loop between CSF3R and TGF-β can induce the expression of CSF3R and TGF-β or increase the expression levels of CSF3R and TGF-β.

In one embodiment, the reciprocal positive-feedback loop between CSF3R and TGF-β may be activated through at least one of the following processes:

The TGF-β mediated signaling pathway is activated and/or the CSF3-associated and non-TGF-β mediated signaling pathways are activated by CSF3R;

The expression level of CSF3R and/or TGF-β is increased by the activated TGF-β mediated signaling pathway and/or the activated CSF3-associated and non-TGF-β mediated signaling pathways;

The TGF-β mediated signaling pathway and/or the CSF3-associated and non-TGF-β mediated signaling pathways are activated by the expressed CSF3R;

The TGF-β mediated signaling pathway is activated by the expressed TGF-β; and

The expression levels of CSF3R and TGF-β are increased by the activated TGF-β mediated signaling pathway and/or the activated CSF3-associated and non-TGF-β mediated signaling pathways.

At this time, the processes of "the expression levels of CSF3R and TGF-β are increased by the activated TGF-β mediated signaling pathway and/or the activated non-TGF-β mediated signaling pathway" and "the TGF-β mediated signaling pathway and/or the non-TGF-β mediated signaling pathway is activated by the expressed CSF3R; and the TGF-β mediated signaling pathway is activated by the expressed TGF-β" may be repeated, and through this repetitive process (or loop structure), the positive-feedback loop in which the expression levels of CSF3R and TGF-β increase may be activated.

In another embodiment, the reciprocal positive-feedback loop between CSF3R and TGF-β may be activated through at least one of the following processes:

The TGF-β mediated signaling pathway is activated by TGF-β;

The expression level of CSF3R and/or TGF-β is increased by the activated TGF-β mediated signaling pathway;

The TGF-β mediated signaling pathway and/or the non-TGF-β mediated signaling pathway is activated by the expressed CSF3R;

The TGF-β mediated signaling pathway is activated by the expressed TGF-β; and

The expression levels of CSF3R and TGF-β are increased by the activated TGF-β mediated signaling pathway and/or the activated non-TGF-β mediated signaling pathway.

At this time, the processes of "the expression levels of CSF3R and TGF-β are increased by the activated TGF-β mediated signaling pathway and/or the activated non-TGF-β mediated signaling pathway" and "the TGF-β mediated signaling pathway and/or the non-TGF-β mediated signaling pathway is activated by the expressed CSF3R; and the TGF-β mediated signaling pathway is activated by the expressed TGF-β" may be repeated, and through this repetitive process (or loop structure), the positive-feedback loop in which the expression levels of CSF3R and TGF-β increase may be activated.

### 2.6.4. Mechanism of Action by Which CSF3R Inhibitor Inhibits Positive-Feedback Loops

The CSF3R inhibitor of the present disclosure can inhibit at least one of the aforementioned positive-feedback loops.

For example, it can inhibit both the positive-feedback loop of CSF3R and the positive-feedback loop of TGF-β expression. Likewise, the CSF3R inhibitor can inhibit the reciprocal positive-feedback loop between CSF3R and TGF-β.

In one embodiment, the CSF3R inhibitor can inhibit both the expression of CSF3R and the expression of TGF-β. In another embodiment, the CSF3R inhibitor can decrease both the expression level of CSF3R and the expression level of TGF-β. That is, by inhibiting CSF3R, both the expression of CSF3R and the expression of TGF-β can be inhibited.

In the positive-feedback loop of CSF3R; the positive-feedback loop of TGF-β expression; and the reciprocal positive-feedback loop between CSF3R and TGF-β, CSF3R and TGF-β are in a close relationship. Within this close relationship, (i) inhibiting CSF3R can meaningfully inhibit the positive-feedback loop of CSF3R; the positive-feedback loop of TGF-β expression; and the reciprocal positive-feedback loop between CSF3R and TGF-β, whereas (ii) inhibiting TGF-β alone makes it difficult to meaningfully inhibit the positive-feedback loop of CSF3R; the positive-feedback loop of TGF-β expression; and the reciprocal positive-feedback loop between CSF3R and TGF-β. That is, compared to the case of inhibiting TGF-β, the positive-feedback loop of CSF3R; the positive-feedback loop of TGF-β expression; and the reciprocal positive-feedback loop between CSF3R and TGF-β are inhibited to a stronger degree when CSF3R is inhibited.

In one embodiment, when TGF-β is inhibited, the expression levels of CSF3R and TGF-β are decreased, and when CSF3R is inhibited, the expression levels of CSF3R and TGF-β are decreased. At this time, compared to inhibition of TGF-β, the expression levels of CSF3R and TGF-β are decreased to a greater extent by inhibition of CSF3R.

The reason is that inhibiting CSF3R inhibits both the TGF-β mediated signaling pathway and the non-TGF-β mediated signaling pathway within the structure of the positive-feedback loop of CSF3R; the positive-feedback loop of TGF-β expression; and the reciprocal positive-feedback loop between CSF3R and TGF-β, whereas inhibiting TGF-β inhibits only the TGF-β mediated signaling pathway.

### 2.7. Key Regulator in Signaling Pathways Inducing Fibrosis: CSF3R

The present inventors were the first to reveal that when CSF3R is inhibited, the TGF-β mediated signaling pathway and the non-TGF-β mediated signaling pathway are inhibited, and when CSF3R increases, the TGF-β mediated signaling pathway and the non-TGF-β mediated signaling pathway are activated. That is, it has been revealed for the first time that CSF3R is a key regulator across all signaling pathways that induce fibrosis or fibrogenesis in tissue. Based on the foregoing description, the present inventors infer that CSF3R is an upstream regulator of TGF-β based on experimental data showing that CSF3R is a key regulator in the induction of fibrosis.

For example, referring to Experimental Examples 2.3 to 2.7, when comparing i) the CSF3R expression level decreased by inhibition of only the TGF-β mediated signaling pathway and ii) the CSF3R expression level decreased by inhibition of both the TGF-β mediated signaling pathway and the non-TGF-β mediated signaling pathway, it can be seen that the magnitude of decrease in the latter (ii) is greater.

For example, referring to Experimental Examples 2.3 to 2.7, when comparing i) the expression levels of α-SMA, Col1A1, CSF3, IL11, and CSF3R, etc. decreased by inhibition of only the TGF-β mediated signaling pathway and ii) the expression levels of α-SMA, Col1A1, CSF3, IL11, and CSF3R, etc. decreased by inhibition of both the TGF-β mediated signaling pathway and the non-TGF-β mediated signaling pathway, it can be seen that the magnitude of decrease in the latter (ii) is greater.

For example, referring to Experimental Example 2.5, FIG. 17, and FIG. 18, when the interaction between TGF-β and its TGF-β receptor (TGF-βR) is inhibited, it can be confirmed that the expression levels of fibrosis-related markers are slightly decreased. In contrast, referring to Experimental Example 2.5, Experimental Example 2.6, FIG. 17, FIG. 18, and FIG. 19, when the interaction between CSF3 and its CSF3 receptor (CSF3R) is inhibited, it can be confirmed that the expression levels of fibrosis-related markers are significantly decreased. That is, when the interaction between CSF3 and its CSF3 receptor (CSF3R) is inhibited, the expression levels of fibrosis-related markers are closer to those of the control group (the normal group in which fibrosis was not induced) than when the interaction between TGF-β and its TGF-β receptor is inhibited.

### 2.8. Effect of CSF3R Inhibitor in Restoring Tissue Where Fibrosis Has Occurred to Normal Tissue

The CSF3R inhibitor of the present disclosure restores fibrotic tissue (or cells) to normal tissue (or cells). In another embodiment, the CSF3R inhibitor causes fibrotic tissue (or cells) to return to normal tissue (or cells).

Referring to the experimental examples herein and FIG. 15, FIG. 20, FIG. 26, FIG. 28, FIG. 29, FIG. 41, FIG. 42, FIG. 43, and FIG. 45, etc., it can be seen that fibrotic tissue (or cells) returns to normal tissue (or cells) due to CSF3R inhibition.

On the other hand, methods of inhibiting the TGF-β mediated signaling pathway through commercialized pulmonary fibrosis therapeutics (e.g., nintedanib or pirfenidone) do not have the effect of restoring fibrotic tissue (or cells) to normal tissue (or cells). That is, the fact that these therapeutics only show an alleviating effect on fibrotic symptoms and do not have a therapeutic effect of restoring tissue to normal is widely acknowledged in the field.

However, the CSF3R inhibitor disclosed in the present specification has a therapeutic effect of restoring fibrotic tissue to normal tissue.

The reason why there is a difference in the effects of commercialized pulmonary fibrosis therapeutics and the CSF3R inhibitor may be that, compared to commercialized pulmonary fibrosis therapeutics, the CSF3R inhibitor not only inhibits the TGF-β mediated signaling pathway but also additionally inhibits the non-TGF-β mediated signaling pathway. From another perspective, the reason why there is a difference in the effects of commercialized pulmonary fibrosis therapeutics and the CSF3R inhibitor may be that, compared to commercialized pulmonary fibrosis therapeutics, the CSF3R inhibitor has the effect of more strongly inhibiting the TGF-β mediated signaling pathway.

### 2.9. Examples of CSF3R Inhibitors

The CSF3R inhibitor described in the present disclosure is not particularly limited as long as it can inhibit the function of CSF3R. The CSF3R inhibitor may 1) interfere with the interaction between the CSF3R protein and other molecules, or 2) inhibit the expression of the CSF3R protein to reduce the absolute amount of CSF3R protein interacting with other molecules, thereby consequently interfering with the function of the CSF3R protein. Examples of CSF3R inhibitors capable of such actions include, but are not limited to, nucleic acids that silence the mRNA of the CSF3R gene, a CRISPR/Cas system that knocks out or knocks down the CSF3R gene, compounds that inhibit the CSF3R protein, or aptamers that inhibit the CSF3R protein.

### 2.9.1. Nucleic Acids Silencing mRNA of CSF3R Gene

The CSF3R inhibitor may silence the mRNA of the CSF3R gene to interfere with the translation process of the CSF3R protein. In one embodiment, the CSF3R inhibitor may be a nucleic acid capable of specifically binding to the mRNA transcribed from the CSF3R gene, and may be selected from siRNA (small interfering RNA), shRNA (small hairpin RNA), miRNA (microRNA), and antisense nucleic acids.

### 2.9.2. CRISPR/Cas System Targeting CSF3R Gene 1 - Nuclease

The CSF3R inhibitor may knock out the CSF3R gene in the intracellular genome to prevent the production of CSF3R protein. For example, a CRISPR/Cas system capable of acting as a target-specific nuclease can cleave a specific gene in the cellular genome, and can induce errors in intracellular DNA repair mechanisms (e.g., non-homologous end joining (NHEJ) and/or homology-directed repair (HDR)) to generate an indel within the specific gene, thereby knocking out the specific gene. In one embodiment, the CSF3R inhibitor may be a composition comprising a CRISPR/Cas system comprising: a Cas protein or a nucleic acid encoding same; and a guide RNA or a nucleic acid encoding same, wherein the guide RNA is capable of targeting the CSF3R gene in the cellular genome and is capable of interacting with the Cas protein to form a complex.

### 2.9.3. CRISPR/Cas System Targeting CSF3R Gene 2 - Regulator

The CSF3R inhibitor may inhibit the expression of the CSF3R gene in the intracellular genome, exerting a knockdown effect to suppress the production of the CSF3R protein. In one embodiment, the CSF3R inhibitor may be a composition comprising a CRISPRi system comprising: a fusion protein in which a Cas protein and at least one gene expression regulatory domain are linked, or a nucleic acid encoding the fusion protein; and a guide RNA or a nucleic acid encoding same, wherein the guide RNA is capable of targeting the CSF3R gene in the cellular genome and is capable of interacting with the fusion protein to form a complex. At this time, the gene expression regulatory domain may be a polypeptide or protein that suppresses the expression of the CSF3R gene in the intracellular genome.

### 2.9.4. Substance Inhibiting CSF3R Protein 1 - Compound

In one embodiment, the CSF3R inhibitor may be a compound and/or small molecule that functions to prevent the CSF3R protein from interacting with other molecules.

### 2.9.5. Substance Inhibiting CSF3R Protein 2 - Aptamer

In one embodiment, the CSF3R inhibitor may be a DNA and/or RNA aptamer that functions to prevent the CSF3R protein from interacting with other molecules.

### 2.9.6. Substance Inhibiting CSF3R Protein 3 - Antibody

In one embodiment, the CSF3R inhibitor may be an anti-CSF3R antibody or a fragment of the anti-CSF3R antibody. The anti-CSF3R antibody refers to an antibody (or immunoglobulin) that recognizes the CSF3R protein as an antigen. The anti-CSF3R antibody refers to an antibody having the function of recognizing all or part of the CSF3R protein; and/or the function of binding to all or part of the CSF3R protein. The fragment of the anti-CSF3R antibody is a molecule comprising a portion of the anti-CSF3R antibody and having the function of the anti-CSF3R antibody (the function of recognizing and binding to all or part of the CSF3R protein). That is, the anti-CSF3R antibody or the fragment of the anti-CSF3R antibody can recognize and/or bind to all or part of the CSF3R protein. Accordingly, the anti-CSF3R antibody or the fragment of the anti-CSF3R antibody can bind to the CSF3R protein expressed in fibrotic tissue, thereby preventing the CSF3R protein from interacting with other molecules (e.g., CSF3), and consequently inhibiting the function of the CSF3R protein. The anti-CSF3R antibody or the fragment of the anti-CSF3R antibody is not particularly limited as long as it can inhibit CSF3R.

In one embodiment, the anti-CSF3R antibody may be immunoglobulin G (IgG). At this time, the IgG may be selected from IgG1, IgG2, IgG3, and IgG4.

In one embodiment, the anti-CSF3R antibody may be any one of a human-derived antibody, a humanized antibody, and a chimeric antibody.

In one embodiment, the anti-CSF3R antibody may be any one selected from a monoclonal antibody, a polyclonal antibody, a hybridoma monoclonal antibody, and a recombinant monoclonal antibody.

In one embodiment, the anti-CSF3R antibody may be a human IgG.

In one embodiment, the anti-CSF3R antibody may be a CSF3R neutralizing antibody.

In one embodiment, the fragment of the anti-CSF3R antibody may be a fragment of a CSF3R neutralizing antibody.

In one embodiment, the fragment of the anti-CSF3R antibody may be selected from F(ab), F(ab'), F(ab')2, monospecific F(ab')2, bispecific F(ab')2, scFv, ScFv-Fc, and sdAb.

### 3. Therapeutic Use of CSF3 Inhibitor for Fibrosis

### 3.1. Background Art - Therapeutic Effect of CSF3 Inhibitor on Pulmonary Fibrosis

Fibrosis can occur in various tissues such as the liver and skin as well as the lung, but it is uncertain whether the same therapeutic strategy will be effective because the environment inside the tissue where fibrosis occurred, the environment surrounding the tissue where fibrosis occurred, the environment inside the cells of the tissue where fibrosis occurred, or the environment outside the cells of the tissue where fibrosis occurred is different. Therefore, until confirmed through actual experiments, it is difficult to predict that a CSF3 inhibitor having a therapeutic effect on pulmonary fibrosis will also have a therapeutic effect on fibrosis in other tissues. For example, while Nintedanib and Pirfenidone are used as therapeutic agents for pulmonary fibrosis, they are not used as therapeutic agents for fibrosis in other tissues.

### 3.2. Overview of Therapeutic Use of CSF3 Inhibitor for Fibrosis

In one aspect, the present disclosure discloses a therapeutic use of a CSF3 inhibitor for fibrosis. The CSF3 inhibitor of the present disclosure functions to directly and/or indirectly inhibit or interfere with the proper functioning of CSF3 in vivo. Specifically, the CSF3 inhibitor may inhibit the interaction of the CSF3 protein with other molecules or inhibit the expression of the CSF3 protein. The inventors of the present disclosure have discovered that when the function of CSF3 within a tissue where fibrosis has occurred is inhibited, the fibrosed tissue is restored to normal tissue and fibrosis is treated, and have identified the mechanism of action for treating fibrosis by the CSF3 inhibitor through research.

The CSF3 inhibitor has a therapeutic mechanism of inhibiting the induction of epithelial to mesenchymal transition (EMT) in tissue epithelial cells, inhibiting differentiation into myofibroblasts through inhibition of fibroblast activity, and/or directly acting on myofibroblasts to reduce myofibroblasts. The CSF3 inhibitor can inhibit a TGF-β mediated signaling pathway; a non-TGF-β mediated signaling pathway; a positive-feedback loop of CSF3; and/or a positive-feedback loop of TGF-β.

In one embodiment, the CSF3 inhibitor can inhibit EMT of tissue epithelial cells in which the TGF-β mediated signaling pathway is involved, fibroblast-to-myofibroblast transdifferentiation (FMT) in which the TGF-β mediated signaling pathway is involved, and/or extracellular matrix (ECM) deposition in which the TGF-β mediated signaling pathway is involved.

In one embodiment, the CSF3 inhibitor can inhibit EMT of tissue epithelial cells in which the CSF3-associated and non-TGF-β mediated signaling pathway is involved, FMT in which the non-TGF-β mediated signaling pathway is involved, and/or ECM deposition in which the non-TGF-β mediated signaling pathway is involved.

A detailed description of the inhibition of the TGF-β mediated signaling pathway; the non-TGF-β mediated signaling pathway; the positive-feedback loop of CSF3; and/or the positive-feedback loop of TGF-β by the CSF3 inhibitor is provided in the section <1. Mechanism of Action of CSF3 Inhibitor Effect for Returning Fibrosis-Occurred Tissue to Normal Tissue> and its sub-sections.

When the CSF3 inhibitor is administered to a tissue where fibrosis has occurred, the extracellular matrix accumulated in the tissue where fibrosis has occurred is decomposed and myofibroblasts are reduced, showing a therapeutic effect in which the tissue where fibrosis has occurred returns to normal tissue.

In one embodiment, when the CSF3 inhibitor is administered to a tissue where fibrosis has occurred, 1) abnormally accumulated extracellular matrix, particularly accumulated collagen, is removed to restore the extracellular matrix of the tissue to a normal state, and 2) abnormal myofibroblasts are reduced to restore the tissue to a normal state. In one embodiment, the CSF3 inhibitor treats fibrosis by 1) decomposing the myofibroblast-derived extracellular matrix accumulated in the tissue where fibrosis has occurred, 2) preventing myofibroblasts in the tissue where fibrosis has occurred from secreting extracellular matrix, and 3) reducing myofibroblasts in the tissue where fibrosis has occurred. More specifically, the CSF3 inhibitor may have a therapeutic mechanism of directly acting on myofibroblasts to inactivate them. The inactivation of myofibroblasts may include 1) an extracellular matrix remodeling mechanism, 2) a mechanism for inhibiting extracellular matrix production by myofibroblasts, and 3) a mechanism for reducing myofibroblasts in the fibrosed tissue. The CSF3 inhibitor can inactivate myofibroblasts and/or induce them to be inactivated. The therapeutic effects and therapeutic mechanisms will be described in more detail in the following sections.

### 3.3. Definition of CSF3 Inhibitor

The CSF3 inhibitor of the present disclosure refers to a substance that ultimately inhibits the function of CSF3. As long as the CSF3 inhibitor can inhibit the function of CSF3, its type, form, and composition are not otherwise limited. Specifically, inhibiting the function of CSF3 means 1) binding to the CSF3 protein to inhibit the function of the protein itself, 2) inhibiting the interaction of the CSF3 protein with other molecules, 3) inhibiting the expression of CSF3 to consequently block the opportunity for CSF3 to function, or 4) inhibiting the function of CSF3 through other direct or indirect pathways.

In one embodiment, the CSF3 inhibitor may be an anti-CSF3 antibody. In another embodiment, the CSF3 inhibitor may be a nucleic acid molecule capable of silencing the mRNA of a CSF3 gene or performing RNA interference. Specifically, the nucleic acid molecule may include an antisense oligonucleotide; iRNA (interfering RNA), such as miRNA, siRNA (small interfering RNA), shRNA (short hairpin RNA), and the like. In another embodiment, the CSF3 inhibitor may be a compound that inhibits the function of CSF3.

### 3.4. Therapeutic Mechanism 1 of CSF3 Inhibitor for Fibrosis - Extracellular Matrix Degradation

### 3.4.1. Overview of Extracellular Matrix Degradation

The CSF3 inhibitor can restore a tissue where fibrosis has progressed to a normal tissue. Specifically, the CSF3 inhibitor can decompose or remove the extracellular matrix secreted and accumulated by myofibroblasts in the tissue where fibrosis has occurred (or fibrosed tissue). At this time, the CSF3 inhibitor restores the extracellular matrix environment of the tissue where fibrosis has occurred to the extracellular matrix environment of normal tissue by decomposing or removing the accumulated extracellular matrix. In the present disclosure, this therapeutic mechanism is referred to as "extracellular matrix degradation."

The extracellular matrix accumulated in the tissue where fibrosis has occurred may be collagen, fibronectin, hyaluronan, Versican, Osteopontin (OPN), and the like. The accumulation of the extracellular matrix prevents the tissue from functioning normally and can cause various pathological symptoms. In one embodiment, the extracellular matrix degradation may be decomposing collagen accumulated in the fibrotic tissue.

When the CSF3 inhibitor is administered to a tissue where fibrosis has occurred, it removes the extracellular matrix secreted and accumulated by myofibroblasts through various pathways. In one embodiment, the CSF3 inhibitor can promote the secretion and/or activation of matrix metalloproteinase (MMP) in the tissue. In another embodiment, the CSF3 inhibitor can inhibit the secretion and/or activation of tissue inhibitor of metalloproteinase (TIMP) in the tissue. In another embodiment, the CSF3 inhibitor can inhibit the secretion and/or activation of Hyaluronan Synthase 3 (HAS3) in the tissue.

### 3.4.2. Example 1 of Extracellular Matrix Degradation Pathway - Promotion of MMP Secretion

Matrix metalloproteinase (MMP) is a type of proteinase, also called matrixin, and is an enzyme related to extracellular matrix degradation. Therefore, when MMP is secreted and activated in a tissue, the accumulated extracellular matrix, particularly collagen, is decomposed. In one embodiment, the CSF3 inhibitor can promote the secretion and/or activation of MMP in the tissue where fibrosis has occurred. Specifically, the MMP may be at least one selected from MMP2, MMP9, and MMP13.

### 3.4.3. Example 2 of Extracellular Matrix Degradation Pathway - Inhibition of TIMP Secretion

Tissue inhibitor of metalloproteinase (TIMP), as the name suggests, is a protein that functions to inhibit the activity of MMP. TIMP functions to regulate the activity of the aforementioned MMP, and specifically, functions to inhibit the activity of MMP. Therefore, when the secretion and/or activation of TIMP in a tissue is inhibited, the activity of MMP increases, and thus the accumulated extracellular matrix can be decomposed.

### 3.5. Therapeutic Mechanism 2 of CSF3 Inhibitor for Fibrosis - Inhibition of Extracellular Matrix Production by Myofibroblasts

### 3.5.1. Overview of Inhibition of Extracellular Matrix Secretion

The CSF3 inhibitor can inhibit the production and secretion of extracellular matrix by myofibroblasts in a tissue where fibrosis has occurred (or fibrosed tissue). This prevents the extracellular matrix secreted by the myofibroblasts from further accumulating in the tissue where fibrosis has occurred. Here, the extracellular matrix may be collagen, fibronectin, hyaluronan, Versican, and/or Osteopontin (OPN). In order to prevent the extracellular matrix from further accumulating in the tissue where fibrosis has occurred, the production and secretion of the extracellular matrix by the myofibroblasts must be blocked. When the CSF3 inhibitor is administered to a tissue where fibrosis has occurred, it inhibits the production and secretion of the extracellular matrix by myofibroblasts through various pathways. In one embodiment, the CSF3 inhibitor can inhibit myofibroblasts in the tissue where fibrosis has occurred from expressing and secreting collagen and/or Osteopontin (OPN). In another embodiment, the CSF3 inhibitor can inhibit myofibroblasts in the tissue where fibrosis has occurred from producing hyaluronan synthase (HAS), thereby consequently inhibiting the synthesis and secretion of hyaluronan. This works together with therapeutic mechanism 1 to remove abnormally accumulated extracellular matrix in the tissue where fibrosis has occurred and restore it to a normal state.

### 3.5.2. Example 1 of Extracellular Matrix Secretion Inhibition Pathway - Inhibition of Collagen Expression

The CSF3 inhibitor inhibits the expression of collagen. In the tissue where fibrosis has occurred, one of the most actively secreted extracellular matrices from myofibroblasts is collagen. Therefore, it is as important to block the expression and secretion of collagen by the myofibroblasts as it is to remove the already accumulated collagen. The CSF3 inhibitor can inhibit myofibroblasts from expressing and secreting collagen in the tissue where fibrosis has occurred. Referring to Experimental Example 3, when the CSF3 inhibitor is administered to a tissue and/or cells where fibrosis has occurred, it can be seen that the expression of collagen in myofibroblasts decreases.

### 3.5.3. Example 2 of Extracellular Matrix Secretion Inhibition Pathway - Inhibition of HAS Expression

The CSF3 inhibitor inhibits the expression of hyaluronan synthase. Hyaluronan synthase (HAS) is an enzyme that synthesizes hyaluronan and is involved in synthesizing hyaluronan at the cell plasma membrane. Therefore, when the expression of HAS in cells is inhibited, the synthesis and secretion of hyaluronan are inhibited, thereby inhibiting myofibroblasts from producing hyaluronan. The HAS may be HAS1, HAS2, and/or HAS3.

### 3.5.4. Example 3 of Extracellular Matrix Secretion Inhibition Pathway - Inhibition of OPN Expression

The CSF3 inhibitor inhibits the expression of osteopontin. Osteopontin (OPN) is a major phosphoprotein constituting the skeleton and is involved in bone remodeling. In addition, OPN is known to be associated with the occurrence and progression of tissue fibrosis. The CSF3 inhibitor can inhibit myofibroblasts from expressing and secreting OPN in a tissue where fibrosis has occurred.

### 3.6. Therapeutic Mechanism 3 of CSF3 Inhibitor for Fibrosis - Reduction of Myofibroblasts in Fibrosed Tissue

### 3.6.1. Overview of Reduction of Myofibroblasts in Fibrosed Tissue

The CSF3 inhibitor can reduce myofibroblasts in a tissue where fibrosis has occurred (or fibrosed tissue). The reduction of myofibroblasts may be a result of at least one of 1) apoptosis of myofibroblasts in the tissue where fibrosis has occurred, 2) inhibition of differentiation of epithelial cells and/or fibroblasts into myofibroblasts in the tissue where fibrosis has occurred, and 3) reversion of myofibroblasts in the tissue where fibrosis has occurred to tissue epithelial cells and/or fibroblasts. As a result, the tissue where fibrosis has occurred can be restored to a normal tissue. This effect of reducing myofibroblasts in the fibrosed tissue is well demonstrated in Experimental Example 3, etc.

### 3.6.2. Reduction of Myofibroblasts Due to Apoptosis

As described above, when the CSF3 inhibitor is administered into a tissue where fibrosis has occurred, it inactivates myofibroblasts existing in the fibrosed tissue. As a result, the myofibroblasts themselves undergo apoptosis, resulting in a reduction of myofibroblasts.

### 3.6.3. Inhibition of Epithelial to Mesenchymal Transition in Tissue

The CSF3 inhibitor can also inhibit epithelial to mesenchymal transition (EMT) in which normal epithelial cells in a tissue where fibrosis has occurred de-differentiate into mesenchymal cells. As described above, since normal epithelial cells de-differentiate into mesenchymal cells through EMT and then differentiate into myofibroblasts, if EMT is inhibited, myofibroblasts no longer increase.

### 3.6.4. Inhibition of Differentiation of Fibroblasts into Myofibroblasts in Tissue

The CSF3 inhibitor can also inhibit the differentiation of fibroblasts in a tissue where fibrosis has occurred into myofibroblasts. As described above, since fibroblasts differentiate into myofibroblasts during the progression of fibrosis, if the differentiation of fibroblasts into myofibroblasts is inhibited, myofibroblasts no longer increase.

### 3.6.5. Reversion of Myofibroblasts to Tissue Epithelial Cells

The CSF3 inhibitor can induce or promote the reversion of the myofibroblasts back to epithelial cells. Just as epithelial cells can change into myofibroblasts through EMT, the reverse process of EMT can occur, and myofibroblasts can revert to epithelial cells of normal tissue. This can act in addition to the reduction of myofibroblasts due to apoptosis and the inhibition of de-differentiation of epithelial cells into myofibroblasts, and the results of Experimental Example 3 are indirect evidence that the myofibroblasts revert to tissue epithelial cells.

### 3.6.6. Reversion of Myofibroblasts to Fibroblasts

The CSF3 inhibitor can induce or promote the reversion of the myofibroblasts back to fibroblasts. Just as fibroblasts can differentiate into myofibroblasts, the reverse process of differentiation can occur, and myofibroblasts can revert to fibroblasts. This can act in addition to the reduction of myofibroblasts due to apoptosis and the inhibition of differentiation of fibroblasts into myofibroblasts.

### 3.6.7. Biomarkers Related to Inhibition of EMT, or Reversion of Myofibroblasts to Tissue Epithelial Cells and/or Fibroblasts

Biomarkers related to EMT or reversion of myofibroblasts to tissue epithelial cells include E-cadherin, Snail1, Snail2, Zeb1, Zeb2, Vimentin, N-cadherin, Fibronectin, and the like. Specifically, when EMT occurs, the expression level of E-cadherin may decrease, and/or the expression level of at least one marker selected from Snail1, Snail2, Zeb1, Zeb2, Vimentin, N-cadherin, and Fibronectin may increase. Conversely, when the expression level of E-cadherin increases, and/or the expression level of at least one marker selected from Snail1, Snail2, Zeb1, Zeb2, Vimentin, N-cadherin, and Fibronectin decreases, it can be interpreted as 1) EMT being inhibited so that differentiation into myofibroblasts no longer occurs, or 2) myofibroblasts reverting to tissue epithelial cells.

### 3.7. Advantages of Fibrosis Therapeutic Agent Using CSF3 Inhibitor 1 - Therapeutic Effect on Fibrosis

When a therapeutic agent for fibrosis comprising the CSF3 inhibitor is administered to a tissue where fibrosis has occurred, it has the effect of 1) decomposing the accumulated myofibroblast-derived extracellular matrix, particularly collagen, and 2) reducing myofibroblasts in the tissue to restore it to a normal tissue. This means that the therapeutic agent for fibrosis comprising the CSF3 inhibitor has a direct and complete therapeutic effect on fibrosis.

The inventors of the present disclosure have confirmed through experiments that the extracellular matrix accumulated in the tissue where fibrosis occurred is decomposed and the fibrosis-occurred tissue is restored to a normal tissue. Compared to the fact that conventional therapeutic agents for fibrosis have only an incomplete therapeutic effect on fibrosis, the therapeutic agent for fibrosis comprising the CSF3 inhibitor has a complete therapeutic effect compared to conventional therapeutic agents for fibrosis, and thus can be a superior therapeutic agent.

The reason why the therapeutic agent for fibrosis comprising the CSF3 inhibitor has a complete therapeutic effect compared to conventional therapeutic agents for fibrosis may be that the level of inhibition of the TGF-β mediated signaling pathway by treatment with the CSF3 inhibitor is superior to that of conventional therapeutic agents for fibrosis. Another reason may be that while conventional therapeutic agents for fibrosis only inhibit the TGF-β mediated signaling pathway, the therapeutic agent for fibrosis comprising the CSF3 inhibitor can inhibit the non-TGF-β mediated signaling pathway, the positive-feedback loop of CSF3, and the positive-feedback loop of TGF-β in addition to the TGF-β mediated signaling pathway.

### 3.8. Advantages of Fibrosis Therapeutic Agent Using CSF3 Inhibitor 2 - Therapeutic Effect Independent of the Type of Tissue where Fibrosis Occurred

The present inventors have revealed that CSF3 functions as a key regulator in the pathogenesis of pulmonary fibrosis. Accordingly, a therapeutic agent for pulmonary fibrosis comprising a CSF3 inhibitor has a more fundamental mechanism of action than conventional therapeutic agents for pulmonary fibrosis and exhibits a true therapeutic effect. Furthermore, the present inventors have revealed through experiments that CSF3 is also crucially involved in the fibrosis of skin, liver, and kidney cells. These findings allow for a reasonable expectation that CSF3 is a key regulator (or upstream regulator) involved in tissue fibrosis regardless of the tissue type. Furthermore, it serves as a basis for predicting that a CSF3 inhibitor can have a therapeutic effect on fibrosis regardless of the tissue type.

Specifically, the inventors of the present disclosure have identified the pathogenesis and/or therapeutic mechanism of fibrosis through Experimental Example 2 and confirmed through Experimental Example 3 that the CSF3 inhibitor actually has a therapeutic effect on fibrosis occurring in various tissues. For example, it was confirmed through Experimental Examples 3.1 and 3.2 that the CSF3 inhibitor has a therapeutic effect on pulmonary fibrosis. For example, it was confirmed through Experimental Examples 3.3 and 3.4 that the CSF3 inhibitor has a therapeutic effect on skin fibrosis. For example, it was confirmed through Experimental Example 3.5 that the CSF3 inhibitor has a therapeutic effect on liver fibrosis. For example, it was confirmed through Experimental Example 3.6 that the CSF3 inhibitor has a therapeutic effect on renal fibrosis.

Therefore, the therapeutic agent for fibrosis comprising the CSF3 inhibitor disclosed in the present specification can function as an effective therapeutic agent regardless of the type of tissue where fibrosis has occurred.

In one embodiment, the tissue where fibrosis occurred may be any one tissue selected from skin; brain; nervous system; heart; bone marrow; liver; gut; joint; eye; lung; kidney; retroperitoneum; mediastinum; and pancreas.

In one embodiment, the CSF3 inhibitor has a therapeutic effect on skin fibrosis.

In one embodiment, the CSF3 inhibitor has a therapeutic effect on brain fibrosis.

In one embodiment, the CSF3 inhibitor has a therapeutic effect on nervous system fibrosis.

In one embodiment, the CSF3 inhibitor has a therapeutic effect on cardiac fibrosis.

In one embodiment, the CSF3 inhibitor has a therapeutic effect on bone marrow fibrosis.

In one embodiment, the CSF3 inhibitor has a therapeutic effect on liver fibrosis.

In one embodiment, the CSF3 inhibitor has a therapeutic effect on intestinal fibrosis.

In one embodiment, the CSF3 inhibitor has a therapeutic effect on joint fibrosis.

In one embodiment, the CSF3 inhibitor has a therapeutic effect on fibrosis.

In one embodiment, the CSF3 inhibitor has a therapeutic effect on ocular fibrosis.

In one embodiment, the CSF3 inhibitor has a therapeutic effect on pulmonary fibrosis.

In one embodiment, the CSF3 inhibitor has a therapeutic effect on renal fibrosis.

In one embodiment, the CSF3 inhibitor has a therapeutic effect on retroperitoneal fibrosis.

In one embodiment, the CSF3 inhibitor has a therapeutic effect on mediastinal fibrosis.

In one embodiment, the CSF3 inhibitor has a therapeutic effect on pancreatic fibrosis.

### 3.9. Pharmaceutical Composition for Treating Fibrosis Comprising CSF3 Inhibitor

### 3.9.1. Overview of Pharmaceutical Composition for Treating Fibrosis Comprising CSF3 Inhibitor

The present disclosure discloses a pharmaceutical composition for treating fibrosis comprising a CSF3 inhibitor. The pharmaceutical composition for treating fibrosis comprises a therapeutically effective amount of a CSF3 inhibitor. In one embodiment, the pharmaceutical composition for treating fibrosis may comprise a therapeutically effective amount of a CSF3 inhibitor and a pharmaceutically acceptable carrier. In another embodiment, the pharmaceutical composition for treating fibrosis may comprise a therapeutically effective amount of a CSF3 inhibitor and an adjuvant. In another embodiment, the pharmaceutical composition for treating fibrosis may comprise a therapeutically effective amount of a CSF3 inhibitor; a pharmaceutically acceptable carrier; and an adjuvant. Here, the pharmaceutically acceptable carrier may be for appropriate formulation of the pharmaceutical composition for treating fibrosis.

### 3.9.2. Target Disease - Fibrosis

The pharmaceutical composition for treating fibrosis is for treating fibrosis, or fibrosis-related diseases, disorders, and/or symptoms. The fibrosis or fibrosis-related diseases, disorders, and/or symptoms include all those recognizable by those of ordinary skill in the art.

In one embodiment, the fibrosis or fibrosis-related diseases, disorders, and/or symptoms may be those described in the section <3.14. Examples of Fibrosis Diseases>.

In one embodiment, the tissue where fibrosis occurred may be those described in the section <3.13. Examples of Tissues where Fibrosis Occurs>.

### 3.9.3. Examples of Formulation of Pharmaceutical Composition for Treating Fibrosis

In one embodiment, the pharmaceutical composition for treating fibrosis comprising the CSF3 inhibitor may be formulated into troches, lozenges, tablets, aqueous suspensions, oily suspensions, prepared powders, granules, emulsions, hard capsules, soft capsules, syrups, or elixirs. In another embodiment, the pharmaceutical composition for treating fibrosis comprising the CSF3 inhibitor may be formulated into injection solutions, suppositories, powders for respiratory inhalation, aerosolized sprays, ointments, powders for application, oils, and creams. In another embodiment, the pharmaceutical composition for treating fibrosis comprising the CSF3 inhibitor may be formulated as an injection solution. Specifically, it may be formulated as an injection solution by mixing a therapeutically effective amount of the CSF3 inhibitor with a stabilizer or a buffer in water to prepare a solution or suspension, which is then formulated into a unit dose of an ampoule or vial. In another embodiment, the pharmaceutical composition for treating fibrosis comprising the CSF3 inhibitor may be formulated as an aerosol by mixing a propellant or the like with an additive to prepare an aqueous concentrated product or a wet powder. In another embodiment, when formulating the pharmaceutical composition for treating fibrosis comprising the CSF3 inhibitor for transdermal use, ointments, creams, powders for application, oils, external preparations for skin, etc. may be prepared by adding animal oils, vegetable oils, waxes, paraffin, starch, tragacanth, cellulose derivatives, polyethylene glycol, silicone, bentonite, silica, talc, zinc oxide, etc. as a carrier to a therapeutically effective amount of the CSF3 inhibitor.

### 3.9.4. Examples of Pharmaceutically Acceptable Carriers

Pharmaceutically acceptable carriers are those commonly used in formulation and include, but are not limited to, saline, sterile water, Ringer's solution, buffered saline, cyclodextrin, dextrose solution, maltodextrin solution, glycerol, ethanol, liposomes, and the like, and may further include other conventional additives such as antioxidants and buffers as needed. In addition, diluents, dispersants, surfactants, binders, lubricants, etc., may be additionally added to formulate injectable formulations such as aqueous solutions, suspensions, and emulsions, or pills, capsules, granules, or tablets. For suitable pharmaceutically acceptable carriers and formulations, the methods disclosed in Remington's Pharmaceutical Sciences (19th edition, 1995) can be preferably used according to each component. In one embodiment, the pharmaceutical composition for treating fibrosis comprising the CSF3 inhibitor may include, as a pharmaceutically acceptable carrier, binders such as lactose, saccharose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol wax; sweeteners; flavorings; syrups; liquid carriers such as fatty oils; sterile aqueous solutions; propylene glycol; polyethylene glycol; injectable esters such as ethyl oleate; suspensions; emulsions; lyophilized preparations; external preparations; stabilizers; buffers; animal oils; vegetable oils; waxes; paraffin; starch; tragacanth; cellulose derivatives; polyethylene glycol; silicone; bentonite; silica; talc; zinc oxide; or appropriate combinations thereof.

### 3.10. Method for Treating Fibrosis Using CSF3 Inhibitor

### 3.10.1. Overview of Method for Treating Fibrosis Using CSF3 Inhibitor

The present disclosure discloses a method for treating fibrosis using the CSF3 inhibitor. The method for treating fibrosis comprises administering a therapeutically effective amount of a CSF3 inhibitor to a subject by an appropriate administration method, an appropriate administration regimen, and an appropriate dose. In one embodiment, the method for treating fibrosis comprises administering a suitably formulated therapeutically effective CSF3 inhibitor, that is, the pharmaceutical composition for treating fibrosis, to a subject by an appropriate administration method, an appropriate administration regimen, and an appropriate dose. In one embodiment, the subject of the method for treating fibrosis may be a human or a non-human animal. In one embodiment, the subject of the method for treating fibrosis may be a subject having fibrosis, or a fibrosis-related disease, disorder, and/or symptom.

### 3.10.2. Target Disease - Fibrosis

The method for treating fibrosis is for treating fibrosis, or fibrosis-related diseases, disorders, and/or symptoms. The fibrosis or fibrosis-related diseases, disorders, and/or symptoms include all those recognizable by those of ordinary skill in the art.

In one embodiment, the fibrosis or fibrosis-related diseases, disorders, and/or symptoms may be those described in the section <3.14. Examples of Fibrosis Diseases>.

In one embodiment, the tissue where fibrosis occurred may be those described in the section <3.13. Examples of Tissues where Fibrosis Occurs>.

### 3.10.3. Examples of Administration Methods

The method for treating fibrosis may be administering a suitably formulated therapeutically effective CSF3 inhibitor to a subject by an appropriate administration method. In one embodiment, the method for treating fibrosis may involve oral administration or parenteral administration of a suitably formulated therapeutically effective CSF3 inhibitor. The parenteral administration method may include intravenous administration, intraperitoneal administration, intramuscular administration, transdermal administration, subcutaneous administration, and the like.

Various criteria related to administration (dose, cycle, frequency, etc.) can be adjusted by a physician according to the individual patient's needs following clinical examinations during the administration process.

### 3.10.4. Examples of Dosage

The method for treating fibrosis may be administering a suitably formulated therapeutically effective CSF3 inhibitor to a subject in an appropriate dose. In one embodiment, the dose may be about 0.01 mg to 1000 mg per 1 kg of the subject's body weight, based on the CSF3 inhibitor.

### 3.10.5. Examples of Administration Cycle

The method for treating fibrosis may be administering a suitably formulated therapeutically effective CSF3 inhibitor to a subject at an appropriate administration cycle. In one embodiment, it may be administering the pharmaceutical composition for treating fibrosis comprising an appropriate dose of the CSF3 inhibitor once a day. As another example, the administration cycle may be administering the pharmaceutical composition for treating fibrosis comprising an appropriate dose of the CSF3 inhibitor in two or more divided doses a day. As another example, the administration cycle may be administering the pharmaceutical composition for treating fibrosis comprising an appropriate dose of the CSF3 inhibitor at intervals of 1 hour, 2 hours, 6 hours, 12 hours, 24 hours, 2 days, 3 days, 1 week, 2 weeks, 1 month, and/or 3 months.

### 3.11. Therapeutic Use of CSF3 Inhibitor

The present disclosure discloses a therapeutic use of the CSF3 inhibitor for fibrosis. In one embodiment, the present disclosure discloses the CSF3 inhibitor for treating fibrosis, or fibrosis-related diseases, disorders, and/or symptoms.

In one embodiment, the fibrosis or fibrosis-related diseases, disorders, and/or symptoms may be those described in the section <3.14. Examples of Fibrosis Diseases>.

In one embodiment, the tissue where fibrosis occurred may be those described in the section <3.13. Examples of Tissues where Fibrosis Occurs>.

### 3.12. Use of CSF3 Inhibitor for Manufacturing a Therapeutic Agent

The present disclosure discloses a use of the CSF3 inhibitor for manufacturing a therapeutic agent for fibrosis. In one embodiment, the present disclosure discloses a use of the CSF3 inhibitor for manufacturing a medicament for treating fibrosis, or fibrosis-related diseases, disorders, and/or symptoms.

In one embodiment, the fibrosis or fibrosis-related diseases, disorders, and/or symptoms may be those described in the section <3.14. Examples of Fibrosis Diseases>.

In one embodiment, the tissue where fibrosis occurred may be those described in the section <3.13. Examples of Tissues where Fibrosis Occurs>.

### 3.13. Examples of Tissues where Fibrosis Occurs

Fibrosis can occur in almost all tissues in the body, and it is known that many tissues in the body can be fibrosed, causing various diseases, disorders, and/or conditions. For example, it has been reported that fibrosis can occur in tissues such as skin, brain, nervous system, heart, bone marrow, liver, gut, joint, eye, lung, kidney, retroperitoneum, mediastinum, and/or pancreas.

### 3.14. Examples of Fibrosis Diseases

The fibrosis or fibrosis-related diseases, disorders, and/or symptoms described in the present disclosure include all those recognizable by those of ordinary skill in the art. In one embodiment, the fibrosis or fibrosis-related diseases, disorders, and/or symptoms may be selected from: hypertrophic scar; systemic sclerosis; multiple cancers; pulmonary arterial hypertension; glial scar; Alzheimer's disease; cardiac fibrosis; hypertrophic cardiomyopathy; cardiac dysfunction; valvular disease; arrhythmia; myelofibrosis; myelodysplastic syndrome; chronic myelogenous leukemia; cirrhosis; portal hypertension; hepatocellular carcinoma; nonalcoholic steatohepatitis (NASH); intestinal fibrosis; enteropathies; inflammatory bowel disease; arthrofibrosis; subretinal fibrosis; epiretinal fibrosis; vision loss; idiopathic pulmonary fibrosis; cystic fibrosis; pulmonary hypertension; thromboembolic disease; emphysema; renal fibrosis; cystic fibrosis; nephrogenic systemic fibrosis; chronic kidney disease; renal anemia; retroperitoneal fibrosis; mediastinal fibrosis; pancreatic fibrosis; cystic fibrosis; chronic pancreatitis; duct obstruction; autoimmune-interstitial lung disease such as ankylosing spondylitis and rheumatoid arthritis; connective tissue disease-interstitial lung disease such as rheumatoid arthritis-interstitial lung disease; and non-idiopathic pulmonary fibrosis-interstitial lung disease.

### 3.15. Examples of CSF3 Inhibitors

The CSF3 inhibitor described in the present disclosure is not particularly limited as long as it can inhibit the function of CSF3. As described above, the CSF3 inhibitor may 1) interfere with the interaction between the CSF3 protein and other molecules, or 2) inhibit the expression of the CSF3 protein to reduce the absolute amount of CSF3 protein interacting with other molecules, thereby consequently interfering with the function of the CSF3 protein. Examples of CSF3 inhibitors capable of such actions include, but are not limited to, nucleic acids that silence the mRNA of the CSF3 gene, a CRISPR/Cas system that knocks out or knocks down the CSF3 gene, compounds that inhibit the CSF3 protein, or aptamers that inhibit the CSF3 protein.

In one embodiment, the CSF3 inhibitor may be those described in the section <1.9. Examples of CSF3 Inhibitors> and its sub-sections <1.9.1. Nucleic Acids Silencing mRNA of CSF3 Gene> to <1.9.6. Substance Inhibiting CSF3 Protein 3 - Antibody>.

In one embodiment, the anti-CSF3 antibody may be as described in the section <5. Anti-CSF3 Antibody>.

### 4. Therapeutic Use of CSF3R Inhibitor for Fibrosis

### 4.1. Background Art - Therapeutic Effect of CSF3R Inhibitor on Pulmonary Fibrosis

Fibrosis can occur in various tissues such as the liver and skin as well as the lung, but it is uncertain whether the same therapeutic strategy will be effective because the environment inside the tissue where fibrosis occurred, the environment surrounding the tissue where fibrosis occurred, the environment inside the cells of the tissue where fibrosis occurred, or the environment outside the cells of the tissue where fibrosis occurred is different. Therefore, until confirmed through actual experiments, it is difficult to predict that a CSF3R inhibitor having a therapeutic effect on pulmonary fibrosis will also have a therapeutic effect on fibrosis in other tissues. For example, while Nintedanib and Pirfenidone are used as therapeutic agents for pulmonary fibrosis, they are not used as therapeutic agents for fibrosis in other tissues.

### 4.2. Overview of Therapeutic Use of CSF3R Inhibitor for Fibrosis

In one aspect, the present disclosure discloses a therapeutic use of a CSF3R inhibitor for fibrosis. The CSF3R inhibitor of the present disclosure functions to directly and/or indirectly inhibit or interfere with the proper functioning of CSF3R in vivo. Specifically, the CSF3R inhibitor may inhibit the interaction of the CSF3R protein with other molecules or inhibit the expression of the CSF3R protein.

The inventors of the present disclosure have previously confirmed that treating pulmonary fibrosis-affected tissue with a CSF3R inhibitor has the effect of returning it to normal tissue, and confirmed that this effect is the same as or similar to the effect that appears when pulmonary fibrosis-affected tissue is treated with a CSF3 inhibitor. It is a known fact that CSF3 and CSF3R interact with each other, and as the present inventors have discovered, since the effects found when CSF3 is inhibited in pulmonary fibrosis are also found when CSF3R is inhibited, the CSF3R inhibitor has a therapeutic effect on fibrosis in all tissues, just as the CSF3 inhibitor does. Accordingly, the inventors of the present disclosure conducted further research on CSF3R and discovered that when the function of CSF3R in fibrotic tissue is inhibited, the fibrosed tissue is restored to normal tissue and fibrosis is treated, and identified the mechanism of action by which the CSF3R inhibitor treats fibrosis.

The CSF3R inhibitor has a therapeutic mechanism of inhibiting the induction of epithelial to mesenchymal transition (EMT) in tissue epithelial cells, inhibiting differentiation into myofibroblasts through inhibition of fibroblast activity, and/or directly acting on myofibroblasts to reduce myofibroblasts. The CSF3R inhibitor can inhibit a TGF-β mediated signaling pathway; a non-TGF-β mediated signaling pathway; a positive-feedback loop of CSF3; and/or a positive-feedback loop of TGF-β. In one embodiment, the CSF3R inhibitor can inhibit EMT of tissue epithelial cells in which the TGF-β mediated signaling pathway is involved, fibroblast-to-myofibroblast transdifferentiation (FMT) in which the TGF-β mediated signaling pathway is involved, and/or extracellular matrix (ECM) deposition in which the TGF-β mediated signaling pathway is involved. In one embodiment, the CSF3R inhibitor can inhibit EMT of tissue epithelial cells in which the non-TGF-β mediated signaling pathway is involved, FMT in which the non-TGF-β mediated signaling pathway is involved, and/or ECM deposition in which the non-TGF-β mediated signaling pathway is involved. A detailed description of the inhibition of the TGF-β mediated signaling pathway; the non-TGF-β mediated signaling pathway; the positive-feedback loop of CSF3R; and/or the positive-feedback loop of TGF-β by the CSF3R inhibitor is provided in the section <2. Mechanism of Action of CSF3R Inhibitor Effect for Returning Fibrosis-Occurred Tissue to Normal Tissue> and its sub-sections.

When the CSF3R inhibitor is administered to a tissue where fibrosis has occurred, the extracellular matrix accumulated in the tissue where fibrosis has occurred is decomposed and myofibroblasts are reduced, showing a therapeutic effect in which the tissue where fibrosis has occurred returns to normal tissue. In one embodiment, when the CSF3R inhibitor is administered to a tissue where fibrosis has occurred, 1) abnormally accumulated extracellular matrix, particularly accumulated collagen, is removed to restore the extracellular matrix of the tissue to a normal state, and 2) abnormal myofibroblasts are reduced to restore the tissue to a normal state. In one embodiment, the CSF3R inhibitor treats fibrosis by 1) decomposing the myofibroblast-derived extracellular matrix accumulated in the tissue where fibrosis has occurred, 2) preventing myofibroblasts in the tissue where fibrosis has occurred from secreting extracellular matrix, and 3) reducing myofibroblasts in the tissue where fibrosis has occurred. More specifically, the CSF3R inhibitor may have a therapeutic mechanism of directly acting on myofibroblasts to inactivate them. The inactivation of myofibroblasts may include 1) an extracellular matrix remodeling mechanism, 2) a mechanism for inhibiting extracellular matrix production by myofibroblasts, and 3) a mechanism for reducing myofibroblasts in the fibrosed tissue. The CSF3R inhibitor can inactivate myofibroblasts and/or induce them to be inactivated. The therapeutic effects and therapeutic mechanisms will be described in more detail in the following sections.

### 4.3. Definition of CSF3R Inhibitor

The CSF3R inhibitor of the present disclosure refers to a substance that ultimately inhibits the function of CSF3R. As long as the CSF3R inhibitor can inhibit the function of CSF3R, its type, form, and composition are not otherwise limited. Specifically, inhibiting the function of CSF3R means 1) binding to the CSF3R protein to inhibit the function of the protein itself, 2) inhibiting the interaction of the CSF3R protein with other molecules, 3) inhibiting the expression of CSF3R to consequently block the opportunity for CSF3R to function, or 4) inhibiting the function of CSF3R through other direct or indirect pathways.

In one embodiment, the CSF3R inhibitor may be an anti-CSF3R antibody. In another embodiment, the CSF3R inhibitor may be a nucleic acid molecule capable of silencing the mRNA of the CSF3R gene or performing RNA interference. Specifically, the nucleic acid molecule may include an antisense oligonucleotide; iRNA (interfering RNA), such as miRNA, siRNA (small interfering RNA), shRNA (short hairpin RNA), and the like. In another embodiment, the CSF3R inhibitor may be a compound that inhibits the function of CSF3R.

### 4.4. Therapeutic Mechanism 1 of CSF3R Inhibitor for Fibrosis - Extracellular Matrix Degradation

### 4.4.1. Overview of Extracellular Matrix Degradation

The CSF3R inhibitor can restore a tissue where fibrosis has progressed to a normal tissue. Specifically, the CSF3R inhibitor can decompose or remove the extracellular matrix secreted and accumulated by myofibroblasts in the tissue where fibrosis has occurred (or fibrosed tissue). At this time, the CSF3R inhibitor can restore the extracellular matrix environment of the tissue where fibrosis has occurred to the extracellular matrix environment of normal tissue by decomposing or removing the accumulated extracellular matrix. In the present disclosure, this therapeutic mechanism is referred to as "extracellular matrix degradation."

The extracellular matrix accumulated in the tissue where fibrosis has occurred may be collagen, fibronectin, hyaluronan, Versican, Osteopontin (OPN), and the like. The accumulation of the extracellular matrix prevents the tissue from functioning normally and can cause various pathological symptoms. In one embodiment, the extracellular matrix degradation may be decomposing collagen accumulated in the fibrotic tissue.

When the CSF3R inhibitor is administered to a tissue where fibrosis has occurred, it removes the extracellular matrix secreted and accumulated by myofibroblasts through various pathways. In one embodiment, the CSF3R inhibitor can promote the secretion and/or activation of matrix metalloproteinase (MMP) in the tissue. In another embodiment, the CSF3R inhibitor can inhibit the secretion and/or activation of tissue inhibitor of metalloproteinase (TIMP) in the tissue. In another embodiment, the CSF3R inhibitor can inhibit the secretion and/or activation of Hyaluronan Synthase 3 (HAS3) in the tissue.

### 4.4.2. Example 1 of Extracellular Matrix Degradation Pathway - Promotion of MMP Secretion

Matrix metalloproteinase (MMP) is a type of proteinase, also called matrixin, and is an enzyme related to extracellular matrix degradation. Therefore, when MMP is secreted and activated in a tissue, the accumulated extracellular matrix, particularly collagen, is decomposed. In one embodiment, the CSF3R inhibitor can promote the secretion and/or activation of MMP in the tissue where fibrosis has occurred. Specifically, the MMP may be at least one selected from MMP2, MMP9, and MMP13.

### 4.4.3. Example 2 of Extracellular Matrix Degradation Pathway - Inhibition of TIMP Secretion

Tissue inhibitor of metalloproteinase (TIMP), as the name suggests, is a protein that functions to inhibit the activity of MMP. TIMP functions to regulate the activity of the aforementioned MMP, and specifically, functions to inhibit the activity of MMP. Therefore, when the secretion and/or activation of TIMP in a tissue is inhibited, the activity of MMP increases, and thus the accumulated extracellular matrix can be decomposed.

### 4.5. Therapeutic Mechanism 2 of CSF3R Inhibitor for Fibrosis - Inhibition of Extracellular Matrix Production by Myofibroblasts

### 4.5.1. Overview of Inhibition of Extracellular Matrix Secretion

The CSF3R inhibitor can inhibit the production and secretion of extracellular matrix by myofibroblasts in a tissue where fibrosis has occurred (or fibrosed tissue). This prevents the extracellular matrix secreted by the myofibroblasts from further accumulating in the tissue where fibrosis has occurred. Here, the extracellular matrix may be collagen, fibronectin, hyaluronan, Versican, and/or Osteopontin (OPN). In order to prevent the extracellular matrix from further accumulating in the tissue where fibrosis has occurred, the production and secretion of the extracellular matrix by the myofibroblasts must be blocked. When the CSF3R inhibitor is administered to a tissue where fibrosis has occurred, it inhibits the production and secretion of the extracellular matrix by myofibroblasts through various pathways. In one embodiment, the CSF3R inhibitor can inhibit myofibroblasts in the tissue where fibrosis has occurred from expressing and secreting collagen and/or Osteopontin (OPN). In another embodiment, the CSF3R inhibitor can inhibit myofibroblasts in the tissue where fibrosis has occurred from producing hyaluronan synthase (HAS), thereby consequently inhibiting the synthesis and secretion of hyaluronan. This works together with therapeutic mechanism 1 to remove abnormally accumulated extracellular matrix in the tissue where fibrosis has occurred and restore it to a normal state.

### 4.5.2. Example 1 of Extracellular Matrix Secretion Inhibition Pathway - Inhibition of Collagen Expression

The CSF3R inhibitor inhibits the expression of collagen. In the tissue where fibrosis has occurred, one of the most actively secreted extracellular matrices from myofibroblasts is collagen. Therefore, it is as important to block the expression and secretion of collagen by the myofibroblasts as it is to remove the already accumulated collagen. The CSF3R inhibitor can inhibit myofibroblasts from expressing and secreting collagen in the tissue where fibrosis has occurred. Referring to Experimental Example 3, when the CSF3R inhibitor is administered to a tissue and/or cells where fibrosis has occurred, it can be seen that the expression of collagen in myofibroblasts decreases.

### 4.5.3. Example 2 of Extracellular Matrix Secretion Inhibition Pathway - Inhibition of HAS Expression

The CSF3R inhibitor inhibits the expression of hyaluronan synthase. Hyaluronan synthase (HAS) is an enzyme that synthesizes hyaluronan and is involved in synthesizing hyaluronan at the cell plasma membrane. Therefore, when the expression of HAS in cells is inhibited, the synthesis and secretion of hyaluronan are inhibited, thereby inhibiting myofibroblasts from producing hyaluronan. The HAS may be HAS1, HAS2, and/or HAS3.

### 4.5.4. Example 3 of Extracellular Matrix Secretion Inhibition Pathway - Inhibition of OPN Expression

The CSF3R inhibitor inhibits the expression of osteopontin. Osteopontin (OPN) is a major phosphoprotein constituting the skeleton and is involved in bone remodeling. In addition, OPN is known to be associated with the occurrence and progression of tissue fibrosis. The CSF3R inhibitor can inhibit myofibroblasts from expressing and secreting OPN in a tissue where fibrosis has occurred.

### 4.6. Therapeutic Mechanism 3 of CSF3R Inhibitor for Fibrosis - Reduction of Myofibroblasts in Fibrosed Tissue

### 4.6.1. Overview of Reduction of Myofibroblasts in Fibrosed Tissue

The CSF3R inhibitor can reduce myofibroblasts in a tissue where fibrosis has occurred (or fibrosed tissue). The reduction of myofibroblasts may be a result of at least one of 1) apoptosis of myofibroblasts in the tissue where fibrosis has occurred, 2) inhibition of differentiation of epithelial cells and/or fibroblasts into myofibroblasts in the tissue where fibrosis has occurred, and 3) reversion of myofibroblasts in the tissue where fibrosis has occurred to tissue epithelial cells and/or fibroblasts. As a result, the tissue where fibrosis has occurred can be restored to a normal tissue. This effect of reducing myofibroblasts in the fibrosed tissue is well demonstrated in Experimental Example 3, etc.

### 4.6.2. Reduction of Myofibroblasts Due to Apoptosis

As described above, when the CSF3R inhibitor is administered into a tissue where fibrosis has occurred, it inactivates myofibroblasts existing in the fibrosed tissue. As a result, the myofibroblasts themselves undergo apoptosis, resulting in a reduction of myofibroblasts.

### 4.6.3. Inhibition of Epithelial to Mesenchymal Transition in Tissue

The CSF3R inhibitor can also inhibit epithelial to mesenchymal transition (EMT) in which normal epithelial cells in a tissue where fibrosis has occurred de-differentiate into mesenchymal cells. As described above, since normal epithelial cells de-differentiate into mesenchymal cells through EMT and then differentiate into myofibroblasts, if EMT is inhibited, myofibroblasts no longer increase.

### 4.6.4. Inhibition of Differentiation of Fibroblasts into Myofibroblasts in Tissue

The CSF3R inhibitor can also inhibit the differentiation of fibroblasts in a tissue where fibrosis has occurred into myofibroblasts. As described above, since fibroblasts differentiate into myofibroblasts during the progression of fibrosis, if the differentiation of fibroblasts into myofibroblasts is inhibited, myofibroblasts no longer increase.

### 4.6.5. Reversion of Myofibroblasts to Tissue Epithelial Cells

The CSF3R inhibitor can induce or promote the reversion of the myofibroblasts back to epithelial cells. Just as epithelial cells can change into myofibroblasts through EMT, the reverse process of EMT can occur, and myofibroblasts can revert to epithelial cells of normal tissue. This can act in addition to the reduction of myofibroblasts due to apoptosis and the inhibition of de-differentiation of epithelial cells into myofibroblasts, and the results of Experimental Example 3 are indirect evidence that the myofibroblasts revert to tissue epithelial cells.

### 4.6.6. Reversion of Myofibroblasts to Fibroblasts

The CSF3R inhibitor can induce or promote the reversion of the myofibroblasts back to fibroblasts. Just as fibroblasts can differentiate into myofibroblasts, the reverse process of differentiation can occur, and myofibroblasts can revert to fibroblasts. This can act in addition to the reduction of myofibroblasts due to apoptosis and the inhibition of differentiation of fibroblasts into myofibroblasts.

### 4.6.7. Biomarkers Related to Inhibition of EMT, or Reversion of Myofibroblasts to Tissue Epithelial Cells and/or Fibroblasts

Biomarkers related to EMT or reversion of myofibroblasts to tissue epithelial cells include E-cadherin, Snail1, Snail2, Zeb1, Zeb2, Vimentin, N-cadherin, Fibronectin, and the like. Specifically, when EMT occurs, the expression level of E-cadherin may decrease, and/or the expression level of at least one marker selected from Snail1, Snail2, Zeb1, Zeb2, Vimentin, N-cadherin, and Fibronectin may increase. Conversely, when the expression level of E-cadherin increases, and/or the expression level of at least one marker selected from Snail1, Snail2, Zeb1, Zeb2, Vimentin, N-cadherin, and Fibronectin decreases, it can be interpreted as 1) EMT being inhibited so that differentiation into myofibroblasts no longer occurs, or 2) myofibroblasts reverting to tissue epithelial cells.

### 4.7. Advantages of Fibrosis Therapeutic Agent Using CSF3R Inhibitor 1 - Therapeutic Effect on Fibrosis

When a therapeutic agent for fibrosis comprising the CSF3R inhibitor is administered to a tissue where fibrosis has occurred, it has the effect of 1) decomposing the accumulated myofibroblast-derived extracellular matrix, particularly collagen, and 2) reducing myofibroblasts in the tissue to restore it to a normal tissue. This means that the therapeutic agent for fibrosis comprising the CSF3R inhibitor has a direct and complete therapeutic effect on fibrosis. The inventors of the present disclosure have confirmed through experiments that the extracellular matrix accumulated in the tissue where fibrosis occurred is decomposed and the fibrosis-occurred tissue is restored to a normal tissue. Compared to the fact that conventional therapeutic agents for fibrosis have only an incomplete therapeutic effect on fibrosis, the therapeutic agent for fibrosis comprising the CSF3R inhibitor has a complete therapeutic effect compared to conventional therapeutic agents for fibrosis, and thus can be a superior therapeutic agent.

The reason why the therapeutic agent for fibrosis comprising the CSF3R inhibitor has a complete therapeutic effect compared to conventional therapeutic agents for fibrosis may be that the level of inhibition of the TGF-β mediated signaling pathway by treatment with the CSF3R inhibitor is superior to that of conventional therapeutic agents for fibrosis. Another reason may be that while conventional therapeutic agents for fibrosis only inhibit the TGF-β mediated signaling pathway, the therapeutic agent for fibrosis comprising the CSF3R inhibitor can inhibit the non-TGF-β mediated signaling pathway, the positive-feedback loop of CSF3R, and the positive-feedback loop of TGF-β in addition to the TGF-β mediated signaling pathway.

### 4.8. Advantages of Fibrosis Therapeutic Agent Using CSF3R Inhibitor 2 - Therapeutic Effect Independent of the Type of Tissue where Fibrosis Occurred

The present inventors have revealed that CSF3R functions as a key regulator in the pathogenesis of pulmonary fibrosis. Accordingly, a therapeutic agent for pulmonary fibrosis comprising a CSF3R inhibitor has a more fundamental mechanism of action than conventional therapeutic agents for pulmonary fibrosis and exhibits a true therapeutic effect. Furthermore, the present inventors have revealed through experiments that CSF3R is also crucially involved in the fibrosis of skin, liver, and kidney cells. These findings allow for a reasonable expectation that CSF3R is a key regulator (or upstream regulator) involved in tissue fibrosis regardless of the tissue type. Furthermore, it serves as a basis for predicting that a CSF3R inhibitor can have a therapeutic effect on fibrosis regardless of the tissue type.

Specifically, the inventors of the present disclosure have identified the pathogenesis and/or therapeutic mechanism of fibrosis through Experimental Example 2 and confirmed through Experimental Example 3 that the CSF3R inhibitor actually has a therapeutic effect on fibrosis occurring in various tissues. For example, it was confirmed through Experimental Examples 3.1 and 3.2 that the CSF3R inhibitor has a therapeutic effect on pulmonary fibrosis. For example, it was confirmed through Experimental Examples 3.3 and 3.4 that the CSF3R inhibitor has a therapeutic effect on skin fibrosis. For example, it was confirmed through Experimental Example 3.5 that the CSF3R inhibitor has a therapeutic effect on liver fibrosis. For example, it was confirmed through Experimental Example 3.6 that the CSF3R inhibitor has a therapeutic effect on renal fibrosis.

Therefore, the therapeutic agent for fibrosis comprising the CSF3R inhibitor disclosed in the present specification can function as an effective therapeutic agent regardless of the type of tissue where fibrosis has occurred.

In one embodiment, the tissue where fibrosis occurred may be any one tissue selected from skin; brain; nervous system; heart; bone marrow; liver; gut; joint; eye; lung; kidney; retroperitoneum; mediastinum; and pancreas.

In one embodiment, the CSF3R inhibitor has a therapeutic effect on skin fibrosis.

In one embodiment, the CSF3R inhibitor has a therapeutic effect on brain fibrosis.

In one embodiment, the CSF3R inhibitor has a therapeutic effect on nervous system fibrosis.

In one embodiment, the CSF3R inhibitor has a therapeutic effect on cardiac fibrosis.

In one embodiment, the CSF3R inhibitor has a therapeutic effect on bone marrow fibrosis.

In one embodiment, the CSF3R inhibitor has a therapeutic effect on liver fibrosis.

In one embodiment, the CSF3R inhibitor has a therapeutic effect on intestinal fibrosis.

In one embodiment, the CSF3R inhibitor has a therapeutic effect on joint fibrosis.

In one embodiment, the CSF3R inhibitor has a therapeutic effect on fibrosis.

In one embodiment, the CSF3R inhibitor has a therapeutic effect on ocular fibrosis.

In one embodiment, the CSF3R inhibitor has a therapeutic effect on pulmonary fibrosis.

In one embodiment, the CSF3R inhibitor has a therapeutic effect on renal fibrosis.

In one embodiment, the CSF3R inhibitor has a therapeutic effect on retroperitoneal fibrosis.

In one embodiment, the CSF3R inhibitor has a therapeutic effect on mediastinal fibrosis.

In one embodiment, the CSF3R inhibitor has a therapeutic effect on pancreatic fibrosis.

### 4.9. Pharmaceutical Composition for Treating Fibrosis Comprising CSF3R Inhibitor

### 4.9.1. Overview of Pharmaceutical Composition for Treating Fibrosis Comprising CSF3R Inhibitor

The present disclosure discloses a pharmaceutical composition for treating fibrosis comprising a CSF3R inhibitor. The pharmaceutical composition for treating fibrosis comprises a therapeutically effective amount of a CSF3R inhibitor. In one embodiment, the pharmaceutical composition for treating fibrosis may comprise a therapeutically effective amount of a CSF3R inhibitor and a pharmaceutically acceptable carrier. In another embodiment, the pharmaceutical composition for treating fibrosis may comprise a therapeutically effective amount of a CSF3R inhibitor and an adjuvant. In another embodiment, the pharmaceutical composition for treating fibrosis may comprise a therapeutically effective amount of a CSF3R inhibitor; a pharmaceutically acceptable carrier; and an adjuvant. Here, the pharmaceutically acceptable carrier may be for appropriate formulation of the pharmaceutical composition for treating fibrosis.

### 4.9.2. Target Disease - Fibrosis

The pharmaceutical composition for treating fibrosis is for treating fibrosis, or fibrosis-related diseases, disorders, and/or symptoms. The fibrosis or fibrosis-related diseases, disorders, and/or symptoms include all those recognizable by those of ordinary skill in the art.

In one embodiment, the fibrosis or fibrosis-related diseases, disorders, and/or symptoms may be those described in the section <3.14. Examples of Fibrosis Diseases>.

In one embodiment, the tissue where fibrosis occurred may be those described in the section <3.13. Examples of Tissues where Fibrosis Occurs>.

### 4.9.3. Examples of Formulation of Pharmaceutical Composition for Treating Fibrosis

In one embodiment, the pharmaceutical composition for treating fibrosis comprising the CSF3R inhibitor may be formulated into troches, lozenges, tablets, aqueous suspensions, oily suspensions, prepared powders, granules, emulsions, hard capsules, soft capsules, syrups, or elixirs. In another embodiment, the pharmaceutical composition for treating fibrosis comprising the CSF3R inhibitor may be formulated into injection solutions, suppositories, powders for respiratory inhalation, aerosolized sprays, ointments, powders for application, oils, and creams. In another embodiment, the pharmaceutical composition for treating fibrosis comprising the CSF3R inhibitor may be formulated as an injection solution. Specifically, it may be formulated as an injection solution by mixing a therapeutically effective amount of the CSF3R inhibitor with a stabilizer or a buffer in water to prepare a solution or suspension, which is then formulated into a unit dose of an ampoule or vial. In another embodiment, the pharmaceutical composition for treating fibrosis comprising the CSF3R inhibitor may be formulated as an aerosol by mixing a propellant or the like with an additive to prepare an aqueous concentrated product or a wet powder. In another embodiment, when formulating the pharmaceutical composition for treating fibrosis comprising the CSF3R inhibitor for transdermal use, ointments, creams, powders for application, oils, external preparations for skin, etc. may be prepared by adding animal oils, vegetable oils, waxes, paraffin, starch, tragacanth, cellulose derivatives, polyethylene glycol, silicone, bentonite, silica, talc, zinc oxide, etc. as a carrier to a therapeutically effective amount of the CSF3R inhibitor.

### 4.9.4. Examples of Pharmaceutically Acceptable Carriers

Pharmaceutically acceptable carriers are those commonly used in formulation and include, but are not limited to, saline, sterile water, Ringer's solution, buffered saline, cyclodextrin, dextrose solution, maltodextrin solution, glycerol, ethanol, liposomes, and the like, and may further include other conventional additives such as antioxidants and buffers as needed. In addition, diluents, dispersants, surfactants, binders, lubricants, etc., may be additionally added to formulate injectable formulations such as aqueous solutions, suspensions, and emulsions, or pills, capsules, granules, or tablets. For suitable pharmaceutically acceptable carriers and formulations, the methods disclosed in Remington's Pharmaceutical Sciences (19th edition, 1995) can be preferably used according to each component. In one embodiment, the pharmaceutical composition for treating fibrosis comprising the CSF3R inhibitor may include, as a pharmaceutically acceptable carrier, binders such as lactose, saccharose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol wax; sweeteners; flavorings; syrups; liquid carriers such as fatty oils; sterile aqueous solutions; propylene glycol; polyethylene glycol; injectable esters such as ethyl oleate; suspensions; emulsions; lyophilized preparations; external preparations; stabilizers; buffers; animal oils; vegetable oils; waxes; paraffin; starch; tragacanth; cellulose derivatives; polyethylene glycol; silicone; bentonite; silica; talc; zinc oxide; or appropriate combinations thereof.

### 4.10. Method for Treating Fibrosis Using CSF3R Inhibitor

### 4.10.1. Overview of Method for Treating Fibrosis Using CSF3R Inhibitor

The present disclosure discloses a method for treating fibrosis using the CSF3R inhibitor. The method for treating fibrosis comprises administering a therapeutically effective amount of a CSF3R inhibitor to a subject by an appropriate administration method, an appropriate administration regimen, and an appropriate dose. In one embodiment, the method for treating fibrosis comprises administering a suitably formulated therapeutically effective CSF3R inhibitor, that is, the pharmaceutical composition for treating fibrosis, to a subject by an appropriate administration method, an appropriate administration regimen, and an appropriate dose. In one embodiment, the subject of the method for treating fibrosis may be a human or a non-human animal. In one embodiment, the subject of the method for treating fibrosis may be a subject having fibrosis, or a fibrosis-related disease, disorder, and/or symptom.

### 4.10.2. Target Disease - Fibrosis

The method for treating fibrosis is for treating fibrosis, or fibrosis-related diseases, disorders, and/or symptoms. The fibrosis or fibrosis-related diseases, disorders, and/or symptoms include all those recognizable by those of ordinary skill in the art.

In one embodiment, the fibrosis or fibrosis-related diseases, disorders, and/or symptoms may be those described in the section <3.14. Examples of Fibrosis Diseases>.

In one embodiment, the tissue where fibrosis occurred may be those described in the section <3.13. Examples of Tissues where Fibrosis Occurs>.

### 4.10.3. Examples of Administration Methods

The method for treating fibrosis may be administering a suitably formulated therapeutically effective CSF3R inhibitor to a subject by an appropriate administration method. In one embodiment, the method for treating fibrosis may involve oral administration or parenteral administration of a suitably formulated therapeutically effective CSF3R inhibitor. The parenteral administration method may include intravenous administration, intraperitoneal administration, intramuscular administration, transdermal administration, subcutaneous administration, and the like.

Various criteria related to administration (dose, cycle, frequency, etc.) can be adjusted by a physician according to the individual patient's needs following clinical examinations during the administration process.

### 4.10.4. Examples of Dosage

The method for treating fibrosis may be administering a suitably formulated therapeutically effective CSF3R inhibitor to a subject in an appropriate dose. In one embodiment, the dose may be about 0.01 mg to 1000 mg per 1 kg of the subject's body weight, based on the CSF3R inhibitor.

### 4.10.5. Examples of Administration Cycle

The method for treating fibrosis may be administering a suitably formulated therapeutically effective CSF3R inhibitor to a subject at an appropriate administration cycle. In one embodiment, it may be administering the pharmaceutical composition for treating fibrosis comprising an appropriate dose of the CSF3R inhibitor once a day. As another example, the administration cycle may be administering the pharmaceutical composition for treating fibrosis comprising an appropriate dose of the CSF3R inhibitor in two or more divided doses a day. As another example, the administration cycle may be administering the pharmaceutical composition for treating fibrosis comprising an appropriate dose of the CSF3R inhibitor at intervals of 1 hour, 2 hours, 6 hours, 12 hours, 24 hours, 2 days, 3 days, 1 week, 2 weeks, 1 month, and/or 3 months.

### 4.11. Therapeutic Use of CSF3R Inhibitor

The present disclosure discloses a therapeutic use of the CSF3R inhibitor for fibrosis. In one embodiment, the present disclosure discloses the CSF3R inhibitor for treating fibrosis, or fibrosis-related diseases, disorders, and/or symptoms.

In one embodiment, the fibrosis or fibrosis-related diseases, disorders, and/or symptoms may be those described in the section <3.14. Examples of Fibrosis Diseases>.

In one embodiment, the tissue where fibrosis occurred may be those described in the section <3.13. Examples of Tissues where Fibrosis Occurs>.

### 4.12. Use of CSF3R Inhibitor for Manufacturing a Therapeutic Agent

The present disclosure discloses a use of the CSF3R inhibitor for manufacturing a therapeutic agent for fibrosis. In one embodiment, the present disclosure discloses a use of the CSF3R inhibitor for manufacturing a medicament for treating fibrosis, or fibrosis-related diseases, disorders, and/or symptoms.

In one embodiment, the fibrosis or fibrosis-related diseases, disorders, and/or symptoms may be those described in the section <3.14. Examples of Fibrosis Diseases>.

In one embodiment, the tissue where fibrosis occurred may be those described in the section <3.13. Examples of Tissues where Fibrosis Occurs>.

### 4.13. Examples of Tissues where Fibrosis Occurs

Fibrosis can occur in almost all tissues in the body, and it is known that many tissues in the body can be fibrosed, causing various diseases, disorders, and/or conditions. For example, it has been reported that fibrosis can occur in tissues such as skin, brain, nervous system, heart, bone marrow, liver, gut, joint, eye, lung, kidney, retroperitoneum, mediastinum, and/or pancreas.

### 4.14. Examples of Fibrosis Diseases

The fibrosis or fibrosis-related diseases, disorders, and/or symptoms described in the present disclosure include all those recognizable by those of ordinary skill in the art. In one embodiment, the fibrosis or fibrosis-related diseases, disorders, and/or symptoms may be those described in the section <3.14. Examples of Fibrosis Diseases>.

### 4.15. Examples of CSF3R Inhibitors

The CSF3R inhibitor described in the present disclosure is not particularly limited as long as it can inhibit the function of CSF3R. As described above, the CSF3R inhibitor may 1) interfere with the interaction between the CSF3R protein and other molecules, or 2) inhibit the expression of the CSF3R protein to reduce the absolute amount of CSF3R protein interacting with other molecules, thereby consequently interfering with the function of the CSF3R protein. Examples of CSF3R inhibitors capable of such actions include, but are not limited to, nucleic acids that silence the mRNA of the CSF3R gene, a CRISPR/Cas system that knocks out or knocks down the CSF3R gene, compounds that inhibit the CSF3R protein, or aptamers that inhibit the CSF3R protein.

In one embodiment, the CSF3R inhibitor may be those described in the section <2.9. Examples of CSF3R Inhibitors> and its sub-sections <2.9.1. Nucleic Acids Silencing mRNA of CSF3R Gene> to <2.9.6. Substance Inhibiting CSF3R Protein 3 - Antibody>.

### 5. Anti-CSF3 Antibody

### 5.1. Anti-CSF3 Antibody - Overview

In one aspect, the present disclosure discloses an antibody against colony-stimulating factor 3 (CSF3). In the present disclosure, the antibody may be referred to as an anti-CSF3 antibody.

The anti-CSF3 antibody can bind to CSF3 or a fragment of CSF3. In one embodiment, the anti-CSF3 antibody can specifically bind to CSF3 or a fragment of CSF3. In another embodiment, the anti-CSF3 antibody can inhibit or interfere with the binding of CSF3 to CSF3R. In another embodiment, the anti-CSF3 antibody can inhibit the function of CSF3. In another embodiment, the anti-CSF3 antibody may be an anti-CSF3 neutralizing antibody. In another embodiment, the anti-CSF3 antibody may be a monoclonal antibody that specifically binds to human CSF3 protein.

Table 1 below lists amino acid sequences that can be included in the CDRH1, CDRH2, CDRH3, CDRL1, CDRL2, CDRL3, FRH1, FRH2, FRH3, FRH4, FRL1, FRL2, FRL3, or FRL4 of the anti-CSF3 antibody.

Table 2 below lists amino acid sequences that can be included in the VH and VL of the anti-CSF3 antibody.

**[Table 1]**

| **Region** | **Amino Acid Sequence (N terminal → C terminal)** |
|---|---|
| CDRH1 | GYTFTNYG (SEQ ID NO: 1) |
| CDRH2 | INPNSGGT (SEQ ID NO: 2) |
| CDRH3 | SRGSGGRDAFDV (SEQ ID NO: 3) |
| | SRGSGGHLAFDV (SEQ ID NO: 4) |
| | SRGSGGMSGFDV (SEQ ID NO: 5) |
| | SRGSGGRNGFDV (SEQ ID NO: 6) |
| | SRGSGGTTGFDV (SEQ ID NO: 7) |
| | SRGSGGMHPFDV (SEQ ID NO: 8) |
| | SRGSGGTSPFDV (SEQ ID NO: 9) |
| | SRGSGGIKPFDV (SEQ ID NO: 10) |
| | SRGSGGAVPFDV (SEQ ID NO: 11) |
| | SRGSGGIAPFDV (SEQ ID NO: 12) |
| | SRGSGGLLPFDV (SEQ ID NO: 13) |
| | SRGSGGMVPFDV (SEQ ID NO: 14) |
| | SRGSGGRSGFDV (SEQ ID NO: 15) |
| | SRGSGGFQAFDV (SEQ ID NO: 16) |
| | SRGSGGLVPFDV (SEQ ID NO: 17) |
| | SRGSGGIRPFDV (SEQ ID NO: 18) |
| | SRGSGGFFPFDV (SEQ ID NO: 19) or |
| | SRGSGGYSGFDV (SEQ ID NO: 20) |
| CDRL1 | QTISTY (SEQ ID NO: 21) |
| | QTTGQY (SEQ ID NO: 22) |
| | QTISNR (SEQ ID NO: 23) |
| | KSISTY (SEQ ID NO: 24) |
| CDRL2 | YAS (SEQ ID NO: 25) |
| CDRL3 | QGGYSSPRT (SEQ ID NO: 26) |
| FRH1 | QMLVQSGAEARKPGSSVKVSCKAS (SEQ ID NO: 27) or |
| | EMLVQLGQSAEARKPGSSVKVSCKAS (SEQ ID NO: 28) |
| FRH2 | ISWLKQRPGQGLEWMGW (SEQ ID NO: 29) |
| FRH3 | IYAQKFGQRYTMRTDTTSITAYMELSTL RSEDTAVYYC (SEQ ID NO: 30); or |
| | IYAQKFGQRYTMRTDTPTTAYMELSTL RSEDTAVYYC (SEQ ID NO: 31) |
| FRH4 | WGQGTMVTVSS (SEQ ID NO: 32) |
| FRL1 | DIQMTQSPFSLASAVGDRVTITCRAS (SEQ ID NO: 33) |
| FRL2 | LNWYQHKPGKAPKLFY (SEQ ID NO: 34) |
| FRL3 | SLQSGVPSRFGSGSGSGSTDFALTISSLQPEDFATYYC (SEQ ID NO: 35) |
| FRL4 | FGQGTKVEIK (SEQ ID NO: 36); or |
| | FGQGTKVEIT (SEQ ID NO: 37) |

**[Table 2]**

| **Region** | **Amino Acid Sequence (N terminal → C terminal)** |
|---|---|
| VH | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGRDAFDVWGQGT MVTVSS (SEQ ID NO: 38); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGHLAFDVWGQGT MVTVSS (SEQ ID NO: 39); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGMSGFDVWGQGT MVTVSS (SEQ ID NO: 40); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGRNGFDVWGQGT MVTVSS (SEQ ID NO: 41); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGTTGFDVWGQGT MVTVSS (SEQ ID NO: 42); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGMHPFDVWGQGT MVTVSS (SEQ ID NO: 43); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGTSPFDVWGQGTM VTVSS (SEQ ID NO: 44); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGIKPFDVWGQGTM VTVSS (SEQ ID NO: 45); |
| | EMQLVQSGAEARKPGSSVKVSCKASGYTFTNYGISWLRQTPGQGLEWMGWINPNSG GTIYAQKFQGRVTMTRDTPITTAYMELSTLRSEDTAVYYCSR GSGGAVPFDVWGQGT MVTVSS (SEQ ID NO: 46); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGIAPFDVWGQGTM VTVSS (SEQ ID NO: 47); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGLLPFDVWGQGTM VTVSS (SEQ ID NO: 48); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGMVPFDVWGQGT MVTVSS (SEQ ID NO: 49); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGRSGFDVWGQGT |
| | MVTVSS (SEQ ID NO: 50); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGFQAFDVWGQGT MVTVSS (SEQ ID NO: 51); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGLVPFDVWGQGTM VTVSS (SEQ ID NO: 52); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGIRPFDVWGQGTM VTVSS (SEQ ID NO: 53); |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGFFPFDVWGQGTM VTVSS (SEQ ID NO: 54); or |
| | QMQLVQSGAEARKPGSSVKVSCKASGYTFTNYGISWLRQTPGQGLEWMGWINPNSG GTIYAQKFQGRVTMTRDTSITTAYMELSTLRSEDTAVYYCSR GSGGYSGFDVWGQGT MVTVSS (SEQ ID NO: 55) |
| VL | DIQMTQSPFSLSASVGDRVTITCRASQTISTYLNWYQHKPGKAPKLFIYYASSLQSGVPSR FSGSGSGTDFALTISSLQPEDFATYYCQQGYSSPRTFGQGTKVEIK (SEQ ID NO: 56); |
| | DIQMTQSPFSLSASVGDRVTITCRASQTISTYLNWYQHKPGKAPKLFIYYASSLQSGVPSR FSGSGSGTDFALTISSLQPEDFATYYCQQGYSSPRTFGQGTKVEIT (SEQ ID NO: 57); |
| | DIQMTQSPFSLSASVGDRVTITCRASQTTGQYLNWYQHKPGKAPKLFIYYASSLQSGVP SRFSGSGSGTDFALTISSLQPEDFATYYCQQGYSSPRTFGQGTKVEIK (SEQ ID NO: 58); |
| | DIQMTQSPFSLSASVGDRVTITCRASQTISNRLNWYQHKPGKAPKLFIYYASSLQSGVPS RFSGSGSGTDFALTISSLQPEDFATYYCQQGYSSPRTFGQGTKVEIK (SEQ ID NO: 59); |
| | or |
| | DIQMTQSPFSLSASVGDRVTITCRASKSISTYLNWYQHKPGKAPKLFIYYASSLQSGVPSR FSGSGSGTDFALTISSLQPEDFATYYCQQGYSSPRTFGQGTKVEIK (SEQ ID NO: 60) |

### 5.2. Sequences of Variable Regions of Anti-CSF3 Antibody

### Sequence of CDRH1

In one embodiment, the amino acid sequence of the CDRH1 of the anti-CSF3 antibody may be the amino acid sequence of SEQ ID NO: 1, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 1.

In one embodiment, the amino acid sequence of the CDRH1 of the anti-CSF3 antibody may comprise the amino acid sequence of SEQ ID NO: 1.

In one embodiment, the amino acid sequence of the CDRH1 of the anti-CSF3 antibody may be the amino acid sequence of SEQ ID NO: 1.

In one embodiment, the VH of the anti-CSF3 antibody may comprise a CDRH1 comprising the amino acid sequence of SEQ ID NO: 1, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 1.

In one embodiment, the VH of the anti-CSF3 antibody may comprise a CDRH1 comprising the same amino acid sequence as SEQ ID NO: 1.

In one embodiment, the VH of the anti-CSF3 antibody may comprise a CDRH1 represented by the amino acid sequence of SEQ ID NO: 1.

### Sequence of CDRH2

In one embodiment, the amino acid sequence of the CDRH2 of the anti-CSF3 antibody may be the amino acid sequence of SEQ ID NO: 2, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 2.

In one embodiment, the amino acid sequence of the CDRH2 of the anti-CSF3 antibody may comprise the amino acid sequence of SEQ ID NO: 2.

In one embodiment, the amino acid sequence of the CDRH2 of the anti-CSF3 antibody may be the amino acid sequence of SEQ ID NO: 2.

In one embodiment, the VH of the anti-CSF3 antibody may comprise a CDRH2 comprising the amino acid sequence of SEQ ID NO: 2, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 2.

In one embodiment, the VH of the anti-CSF3 antibody may comprise a CDRH2 comprising the same amino acid sequence as SEQ ID NO: 2.

In one embodiment, the VH of the anti-CSF3 antibody may comprise a CDRH2 represented by the amino acid sequence of SEQ ID NO: 2.

### Sequence of CDRH3

In one embodiment, the amino acid sequence of the CDRH3 of the anti-CSF3 antibody may be any one amino acid sequence of SEQ ID NOs: 3 to 20, or an amino acid sequence having at least 80, 81, 82, 83, 84, 85, 86, 87, 88, 89, 90, 91, 92, 93, 94, 95, 96, 97, 98, or 99% or more sequence similarity to any one amino acid sequence of SEQ ID NOs: 3 to 20.

In one embodiment, the amino acid sequence of the CDRH3 of the anti-CSF3 antibody may comprise any one amino acid sequence of SEQ ID NOs: 3 to 20.

In one embodiment, the amino acid sequence of the CDRH3 of the anti-CSF3 antibody may be any one amino acid sequence of SEQ ID NOs: 3 to 20.

In one embodiment, the VH of the anti-CSF3 antibody may comprise a CDRH3 comprising any one amino acid sequence of SEQ ID NOs: 3 to 20, or an amino acid sequence having at least 80, 81, 82, 83, 84, 85, 86, 87, 88, 89, 90, 91, 92, 93, 94, 95, 96, 97, 98, or 99% or more sequence similarity to any one sequence of SEQ ID NOs: 3 to 20.

In one embodiment, the VH of the anti-CSF3 antibody may comprise a CDRH3 comprising any one amino acid sequence of SEQ ID NOs: 3 to 20.

In one embodiment, the VH of the anti-CSF3 antibody may comprise a CDRH3 represented by any one amino acid sequence of SEQ ID NOs: 3 to 20.

### Sequence of CDRL1

In one embodiment, the amino acid sequence of the CDRL1 of the anti-CSF3 antibody may be any one amino acid sequence of SEQ ID NOs: 21 to 24, or an amino acid sequence having at least 80, 81, 82, 83, 84, 85, 86, 87, 88, 89, 90, 91, 92, 93, 94, 95, 96, 97, 98, or 99% or more sequence similarity to any one amino acid sequence of SEQ ID NOs: 21 to 24.

In one embodiment, the amino acid sequence of the CDRL1 of the anti-CSF3 antibody may comprise any one amino acid sequence of SEQ ID NOs: 21 to 24.

In one embodiment, the amino acid sequence of the CDRL1 of the anti-CSF3 antibody may be any one amino acid sequence of SEQ ID NOs: 21 to 24.

In one embodiment, the VL of the anti-CSF3 antibody may comprise a CDRL1 comprising any one amino acid sequence of SEQ ID NOs: 21 to 24, or an amino acid sequence having at least 80, 81, 82, 83, 84, 85, 86, 87, 88, 89, 90, 91, 92, 93, 94, 95, 96, 97, 98, or 99% or more sequence similarity to any one sequence of SEQ ID NOs: 21 to 24.

In one embodiment, the VL of the anti-CSF3 antibody may comprise a CDRL1 comprising any one amino acid sequence of SEQ ID NOs: 21 to 24.

In one embodiment, the VL of the anti-CSF3 antibody may comprise a CDRL1 represented by any one amino acid sequence of SEQ ID NOs: 21 to 24.

### Sequence of CDRL2

In one embodiment, the amino acid sequence of the CDRL2 of the anti-CSF3 antibody may be YAS (SEQ ID NO: 25), or an amino acid sequence having at least 80, 81, 82, 83, 84, 85, 86, 87, 88, 89, 90, 91, 92, 93, 94, 95, 96, 97, 98, or 99% or more sequence similarity to YAS (SEQ ID NO: 25).

In one embodiment, the amino acid sequence of the CDRL2 of the anti-CSF3 antibody may comprise the amino acid sequence of YAS (SEQ ID NO: 25).

In one embodiment, the amino acid sequence of the CDRL2 of the anti-CSF3 antibody may be the amino acid sequence of YAS (SEQ ID NO: 25).

In one embodiment, the VL of the anti-CSF3 antibody may comprise a CDRL2 comprising the amino acid sequence of YAS (SEQ ID NO: 25), or an amino acid sequence having at least 80, 81, 82, 83, 84, 85, 86, 87, 88, 89, 90, 91, 92, 93, 94, 95, 96, 97, 98, or 99% or more sequence similarity to the sequence of YAS (SEQ ID NO: 25).

In one embodiment, the VL of the anti-CSF3 antibody may comprise a CDRL2 comprising the amino acid sequence of YAS (SEQ ID NO: 25).

In one embodiment, the VL of the anti-CSF3 antibody may comprise a CDRL2 represented by the amino acid sequence of YAS (SEQ ID NO: 25).

### Sequence of CDRL3

In one embodiment, the amino acid sequence of the CDRL3 of the anti-CSF3 antibody may be the amino acid sequence of SEQ ID NO: 26, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 26.

In one embodiment, the amino acid sequence of the CDRL3 of the anti-CSF3 antibody may comprise the amino acid sequence of SEQ ID NO: 26.

In one embodiment, the amino acid sequence of the CDRL3 of the anti-CSF3 antibody may be the amino acid sequence of SEQ ID NO: 26.

In one embodiment, the VL of the anti-CSF3 antibody may comprise a CDRL3 comprising the amino acid sequence of SEQ ID NO: 26, or an amino acid sequence having at least 80, 81, 82, 83, 84, 85, 86, 87, 88, 89, 90, 91, 92, 93, 94, 95, 96, 97, 98, or 99% or more sequence similarity to the sequence of SEQ ID NO: 26.

In one embodiment, the VL of the anti-CSF3 antibody may comprise a CDRL3 comprising the amino acid sequence of SEQ ID NO: 26.

In one embodiment, the VL of the anti-CSF3 antibody may comprise a CDRL3 represented by the amino acid sequence of SEQ ID NO: 26.

### CDRH1, CDRH2, and CDRH3

In one embodiment, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) any one amino acid sequence of SEQ ID NOs: 3 to 20.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 3.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 4.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 5.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 6.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 7.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 8.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 9.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 10.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 11.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 12.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 13.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 14.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 15.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 16.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 17.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 18.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 19.

Preferably, the VH of the anti-CSF3 antibody may comprise: a CDRH1 comprising (or represented by) the amino acid sequence of SEQ ID NO: 1; a CDRH2 comprising (or represented by) the amino acid sequence of SEQ ID NO: 2; and a CDRH3 comprising (or represented by) the amino acid sequence of SEQ ID NO: 20.

### CDRL1, CDRL2, and CDRL3

In one embodiment, the VL of the anti-CSF3 antibody may comprise: a CDRL1 comprising (or represented by) any one amino acid sequence of SEQ ID NOs: 21 to 24; a CDRL2 comprising (or represented by) the amino acid sequence of YAS (SEQ ID NO: 25); and a CDRL3 comprising (or represented by) the amino acid sequence of SEQ ID NO: 26.

Preferably, the VL of the anti-CSF3 antibody may comprise: a CDRL1 comprising (or represented by) the amino acid sequence of SEQ ID NO: 21; a CDRL2 comprising (or represented by) the amino acid sequence of YAS (SEQ ID NO: 25); and a CDRL3 comprising (or represented by) the amino acid sequence of SEQ ID NO: 26.

Preferably, the VL of the anti-CSF3 antibody may comprise: a CDRL1 comprising (or represented by) the amino acid sequence of SEQ ID NO: 22; a CDRL2 comprising (or represented by) the amino acid sequence of YAS (SEQ ID NO: 25); and a CDRL3 comprising (or represented by) the amino acid sequence of SEQ ID NO: 26.

Preferably, the VL of the anti-CSF3 antibody may comprise: a CDRL1 comprising (or represented by) the amino acid sequence of SEQ ID NO: 23; a CDRL2 comprising (or represented by) the amino acid sequence of YAS (SEQ ID NO: 25); and a CDRL3 comprising (or represented by) the amino acid sequence of SEQ ID NO: 26.

Preferably, the VL of the anti-CSF3 antibody may comprise: a CDRL1 comprising (or represented by) the amino acid sequence of SEQ ID NO: 24; a CDRL2 comprising (or represented by) the amino acid sequence of YAS (SEQ ID NO: 25); and a CDRL3 comprising (or represented by) the amino acid sequence of SEQ ID NO: 26.

CDRH1, CDRH2, CDRH3, CDRL1, CDRL2, and CDRL3

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 3, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 4, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 5, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 6, SEQ ID NO: 22, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 7, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 8, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 9, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 10, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 11, SEQ ID NO: 23, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 12, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 13, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 14, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 15, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 16, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 17, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 18, SEQ ID NO: 21, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 19, SEQ ID NO: 24, YAS (SEQ ID NO: 25), and SEQ ID NO: 26, respectively.

In one embodiment, the amino acid sequences of CDRH1, CDRH2, CDRH3, CDRL1, CDRL2, and CDRL3 of the variable region of the anti-CSF3 antibody may be the amino acid sequences of SEQ ID NO: 1, SEQ ID NO: 2, SEQ ID NO: 20, SEQ ID NO: 21, YAS (SEQ ID NO: 25), and SEQ ID NO: 26, respectively.

### Sequence of FRH1

In one embodiment, the amino acid sequence of the FRH1 of the anti-CSF3 antibody may be any one amino acid sequence of SEQ ID NOs: 27 to 28, or an amino acid sequence having at least 80, 81, 82, 83, 84, 85, 86, 87, 88, 89, 90, 91, 92, 93, 94, 95, 96, 97, 98, or 99% or more sequence similarity to any one of SEQ ID NOs: 27 to 28.

In one embodiment, the amino acid sequence of the FRH1 of the anti-CSF3 antibody may comprise any one amino acid sequence of SEQ ID NOs: 27 to 28.

In one embodiment, the amino acid sequence of the FRH1 of the anti-CSF3 antibody may be any one amino acid sequence of SEQ ID NOs: 27 to 28.

In one embodiment, the VH of the anti-CSF3 antibody may comprise a FRH1 comprising any one amino acid sequence of SEQ ID NOs: 27 to 28, or an amino acid sequence having at least 80, 81, 82, 83, 84, 85, 86, 87, 88, 89, 90, 91, 92, 93, 94, 95, 96, 97, 98, or 99% or more sequence similarity to any one of SEQ ID NOs: 27 to 28.

In one embodiment, the VH of the anti-CSF3 antibody may comprise a FRH1 comprising the same amino acid sequence as any one of SEQ ID NOs: 27 to 28.

In one embodiment, the VH of the anti-CSF3 antibody may comprise a FRH1 represented by any one amino acid sequence of SEQ ID NOs: 27 to 28.

### Sequence of FRH2

In one embodiment, the amino acid sequence of the FRH2 of the anti-CSF3 antibody may be the amino acid sequence of SEQ ID NO: 29, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 29.

In one embodiment, the amino acid sequence of the FRH2 of the anti-CSF3 antibody may comprise the amino acid sequence of SEQ ID NO: 29.

In one embodiment, the amino acid sequence of the FRH2 of the anti-CSF3 antibody may be the amino acid sequence of SEQ ID NO: 29.

In one embodiment, the VH of the anti-CSF3 antibody may comprise a FRH2 comprising the amino acid sequence of SEQ ID NO: 29, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 29.

In one embodiment, the VH of the anti-CSF3 antibody may comprise a FRH2 comprising the same amino acid sequence as SEQ ID NO: 29.

In one embodiment, the VH of the anti-CSF3 antibody may comprise a FRH2 represented by the amino acid sequence of SEQ ID NO: 29.

### Sequence of FRH3

In one embodiment, the amino acid sequence of the FRH3 of the anti-CSF3 antibody may be any one amino acid sequence of SEQ ID NOs: 30 to 31, or an amino acid sequence having at least 80, 81, 82, 83, 84, 85, 86, 87, 88, 89, 90, 91, 92, 93, 94, 95, 96, 97, 98, or 99% or more sequence similarity to any one of SEQ ID NOs: 30 to 31.

In one embodiment, the amino acid sequence of the FRH3 of the anti-CSF3 antibody may comprise any one amino acid sequence of SEQ ID NOs: 30 to 31.

In one embodiment, the amino acid sequence of the FRH3 of the anti-CSF3 antibody may be any one amino acid sequence of SEQ ID NOs: 30 to 31.

In one embodiment, the VH of the anti-CSF3 antibody may comprise a FRH3 comprising any one amino acid sequence of SEQ ID NOs: 30 to 31, or an amino acid sequence having at least 80, 81, 82, 83, 84, 85, 86, 87, 88, 89, 90, 91, 92, 93, 94, 95, 96, 97, 98, or 99% or more sequence similarity to any one of SEQ ID NOs: 30 to 31.

In one embodiment, the VH of the anti-CSF3 antibody may comprise a FRH3 comprising the same amino acid sequence as any one of SEQ ID NOs: 30 to 31.

In one embodiment, the VH of the anti-CSF3 antibody may comprise a FRH3 represented by any one amino acid sequence of SEQ ID NOs: 30 to 31.

### Sequence of FRH4

In one embodiment, the amino acid sequence of the FRH4 of the anti-CSF3 antibody may be the amino acid sequence of SEQ ID NO: 32, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 32.

In one embodiment, the amino acid sequence of the FRH4 of the anti-CSF3 antibody may comprise the amino acid sequence of SEQ ID NO: 32.

In one embodiment, the amino acid sequence of the FRH4 of the anti-CSF3 antibody may be the amino acid sequence of SEQ ID NO: 32.

In one embodiment, the VH of the anti-CSF3 antibody may comprise a FRH4 comprising the amino acid sequence of SEQ ID NO: 32, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 32.

In one embodiment, the VH of the anti-CSF3 antibody may comprise a FRH4 comprising the same amino acid sequence as SEQ ID NO: 32.

In one embodiment, the VH of the anti-CSF3 antibody may comprise a FRH4 represented by the amino acid sequence of SEQ ID NO: 32.

### Sequence of FRL1

In one embodiment, the amino acid sequence of the FRL1 of the anti-CSF3 antibody may be the amino acid sequence of SEQ ID NO: 33, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 33.

In one embodiment, the amino acid sequence of the FRL1 of the anti-CSF3 antibody may comprise the amino acid sequence of SEQ ID NO: 33.

In one embodiment, the amino acid sequence of the FRL1 of the anti-CSF3 antibody may be the amino acid sequence of SEQ ID NO: 33.

In one embodiment, the VL of the anti-CSF3 antibody may comprise a FRL1 comprising the amino acid sequence of SEQ ID NO: 33, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 33.

In one embodiment, the VL of the anti-CSF3 antibody may comprise a FRL1 comprising the same amino acid sequence as SEQ ID NO: 33.

In one embodiment, the VL of the anti-CSF3 antibody may comprise a FRL1 represented by the amino acid sequence of SEQ ID NO: 33.

### Sequence of FRL2

In one embodiment, the amino acid sequence of the FRL2 of the anti-CSF3 antibody may be the amino acid sequence of SEQ ID NO: 34, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 34.

In one embodiment, the amino acid sequence of the FRL2 of the anti-CSF3 antibody may comprise the amino acid sequence of SEQ ID NO: 34.

In one embodiment, the amino acid sequence of the FRL2 of the anti-CSF3 antibody may be the amino acid sequence of SEQ ID NO: 34.

In one embodiment, the VL of the anti-CSF3 antibody may comprise a FRL2 comprising the amino acid sequence of SEQ ID NO: 34, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 34.

In one embodiment, the VL of the anti-CSF3 antibody may comprise a FRL2 comprising the same amino acid sequence as SEQ ID NO: 34.

In one embodiment, the VL of the anti-CSF3 antibody may comprise a FRL2 represented by the amino acid sequence of SEQ ID NO: 34.

### Sequence of FRL3

In one embodiment, the amino acid sequence of the FRL3 of the anti-CSF3 antibody may be the amino acid sequence of SEQ ID NO: 35, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 35.

In one embodiment, the amino acid sequence of the FRL3 of the anti-CSF3 antibody may comprise the amino acid sequence of SEQ ID NO: 35.

In one embodiment, the amino acid sequence of the FRL3 of the anti-CSF3 antibody may be the amino acid sequence of SEQ ID NO: 35.

In one embodiment, the VL of the anti-CSF3 antibody may comprise a FRL3 comprising the amino acid sequence of SEQ ID NO: 35, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 35.

In one embodiment, the VL of the anti-CSF3 antibody may comprise a FRL3 comprising the same amino acid sequence as SEQ ID NO: 35.

In one embodiment, the VL of the anti-CSF3 antibody may comprise a FRL3 represented by the amino acid sequence of SEQ ID NO: 35.

### Sequence of FRL4

In one embodiment, the amino acid sequence of the FRL4 of the anti-CSF3 antibody may be any one amino acid sequence of SEQ ID NOs: 36 to 37, or an amino acid sequence having at least 80, 81, 82, 83, 84, 85, 86, 87, 88, 89, 90, 91, 92, 93, 94, 95, 96, 97, 98, or 99% or more sequence similarity to any one of SEQ ID NOs: 36 to 37.

In one embodiment, the amino acid sequence of the FRL4 of the anti-CSF3 antibody may comprise any one amino acid sequence of SEQ ID NOs: 36 to 37.

In one embodiment, the amino acid sequence of the FRL4 of the anti-CSF3 antibody may be any one amino acid sequence of SEQ ID NOs: 36 to 37.

In one embodiment, the VL of the anti-CSF3 antibody may comprise a FRL4 comprising any one amino acid sequence of SEQ ID NOs: 36 to 37, or an amino acid sequence having at least 80, 81, 82, 83, 84, 85, 86, 87, 88, 89, 90, 91, 92, 93, 94, 95, 96, 97, 98, or 99% or more sequence similarity to any one of SEQ ID NOs: 36 to 37.

In one embodiment, the VL of the anti-CSF3 antibody may comprise a FRL4 comprising the same amino acid sequence as any one of SEQ ID NOs: 36 to 37.

In one embodiment, the VL of the anti-CSF3 antibody may comprise a FRL4 represented by any one amino acid sequence of SEQ ID NOs: 36 to 37.

### FRH1, FRH2, FRH3, and FRH4

In one embodiment, the VH of the anti-CSF3 antibody may comprise: a FRH1 comprising (or represented by) any one amino acid sequence of SEQ ID NOs: 27 to 28; a FRH2 comprising (or represented by) the amino acid sequence of SEQ ID NO: 29; a FRH3 comprising (or represented by) any one amino acid sequence of SEQ ID NOs: 30 to 31; and a FRH4 comprising (or represented by) the amino acid sequence of SEQ ID NO: 32.

Preferably, the VH of the anti-CSF3 antibody may comprise: a FRH1 comprising (or represented by) the amino acid sequence of SEQ ID NO: 27; a FRH2 comprising (or represented by) the amino acid sequence of SEQ ID NO: 29; a FRH3 comprising (or represented by) the amino acid sequence of SEQ ID NO: 30; and a FRH4 comprising (or represented by) the amino acid sequence of SEQ ID NO: 32.

Preferably, the VH of the anti-CSF3 antibody may comprise: a FRH1 comprising (or represented by) the amino acid sequence of SEQ ID NO: 28; a FRH2 comprising (or represented by) the amino acid sequence of SEQ ID NO: 29; a FRH3 comprising (or represented by) the amino acid sequence of SEQ ID NO: 31; and a FRH4 comprising (or represented by) the amino acid sequence of SEQ ID NO: 32.

### FRL1, FRL2, FRL3, and FRL4

In one embodiment, the VL of the anti-CSF3 antibody may comprise: a FRL1 comprising (or represented by) the amino acid sequence of SEQ ID NO: 33; a FRL2 comprising (or represented by) the amino acid sequence of SEQ ID NO: 34; a FRL3 comprising (or represented by) the amino acid sequence of SEQ ID NO: 35; and a FRL4 comprising (or represented by) any one amino acid sequence of SEQ ID NOs: 36 to 37.

Preferably, the VL of the anti-CSF3 antibody may comprise: a FRL1 comprising (or represented by) the amino acid sequence of SEQ ID NO: 33; a FRL2 comprising (or represented by) the amino acid sequence of SEQ ID NO: 34; a FRL3 comprising (or represented by) the amino acid sequence of SEQ ID NO: 35; and a FRL4 comprising (or represented by) the amino acid sequence of SEQ ID NO: 36.

Preferably, the VL of the anti-CSF3 antibody may comprise: a FRL1 comprising (or represented by) the amino acid sequence of SEQ ID NO: 33; a FRL2 comprising (or represented by) the amino acid sequence of SEQ ID NO: 34; a FRL3 comprising (or represented by) the amino acid sequence of SEQ ID NO: 35; and a FRL4 comprising (or represented by) the amino acid sequence of SEQ ID NO: 37.

### FRH1, FRH2, FRH3, FRH4, FRL1, FRL2, FRL3, and FRL4

In one embodiment, the amino acid sequences of FRH1, FRH2, FRH3, FRH4, FRL1, FRL2, FRL3, and FRL4 of the variable region of the anti-CSF3 antibody may be the amino acid sequences of SEQ ID NO: 27, SEQ ID NO: 29, SEQ ID NO: 30, SEQ ID NO: 32, SEQ ID NO: 33, SEQ ID NO: 34, SEQ ID NO: 35, and SEQ ID NO: 36, respectively.

In one embodiment, the amino acid sequences of FRH1, FRH2, FRH3, FRH4, FRL1, FRL2, FRL3, and FRL4 of the variable region of the anti-CSF3 antibody may be the amino acid sequences of SEQ ID NO: 27, SEQ ID NO: 29, SEQ ID NO: 30, SEQ ID NO: 32, SEQ ID NO: 33, SEQ ID NO: 34, SEQ ID NO: 35, and SEQ ID NO: 37, respectively.

In one embodiment, the amino acid sequences of FRH1, FRH2, FRH3, FRH4, FRL1, FRL2, FRL3, and FRL4 of the variable region of the anti-CSF3 antibody may be the amino acid sequences of SEQ ID NO: 28, SEQ ID NO: 29, SEQ ID NO: 31, SEQ ID NO: 32, SEQ ID NO: 33, SEQ ID NO: 34, SEQ ID NO: 35, and SEQ ID NO: 36, respectively.

### Sequence of VH

In one embodiment, the VH of the anti-CSF3 antibody may comprise any one amino acid sequence of SEQ ID NOs: 38 to 55, or an amino acid sequence having at least 80, 81, 82, 83, 84, 85, 86, 87, 88, 89, 90, 91, 92, 93, 94, 95, 96, 97, 98, or 99% or more sequence similarity to any one sequence of SEQ ID NOs: 38 to 55.

In one embodiment, the VH of the anti-CSF3 antibody may comprise any one amino acid sequence of SEQ ID NOs: 38 to 55.

In one embodiment, the amino acid sequence of the VH of the anti-CSF3 antibody may be any one amino acid sequence of SEQ ID NOs: 38 to 55.

### Sequence of VL

In one embodiment, the VL of the anti-CSF3 antibody may comprise any one amino acid sequence of SEQ ID NOs: 56 to 60, or an amino acid sequence having at least 80, 81, 82, 83, 84, 85, 86, 87, 88, 89, 90, 91, 92, 93, 94, 95, 96, 97, 98, or 99% or more sequence similarity to any one sequence of SEQ ID NOs: 56 to 60.

In one embodiment, the VL of the anti-CSF3 antibody may comprise any one amino acid sequence of SEQ ID NOs: 56 to 60.

In one embodiment, the amino acid sequence of the VL of the anti-CSF3 antibody may be any one amino acid sequence of SEQ ID NOs: 56 to 60.

### VH and VL

In one embodiment, the variable region of the anti-CSF3 antibody may comprise: a VH comprising (or represented by) any one amino acid sequence of SEQ ID NOs: 38 to 55; and a VL comprising (or represented by) any one amino acid sequence of SEQ ID NOs: 56 to 60.

Preferably, the variable region of the anti-CSF3 antibody may comprise: a VH comprising (or represented by) the amino acid sequence of SEQ ID NO: 39; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 40; and a VL comprising (or represented by) the amino acid sequence of SEQ ID NO: 57.

Preferably, the variable region of the anti-CSF3 antibody may comprise: a VH comprising (or represented by) the amino acid sequence of SEQ ID NO: 41; and a VL comprising (or represented by) the amino acid sequence of SEQ ID NO: 58.

Preferably, the variable region of the anti-CSF3 antibody may comprise: a VH comprising (or represented by) the amino acid sequence of SEQ ID NO: 42; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 43; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 44; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 45; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 46; and a VL comprising (or represented by) the amino acid sequence of SEQ ID NO: 59.

Preferably, the variable region of the anti-CSF3 antibody may comprise: a VH comprising (or represented by) the amino acid sequence of SEQ ID NO: 47; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 48; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 49; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 50; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 51; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 52; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 53; and a VL comprising (or represented by) the amino acid sequence of SEQ ID NO: 56.

Preferably, the variable region of the anti-CSF3 antibody may comprise: a VH comprising (or represented by) the amino acid sequence of SEQ ID NO: 54; and a VL comprising (or represented by) the amino acid sequence of SEQ ID NO: 60.

Preferably, the variable region of the anti-CSF3 antibody may comprise: a VH comprising (or represented by) the amino acid sequence of SEQ ID NO: 55; and a VL comprising (or represented by) the amino acid sequence of SEQ ID NO: 56.

The following sections <<5.3. Sequences of Constant Regions of Anti-CSF3 Antibody>> and <<5.4. Heavy Chain or Light Chain Sequences of Anti-CSF3 Antibody>> are described assuming that the type of the anti-CSF3 antibody of the present disclosure is IgG1.

Table 3 below lists amino acid sequences that can be included in the CH, CL, heavy chain region, and light chain region of the anti-CSF3 antibody when the type of the anti-CSF3 antibody of the present disclosure is IgG1.

**[Table 3]**

| **Region** | **Amino Acid Sequence (N terminal → C terminal)** |
|---|---|
| CH | ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKT HTCPPCPAPELLGG PSVFLFPPKPKDTLMISRTPEVTCVVVDVSHEDPEVKFN WYVDGVEVHNAKTKPREEQYNSTYRVVSVLTVLHQDWLNGKEYKCKVSNK ALPAPIEKTISKAKGQPREPQVYTLPPSREEMTKNQVSLTCLVKGFYPSDIAVE WESNGQPENNYKTTPPVLDSDGSFFLYSKLTVDKSRWQQGNVFSCSVMHE ALHNHYTQKSLSLSPGK (SEQ ID NO: 61) |
| CL | |
| |  (SEQ ID NO: 62) |
| Heavy chain | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GRDAFDVWGQGTMVTVSSASTKGPSVFPLAPSSKSTSGGTAALGCLVKDYF PEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRTP EVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTK NQVSLTCLVKGFYPSDIAVEWESNGQPENNYKTTPPVLDSDGSFFLYSKLTVD KSRWQQGNVFSCSVMHEALHNHYTQKSLSLSPGK (SEQ ID NO: 63); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GHLAFDVWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 64); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GMSGFDVWGQGTMVTVSSASTKGPSVFPLAPSSKSTSGGTAALGCLVKDYF PEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRTP EVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTK NQVSLTCLVKGFYPSDIAVEWESNGQPENNYKTTPPVLDSDGSFFLYSKLTVD KSRWQQGNVFSCSVMHEALHNHYTQKSLSLSPGK (SEQ ID NO: 65); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GRNGFDVWGQGTMVTVSSASTKGPSVFPLAPSSKSTSGGTAALGCLVKDYF PEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRTP EVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTK NQVSLTCLVKGFYPSDIAVEWESNGQPENNYKTTPPVLDSDGSFFLYSKLTVD KSRWQQGNVFSCSVMHEALHNHYTQKSLSLSPGK (SEQ ID NO: 66); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GTTGFDVWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 67); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GMHPFDVWGQGTMVTVSSASTKGPSVFPLAPSSKSTSGGTAALGCLVKDYF PEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRTP EVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTK NQVSLTCLVKGFYPSDIAVEWESNGQPENNYKTTPPVLDSDGSFFLYSKLTVD KSRWQQGNVFSCSVMHEALHNHYTQKSLSLSPGK (SEQ ID NO: 68); QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG |
| | GTSPFDVWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 69); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GIKPFDVWGQGTMVTVSSASTKGPSVFPLAPSSKSTSGGTAALGCLVKDYFPE PVTVSWNSGALTSGVHTFPAVLQSSGLYSLSSVVTVPSSSLGTQTYICNVNHK PSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLTVDKSR WQQGNVFSCSVMHEALHNHYTQKSLSLSPGK (SEQ ID NO: 70); |
| | EMQLVQSGAEARKPGSSVKVSCKASGYTFTNYGISWLRQTPGQGLEWMG WINPNSGGTIYAQKFQGRVTMTRDTPITTAYMELSTLRSEDTAVYYCSRGSG GAVPFDVWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 71); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GIAPFDVWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNH |
| | KPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 72); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GLLPFDVWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 73); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GMVPFDVWGQGTMVTVSSASTKGPSVFPLAPSSKSTSGGTAALGCLVKDYF PEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRTP EVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTK NQVSLTCLVKGFYPSDIAVEWESNGQPENNYKTTPPVLDSDGSFFLYSKLTVD KSRWQQGNVFSCSVMHEALHNHYTQKSLSLSPGK (SEQ ID NO: 74); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GRSGFDVWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVL |
| | HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 75); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GFQAFDVWGQGTMVTVSSASTKGPSVFPLAPSSKSTSGGTAALGCLVKDYF PEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRTP EVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTK NQVSLTCLVKGFYPSDIAVEWESNGQPENNYKTTPPVLDSDGSFFLYSKLTVD KSRWQQGNVFSCSVMHEALHNHYTQKSLSLSPGK (SEQ ID NO: 76); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GLVPFDVWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 77); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GIRPFDVWGQGTMVTVSSASTKGPSVFPLAPSSKSTSGGTAALGCLVKDYFPE PVTVSWNSGALTSGVHTFPAVLQSSGLYSLSSVVTVPSSSLGTQTYICNVNHK PSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLTVDKSR |
| | WQQGNVFSCSVMHEALHNHYTQKSLSLSPGK (SEQ ID NO: 78); |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GFFPFDVWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 79); or |
| | QMQLVQSGAEARKPGSSVKVSCKASGYTFTNYGISWLRQTPGQGLEWMG WINPNSGGTIYAQKFQGRVTMTRDTSITTAYMELSTLRSEDTAVYYCSRGSG GYSGFDVWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 80) |
| Light chain | DIQMTQSPFSLSASVGDRVTITCRASQTISTYLNWYQHKPGKAPKLFIYYASSL QSGVPSRFSGSGSGTDFALTISSLQPEDFATYYCQQGYSSPRTFGQGTKVEIKR TVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGE C (SEQ ID NO: 81) |
| | DIQMTQSPFSLSASVGDRVTITCRASQTISTYLNWYQHKPGKAPKLFIYYASSL QSGVPSRFSGSGSGTDFALTISSLQPEDFATYYCQQGYSSPRTFGQGTKVEITR TVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGE C (SEQ ID NO: 82) |
| | DIQMTQSPFSLSASVGDRVTITCRASQTTGQYLNWYQHKPGKAPKLFIYYASS LQSGVPSRFSGSGSGTDFALTISSLQPEDFATYYCQQGYSSPRTFGQGTKVEIK RTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGE C (SEQ ID NO: 83) |
| | DIQMTQSPFSLSASVGDRVTITCRASQTISNRLNWYQHKPGKAPKLFIYYASS LQSGVPSRFSGSGSGTDFALTISSLQPEDFATYYCQQGYSSPRTFGQGTKVEIK RTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGE C (SEQ ID NO: 84); or |
| | DIQMTQSPFSLSASVGDRVTITCRASKSISTYLNWYQHKPGKAPKLFIYYASSL QSGVPSRFSGSGSGTDFALTISSLQPEDFATYYCQQGYSSPRTFGQGTKVEIKR TVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGE C (SEQ ID NO: 85) |

### 5.3. Sequences of Constant Regions of Anti-CSF3 Antibody

### Sequence of Heavy Chain Constant Region (CH)

In one embodiment, the CH of the anti-CSF3 antibody may comprise the amino acid sequence of SEQ ID NO: 61, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 61.

In one embodiment, the CH of the anti-CSF3 antibody may comprise the amino acid sequence of SEQ ID NO: 61.

In one embodiment, the CH of the anti-CSF3 antibody may be represented by the amino acid sequence of SEQ ID NO: 61.

### Sequence of Light Chain Constant Region (CL)

In one embodiment, the CL of the anti-CSF3 antibody may comprise the amino acid sequence of SEQ ID NO: 62, or an amino acid sequence having at least 80, 81, 82, 83, 84, 85, 86, 87, 88, 89, 90, 91, 92, 93, 94, 95, 96, 97, 98, or 99% or more sequence similarity to SEQ ID NO: 62.

In another embodiment, the CL of the anti-CSF3 antibody may comprise the amino acid sequence of SEQ ID NO: 62.

In another embodiment, the CL of the anti-CSF3 antibody may be represented by the amino acid sequence of SEQ ID NO: 62.

### 5.4. Heavy Chain or Light Chain Sequences of Anti-CSF3 Antibody

### Sequence of Heavy Chain Region

In one embodiment, the heavy chain region of the anti-CSF3 antibody may comprise any one amino acid sequence of SEQ ID NOs: 63 to 80, or an amino acid sequence having at least 80, 81, 82, 83, 84, 85, 86, 87, 88, 89, 90, 91, 92, 93, 94, 95, 96, 97, 98, or 99% or more sequence similarity to any one sequence of SEQ ID NOs: 63 to 80.

In another embodiment, the heavy chain region of the anti-CSF3 antibody may comprise any one amino acid sequence of SEQ ID NOs: 63 to 80.

In another embodiment, the heavy chain region of the anti-CSF3 antibody may be represented by any one amino acid sequence of SEQ ID NOs: 63 to 80.

### Sequence of Light Chain Region

In one embodiment, the light chain region of the anti-CSF3 antibody may comprise any one amino acid sequence of SEQ ID NOs: 81 to 85, or an amino acid sequence having at least 80, 81, 82, 83, 84, 85, 86, 87, 88, 89, 90, 91, 92, 93, 94, 95, 96, 97, 98, or 99% or more sequence similarity to any one sequence of SEQ ID NOs: 81 to 85.

In another embodiment, the light chain region of the anti-CSF3 antibody may comprise any one amino acid sequence of SEQ ID NOs: 81 to 85.

In another embodiment, the light chain region of the anti-CSF3 antibody may be represented by any one amino acid sequence of SEQ ID NOs: 81 to 85.

### Sequences of Heavy Chain Region and Light Chain Region

In one embodiment, the anti-CSF3 antibody may comprise: a heavy chain region comprising (or represented by) any one amino acid sequence of SEQ ID NOs: 63 to 80; and a light chain region comprising (or represented by) any one amino acid sequence of SEQ ID NOs: 81 to 85.

Preferably, the anti-CSF3 antibody may comprise: a heavy chain region comprising (or represented by) the amino acid sequence of SEQ ID NO: 63; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 64; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 65; and a light chain region comprising (or represented by) the amino acid sequence of SEQ ID NO: 82.

Preferably, the anti-CSF3 antibody may comprise: a heavy chain region comprising (or represented by) the amino acid sequence of SEQ ID NO: 66; and a light chain region comprising (or represented by) the amino acid sequence of SEQ ID NO: 83.

Preferably, the anti-CSF3 antibody may comprise: a heavy chain region comprising (or represented by) the amino acid sequence of SEQ ID NO: 67; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 68; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 69; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 70; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 71; and a light chain region comprising (or represented by) the amino acid sequence of SEQ ID NO: 84.

Preferably, the anti-CSF3 antibody may comprise: a heavy chain region comprising (or represented by) the amino acid sequence of SEQ ID NO: 72; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 73; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 74; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 75; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 76; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 77; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 78; and a light chain region comprising (or represented by) the amino acid sequence of SEQ ID NO: 81.

Preferably, the anti-CSF3 antibody may comprise: a heavy chain region comprising (or represented by) the amino acid sequence of SEQ ID NO: 79; and a light chain region comprising (or represented by) the amino acid sequence of SEQ ID NO: 85.

Preferably, the anti-CSF3 antibody may comprise: a heavy chain region comprising (or represented by) the amino acid sequence of SEQ ID NO: 80; and a light chain region comprising (or represented by) the amino acid sequence of SEQ ID NO: 81.

### 5.5. Composition According to the Type of Anti-CSF3 Antibody

The type of anti-CSF3 antibody of the present disclosure may be any one of IgG, IgA, IgE, IgM, and IgD. Depending on the type of the anti-CSF3 antibody, the structure of the antibody may be any one of monomer, dimer, trimer, and pentamer. That is, the number of heavy chains and light chains of the anti-CSF3 antibody may be any one of 2 heavy chains and 2 light chains, 4 heavy chains and 4 light chains, 6 heavy chains and 6 light chains, and 10 heavy chains and 10 light chains. In this case, the two or more heavy chains comprised in the anti-CSF3 antibody may be identical heavy chains or may be different heavy chains. For example, when the anti-CSF3 antibody comprises 2 heavy chains, 1) both 2 heavy chains may be identical, or 2) the 2 heavy chains may be different from each other. As another example, when the anti-CSF3 antibody comprises 4 heavy chains, 1) all 4 heavy chains may be identical, 2) only 3 heavy chains may be identical, 3) only 2 heavy chains may be identical, or 4) all 4 heavy chains may be different from each other.

### 5.6. Origin of Anti-CSF3 Antibody

### When of Human Origin

In one embodiment, the anti-CSF3 antibody may be a human-derived antibody. Specifically, the anti-CSF3 antibody may be a human antibody. In this case, the fact that the anti-CSF3 antibody is a human antibody means that the anti-CSF3 antibody is an antibody derived from human immunoglobulin. That is, it means that the entire amino acid sequence constituting the anti-CSF3 antibody (all types of CDRs, FRs, CH, CL, and hinge regions, etc.) is composed of amino acid sequences derived from human immunoglobulin.

### When of Non-Human Animal Origin

In one embodiment, the anti-CSF3 antibody may be an antibody derived from a non-human animal. In this case, the fact that the anti-CSF3 antibody is an antibody derived from a non-human animal means that the anti-CSF3 antibody is an antibody derived from the immunoglobulin of a non-human animal. That is, it means that the entire amino acid sequence constituting the anti-CSF3 antibody (all types of CDRs, FRs, CH, CL, and hinge regions, etc.) is composed of amino acid sequences derived from the immunoglobulin of a non-human animal. In one embodiment, the non-human animal from which the anti-CSF3 antibody is derived may be a mammal (e.g., mouse, rabbit, cow, goat, or sheep, etc.).

### When of Both Non-Human Animal and Human Origin

In one embodiment, the anti-CSF3 antibody may be an antibody derived from a human and/or non-human animal.

In one embodiment, the anti-CSF3 antibody may be a chimeric antibody. Specifically, the variable region of the anti-CSF3 antibody may be derived from the immunoglobulin of a non-human animal, and the constant region may be derived from human immunoglobulin.

In one embodiment, the anti-CSF3 antibody may be a humanized antibody. Specifically, the constant region and the FR of the variable region of the anti-CSF3 antibody may be derived from human immunoglobulin, and the CDR of the variable region may be derived from the immunoglobulin of a non-human animal. In this case, some residues of the FR of the variable region may be derived from the immunoglobulin of a non-human animal.

### 5.7. Fragment of Anti-CSF3 Antibody

### 5.7.1. Overview

In one aspect, the present disclosure discloses a fragment of an anti-CSF3 antibody. The fragment of the anti-CSF3 antibody comprises a portion of the anti-CSF3 antibody of the present disclosure, and the fragment may have any form capable of binding to CSF3. Preferably, the fragment of the anti-CSF3 antibody may comprise the antigen-binding portion of the anti-CSF3 antibody. The fragment of the anti-CSF3 antibody comprises a portion of any one of the anti-CSF3 antibodies described in the sections <<5.1. Anti-CSF3 Antibody - Overview>> to <<5.6. Origin of Anti-CSF3 Antibody>> above.

Below, examples of the structure that the fragment of the anti-CSF3 antibody may have are described, but are not limited thereto.

### 5.7.2. Examples of Structures of Fragment of Anti-CSF3 Antibody

In one embodiment, the fragment of the anti-CSF3 antibody may be selected from F(ab), F(ab'), F(ab')2, monospecific F(ab')2, bispecific F(ab')2, single-chain variable fragment (scFv), scFv-Fc, and sdAb.

The F(ab) refers to one having a structure comprising VH, CH1, VL, and CL of an antibody and having only one antigen-binding site.

The F(ab') refers to one having a structure comprising VH, CH1, VL, and CL of an antibody and further having a hinge comprising one or more cysteine residues at the C-terminus of CH1 compared to Fab.

The F(ab')2 refers to two F(ab') fragments connected to each other. In this case, the connection of the two F(ab') fragments is due to a disulfide bond of cysteine residues in the hinge region, and the two connected F(ab') fragments may be identical or different. The monospecific F(ab')2 refers to one in which the antigen-binding portions of the two F(ab') fragments connected in F(ab')2 can bind to the same antigen. The bispecific F(ab')2 refers to one in which the antigen-binding portions of the two F(ab') fragments connected in F(ab')2 can bind to different antigens.

The single-chain variable fragment (scFv) comprises only VH and VL and does not include immunoglobulin domains belonging to the constant region of an antibody (CL, CH1, CH2, CH3, and CH4). In this case, the VH and VL contained in the scFv are generally covalently bonded to each other via a peptide linker. Specifically, the peptide linker connects the C-terminus of VH to the N-terminus of VL, or connects the N-terminus of VH to the C-terminus of VL. When 2 to 3 scFvs are connected, there may be types such as tandem di-scFv, diabody, tandem tri-scFv, and tribody. The scFv-Fc refers to one comprising scFv, CH2, and CH3. Specifically, the scFv-Fc refers to one in which scFv, CH2, and CH3 are connected in that order.

The single-domain antibody (sdAb) refers to one comprising only one of VH and VL, which are the variable regions of an antibody.

### 5.7.3. Artificially Engineered Protein Comprising a Fragment of Anti-CSF3 Antibody

In one aspect, the present disclosure discloses an artificially engineered protein comprising a fragment of the anti-CSF3 antibody. The artificially engineered protein comprises a portion of the anti-CSF3 antibody of the present disclosure, and the artificially engineered protein may have any form capable of binding to CSF3. Preferably, the artificially engineered protein may comprise the antigen-binding portion of the anti-CSF3 antibody. Alternatively, the artificially engineered protein may comprise the CDR regions of the anti-CSF3 antibody. The artificially engineered protein comprises a portion of any one of the anti-CSF3 antibodies described in the sections <5.1. Anti-CSF3 Antibody - Overview> to <5.6. Origin of Anti-CSF3 Antibody> above. In one embodiment, the artificially engineered protein may be selected from F(ab), F(ab'), F(ab')2, monospecific F(ab')2, bispecific F(ab')2, single-chain variable fragment (scFv), scFv-Fc, and sdAb. In one embodiment, the artificially engineered protein may comprise any one selected from F(ab), F(ab'), F(ab')2, monospecific F(ab')2, bispecific F(ab')2, single-chain variable fragment (scFv), scFv-Fc, and sdAb.

### 5.8. Functions of Anti-CSF3 Antibody or Fragment Thereof

### 5.8.1. Function of Binding to CSF3

The anti-CSF3 antibody or fragment thereof of the present disclosure may be an antibody or fragment thereof capable of binding to the CSF3 protein. In one embodiment, the anti-CSF3 antibody or fragment thereof may be an antibody or fragment thereof that specifically binds to CSF3. In one embodiment, the anti-CSF3 antibody may be a monoclonal antibody capable of binding to CSF3. In one embodiment, the anti-CSF3 antibody may be a polyclonal antibody capable of binding to CSF3.

In one embodiment, the anti-CSF3 antibody or fragment thereof may exhibit a higher level of binding to the CSF3 protein compared to binding to other antigens. Specifically, the anti-CSF3 antibody or fragment thereof may exhibit a higher level of binding to the CSF3 protein compared to binding to ITGA6, AITR, and/or CD58.

In another embodiment, the anti-CSF3 antibody or fragment thereof may bind more strongly to the CSF3 protein than to other antigens. Specifically, the anti-CSF3 antibody or fragment thereof may bind more strongly to the CSF3 protein than to ITGA6, AITR, and/or CD58.

### 5.8.2. Function of Inhibiting the Function of CSF3

The anti-CSF3 antibody or fragment thereof of the present disclosure can bind to the CSF3 protein, thereby interfering with the interaction of the CSF3 protein with other molecules, and consequently inhibiting the function of the CSF3 protein. In one embodiment, the anti-CSF3 antibody or fragment thereof can inhibit or interfere with the binding of CSF3 to CSF3R. Specifically, the anti-CSF3 antibody or fragment thereof can inhibit or interfere with the binding of CSF3 to CSF3R by binding to CSF3. In another embodiment, the anti-CSF3 antibody or fragment thereof may be a neutralizing antibody against CSF3 or a fragment thereof.

### 5.8.3. Epitope of CSF3

A specific region of an antigen that allows an antibody to recognize the antigen is called an epitope. In this case, the epitope may be referred to as an antigenic determinant.

The epitope of human CSF3 protein for the anti-CSF3 antibody is at least one of KLCATYKLCH (SEQ ID NO: 87); TLDT (SEQ ID NO: 88); SAFQR (SEQ ID NO: 89); and SAFQRR (SEQ ID NO: 90), which are included in the amino acid sequence constituting the human CSF3 protein (SEQ ID NO: 86).

In one embodiment, the anti-CSF3 antibody can bind to at least one of KLCATYKLCH (SEQ ID NO: 87); TLDT (SEQ ID NO: 88); SAFQR (SEQ ID NO: 89); and SAFQRR (SEQ ID NO: 90), which are included in the amino acid sequence constituting the human CSF3 protein (SEQ ID NO: 86). In this case, KLCATYKLCH (SEQ ID NO: 87) refers to the amino acid sequence composed of the 64th to 73rd residues in the amino acid sequence constituting the human CSF3 protein (SEQ ID NO: 86). In this case, TLDT (SEQ ID NO: 88) refers to the amino acid sequence composed of the 132nd to 135th residues in the amino acid sequence constituting the human CSF3 protein (SEQ ID NO: 86). In this case, SAFQR (SEQ ID NO: 89) refers to the amino acid sequence composed of the 172nd to 176th residues in the amino acid sequence constituting the human CSF3 protein (SEQ ID NO: 86). In this case, SAFQRR (SEQ ID NO: 90) refers to the amino acid sequence composed of the 172nd to 177th residues in the amino acid sequence constituting the human CSF3 protein (SEQ ID NO: 86).

In one embodiment, the anti-CSF3 antibody can bind to at least one of: the region composed of the 64th to 73rd residues; the region composed of the 132nd to 135th residues; the region composed of the 172nd to 176th residues; and the region composed of the 172nd to 177th residues in the amino acid sequence constituting the human CSF3 protein (SEQ ID NO: 86).

In one embodiment, the anti-CSF3 antibody can inhibit or interfere with the binding of CSF3 to CSF3R by binding to the region composed of the 64th to 73rd residues from the N-terminus of the human CSF3 protein (KLCATYKLCH; SEQ ID NO: 87). Specifically, since the region where CSF3R binds to CSF3 is the region composed of the 69th to 83rd residues from the N-terminus of the human CSF3 protein, the binding of CSF3 to CSF3R can be inhibited or interfered with by the anti-CSF3 antibody binding to a region overlapping with the region where CSF3R and CSF3 bind.

In one embodiment, the anti-CSF3 antibody can inhibit or interfere with the binding of CSF3 to CSF3R by binding to the region composed of the 132nd to 135th residues from the N-terminus of the human CSF3 protein (TLDT; SEQ ID NO: 88). Specifically, since the region where CSF3R binds to CSF3 is the region composed of the 138th to 142nd residues from the N-terminus of the human CSF3 protein, the binding of CSF3 to CSF3R can be inhibited or interfered with by the anti-CSF3 antibody binding to a region adjacent to the region where CSF3R and CSF3 bind.

In one embodiment, the anti-CSF3 antibody can inhibit or interfere with the binding of CSF3 to CSF3R by binding to the region composed of the 172nd to 176th residues from the N-terminus of the human CSF3 protein (SAFQR; SEQ ID NO: 89). Specifically, since the region where CSF3R binds to CSF3 is the region composed of the 172nd to 177th residues from the N-terminus of the human CSF3 protein, the binding of CSF3 to CSF3R can be inhibited or interfered with by the anti-CSF3 antibody binding to a region overlapping with the region where CSF3R and CSF3 bind.

In one embodiment, the anti-CSF3 antibody can inhibit or interfere with the binding of CSF3 to CSF3R by binding to the region composed of the 172nd to 177th residues from the N-terminus of the human CSF3 protein (SAFQRR; SEQ ID NO: 90). Specifically, since the region where CSF3R binds to CSF3 is the region composed of the 172nd to 177th residues from the N-terminus of the human CSF3 protein, the binding of CSF3 to CSF3R can be inhibited or interfered with by the anti-CSF3 antibody binding to a region overlapping with the region where CSF3R and CSF3 bind.

In one embodiment, the anti-CSF3 antibody can bind to: the region composed of the 64th to 73rd residues; the region composed of the 132nd to 135th residues; and the region composed of the 172nd to 176th residues in the amino acid sequence constituting the human CSF3 protein (SEQ ID NO: 86).

In one embodiment, the anti-CSF3 antibody can bind to: the region composed of the 64th to 73rd residues; the region composed of the 132nd to 135th residues; and the region composed of the 172nd to 177th residues in the amino acid sequence constituting the human CSF3 protein (SEQ ID NO: 86).

### 5.9. Anti-CSF3 Neutralizing Antibody

In one embodiment, the anti-CSF3 antibody may be an anti-CSF3 neutralizing antibody. The anti-CSF3 neutralizing antibody or a fragment of the neutralizing antibody can bind to the CSF3 protein expressed in a tissue where fibrosis has occurred, thereby interfering with the interaction of the CSF3 protein with other molecules, and consequently inhibiting the function of the CSF3 protein. Here, the fragment of the anti-CSF3 neutralizing antibody refers to a fragment that retains the antigen-binding function, and includes all of: 1) a portion of the amino acid sequence of the anti-CSF3 neutralizing antibody; 2) various combinations of one or more fragments of the anti-CSF3 neutralizing antibody; and 3) combinations of one or more fragments of the anti-CSF3 neutralizing antibody and additionally necessary molecules.

In one embodiment, the anti-CSF3 neutralizing antibody may refer to an anti-CSF3 antibody capable of binding to at least one of: the region composed of the 64th to 73rd residues; the region composed of the 132nd to 135th residues; the region composed of the 172nd to 176th residues; and the region composed of the 172nd to 177th residues in the amino acid sequence constituting the human CSF3 protein (SEQ ID NO: 86).

### 5.10. Use of Anti-CSF3 Antibody or Fragment Thereof

The anti-CSF3 antibody or fragment thereof of the present disclosure can be used in a pharmaceutical composition for treating fibrosis. Specifically, the present disclosure discloses a pharmaceutical composition for treating fibrosis comprising the anti-CSF3 antibody or fragment thereof described in the section <5. Anti-CSF3 Antibody>. In this case, the pharmaceutical composition for treating fibrosis comprising the anti-CSF3 antibody or fragment thereof may be as described in the section <3.9. Pharmaceutical Composition for Treating Fibrosis Comprising CSF3 Inhibitor>.

The anti-CSF3 antibody or fragment thereof of the present disclosure can be used in a method for treating fibrosis. Specifically, the present disclosure discloses a method for treating fibrosis comprising the anti-CSF3 antibody or fragment thereof described in the section <5. Anti-CSF3 Antibody>. In this case, the method for treating fibrosis comprising the anti-CSF3 antibody or fragment thereof may be as described in the section <3.10. Method for Treating Fibrosis Using CSF3 Inhibitor>.

The present disclosure discloses a therapeutic use of the anti-CSF3 antibody or fragment thereof described in the section <5. Anti-CSF3 Antibody> for fibrosis. In this case, the therapeutic use of the anti-CSF3 antibody or fragment thereof for fibrosis may be as described in the section <3.11. Therapeutic Use of CSF3 Inhibitor>.

The present disclosure discloses a use of the anti-CSF3 antibody or fragment thereof described in the section <5. Anti-CSF3 Antibody> for manufacturing a therapeutic agent for fibrosis. In this case, the use of the anti-CSF3 antibody or fragment thereof for manufacturing a therapeutic agent for fibrosis may be as described in the section <3.12. Use of CSF3 Inhibitor for Manufacturing a Therapeutic Agent>.

### 5.11. Nucleic Acid Encoding Antibody or Fragment Thereof and Vector Comprising the Same

The present disclosure discloses a nucleic acid encoding the anti-CSF3 antibody or fragment thereof described in the section <5. Anti-CSF3 Antibody>.

The present disclosure discloses a vector into which the nucleic acid encoding the anti-CSF3 antibody or fragment thereof is inserted. In this case, the vector can amplify or express the inserted nucleic acid, and by expressing the inserted nucleic acid, the anti-CSF3 antibody or fragment thereof can be produced. In one embodiment, the nucleic acid encoding the anti-CSF3 antibody or fragment thereof in the vector is operably linked to a promoter.

In one embodiment, the vector may further comprise an expression regulatory element, a selection element, etc. The expression regulatory element may be a promoter, enhancer, polyadenylation signal, Kozak consensus sequence, ITR (inverted terminal repeat), LTR (long terminal repeat), terminator, internal ribosome entry site (IRES), 2A self-cleaving peptides, or replication origin, etc. The selection element may be a fluorescent protein gene, tag, reporter gene, antibiotic resistance gene, etc.

In one embodiment, the vector may be a plasmid and/or viral vector. In one embodiment, the viral vector may be one selected from the group consisting of retrovirus, lentivirus, adenovirus, adeno-associated virus, vaccinia virus, poxvirus, and herpes simplex virus. In one embodiment, the vector may be a non-viral vector. In one embodiment, the non-viral vector may be one or more selected from the group consisting of plasmid, phage, naked DNA, DNA complex, and mRNA. In one embodiment, the plasmid may be one selected from the group consisting of the pcDNA series, pS456, p326, pACYC177, ColE1, pKT230, pME290, pBR322, pUC8/9, pUC6, pBD9, pHC79, pIJ61, pLAFR1, pHV14, pGEX series, pET series, and pUC19. In one embodiment, the phage may be one selected from the group consisting of λgt4λB, λ-Charon, λΔz1, and M13. In one embodiment, the encoding nucleic acid may be a PCR amplicon.

### Possible Embodiments of the Invention

The following lists possible embodiments of the invention provided in this specification. The following embodiments provided in this paragraph are only examples of the invention. Therefore, the invention provided in this specification cannot be construed as being limited to the following embodiments. The brief descriptions provided along with the embodiment numbers are also only for the convenience of distinguishing between embodiments and cannot be construed as limitations on the invention disclosed in this specification.

### Fibrosis

### Embodiment 1, Fibrosis

At least one disease, disorder, and/or symptom selected from the following:
Fibrosis; and fibrosis-related diseases.

### Embodiment 2, Myofibroblast-Related

In Embodiment 1, the fibrosis is induced by myofibroblasts.

### Embodiment 3, Tissue where Fibrosis Occurs

In any one of Embodiments 1 to 2, the fibrosis occurs in one or more tissues selected from the following:
Skin; brain; nervous system; heart; bone marrow; liver; gut; joint; eye; lung; kidney; retroperitoneum; mediastinum; and pancreas.

### Embodiment 4, Specification of Fibrosis-Related Diseases

In any one of Embodiments 1 to 3, the disease, disorder, and/or symptom is one or more selected from the following:
Hypertrophic scar; systemic sclerosis; multiple cancers; pulmonary arterial hypertension; glial scar; Alzheimer's disease; cardiac fibrosis; hypertrophic cardiomyopathy; cardiac dysfunction; valvular disease; arrhythmia; myelofibrosis; myelodysplastic syndrome; chronic myelogenous leukemia; cirrhosis; portal hypertension; hepatocellular carcinoma; nonalcoholic steatohepatitis (NASH); intestinal fibrosis; enteropathies; inflammatory bowel disease; arthrofibrosis; subretinal fibrosis; epiretinal fibrosis; vision loss; idiopathic pulmonary fibrosis; cystic fibrosis; pulmonary hypertension; thromboembolic disease; emphysema; renal fibrosis; cystic fibrosis; nephrogenic systemic fibrosis; chronic kidney disease; renal anemia; retroperitoneal fibrosis; mediastinal fibrosis; pancreatic fibrosis; cystic fibrosis; chronic pancreatitis; duct obstruction; autoimmune-interstitial lung disease such as ankylosing spondylitis and rheumatoid arthritis; connective tissue disease-interstitial lung disease such as rheumatoid arthritis-interstitial lung disease; and non-idiopathic pulmonary fibrosis-interstitial lung disease.

### CSF3 Inhibitor

### Embodiment 4, CSF3 Inhibitor

CSF3 inhibitor.

### Embodiment 5, Human CSF3 Inhibitor

In Embodiment 4, the CSF3 inhibitor is a human CSF3 inhibitor.

### Embodiment 6, Definition of CSF3 Inhibitor

In any one of Embodiments 4 to 5, the CSF3 inhibitor 1) interferes with the interaction between the CSF3 protein and other molecules, or 2) inhibits the expression of the CSF3 protein to reduce the absolute amount of CSF3 protein interacting with other molecules, thereby consequently interfering with the function of the CSF3 protein.

### Embodiment 7, Examples of CSF3 Inhibitor

In any one of Embodiments 4 to 6, the CSF3 inhibitor is any one selected from: i) a nucleic acid capable of specifically binding to the mRNA transcribed from the CSF3 gene, selected from siRNA (small interfering RNA), shRNA (small hairpin RNA), miRNA (microRNA), and antisense nucleic acid; ii) a compound that functions to prevent the CSF3 protein from interacting with other molecules; iii) a DNA and/or RNA aptamer that functions to prevent the CSF3 protein from interacting with other molecules; and iv) an anti-CSF3 antibody or a fragment of the anti-CSF3 antibody.

### Embodiment 8, Anti-CSF3 Antibody

In Embodiment 7, the amino acid sequences of CDRH1, CDRH2, CDRH3, CDRL1, CDRL2, and CDRL3 of the variable region of the anti-CSF3 antibody are as in any one of the following:
SEQ ID NO: 1, SEQ ID NO: 2, SEQ ID NO: 3, SEQ ID NO: 21, YAS (SEQ ID NO: 25), and SEQ ID NO: 26;
SEQ ID NO: 1, SEQ ID NO: 2, SEQ ID NO: 4, SEQ ID NO: 21, YAS (SEQ ID NO: 25), and SEQ ID NO: 26;
SEQ ID NO: 1, SEQ ID NO: 2, SEQ ID NO: 5, SEQ ID NO: 21, YAS (SEQ ID NO: 25), and SEQ ID NO: 26;
SEQ ID NO: 1, SEQ ID NO: 2, SEQ ID NO: 6, SEQ ID NO: 22, YAS (SEQ ID NO: 25), and SEQ ID NO: 26;
SEQ ID NO: 1, SEQ ID NO: 2, SEQ ID NO: 7, SEQ ID NO: 21, YAS (SEQ ID NO: 25), and SEQ ID NO: 26;
SEQ ID NO: 1, SEQ ID NO: 2, SEQ ID NO: 8, SEQ ID NO: 21, YAS (SEQ ID NO: 25), and SEQ ID NO: 26;
SEQ ID NO: 1, SEQ ID NO: 2, SEQ ID NO: 9, SEQ ID NO: 21, YAS (SEQ ID NO: 25), and SEQ ID NO: 26;
SEQ ID NO: 1, SEQ ID NO: 2, SEQ ID NO: 10, SEQ ID NO: 21, YAS (SEQ ID NO: 25), and SEQ ID NO: 26;
SEQ ID NO: 1, SEQ ID NO: 2, SEQ ID NO: 11, SEQ ID NO: 23, YAS (SEQ ID NO: 25), and SEQ ID NO: 26;
SEQ ID NO: 1, SEQ ID NO: 2, SEQ ID NO: 12, SEQ ID NO: 21, YAS (SEQ ID NO: 25), and SEQ ID NO: 26;
SEQ ID NO: 1, SEQ ID NO: 2, SEQ ID NO: 13, SEQ ID NO: 21, YAS (SEQ ID NO: 25), and SEQ ID NO: 26;
SEQ ID NO: 1, SEQ ID NO: 2, SEQ ID NO: 14, SEQ ID NO: 21, YAS (SEQ ID NO: 25), and SEQ ID NO: 26;
SEQ ID NO: 1, SEQ ID NO: 2, SEQ ID NO: 15, SEQ ID NO: 21, YAS (SEQ ID NO: 25), and SEQ ID NO: 26;
SEQ ID NO: 1, SEQ ID NO: 2, SEQ ID NO: 16, SEQ ID NO: 21, YAS (SEQ ID NO: 25), and SEQ ID NO: 26;
SEQ ID NO: 1, SEQ ID NO: 2, SEQ ID NO: 17, SEQ ID NO: 21, YAS (SEQ ID NO: 25), and SEQ ID NO: 26;
SEQ ID NO: 1, SEQ ID NO: 2, SEQ ID NO: 18, SEQ ID NO: 21, YAS (SEQ ID NO: 25), and SEQ ID NO: 26;
SEQ ID NO: 1, SEQ ID NO: 2, SEQ ID NO: 19, SEQ ID NO: 24, YAS (SEQ ID NO: 25), and SEQ ID NO: 26; and
SEQ ID NO: 1, SEQ ID NO: 2, SEQ ID NO: 20, SEQ ID NO: 21, YAS (SEQ ID NO: 25), and SEQ ID NO: 26.

### CSF3R Inhibitor

### Embodiment 9, CSF3R Inhibitor

CSF3R inhibitor.

### Embodiment 10, Human CSF3R Inhibitor

In Embodiment 9, the CSF3R inhibitor is a human CSF3R inhibitor.

### Embodiment 11, Definition of CSF3R Inhibitor

In any one of Embodiments 9 to 10, the CSF3R inhibitor 1) interferes with the interaction between the CSF3R protein and other molecules, or 2) inhibits the expression of the CSF3R protein to reduce the absolute amount of CSF3R protein interacting with other molecules, thereby consequently interfering with the function of the CSF3R protein.

### Embodiment 12, Examples of CSF3R Inhibitor

In any one of Embodiments 9 to 11, the CSF3R inhibitor is any one selected from: i) a nucleic acid capable of specifically binding to the mRNA transcribed from the CSF3R gene, selected from siRNA (small interfering RNA), shRNA (small hairpin RNA), miRNA (microRNA), and antisense nucleic acid; ii) a compound that functions to prevent the CSF3R protein from interacting with other molecules; iii) a DNA and/or RNA aptamer that functions to prevent the CSF3R protein from interacting with other molecules; and iv) an anti-CSF3R antibody or a fragment of the anti-CSF3R antibody.

### Embodiment 13, Anti-CSF3R Antibody

In Embodiment 12, the amino acid sequences of CDRH1, CDRH2, CDRH3, CDRL1, CDRL2, and CDRL3 of the variable region of the anti-CSF3R antibody are as follows:
SEQ ID NO: 124, SEQ ID NO: 125, SEQ ID NO: 126, SEQ ID NO: 127, SEQ ID NO: 128, and SEQ ID NO: 129.

### Mechanism of Action for Returning Fibrosis-Occurred Tissue to Normal Tissue

### Embodiment 14, TGF-β Mediated Signaling Pathway

TGF-β mediated signaling pathway.

### Embodiment 15, Definition of TGF-β Mediated Signaling Pathway

In Embodiment 14, the TGF-β mediated signaling pathway is a signaling pathway in which fibrosis, epithelial to mesenchymal transition (EMT), fibroblast-to-myofibroblast transdifferentiation (FMT), and/or extracellular matrix (ECM) deposition is induced by the interaction of transforming growth factor beta (TGF-β) and the TGF-β receptor.

### Embodiment 16, Effects of TGF-β Mediated Signaling Pathway

In any one of Embodiments 14 to 15, the TGF-β mediated signaling pathway induces fibrosis, epithelial to mesenchymal transition, fibroblast-to-myofibroblast transdifferentiation, and/or extracellular matrix deposition, or fibrosis, epithelial to mesenchymal transition, fibroblast-to-myofibroblast transdifferentiation, and/or extracellular matrix deposition is induced by activation of the TGF-β mediated signaling pathway.

### Embodiment 17, Markers Related to TGF-β Mediated Signaling Pathway

In any one of Embodiments 14 to 16, the TGF-β mediated signaling pathway increases the expression level of at least one of CSF3, CSF3R, TGF-β, α-SMA, COL1A1, and IL-11, or the expression level of at least one of CSF3, CSF3R, TGF-β, α-SMA, COL1A1, and IL-11 is increased by activation of the TGF-β mediated signaling pathway.

### Embodiment 18, Non-TGF-β Mediated Signaling Pathway

Non-TGF-β mediated signaling pathway.

### Embodiment 19, Definition 1 of Non-TGF-β Mediated Signaling Pathway

In Embodiment 18, the non-TGF-β mediated signaling pathway is a signaling pathway separate from the TGF-β mediated signaling pathway that is activated by the interaction of TGF-β and the TGF-β receptor, in which fibrosis, epithelial to mesenchymal transition (EMT), fibroblast-to-myofibroblast transdifferentiation (FMT), and/or extracellular matrix (ECM) deposition is induced by CSF3.

### Embodiment 20, Definition 2 of Non-TGF-β Mediated Signaling Pathway

In any one of Embodiments 18 to 19, the non-TGF-β mediated signaling pathway is a signaling pathway in which fibrosis, EMT, FMT, and/or extracellular matrix deposition is induced by the interaction of CSF3 and CSF3R.

### Embodiment 21, Name of Non-TGF-β Mediated Signaling Pathway

In any one of Embodiments 18 to 20, the non-TGF-β mediated signaling pathway is a CSF3-associated and non-TGF-β-mediated signaling pathway.

### Embodiment 22, Effects of Non-TGF-β Mediated Signaling Pathway

In any one of Embodiments 18 to 21, the non-TGF-β mediated signaling pathway induces fibrosis, epithelial to mesenchymal transition, fibroblast-to-myofibroblast transdifferentiation, and/or extracellular matrix deposition, or fibrosis, epithelial to mesenchymal transition, fibroblast-to-myofibroblast transdifferentiation, and/or extracellular matrix deposition is induced by activation of the non-TGF-β mediated signaling pathway.

### Embodiment 23, Markers Related to Non-TGF-β Mediated Signaling Pathway

In any one of Embodiments 18 to 23, the non-TGF-β mediated signaling pathway increases the expression level of at least one of CSF3, CSF3R, TGF-β, α-SMA, COL1A1, and IL-11, or the expression level of at least one of CSF3, CSF3R, TGF-β, α-SMA, COL1A1, and IL-11 is increased by activation of the non-TGF-β mediated signaling pathway.

### Embodiment 24, Positive-Feedback Loop of CSF3

Positive-feedback loop of CSF3.

### Embodiment 25, Definition of Positive-Feedback Loop of CSF3

In Embodiment 24, the positive-feedback loop of CSF3 is a signaling pathway in which the expression of CSF3 is further induced by an increase in CSF3.

### Embodiment 26, Mechanism of Positive-Feedback Loop of CSF3

In Embodiment 25, the signaling pathway in which the expression of CSF3 is further induced by an increase in CSF3 is activated through the following processes:
the TGF-β mediated signaling pathway of any one of Embodiments 14 to 17 and/or the non-TGF-β mediated signaling pathway of any one of Embodiments 18 to 23 is activated by the increased CSF3;
the expression level of CSF3 and/or TGF-β is increased by the activated TGF-β mediated signaling pathway and/or the activated non-TGF-β mediated signaling pathway.

### Embodiment 27, Positive-Feedback Loop of CSF3R

Positive-feedback loop of CSF3R.

### Embodiment 28, Definition of Positive-Feedback Loop of CSF3R

In Embodiment 27, the positive-feedback loop of CSF3R is a signaling pathway in which the expression of CSF3R is further induced by an increase in CSF3R.

### Embodiment 29, Mechanism of Positive-Feedback Loop of CSF3R

In Embodiment 28, the signaling pathway in which the expression of CSF3R is further induced by an increase in CSF3R is activated through the following processes:
the TGF-β mediated signaling pathway of any one of Embodiments 14 to 17 and/or the non-TGF-β mediated signaling pathway of any one of Embodiments 18 to 23 is activated by the increased CSF3R;
the expression level of CSF3R and/or TGF-β is increased by the activated TGF-β mediated signaling pathway and/or the activated non-TGF-β mediated signaling pathway.

### Embodiment 30, Positive-Feedback Loop of TGF-β

Positive-feedback loop of TGF-β.

### Embodiment 31, Definition of Positive-Feedback Loop of TGF-β

In Embodiment 30, the positive-feedback loop of TGF-β is a signaling pathway in which the expression of TGF-β is further induced by an increase in TGF-β.

### Embodiment 32, Mechanism of Positive-Feedback Loop of TGF-β

In Embodiment 31, the signaling pathway in which the expression of TGF-β is further induced by an increase in TGF-β is activated through the following processes:
the TGF-β mediated signaling pathway of any one of Embodiments 14 to 17 is activated by the increased TGF-β;
the expression level of CSF3 and/or TGF-β is increased by the activated TGF-β mediated signaling pathway.

### Embodiment 33, Reciprocal Positive-Feedback Loop Between CSF3 and TGF-β

Positive-feedback loop between CSF3 and TGF-β.

### Embodiment 34, Definition of Reciprocal Positive-Feedback Loop Between CSF3 and TGF-β

In Embodiment 33, the reciprocal positive-feedback loop between CSF3 and TGF-β is a signaling pathway in which the expression of TGF-β is induced by an increase in CSF3, and the expression of CSF3 is induced by an increase in TGF-β; or a signaling pathway in which the expression of CSF3 is induced by an increase in TGF-β, and the expression of TGF-β is induced by an increase in CSF3.

### Embodiment 35, Mechanism of Reciprocal Positive-Feedback Loop Between CSF3 and TGF-β

In Embodiment 34, the reciprocal positive-feedback loop between CSF3 and TGF-β is activated through the mechanism of Embodiment 29, the mechanism of Embodiment 32, or a combination of the mechanism of Embodiment 29 and the mechanism of Embodiment 32.

### Embodiment 36, Method for Inhibiting Signaling Pathways

A method for inhibiting, reducing, blocking, suppressing, and/or decreasing at least one of: the TGF-β mediated signaling pathway of any one of Embodiments 14 to 17; the non-TGF-β mediated signaling pathway of any one of Embodiments 18 to 23; the positive-feedback loop of CSF3 of any one of Embodiments 24 to 26; the positive-feedback loop of CSF3R of any one of Embodiments 27 to 29; the positive-feedback loop of TGF-β of any one of Embodiments 30 to 32; and the reciprocal positive-feedback loop between CSF3 and TGF-β of any one of Embodiments 33 to 35, wherein the method comprises:
administering to a cell, tissue, or subject the CSF3 inhibitor of any one of Embodiments 4 to 8 or the CSF3R inhibitor of any one of Embodiments 9 to 13.

### Embodiment 37, Method for Inhibiting ECM Deposition and EMT

A method for inhibiting, reducing, blocking, suppressing, and/or decreasing extracellular matrix deposition and epithelial to mesenchymal transition (EMT) in a tissue of a subject, wherein the method comprises:
administering to the subject the CSF3 inhibitor of any one of Embodiments 4 to 8 or the CSF3R inhibitor of any one of Embodiments 9 to 13.

### Embodiment 38, Mechanism of Method for Inhibiting ECM Deposition and EMT

In Embodiment 37, the tissue is a tissue at risk of ECM deposition and EMT, and the CSF3 inhibitor or the CSF3R inhibitor inhibits, reduces, blocks, suppresses, and/or decreases at least one of: the TGF-β mediated signaling pathway of any one of Embodiments 14 to 17; the non-TGF-β mediated signaling pathway of any one of Embodiments 18 to 23; the positive-feedback loop of CSF3 of any one of Embodiments 24 to 26; the positive-feedback loop of CSF3R of any one of Embodiments 27 to 29; the positive-feedback loop of TGF-β of any one of Embodiments 30 to 32; and the reciprocal positive-feedback loop between CSF3 and TGF-β of any one of Embodiments 33 to 35.

### Pharmaceutical Composition for Treating Fibrosis

### Embodiment 39, Pharmaceutical Composition

A pharmaceutical composition for preventing and/or treating the disease, disorder, and/or symptom of any one of Embodiments 1 to 4, comprising:
a therapeutically effective amount of a CSF3 inhibitor or CSF3R inhibitor; and
a pharmaceutically acceptable carrier.

### Embodiment 40, Types of CSF3 Inhibitor or CSF3R Inhibitor

In Embodiment 39, the CSF3 inhibitor is the CSF3 inhibitor of any one of Embodiments 4 to 8, and the CSF3R inhibitor is the CSF3R inhibitor of any one of Embodiments 9 to 13.

### Embodiment 41, Types of Pharmaceutically Acceptable Carriers

In any one of Embodiments 39 to 40, the pharmaceutically acceptable carrier is at least one selected from the following:
binders such as lactose, saccharose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol wax; sweeteners; flavorings; syrups; liquid carriers such as fatty oils; sterile aqueous solutions; propylene glycol; polyethylene glycol; injectable esters such as ethyl oleate; suspensions; emulsions; lyophilized preparations; external preparations; stabilizers; buffers; animal oils; vegetable oils; waxes; paraffin; starch; tragacanth; cellulose derivatives; polyethylene glycol; silicone; bentonite; silica; talc; zinc oxide; and appropriate combinations thereof.

### Embodiment 42, Therapeutic Mechanism 1

In any one of Embodiments 39 to 41, the CSF3 inhibitor or the CSF3R inhibitor inactivates myofibroblasts in the tissue where fibrosis has occurred.

### Embodiment 43, Therapeutic Mechanism 2

In Embodiment 42, inactivating myofibroblasts in the tissue where fibrosis has occurred means at least one selected from the following:
(a) decomposing, reducing, degrading, lowering, removing, and/or decreasing the extracellular matrix secreted by myofibroblasts;
(b) inhibiting, reducing, blocking, suppressing, and/or decreasing the production of extracellular matrix by myofibroblasts; and
(c) reducing, killing, removing, and/or decreasing myofibroblasts in the tissue where fibrosis has occurred.

### Embodiment 44, Therapeutic Mechanism 3

In any one of Embodiments 39 to 43, the CSF3 inhibitor or the CSF3R inhibitor induces the tissue where fibrosis has occurred to return to a normal tissue.

### Embodiment 45, Therapeutic Mechanism 4

In any one of Embodiments 39 to 44, the CSF3 inhibitor or the CSF3R inhibitor inhibits at least one of: the TGF-β mediated signaling pathway of any one of Embodiments 14 to 17; the non-TGF-β mediated signaling pathway of any one of Embodiments 18 to 23; the positive-feedback loop of CSF3 of any one of Embodiments 24 to 26; the positive-feedback loop of CSF3R of any one of Embodiments 27 to 29; the positive-feedback loop of TGF-β of any one of Embodiments 30 to 32; and the reciprocal positive-feedback loop between CSF3 and TGF-β of any one of Embodiments 33 to 35.

### Method for Treating Fibrosis

### Embodiment 46, Method for Treating Fibrosis

A method for preventing and/or treating the disease, disorder, and/or symptom of any one of Embodiments 1 to 4, comprising:
administering, injecting, introducing, and/or delivering a therapeutically effective amount of a CSF3 inhibitor, a therapeutically effective amount of a CSF3R inhibitor, or the pharmaceutical composition of any one of Embodiments 39 to 45 to a subject,
wherein the CSF3 inhibitor is the CSF3 inhibitor of any one of Embodiments 4 to 8, and the CSF3R inhibitor is the CSF3R inhibitor of any one of Embodiments 9 to 13.

### Embodiment 47, Therapeutic Mechanism 1

In Embodiment 46, the CSF3 inhibitor or the CSF3R inhibitor inactivates myofibroblasts in the tissue where fibrosis has occurred, and
inactivating myofibroblasts in the tissue where fibrosis has occurred means at least one selected from the following:
(a) decomposing, reducing, degrading, lowering, removing, and/or decreasing the extracellular matrix secreted by myofibroblasts;
(b) inhibiting, reducing, blocking, suppressing, and/or decreasing the production of extracellular matrix by myofibroblasts; and
(c) reducing, killing, removing, and/or decreasing myofibroblasts in the tissue where fibrosis has occurred.

### Embodiment 48, Therapeutic Mechanism 2

In any one of Embodiments 46 to 47, the CSF3 inhibitor or the CSF3R inhibitor induces the tissue where fibrosis has occurred to return to a normal tissue.

### Embodiment 49, Therapeutic Mechanism 3

In any one of Embodiments 46 to 48, the CSF3 inhibitor or the CSF3R inhibitor inhibits at least one of: the TGF-β mediated signaling pathway of any one of Embodiments 14 to 17; the non-TGF-β mediated signaling pathway of any one of Embodiments 18 to 23; the positive-feedback loop of CSF3 of any one of Embodiments 24 to 26; the positive-feedback loop of CSF3R of any one of Embodiments 27 to 29; the positive-feedback loop of TGF-β of any one of Embodiments 30 to 32; and the reciprocal positive-feedback loop between CSF3 and TGF-β of any one of Embodiments 33 to 35.

### Therapeutic Use of CSF3 Inhibitor for Fibrosis

### Embodiment 50, Use of CSF3 Inhibitor

A CSF3 inhibitor for preventing and/or treating the disease, disorder, and/or symptom of any one of Embodiments 1 to 4.

### Embodiment 51, CSF3 Inhibitor

In Embodiment 50, the CSF3 inhibitor is the CSF3 inhibitor of any one of Embodiments 4 to 8.

### Embodiment 52, Therapeutic Mechanism 1

In any one of Embodiments 50 to 51, the CSF3 inhibitor inactivates myofibroblasts in the tissue where fibrosis has occurred, and
inactivating myofibroblasts in the tissue where fibrosis has occurred means at least one selected from the following:
(a) decomposing, reducing, degrading, lowering, removing, and/or decreasing the extracellular matrix secreted by myofibroblasts;
(b) inhibiting, reducing, blocking, suppressing, and/or decreasing the production of extracellular matrix by myofibroblasts; and
(c) reducing, killing, removing, and/or decreasing myofibroblasts in the tissue where fibrosis has occurred.

### Embodiment 53, Therapeutic Mechanism 2

In any one of Embodiments 50 to 52, the CSF3 inhibitor induces the tissue where fibrosis has occurred to return to a normal tissue.

### Embodiment 54, Therapeutic Mechanism 3

In any one of Embodiments 50 to 53, the CSF3 inhibitor inhibits at least one of: the TGF-β mediated signaling pathway of any one of Embodiments 14 to 17; the non-TGF-β mediated signaling pathway of any one of Embodiments 18 to 23; the positive-feedback loop of CSF3 of any one of Embodiments 24 to 26; the positive-feedback loop of CSF3R of any one of Embodiments 27 to 29; the positive-feedback loop of TGF-β of any one of Embodiments 30 to 32; and the reciprocal positive-feedback loop between CSF3 and TGF-β of any one of Embodiments 33 to 35.

### Embodiment 55, Use Claim

Use of the CSF3 inhibitor of any one of Embodiments 50 to 54 for preventing and/or treating the disease, disorder, and/or symptom of any one of Embodiments 1 to 4.

### Use of CSF3 Inhibitor for Manufacturing a Therapeutic Agent for Fibrosis

### Embodiment 56, Use for Manufacturing a Therapeutic Agent

Use of a CSF3 inhibitor for manufacturing a medicament for treating the disease, disorder, and/or symptom of any one of Embodiments 1 to 4.

### Embodiment 57, CSF3 Inhibitor

In Embodiment 48, the CSF3 inhibitor is the CSF3 inhibitor of any one of Embodiments 4 to 8.

### Embodiment 58, Therapeutic Mechanism 1

In any one of Embodiments 56 to 57, the CSF3 inhibitor inactivates myofibroblasts in the tissue where fibrosis has occurred, and
inactivating myofibroblasts in the tissue where fibrosis has occurred means at least one selected from the following:
(a) decomposing, reducing, degrading, lowering, removing, and/or decreasing the extracellular matrix secreted by myofibroblasts;
(b) inhibiting, reducing, blocking, suppressing, and/or decreasing the production of extracellular matrix by myofibroblasts; and
(c) reducing, killing, removing, and/or decreasing myofibroblasts in the tissue where fibrosis has occurred.

### Embodiment 59, Therapeutic Mechanism 2

In any one of Embodiments 56 to 58, the CSF3 inhibitor induces the tissue where fibrosis has occurred to return to a normal tissue.

### Embodiment 60, Therapeutic Mechanism 3

In any one of Embodiments 56 to 59, the CSF3 inhibitor inhibits at least one of: the TGF-β mediated signaling pathway of any one of Embodiments 14 to 17; the non-TGF-β mediated signaling pathway of any one of Embodiments 18 to 23; the positive-feedback loop of CSF3 of any one of Embodiments 24 to 26; the positive-feedback loop of CSF3R of any one of Embodiments 27 to 29; the positive-feedback loop of TGF-β of any one of Embodiments 30 to 32; and the reciprocal positive-feedback loop between CSF3 and TGF-β of any one of Embodiments 33 to 35.

### Therapeutic Use of CSF3R Inhibitor for Fibrosis

### Embodiment 61, Use of CSF3R Inhibitor

A CSF3R inhibitor for preventing and/or treating the disease, disorder, and/or symptom of any one of Embodiments 1 to 4.

### Embodiment 62, CSF3R Inhibitor

In Embodiment 61, the CSF3R inhibitor is the CSF3R inhibitor of any one of Embodiments 9 to 13.

### Embodiment 63, Therapeutic Mechanism 1

In any one of Embodiments 61 to 62, the CSF3R inhibitor inactivates myofibroblasts in the tissue where fibrosis has occurred, and
inactivating myofibroblasts in the tissue where fibrosis has occurred means at least one selected from the following:
(a) decomposing, reducing, degrading, lowering, removing, and/or decreasing the extracellular matrix secreted by myofibroblasts;
(b) inhibiting, reducing, blocking, suppressing, and/or decreasing the production of extracellular matrix by myofibroblasts; and
(c) reducing, killing, removing, and/or decreasing myofibroblasts in the tissue where fibrosis has occurred.

### Embodiment 64, Therapeutic Mechanism 2

In any one of Embodiments 61 to 63, the CSF3R inhibitor induces the tissue where fibrosis has occurred to return to a normal tissue.

### Embodiment 65, Therapeutic Mechanism 3

In any one of Embodiments 61 to 64, the CSF3R inhibitor inhibits at least one of: the TGF-β mediated signaling pathway of any one of Embodiments 14 to 17; the non-TGF-β mediated signaling pathway of any one of Embodiments 18 to 23; the positive-feedback loop of CSF3 of any one of Embodiments 24 to 26; the positive-feedback loop of CSF3R of any one of Embodiments 27 to 29; the positive-feedback loop of TGF-β of any one of Embodiments 30 to 32; and the reciprocal positive-feedback loop between CSF3 and TGF-β of any one of Embodiments 33 to 35.

### Embodiment 66, Use Claim

Use of the CSF3R inhibitor of any one of Embodiments 61 to 65 for preventing and/or treating the disease, disorder, and/or symptom of any one of Embodiments 1 to 4.

### Use of CSF3R Inhibitor for Manufacturing a Therapeutic Agent for Fibrosis

### Embodiment 67, Use for Manufacturing a Therapeutic Agent

Use of a CSF3R inhibitor for manufacturing a medicament for treating the disease, disorder, and/or symptom of any one of Embodiments 1 to 4.

### Embodiment 68, CSF3R Inhibitor

In Embodiment 67, the CSF3R inhibitor is the CSF3R inhibitor of any one of Embodiments 9 to 13.

### Embodiment 69, Therapeutic Mechanism 1

In any one of Embodiments 67 to 68, the CSF3R inhibitor inactivates myofibroblasts in the tissue where fibrosis has occurred, and
inactivating myofibroblasts in the tissue where fibrosis has occurred means at least one selected from the following:
(a) decomposing, reducing, degrading, lowering, removing, and/or decreasing the extracellular matrix secreted by myofibroblasts;
(b) inhibiting, reducing, blocking, suppressing, and/or decreasing the production of extracellular matrix by myofibroblasts; and
(c) reducing, killing, removing, and/or decreasing myofibroblasts in the tissue where fibrosis has occurred.

### Embodiment 70, Therapeutic Mechanism 2

In any one of Embodiments 67 to 69, the CSF3R inhibitor induces the tissue where fibrosis has occurred to return to a normal tissue.

### Embodiment 71, Therapeutic Mechanism 3

In any one of Embodiments 67 to 70, the CSF3R inhibitor inhibits at least one of: the TGF-β mediated signaling pathway of any one of Embodiments 14 to 17; the non-TGF-β mediated signaling pathway of any one of Embodiments 18 to 23; the positive-feedback loop of CSF3 of any one of Embodiments 24 to 26; the positive-feedback loop of CSF3R of any one of Embodiments 27 to 29; the positive-feedback loop of TGF-β of any one of Embodiments 30 to 32; and the reciprocal positive-feedback loop between CSF3 and TGF-β of any one of Embodiments 33 to 35.

The invention provided in this specification will be described in more detail below through experimental examples. It will be apparent to those of ordinary skill in the art that these experimental examples are only intended to illustrate the content disclosed in this specification and that the scope of the content disclosed in this specification is not to be construed as being limited by these experimental examples.

### Experimental Example 1. Experimental Methods and Materials

### Experimental Example 1.1. Animal Experiments

The mice used in the experimental examples of this application were 7-week-old male C57BL/6 mice weighing approximately 20±2 g, purchased from Orient Bio. After confirming individual monitoring data and appearance from the supplier, the animals were acclimatized in a quarantine room for approximately 1 week, and only healthy individuals were used for testing following observation of general symptoms. Animal care was maintained by auto controller according to the standards of the Seoul Women's University Laboratory Animal Facility, at a temperature of 22±1°C, humidity of 50±10%, and lighting cycle of 12 Light: 12 Dark, and sterilized feed was provided along with sterilized water. Bleomycin sulfate (Bleomycin Sulfate; B1141000) used for fibrosis model preparation was purchased from Sigma-Aldrich. In the experimental examples of this application, the CSF3 antibody treated on mice or mouse tissues refers to the CSF3-4E5 antibody. Information on the CSF3-4E5 antibody is described in Experimental Example 3.2.1 of this application.

### Experimental Example 1.2. Cell Culture

Human normal lung fibroblast cell line (MRC5) was purchased from American Type Culture Collection (ATCC, U.S.); human skin fibroblasts (HDF, human foreskin fibroblasts) were purchased from STEMGENT; human hepatic stellate cell line (LX-2, Human Hepatic Stellate Cell Line) was purchased from Sigma-Aldrich; lung fibroblasts isolated from the lungs of pulmonary fibrosis patients (DHLF-IPF, Diseased Human Lung Fibroblasts, Idiopathic Pulmonary Fibrosis) were purchased from Lonza (Lonza, U.S.); and normal human renal proximal tubule epithelial cells (HK2) were purchased from the Korean Cell Line Bank. MRC5, HDF, LX-2, and DHLF-IPF cells were cultured in DMEM media supplemented with penicillin-streptomycin (Gibco, U.S.) and 10% heat-inactivated fetal bovine serum (Gibco, U.S.; heat-inactivated at 56°C for 30 minutes) in a temperature- and humidity-controlled incubator (Sanyo, Japan) maintained at 5% CO2 and 37°C. HK2 cells were cultured in RPMI 1640 media supplemented with 1% penicillin-streptomycin (Gibco, U.S.) and 10% heat-inactivated fetal bovine serum (Gibco, U.S.; heat-inactivated at 56°C for 30 minutes) in a temperature- and humidity-controlled incubator (Sanyo, Japan) maintained at 5% CO2 and 37°C. Each cell type was seeded in culture plates at a density of 3×10⁵ cells. Bleomycin sulfate was treated at a dose of 2 µg/mL. Recombinant Human TGF-beta 1 Protein (TGF-β) was treated at a dose of 2 ng/mL. Recombinant Human Granulocyte colony-stimulating factor (CSF3) was treated at 200 ng/mL, and cells were harvested after 24 or 48 hours. Collagen levels were measured by performing hydroxyproline assay on the harvested cells, protein changes were confirmed by Western blot, and gene changes were confirmed using qPCR. In the experimental examples of this application, the anti-CSF3 antibody treated on human-derived cells refers to the Y41 antibody. Information on the Y41 antibody is described in Experimental Example 4.3 or Experimental Example 6.2.1 of this application. In the experimental examples of this application, the anti-CSF3R antibody treated on human-derived cells refers to a monoclonal antibody that specifically binds to human CSF3R protein, and the sequence information of the anti-CSF3R antibody used is as follows.

The sequence of CDRH1 of the anti-CSF3R antibody is GFIFSNYA (SEQ ID NO: 124).

The sequence of CDRH2 of the anti-CSF3R antibody is INGNGGVT (SEQ ID NO: 125).

The sequence of CDRH3 of the anti-CSF3R antibody is ARESAMYGMDV (SEQ ID NO: 126).

The sequence of CDRL1 of the anti-CSF3R antibody is QSILTY (SEQ ID NO: 127).

The sequence of CDRL2 of the anti-CSF3R antibody is AAS (SEQ ID NO: 128).

The sequence of CDRL3 of the anti-CSF3R antibody is QQSYSSPYS (SEQ ID NO: 129).

The sequence of the heavy chain variable region of the anti-CSF3R antibody is

QMQLVQSGGGLVQPGGSLRLSCAASGFIFSNYAMNWVRQAPGKGLEWVSTINGNGGVTYYADSV
KGRFTISRDNSKNSLYLQMNSLRAEDTAVYYCARESAMYGMDVWGQGTTVTVSS (SEQ ID NO:
130).

The sequence of the light chain variable region of the anti-CSF3R antibody is

DIVMTQTPLSLSASVGDRLTITCRASQSILTYLNWYQQKPGKAPKLLVYAASSLQRGVPSRFSGRGSG
TDFTLTISGLQPDDFALYYCQQSYSSPYSFGPGTKVDIK (SEQ ID NO: 131).

The heavy chain sequence of the anti-CSF3R antibody is

QMQLVQSGGGLVQPGGSLRLSCAASGFIFSNYAMNWVRQAPGKGLEWVSTINGNGGVTYYADSV
KGRFTISRDNSKNSLYLQMNSLRAEDTAVYYCARESAMYGMDVWGQGTTVTVSSASTKGPSVFPLA
PSSKSTSGGTAALGCLVKDYFPEPVTVSWNSGALTSGVHTFPAVLQSSGLYSLSSVVTVPSSSLGTQTY

ICNVNHKPSNTKVDKKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGK (SEQ ID NO: 132).

The light chain sequence of the anti-CSF3R antibody is

DIVMTQTPLSLSASVGDRLTITCRASQSILTYLNWYQQKPGKAPKLLVYAASSLQRGVPSRFSGRGSG
TDFTLTISGLQPDDFALYYCQQSYSSPYSFGPGTKVDIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPV
TKSFNRGEC (SEQ ID NO: 133).

### Experimental Example 1.3. Hydroxyproline Assay for Measuring Collagen Levels

Collagen levels were measured by performing a hydroxyproline assay on cells harvested after treatment with bleomycin sulfate, TGF-β, or CSF3. The experimental procedure involved dissolving the harvested cells in 6N HCl, followed by acid hydrolysis at 110°C for 24 hours. Absorbance (OD = 560 nm) was measured using 500 µL each of Oxidant Sol. and Ehrlich's Sol.

### Experimental Example 1.4. Tissue Staining

Tissues fixed in 10% neutral formalin were paraffin-embedded and sectioned at a thickness of 4 µm, then subjected to hematoxylin and eosin (H&E) staining, Masson's trichrome staining, and immunostaining (α-SMA, collagen 1) to observe tissue changes. Alpha smooth muscle actin (α-SMA) and Collagen I antibodies were purchased from Abcam (Cambridge, UK).

### Experimental Example 1.5. Western Blot

For Western blot, 20 µg of protein was electrophoresed on a 10% SDS-polyacrylamide gel. After protein separation, transfer to a nitrocellulose membrane was performed at 100 V for 1 hour, and the membrane was washed with 1X PBST (2.67 mM potassium chloride, 1.47 mM potassium phosphate monobasic, 137.93 mM sodium chloride, 8.1 mM sodium phosphate dibasic anhydrous, 0.1% Tween 20) and blocked with 5% skim milk (BD, U.S.) for 30 minutes. Primary antibody was diluted 1:1,000 in 1X PBST and incubated at 4°C overnight. The reacted membrane was washed with 1X PBST, then incubated with secondary antibody diluted 1:10,000 in 1X PBST at room temperature for 1 hour. Proteins were detected using ECL solution (PerkinElmer, U.S.) and developed on AGFA film (Agfa, Belgium).

### Experimental Example 1.6. Gene Expression Analysis (qPCR)

Total RNA was extracted from tissues (or cells) using an RNA minikit (Qiagen, Inc.), and then converted to cDNA using Accupower RT mix reagent (Bioneer Corp., Seoul, Korea). Real-time PCR was performed using FastStart Essential DNA Green Master (Roche, Indianapolis, IN, USA). The primer sequences used for qPCR analysis are as shown in Table 4 below.

**[Table 4]**

| **Gene** | **Primer sequence (5'-3') for qPCR** |
|---|---|
| α-SMA | AATCCCATCACCATCTTCCAG (SEQ ID NO: 100) |
| | GCAGTGGCCATCTCATTTTCA (SEQ ID NO: 101) |
| Collagen 1A1 | CCTGGATGCCATCAAAGTCT (SEQ ID NO: 102) |
| | AATCCATCGGTCATGCTCTC (SEQ ID NO: 103) |
| CSF3 | GCTGCTTGAGCCAACTCCAT (SEQ ID NO: 104) |
| | CATTCCCAGTTCTTCCATCTGCT (SEQ ID NO: 105) |
| CSF3R | CAGATACGCTGCATCCGCT (SEQ ID NO: 106) |
| | CACTGGCTTCCAGAACAGCT (SEQ ID NO: 107) |
| TGF-β | CCCAGCATCTGCAAAGCTC (SEQ ID NO: 108) |
| | GTCAATGTACAGCTGCCGCA (SEQ ID NO: 109) |
| IL-11 | ACAGCTGAGGGACAAATTCC (SEQ ID NO: 110) |
| | AGCACACCTGGGAGCTGTA (SEQ ID NO: 111) |
| GAPDH | AATCCCATCACCATCTTCCAG (SEQ ID NO: 112) |
| | AAATGAGCCCCAGCCTTC (SEQ ID NO: 113) |

### Experimental Example 1.7. Immunofluorescence Staining

Cultured cells were fixed in 4% neutral formalin, reacted with primary antibodies (α-SMA, collagen 1), and then reacted with secondary antibodies conjugated with fluorescent materials. Fluorescence images were captured and analyzed using a fluorescence microscope (Olympus, Shinjuku, Tokyo, Japan).

### Experimental Example 1.8. Invasion Assay

Cell invasiveness was examined in a transwell cell culture chamber with an 8 µm pore size polyvinylpyrrolidone (PVP)-free polycarbonate filter pre-coated with Matrigel. First, the inside of the chamber was coated with Matrigel. Then, cells were prepared in serum-free medium at a concentration of 5×10⁴ cells/mL, and 100 µL was added to the inside of the chamber, while 700 µL of medium containing 10% FBS as a nutrient to induce cell infiltration was added to the outside of the chamber, and the cells were cultured at 37°C for 24 hours. Non-invaded cells on the upper part of the filter were removed with a cotton swab, and cells that had invaded through the polycarbonate filter were stained with crystal violet. The number of invaded cells was quantified by counting stained cells on the outside of the chamber using a microscope (×100).

### Experimental Example 2. Mechanisms of Action of CSF3 Inhibitor and CSF3R Inhibitor

### Experimental Example 2.1. Induction of Fibrosis by CSF3

Normal lung fibroblasts (MRC5) were treated with CSF3 recombinant protein at 50, 100, and 200 ng/mL, and after 24 hours, the gene expression and protein expression levels of fibrosis markers α-SMA, Col1A, and Fibronectin were observed, and the activation of Stat3, a key signaling factor, was confirmed at the protein level. The expression of each gene was confirmed by qPCR, and Figure 1 shows the qPCR results. The expression of each protein was confirmed by Western blot, and Figure 2 shows the Western blot results. In addition, immunofluorescence staining was performed to directly confirm α-SMA and Col1A protein expression in cells treated with CSF3 recombinant protein. α-SMA and Col1A1 were fluorescently stained in green to confirm the expression levels of these proteins. Figure 3 shows the immunofluorescence staining results. In addition, a hydroxyproline assay was performed, and it was confirmed that collagen secretion and accumulation were increased in the group treated with CSF3 recombinant protein in normal lung fibroblasts. Figure 4 shows the hydroxyproline assay results.

As a result of the experiment, it was confirmed that in the group of normal lung fibroblasts treated with CSF3 recombinant protein, the protein expression levels of α-SMA, Col1A, Fibronectin, Stat3, and/or β-actin were increased, and the gene expression levels were also increased. In addition, these results demonstrate that fibrosis is induced when CSF3 protein is treated.

### Experimental Example 2.2. Increase in TGF-β Expression and Activation of TGF-β Mediated Signaling Pathway by CSF3

Normal lung fibroblasts (MRC5) were treated with CSF3 recombinant protein at 50, 100, and 200 ng/mL, and after 24 hours, the mRNA expression level of TGF-β (a cytokine known to be important for fibrosis induction), the phosphorylation level of SMAD3 protein (p-SMAD3), and the secretion level of TGF-β protein were confirmed. The mRNA expression level was confirmed by qPCR, and Figure 5 shows the qPCR results. The phosphorylation level of SMAD3 protein (p-SMAD3) was confirmed by Western blot, and Figure 6 shows the Western blot results. The TGF-β protein secretion level was confirmed by TGF-β ELISA assay in cell culture medium, and Figure 7 shows the TGF-β ELISA assay results.

As a result of the experiment, it was confirmed that in the group of normal lung fibroblasts treated with CSF3 recombinant protein, the mRNA expression of TGF-β was increased and the phosphorylation of SMAD3 protein (p-SMAD3), indicating TGF-β signaling activity, was increased. In addition, it was confirmed that TGF-β protein secretion was significantly increased in the group treated with CSF3 recombinant protein.

These experimental results demonstrate that CSF3 (or an increase in CSF3 protein) increases the expression of TGF-β, a cytokine important for fibrosis induction, and accelerates fibrosis induction by activating the TGF-β mediated signaling pathway.

### Experimental Example 2.3. Inhibition of TGF-β Expression and TGF-β Mediated Signaling Pathway by CSF3 Inhibition

CSF3 was inhibited by treating lung fibroblasts derived from pulmonary fibrosis patients (DHLF-IPF) and lung fibroblasts isolated from bleomycin-induced pulmonary fibrosis mouse lung tissue (BLM-MLF) with CSF3 siRNA or a CSF3 antibody. After inhibiting CSF3, the mRNA expression level of TGF-β (a cytokine known to be important for fibrosis induction) and the phosphorylation level of SMAD3 protein (p-SMAD3) were confirmed. The mRNA expression level was confirmed by qPCR, and Figures 8 and 9 show the qPCR results. The phosphorylation level of SMAD3 protein (p-SMAD3) was confirmed by Western blot, and Figures 10 and 11 show the Western blot results.

As a result of the experiment, it was confirmed that inhibiting CSF3 using CSF3 siRNA or a CSF3 antibody significantly reduced the expression level of TGF-β and the phosphorylation of SMAD3 protein (p-SMAD3), which indicates TGF-β signaling activity.

These experimental results demonstrate that inhibiting CSF3 can suppress fibrosis induction by inhibiting the expression of TGF-β, a cytokine important for fibrosis induction, and suppressing the activation of the TGF-β mediated signaling pathway.

### Experimental Example 2.4. Inhibition of TGF-β Expression and TGF-β Mediated Signaling Pathway by CSF3R Inhibition

Normal lung fibroblasts (MRC5) were treated with 200 ng/mL of CSF3 recombinant protein, and after 24 hours, fibrosis induction was confirmed. To verify whether this phenomenon is induced by the interaction of CSF3 and CSF3R, CSF3R was inhibited using siRNA against CSF3R or a CSF3R antibody. After inhibiting CSF3R, the mRNA expression levels of fibrosis markers α-SMA, Col1A, and FN, as well as CSF3, CSF3R, TGF-β, and IL11, were confirmed. The mRNA expression level was confirmed by qPCR, and Figures 12 and 13 show the qPCR results. The protein expression levels of α-SMA and Col1A1 were confirmed by Western blot, and Figure 14 shows the Western blot results. Immunofluorescence staining was performed to confirm the expression of α-SMA and Col1A, and Figure 15 shows the immunofluorescence staining results. The TGF-β protein secretion level in the culture medium was confirmed by TGF-β ELISA assay, and Figure 16 shows the TGF-β ELISA assay results.

The experimental results demonstrate that when CSF3 recombinant protein is treated, the expression levels of α-SMA, Col1A1, FN, CSF3, CSF3R, TGF-β, and IL11 are elevated. This indicates that CSF3 induces the activation of the TGF-β mediated signaling pathway, which can cause fibrosis.

The experimental results confirm that, compared to the group treated only with CSF3 recombinant protein, the expression levels of α-SMA, Col1A1, FN, CSF3, CSF3R, TGF-β, and IL11 were lower in the group treated with CSF3 recombinant protein together with siRNA against CSF3R or a CSF3R antibody. This demonstrates that the interaction (or binding) of CSF3 and CSF3R induces activation of the TGF-β mediated signaling pathway that can cause fibrosis. In addition, it shows that inhibiting CSF3R can inhibit the TGF-β mediated signaling pathway, and that inhibiting the interaction (or binding) of CSF3 and CSF3R can inhibit the TGF-β mediated signaling pathway.

### Experimental Example 2.5. Upstream Action of CSF3-Mediated Signaling Pathway on TGF-β Mediated Signaling Pathway

Normal lung fibroblasts (MRC5) were treated with: CSF3 recombinant protein; CSF3 recombinant protein and siRNA against CSF3R; or CSF3 recombinant protein and siRNA against TGF-β receptor 2 (TGF-βRII). After 24 hours, the mRNA expression levels of fibrosis markers α-SMA and Col1A, and of CSF3, TGF-β, IL11, CSF3R, and TGF-βRII were confirmed. The mRNA expression level was confirmed by qPCR, and Figure 17 shows the qPCR results. In addition, the TGF-β protein secretion level in the culture medium was confirmed by TGF-β ELISA assay, and Figure 18 shows the TGF-β ELISA assay results.

The experimental results confirmed that, compared to the group treated with siRNA against TGF-βRII, the expression levels of α-SMA, Col1A1, CSF3, TGF-β, IL11, and CSF3R were lower in the group treated with siRNA against CSF3R. These results indicate that inhibiting CSF3R suppresses not only the TGF-β mediated signaling pathway that induces fibrosis but also another signaling pathway that induces fibrosis (i.e., the non-TGF-β mediated signaling pathway).

The experimental results confirmed that, compared to the control group, the expression level of TGF-β was significantly lower in the group treated with siRNA against CSF3R, whereas the expression levels of CSF3 and CSF3R were not significantly lower in the group treated with siRNA against TGF-βRII. These results indicate that, with respect to fibrosis induction, the CSF3-mediated signaling pathway acts upstream of the TGF-β mediated signaling pathway.

The experimental results confirmed that the gene expression of fibrosis markers α-SMA and Col1A1 decreased in the group in which the TGF-β mediated signaling pathway was inhibited using siRNA against TGF-βRII. These results indicate that the TGF-β mediated signaling pathway activated by CSF3 can influence the CSF3-mediated signaling pathway.

### Experimental Example 2.6. Relationship Between TGF-β and CSF3 in Fibrosis Induction

Lung fibroblasts were treated with: TGF-β; TGF-β and a CSF3 antibody; or TGF-β and a CSF3R antibody. After 24 hours, the mRNA expression levels of fibrosis markers α-SMA, Col1A1, and FN, as well as CSF3, TGF-β, IL11, and CSF3R, were confirmed. The mRNA expression level was confirmed by qPCR, and Figure 19 shows the qPCR results. In addition, immunofluorescence staining was performed to confirm the expression of α-SMA and Col1A, and Figure 20 shows the immunofluorescence staining results.

The experimental results confirmed that the expression levels of α-SMA, Col1A1, CSF3, TGF-β, and IL11 were increased in the group treated with TGF-β. In addition, compared to the group treated with TGF-β alone, the expression levels of α-SMA, Col1A1, CSF3, TGF-β, and IL11 were lower in the group additionally treated with an anti-CSF3 antibody or an anti-CSF3R antibody. These results indicate that the TGF-β signaling pathway and the CSF3 signaling pathway interact with each other to induce fibrosis, and that fibrosis can be treated by inhibiting the CSF3 signaling pathway using the anti-CSF3 antibody or the anti-CSF3 receptor antibody.

In addition, lung fibroblasts were treated with: TGF-β; TGF-β and si-CSF3#1; or TGF-β and si-CSF3#2. After 24 hours, the mRNA expression levels of fibrosis markers α-SMA, Col1A1, and FN, as well as CSF3, TGF-β, IL11, and CSF3R, were confirmed. The mRNA expression level was confirmed by qPCR, and Figure 91 shows the qPCR results. Here, si-CSF3#1 and si-CSF3#2 each refer to siRNA against CSF3 (siRNA having a nucleic acid sequence complementary to the nucleic acid sequence of the mRNA encoding CSF3). si-CSF3#1 and si-CSF3#2 correspond to siRNA 1 and siRNA 2 in Table 5, respectively. From the experimental results with si-CSF3 treatment, it was confirmed that the expression levels of α-SMA, Col1A1, FN, CSF3, TGF-β, and IL11 were increased in the group treated with TGF-β. In addition, compared to the group treated with TGF-β alone, the expression levels of α-SMA, Col1A1, FN, CSF3, TGF-β, and IL11 were lower in the group additionally treated with si-CSF3. These results indicate that the TGF-β signaling pathway and the CSF3 signaling pathway interact with each other to induce fibrosis, and that fibrosis can be treated by inhibiting the CSF3 signaling pathway using a CSF3 inhibitor (e.g., siRNA against CSF3).

As described above, the results of Experimental Example 2.5 indicate that inhibiting CSF3R suppresses not only the TGF-β mediated signaling pathway that induces fibrosis but also another signaling pathway that induces fibrosis (i.e., the non-TGF-β mediated signaling pathway). In this context, from the results of Experimental Example 2.6, when comparing the group treated with an anti-CSF3 antibody and the group treated with an anti-CSF3R antibody, it was confirmed that the expression levels of α-SMA, Col1A1, CSF3, TGF-β, and IL11 were similar, or the expression levels were even lower in the group treated with the anti-CSF3 antibody. Therefore, the results of Experimental Example 2.5 indicate that inhibiting CSF3 suppresses not only the TGF-β mediated signaling pathway that induces fibrosis but also another signaling pathway that induces fibrosis (i.e., the non-TGF-β mediated signaling pathway). That is, these results more strongly support the conclusion that the CSF3-mediated signaling pathway acts upstream of the TGF-β mediated signaling pathway with respect to fibrosis induction.

In addition, the results of Experimental Example 2.5 confirmed that the gene expression of fibrosis markers α-SMA and Col1A1 decreased in the group in which the TGF-β mediated signaling pathway was inhibited using siRNA against TGF-βRII. These results indicate that the TGF-β mediated signaling pathway activated by CSF3 can influence the CSF3-mediated signaling pathway.

### Experimental Example 2.7. Inhibition of CSF3-Mediated Signaling Pathway

Lung fibroblasts were treated with: CSF3 recombinant protein (200 ng/mL); or CSF3 recombinant protein (200 ng/mL) and a STAT3 inhibitor (STAT3 Inhibitor, WP 1066, 10 µM). After 24 hours, the mRNA expression levels of fibrosis markers α-SMA, Col1A1, and FN, as well as CSF3, TGF-β, and IL11, were confirmed. The mRNA expression level was confirmed by qPCR, and Figure 89 shows the qPCR results. In addition, Western blot was performed to confirm the expression of α-SMA, Col1A, and FN, and Figure 90 shows the Western blot results.

As a result of the experiment, it was confirmed that in the group of normal lung fibroblasts treated with CSF3 recombinant protein, the gene and protein expression of pulmonary fibrosis markers α-SMA and Col1A1 were increased, whereas in the group treated with both CSF3 recombinant protein and a Stat3 inhibitor, the gene and protein expression of α-SMA and Col1A1 were significantly decreased. This indicates that inhibiting the CSF3-mediated signaling pathway can inhibit the TGF-β mediated signaling pathway.

In addition, the results of Experimental Examples 2.2 to 2.6 demonstrate that inhibiting the CSF3-mediated signaling pathway can inhibit the positive-feedback loop of CSF3 and the positive-feedback loop of TGF-β. That is, inhibiting CSF3 or CSF3R can inhibit the positive-feedback loop of CSF3 and the positive-feedback loop of TGF-β.

### Experimental Example 3. Therapeutic Effects of CSF3 Inhibitor and CSF3R Inhibitor on Fibrosis

The results of Experimental Examples 2.1 to 2.7 demonstrate not only the mechanisms of action of CSF3 inhibitor and CSF3R inhibitor, but also the therapeutic effects of CSF3 inhibitor and CSF3R inhibitor on pulmonary fibrosis. Accordingly, the inventors of the present application conducted the experiments of Experimental Example 3 to verify whether CSF3 inhibitor and CSF3R inhibitor have therapeutic effects on fibrosis regardless of the type of tissue in which fibrosis has occurred, and as a result confirmed that CSF3 inhibitor and CSF3R inhibitor have therapeutic effects independent of the type of tissue in which fibrosis has occurred.

### Experimental Example 3.1. Experiment 1 on Therapeutic Effects on Pulmonary Fibrosis

### Experimental Example 3.1.1. CSF3 Inhibition in Activated Lung Fibroblasts Derived from Pulmonary Fibrosis Patients

Activated lung fibroblasts (IPF) derived from pulmonary fibrosis patients were treated with siRNA against CSF3 or an anti-CSF3 antibody to inhibit CSF3, and then changes in fibrosis marker expression and changes in the invasive ability of the activated lung fibroblasts were confirmed.

The mRNA expression levels of α-SMA, Col1A1, FN, and CSF3 were confirmed by qPCR, and Figure 21 shows the qPCR results. Immunofluorescence staining was performed to confirm the expression of α-SMA and Col1A, and Figure 22 shows the immunofluorescence staining results. Changes in the invasive ability of the activated lung fibroblasts were observed through a transwell cell culture chamber, and Figure 23 shows the invasion assay results.

The experimental results confirmed that inhibiting CSF3 in fibrosis-induced cells reduced the expression levels of fibrosis markers α-SMA and Col1A1. In addition, when lung fibroblasts are converted to myofibroblasts due to fibrosis, their invasive ability increases; however, it was confirmed that inhibiting CSF3 significantly reduced the invasive ability. These results demonstrate that CSF3 is an important cytokine in fibrosis induction. In addition, it indicates that inhibiting CSF3 can suppress fibrosis.

### Experimental Example 3.1.2. CSF3 Inhibition in Activated Lung Fibroblasts Derived from Mice with Bleomycin-Induced Pulmonary Fibrosis

Activated fibroblasts (hereinafter referred to as 'BLM-MLF') were isolated and cultured from lung tissue of mice in which pulmonary fibrosis was induced by bleomycin. The cultured BLM-MLF were treated with siRNA against CSF3 or an anti-CSF3 antibody to inhibit CSF3, and then changes in fibrosis marker expression and changes in the invasive ability of the activated lung fibroblasts were confirmed.

The mRNA expression levels of α-SMA, Col1A1, FN, and CSF3 were confirmed by qPCR, and Figure 24 shows the qPCR results. Immunofluorescence staining was performed to confirm the expression of α-SMA and Col1A, and Figure 25 shows the immunofluorescence staining results. Changes in the invasive ability of the activated lung fibroblasts were observed through a transwell cell culture chamber, and Figure 26 shows the invasion assay results.

The experimental results confirmed that inhibiting CSF3 in fibrosis-induced cells reduced the expression levels of fibrosis markers α-SMA and Col1A1. In addition, when lung fibroblasts are converted to myofibroblasts due to fibrosis, their invasive ability increases; however, it was confirmed that inhibiting CSF3 significantly reduced the invasive ability. These results demonstrate that CSF3 is an important cytokine in fibrosis induction. In addition, it indicates that inhibiting CSF3 can suppress fibrosis.

### Experimental Example 3.2. Experiment 2 on Therapeutic Effects on Pulmonary Fibrosis

### Experimental Example 3.2.1. Overview of Animal Experiment Method for Pulmonary Fibrosis

To confirm the therapeutic effects on pulmonary fibrosis, a mouse model was used. The mouse models used are as follows: a negative control mouse model not treated with bleomycin; and a BLM-induced pulmonary fibrosis mouse model in which pulmonary fibrosis was induced by injecting bleomycin (BLM, 2 mg/kg, 1 time) into the mouse airway.

To verify the fibrosis therapeutic effects of the antibody, CSF3-4E5 was intraperitoneally injected at 2 mg/kg at 2-day intervals for 4 times starting from day 5 after pulmonary fibrosis was induced by BLM in the BLM-induced pulmonary fibrosis mouse model. On day 14 of the experiment, the mice were necropsied, the lungs were excised, and pathological analysis of lung tissue and analysis of gene expression of pulmonary fibrosis markers were performed. The experimental method for verifying the fibrosis therapeutic effects of the antibody is shown in Figure 27. The specific details of experiments using the mouse model and experimental methods described in Experimental Example 3.2.1 are described in Experimental Examples 3.2.2 to 3.2.4 below.

The CSF3-4E5 used in the experiments of the present application refers to an antibody capable of binding to mouse CSF3 protein. The CSF3-4E5 was prepared as a surrogate antibody for a monoclonal antibody (Y41 antibody) that specifically binds to human CSF3 protein. Information on the monoclonal antibody (Y41 antibody) that specifically binds to human CSF3 protein is described in Experimental Example 6. The CSF3-4E5 used in the experiments was an antibody manufactured by AbClon Inc., and the sequence information of CSF3-4E5 is as follows.

The sequence of CDRH1 of CSF3-4E5 is GYTFTSYD (SEQ ID NO: 91).

The sequence of CDRH2 of CSF3-4E5 is IYPGDGST (SEQ ID NO: 92).

The sequence of CDRH3 of CSF3-4E5 is ARGGWFAY (SEQ ID NO: 93).

The sequence of CDRL1 of CSF3-4E5 is QSLLNSRTRKNY (SEQ ID NO: 94).

The sequence of CDRL2 of CSF3-4E5 is WAS (SEQ ID NO: 95).

The sequence of CDRL3 of CSF3-4E5 is KQSYNLYT (SEQ ID NO: 96).

The sequence of the heavy chain variable region of CSF3-4E5 is

QVQLKQSGPELVKPGALVKISCKASGYTFTSYDINWVKQRPGQGLEWIGWIYPGDGSTKYNEKFKGK
ATLTADKSSSTAYMQLSSLTSENSAVYFCARGGWFAYWGQGTLVTVSA (SEQ ID NO: 97).

The sequence of the light chain variable region of CSF3-4E5 is

DIVMTQSPSSLAVSAGEKVTMSCKSSQSLLNSRTRKNYLAWYQQKPGQSPKLLIYWASTRESGVPDR
FTGSGSGTDFTLTISSVQAEDLAVYYCKQSYNLYTFGGGTKLEIK (SEQ ID NO: 98).

### Experimental Example 3.2.2. Analysis of Morphological Changes in the Lung and Collagen Accumulation Levels

Paraffin blocks were prepared from the lung tissue examined in Experimental Example 3.2.1, and H&E staining for analyzing morphological changes in the lung tissue and Masson's trichrome staining for confirming collagen accumulation were performed to confirm the degree of pulmonary fibrosis.

As a result of staining, compared to the negative control group (PBS), the positive control group treated only with bleomycin (BLM) showed a higher level of lung tissue deformation and increased collagen accumulation. In addition, compared to the positive control group treated only with BLM, the experimental group additionally treated with CSF3-4E5 (BLM_+CSF3-4E5) showed a lower level of lung tissue deformation and decreased collagen accumulation.

These H&E staining and Masson's trichrome staining results for lung tissue confirmed that the degree of bleomycin-induced pulmonary fibrosis was significantly reduced by CSF3-4E5 treatment, and the results are shown in Figures 28 and 29.

### Experimental Example 3.2.3. Hydroxyproline Content Measurement Results

To indirectly analyze collagen levels, the inventors measured hydroxyproline contents. As a result of the measurement, it was confirmed that the collagen level was higher in the positive control group treated only with BLM (BLM) compared to the negative control group (PBS), and the collagen level was lower in the experimental group additionally treated with CSF3-4E5 (BLM+4E5) compared to the positive control group treated only with BLM. The result graph is shown in Figure 30. Here, G1-1, G1-2, G1-3, G1-4, and G1-5 in Figure 30 each refer to negative control group (PBS) mice not treated with BLM. G2-1, G2-3, and G2-5 in Figure 30 each refer to positive control group (BLM) mice treated only with BLM. G3-1, G3-2, and G3-3 in Figure 30 each refer to experimental group (BLM+4E5) mice additionally treated with CSF3-4E5.

### Experimental Example 3.2.4. Confirmation of Pulmonary Fibrosis Marker Expression

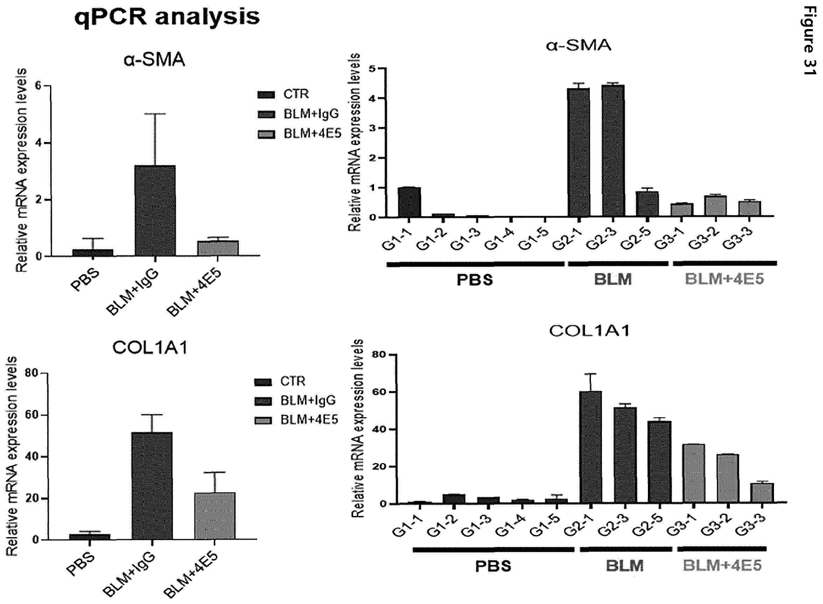

The inventors confirmed the gene expression levels of pulmonary fibrosis markers α-SMA and Col1A1 in mouse lung tissue. Specifically, the expression of α-SMA and Col1A1 genes was measured through qPCR analysis. As a result, it was confirmed that the expression levels of α-SMA and Col1A1 genes were higher in the positive control group treated only with BLM compared to the negative control group (PBS), and the expression levels of α-SMA and Col1A1 genes were markedly lower in the experimental group treated with CSF3-4E5 (BLM+4E5) compared to the positive control group. The result graph is shown in Figure 31. Here, G1-1, G1-2, G1-3, G1-4, and G1-5 in Figure 31 each refer to negative control group (PBS) mice not treated with BLM. G2-1, G2-3, and G2-5 in Figure 31 each refer to positive control group (BLM) mice treated only with BLM. G3-1, G3-2, and G3-3 in Figure 31 each refer to experimental group (BLM+4E5) mice additionally treated with CSF3-4E5.

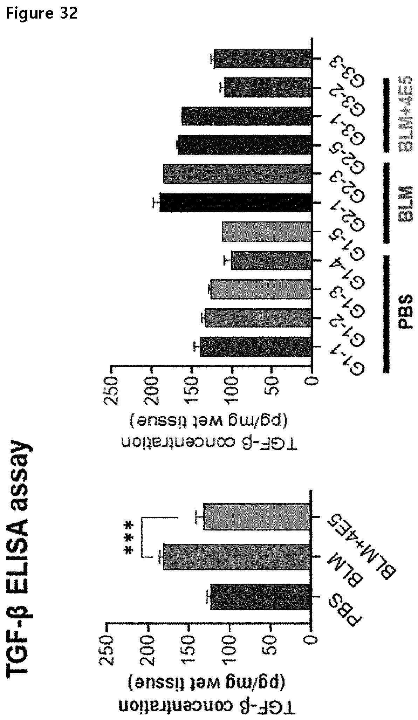

In addition, the inventors confirmed the secretion level of TGF-β, a key factor in pulmonary fibrosis induction, by ELISA assay on mouse lung tissue. As a result of the analysis, it was confirmed that TGF-β secretion was higher in the positive control group treated only with BLM compared to the negative control group, and was significantly lower in the experimental group treated with CSF3-4E5 (BLM+4E5) compared to the positive control group. The result graph is shown in Figure 32. Here, G1-1, G1-2, G1-3, G1-4, and G1-5 in Figure 32 each refer to negative control group (PBS) mice not treated with BLM. G2-1, G2-3, and G2-5 in Figure 32 each refer to positive control group (BLM) mice treated only with BLM. G3-1, G3-2, and G3-3 in Figure 32 each refer to experimental group (BLM+4E5) mice additionally treated with CSF3-4E5.

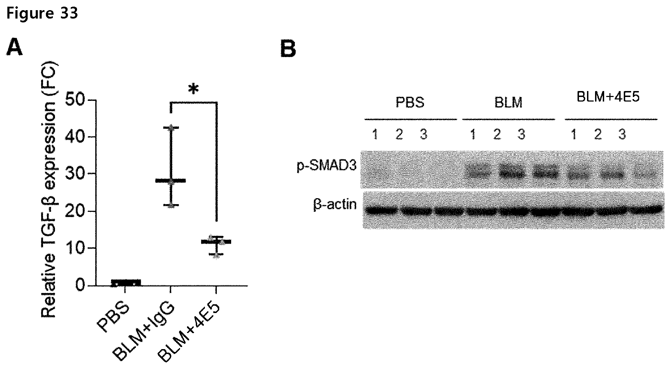

In addition, the inventors confirmed TGF-β gene expression (a key factor in pulmonary fibrosis induction) and TGF-β signaling activity in mouse lung tissue through qPCR of the TGF-β gene and Western blot analysis of SMAD3 protein phosphorylation (p-SMAD3). As a result of the analysis, it was confirmed that TGF-β gene expression was higher in the positive control group treated only with BLM compared to the negative control group, and was significantly lower in the experimental group treated with CSF3-4E5 (BLM+4E5) compared to the positive control group. In addition, as a result of analyzing the phosphorylation of SMAD3 protein (p-SMAD3) indicating TGF-β signaling activity, it was confirmed that p-SMAD3 expression was higher in the positive control group treated only with BLM compared to the negative control group, and was significantly lower in the experimental group treated with CSF3-4E5 (BLM+4E5) compared to the positive control group. The result graph is shown in Figure 33. Here, G1-1, G1-2, G1-3, G1-4, and G1-5 in Figure 33 each refer to negative control group (PBS) mice not treated with BLM. G2-1, G2-3, and G2-5 in Figure 33 each refer to positive control group (BLM) mice treated only with BLM. G3-1, G3-2, and G3-3 in Figure 33 each refer to experimental group (BLM+4E5) mice additionally treated with CSF3-4E5.

The results of Experimental Examples 3.2.2 to 3.2.4 demonstrate that pulmonary fibrosis can be treated with a CSF3 inhibitor.

### Experimental Example 3.3. Experiment 1 on Therapeutic Effects on Skin Fibrosis

### Experimental Example 3.3.1. Confirmation of Skin Fibrosis Markers

Human skin fibroblasts HDF (human foreskin fibroblasts) were used for experiments on skin fibrosis. Skin fibrosis was induced by treating skin fibroblasts with TGF-β for 48 hours, and changes in the expression of fibrosis markers α-SMA and Col1A1 protein and gene expression were observed. At this time, the skin fibroblasts were divided into a Cont group treated only with PBS, a TGF-β group treated only with TGF-β, and a TGF-β+CSF3 Ab group treated with both TGF-β and a CSF3 antibody, and changes in protein expression and gene expression in each group were observed.

The expression of each protein was confirmed by Western blot (Figure 34, left), and α-SMA was fluorescently stained in green and Col1A1 in red to confirm the expression levels of these proteins (Figure 34, right). In addition, the expression levels of α-SMA and Col1A1 in each group were confirmed by qPCR (Figure 35).

As a result of the experiment, it was confirmed that in the TGF-β group to which TGF-β was added to skin fibroblasts, the protein expression levels of α-SMA and Col1A1 were increased, and the gene expression levels were also increased. This indicates that fibrosis is induced when TGF-β is added. On the other hand, in the group co-administered with a CSF3 antibody, it was confirmed that both the protein and gene expression levels of α-SMA and Col1A1 were significantly suppressed, showing results similar to the normal state (Cont group). This indicates that administering an anti-CSF3 antibody to cells where fibrosis has occurred has the effect of inhibiting myofibroblast differentiation, collagen secretion and accumulation, or restoring these to the normal tissue state.

### Experimental Example 3.3.2. Confirmation of Effects of Anti-CSF3 Antibody

Skin fibrosis was induced by treating skin fibroblasts with TGF-β for 48 hours, and the gene expression levels of cytokines related to inflammation were confirmed using qPCR. At this time, the skin fibroblasts were divided into a Cont group treated only with PBS, a TGF-β group treated only with TGF-β, and a TGF-β+CSF3 Ab group treated with both TGF-β and an anti-CSF3 antibody, and changes in protein expression and gene expression in each group were observed. Specifically, changes in gene expression of TGF-β, CTGF, and inflammation-related TNF-α, IL8, and IL11 in skin fibroblasts were measured (Figure 36).

As a result of the experiment, it was confirmed that when TGF-β was treated to skin fibroblasts, the gene expression levels of TGF-β, CTGF, and inflammation-related TNF-α, IL8, and IL11 were increased.

On the other hand, when a CSF3 antibody was co-treated, it was confirmed that the gene expression levels of TGF-β, CTGF, and inflammation-related TNF-α, IL8, and IL11 were significantly suppressed.

This indicates that the anti-CSF3 antibody can reduce the expression of TGF-β, a key signaling factor in skin fibrosis induction, and consequently inhibit the signaling of important inflammatory cytokines such as CTGF and TNF-α, IL8, and IL11, thereby exerting a therapeutic effect on skin fibrosis.

### Experimental Example 3.3.3. Confirmation of Effects of Anti-CSF3R Antibody

Skin fibrosis was induced by treating skin fibroblasts with TGF-β for 48 hours, and the mRNA expression levels of fibrosis markers α-SMA, Col1A1, and FN, as well as TGF-β, IL11, CSF3, and CSF3R, were observed. At this time, the skin fibroblasts were divided into an HDF group treated only with PBS, a TGF-β group treated only with TGF-β, and a TGF-β+CSF3R Ab group treated with both TGF-β and an anti-CSF3R antibody, and changes in gene expression in each group were observed.

The gene expression was confirmed by qPCR for the expression levels of α-SMA, Col1A1, TGF-β, IL11, CSF3, and CSF3R in each group (Figure 37).

As a result of the experiment, it was confirmed that in the TGF-β group to which TGF-β was added to skin fibroblasts, the mRNA expression levels of α-SMA, Col1A1, FN, TGF-β, IL11, CSF3, and CSF3R were increased. This indicates that fibrosis is induced when TGF-β is added. On the other hand, in the group co-administered with a CSF3R antibody, it was confirmed that the mRNA expression levels of α-SMA, Col1A1, FN, TGF-β, IL11, CSF3, and CSF3R were all significantly suppressed. This indicates that administering an anti-CSF3R antibody to cells where fibrosis has occurred has the effect of inhibiting myofibroblast differentiation, collagen secretion and accumulation, or restoring these to the normal tissue state.

### Experimental Example 3.3.4. Confirmation of CSF3 Expression Inhibitory Effect of siRNA Against CSF3

Skin fibroblasts were used to compare the experimental group treated only with bleomycin and the experimental group treated with both bleomycin and siRNA against CSF3 (siRNA having a nucleic acid sequence complementary to the nucleic acid sequence of the mRNA encoding CSF3), in order to verify the therapeutic effect of siRNA against CSF3 on skin fibrosis.

First, siRNA against CSF3 was used at a concentration of 100 nM to inhibit gene expression in skin fibroblasts, and then Western blot was performed to confirm whether CSF3 protein expression was also inhibited.

The mRNA sequence of the human CSF3 gene is
atggctggacctgccacccagagccccatgaagctgatggccctgcagctgctgctgtggcacagtgcactctggacagtgc aggaagccacccccctgggccctgccagctccctgccccagagcttcctgctcaagtgcttagagcaagtgaggaagatcca gggcgatggcgcagcgctccaggagaagctgtgtgccacctacaagctgtgccaccccgaggagctggtgctgctcggac actctctgggcatcccctgggctcccctgagcagctgccccagccaggccctgcagctggcaggctgcttgagccaactccat agcggccttttcctctaccaggggctcctgcaggccctggaagggatctcccccgagttgggtcccaccttggacacactgca gctggacgtcgccgactttgccaccaccatctggcagcagatggaagaactgggaatggcccctgccctgcagcccacccag ggtgccatgccggccttcgcctctgctttccagcgccgggcaggaggggtcctggttgcctcccatctgcagagcttcctgga ggtgtcgtaccgcgttctacgccaccttgcccagccctga (SEQ ID NO: 99), and the siRNA sequences used to silence it are as shown in Table 5.

**[Table 5]**

| **Gene Name** | **Gene ID/ Accession no.** | **siRNA name** | **Primer sequence (5'-3') for qPCR** |
|---|---|---|---|
| CSF3 | NM_172219.3 | siRNA 1 | Sense: GUUUGACUCCCGAACAUCAdTdT (SEQ ID NO: 114) |
| | | | Antisense: UGAUGUUCGGGAGUCAAACdTdT (SEQ ID N O: 115) |
| CSF3 | NM_172219.3 | siRNA 2 | Sense: CCAUCUGGCAGCAGAUGGAdTdT (SEQ ID NO: 116) |
| | | | Antisense: UCCAUCUGCUGCCAGAUGGdTdT (SEQ ID N O: 117) |
| CSF3 | NM_172219.3 | siRNA 3 | Sense: GGUGUCGUACCGCGUUCUAdTdT (SEQ ID NO: 118) |
| | | | Antisense: UAGAACGCGGUACGACACCdTdT (SEQ ID N O: 119) |

| | | | |
|---|---|---|---|
| Note: siRNAs having sequences in which dTdT is omitted from SEQ ID NOs: 114 to 119 in Table 5 may be used. | | | |

As a result of the experiment, it was confirmed that the siRNA against CSF3 used in this experiment effectively inhibits the expression of CSF3 protein when administered intracellularly (Figure 38).

In addition, the expression levels of CSF3, α-SMA, Col1A1, and TGF-β were measured in the control group treated with unrelated siRNA, the experimental group treated with unrelated siRNA and bleomycin, and the experimental group treated with siRNA against CSF3 and bleomycin. As a result of the experiment, it was confirmed that when unrelated siRNA and bleomycin were treated, the expression levels of CSF3, α-SMA, Col1A1, and TGF-β were increased. On the other hand, when siRNA against CSF3 was co-treated, it was confirmed that the expression levels of CSF3, α-SMA, Col1A1, and TGF-β were significantly suppressed (Figure 39).

This indicates that siRNA against CSF3 can reduce the expression of TGF-β, a key signaling factor in skin fibrosis induction, and consequently exert a therapeutic effect on skin fibrosis.

When CSF3 protein expression is reduced, the function of CSF3 in the cell will consequently be inhibited or decreased. Therefore, as with the antibody experimental results, administering siRNA against CSF3 to fibrosis-affected skin cells and/or skin tissue is expected to show the same therapeutic effects on fibrosis as administering a CSF3 antibody.

### Experimental Example 3.4. Experiment 2 on Therapeutic Effects on Skin Fibrosis

### Experimental Example 3.4.1. Animal Experiment Model

Animal experiments were conducted on C57BL/6 mice using a bleomycin-induced skin fibrosis treatment model.

The experiment was conducted by dividing the mice into a control group administered only PBS, an experimental group administered only bleomycin, and an experimental group administered bleomycin together with a CSF3 antibody.

The control group administered only PBS (PBS group) was subcutaneously injected with PBS (0.5 mg/mL) 3 times per week for 6 weeks.

The experimental group administered only bleomycin (BLM group) was subcutaneously injected with BLM (0.5 mg/mL) 3 times per week for 6 weeks to induce skin fibrosis.

The experimental group administered bleomycin together with a CSF3 antibody (BLM + CSF3 Ab group) was subcutaneously injected with BLM (0.5 mg/mL) 3 times per week for 6 weeks to induce skin fibrosis, and starting from week 4, the CSF3 antibody (250 µg/kg) was intraperitoneally injected 3 times per week for 3 weeks, for a total of 9 injections (Figure 40).

### Experimental Example 3.4.2. Confirmation of Therapeutic Effects on Skin Fibrosis 1 - Histological Observation

To confirm the therapeutic effects of the anti-CSF3 antibody on skin fibrosis, histological observation was performed on each group of the animal model of Experimental Example 3.4.1 to confirm the skin tissue morphology and collagen accumulation status.

Specifically, for tissue morphology confirmation, skin tissue from each group of mice was stained by H&E staining and observed under a microscope (Figure 41); collagen in the skin tissue of each group of mice was stained by Masson's trichrome staining and observed under a microscope (Figure 42). In addition, α-SMA and Col1A1 proteins in the skin tissue of each group of mice were subjected to immunohistochemistry, stained brown, and observed under a microscope (Figure 43).

As a result of the experiment, in the BLM group mice with induced skin fibrosis, the dermis layer was thickened, the distribution of tissue organelles was altered, collagen was accumulated, and furthermore, α-SMA and Col1A1 proteins were found to be accumulated, confirming that skin fibrosis had occurred.

On the other hand, in the BLM+CSF3 Ab group administered with the anti-CSF3 antibody, the dermis layer thickness was reduced to a level similar to that of normal tissue (PBS group), the fat layer was distributed, the accumulated collagen was significantly reduced, and furthermore, the α-SMA and Col1A1 proteins were found to be very greatly reduced to a level similar to that of normal tissue.

This indicates that when an anti-CSF3 antibody is administered to tissue with induced skin fibrosis, it has the effect of treating skin fibrosis and restoring the tissue to a normal state.

### Experimental Example 3.4.3. Confirmation of Therapeutic Effects on Skin Fibrosis 2 - Observation of Hair Loss Recovery Phenomenon

Similar to Experimental Example 3.4.1, a BLM-induced skin fibrosis animal model experiment was conducted. At this time, as in Experimental Example 3.4.1, the experiment was conducted by dividing the mice into a control group administered only PBS, an experimental group administered only bleomycin, and an experimental group administered bleomycin together with an anti-CSF3 antibody. The specific administration schedule and dosage are as shown in Figure 44.

As a result of the experiment, as bleomycin was intraperitoneally injected into mice, fibrosis was induced in various organs, and in particular, it was observed that collagen accumulated in the dermis layer of mouse skin due to skin fibrosis, causing thickening of the dermis and hair loss (BLM group in Figure 45).

However, when the anti-CSF3 antibody was administered, it was confirmed that the skin layer that had undergone hair loss recovered to a level similar to the normal state (PBS group in Figure 45) (BLM+CSF3 Ab group in Figure 45).

This indicates that CSF3 inhibitor has therapeutic effects on skin fibrosis.

### Experimental Example 3.4.4. Confirmation of Therapeutic Effects on Skin Fibrosis 3 - Analysis of Skin Fibrosis-Related Gene Expression

Skin fibroblasts isolated and cultured from skin tissue of male C57BL/6 mice were used to compare the control group treated only with PBS, the experimental group treated only with bleomycin, and the experimental group treated with both bleomycin and an anti-CSF3 antibody, in order to verify the therapeutic effect of the anti-CSF3 antibody on skin fibrosis (Figure 46).

Mouse skin fibroblasts were treated with bleomycin for 48 hours to induce skin fibrosis, and changes in the gene expression of fibrosis markers α-SMA and Col1A1 and changes in TGF-β gene expression were observed. Gene expression levels in mouse skin fibroblasts were confirmed using qPCR.

As a result of the experiment, it was confirmed that treating mouse skin fibroblasts with bleomycin increased the gene expression of CSF3, α-SMA, Col1A1, and TGF-β (Figure 47).

On the other hand, when mouse skin fibroblasts were co-treated with the CSF3 antibody, it was confirmed that the gene expression levels of CSF3, α-SMA, Col1A1, and TGF-β were significantly suppressed.

This indicates that the CSF3 antibody can reduce the expression of TGF-β, a key signaling factor in skin fibrosis induction, and consequently exert a therapeutic effect on skin fibrosis.

### Experimental Example 3.4.5. Confirmation of Skin Fibrosis Inhibitory Effect of CSF3 Antibody in Hypertrophic Scar Tissue of Burn Patients

Most wounds develop scars during the healing process, which occurs through inflammatory and fibrotic reactions. During the wound healing process, when new collagen fibers are produced in appropriate amounts, a normal scar forms in the skin, but when collagen fibers are produced excessively, a hypertrophic scar forms. In this case, a hypertrophic scar is a scar that is larger and thicker overall compared to a normal scar. Hypertrophic scars and keloids are representative fibrotic diseases of the skin.

In burn patients as well, excessive collagen can accumulate in scar tissue, leading to the formation of hypertrophic scar tissue.

The inventors cultured hypertrophic scar tissue from two burn patients and verified the therapeutic effects of the anti-CSF3 antibody on skin fibrosis using the cultured tissue. Here, the hypertrophic scar tissue used in the experiment is the hypertrophic scar tissue removed during surgical procedures to remove scar tissue from burn patients.

The expression levels of fibrosis markers α-SMA and Col1A1 genes, and the CSF3 gene in hypertrophic scar tissue were confirmed using qPCR.

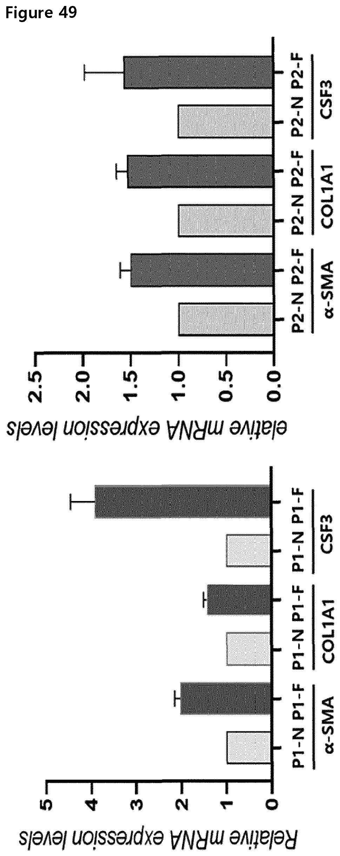

As a result of the experiment, it was confirmed that the expression of α-SMA, Col1A1, and CSF3 genes was higher in the hypertrophic scar tissue of the two burn patients (P1-F and P2-F) compared to the normal tissue (P1-N and P2-N) (Figures 48 and 49). Here, P1-F and P1-N refer to tissue derived from the same patient, and P2-F and P2-N refer to tissue derived from the same patient.

Skin fibroblasts were isolated and cultured from normal tissue and hypertrophic scar tissue of the burn patients, treated with the anti-CSF3 antibody, and changes in the gene expression of fibrosis markers α-SMA and Col1A1 and changes in TGF-β gene expression were observed. At this time, gene expression levels in skin fibroblasts were confirmed using qPCR.

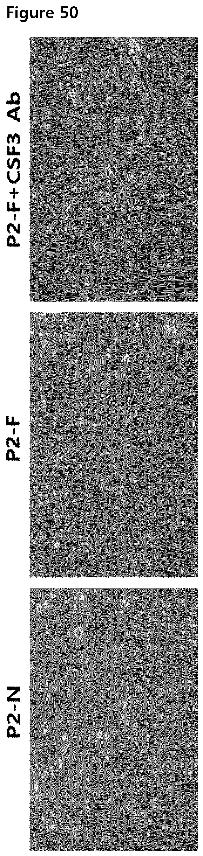

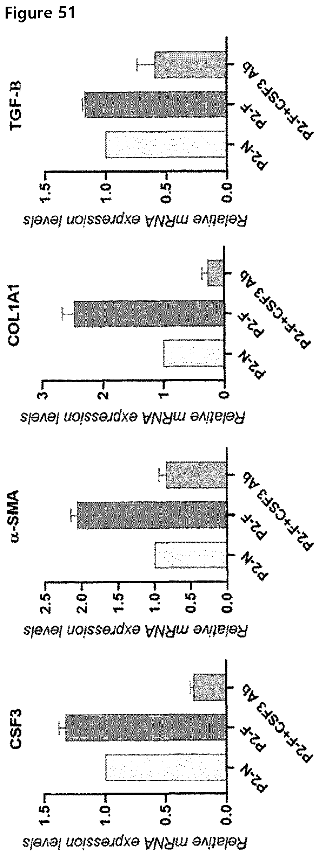

As a result of the experiment, it was confirmed that the expression levels of fibrosis markers α-SMA and Col1A1 and the TGF-β gene expression level were higher in skin fibroblasts isolated from hypertrophic scar tissue compared to skin fibroblasts isolated from normal tissue. Moreover, when the anti-CSF3 antibody was treated on skin fibroblasts isolated from hypertrophic scar tissue, the expression levels of fibrosis markers α-SMA and Col1A1 genes and the TGF-β gene were all markedly reduced (Figures 50 and 51).

Skin fibroblasts isolated from hypertrophic scar tissue were treated with siRNA against CSF3 for 48 hours, and changes in the gene expression of fibrosis markers α-SMA and Col1A1 and changes in TGF-β gene expression were observed. At this time, gene expression levels in skin fibroblasts were confirmed using qPCR.

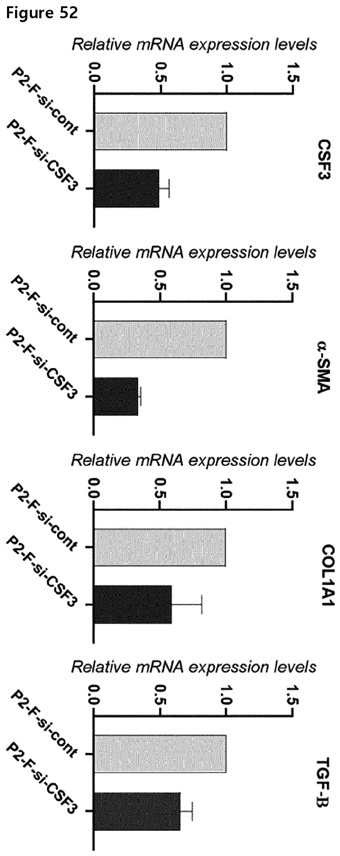

As a result of the experiment, it was confirmed that when siRNA against CSF3 was treated, the gene expression levels of CSF3, α-SMA, Col1A1, and TGF-β were significantly reduced (Figure 52).

This indicates that CSF3 inhibitor can reduce the expression of TGF-β, a key signaling factor in skin fibrosis induction, even in hypertrophic scar tissue of burn patients, and consequently exert a therapeutic effect on skin fibrosis.

### Experimental Example 3.5. Therapeutic Effects on Liver Fibrosis

LX-2 (Human Hepatic Stellate Cell) cells, a human hepatic stellate cell line, were used for experiments on liver fibrosis. The LX-2 cells were divided into four groups: 1) a control group administered only PBS (Cont); 2) a comparative group to which only TGF-β was treated to induce fibrosis (TGF-β); 3) an experimental group administered both TGF-β and a CSF3 antibody (TGF-β+CSF3 Ab); and 4) an experimental group administered both TGF-β and a CSF3R antibody (TGF-β+CSF3R Ab), and each reagent was treated accordingly.

For each group, the gene expression of α-SMA, Col1A1, FN, CSF3, CSF3R, TGF-β, and IL11 was confirmed by qPCR (Figure 53).

As a result of the experiment, it was confirmed that in the TGF-β group to which TGF-β was added, the mRNA expression levels of α-SMA, Col1A1, FN, CSF3, CSF3R, TGF-β, and IL11 were increased. On the other hand, in the group co-administered with a CSF3 antibody or a CSF3R antibody, it was confirmed that the mRNA expression levels of α-SMA, Col1A1, FN, CSF3, CSF3R, TGF-β, and IL11 were all significantly suppressed. This indicates that the CSF3 antibody and the CSF3 receptor (CSF3R) antibody have a therapeutic effect on fibrosis. Specifically, the CSF3 antibody and the CSF3 receptor (CSF3R) antibody reduce myofibroblasts in the tissue where fibrosis has occurred, inhibit the expression and secretion of collagen in the tissue to prevent collagen from accumulating in the tissue, and show an effect of removing accumulated collagen.

### Experimental Example 3.6. Therapeutic Effects on Renal Fibrosis

HK-2 (human renal proximal tubule epithelial cells), normal human renal tubular epithelial cells, were used for experiments on renal fibrosis. The HK-2 cells were divided into four groups: 1) a control group administered only PBS (Cont); 2) a comparative group to which only TGF-β was treated to induce fibrosis (TGF-β); 3) an experimental group administered both TGF-β and a CSF3 antibody (TGF-β+CSF3 Ab); and 4) an experimental group administered both TGF-β and an anti-CSF3R antibody (TGF-β+CSF3R Ab), and each reagent was treated accordingly.

For each group, the gene expression of α-SMA, Col1A1, FN, CSF3, CSF3R, TGF-β, and IL11 was confirmed by qPCR (Figure 54).

As a result of the experiment, it was confirmed that in the TGF-β group to which TGF-β was added, the mRNA expression levels of α-SMA, Col1A1, FN, CSF3, CSF3R, TGF-β, and IL11 were increased. On the other hand, in the group co-administered with a CSF3 antibody or a CSF3R antibody, it was confirmed that the mRNA expression levels of α-SMA, Col1A1, FN, CSF3, CSF3R, TGF-β, and IL11 were all significantly suppressed. This indicates that the anti-CSF3 antibody and the CSF3 receptor (CSF3R) antibody have a therapeutic effect on fibrosis. Specifically, the anti-CSF3 antibody and the anti-CSF3 receptor (CSF3R) antibody reduce myofibroblasts in the tissue where fibrosis has occurred, inhibit the expression and secretion of collagen in the tissue to prevent collagen from accumulating in the tissue, and show an effect of removing accumulated collagen.

### Experimental Example 4. Selection of Anti-CSF3 Antibody

The process of selecting monoclonal antibodies (human IgG1 antibodies) capable of specifically binding to human CSF3 protein (human colony-stimulating factor 3) was entrusted to Y-Biologics Inc. As the first step, to select the mother antibody, the phage display method described in Experimental Example 4 was used on Y-Biologics' Ymax^{®}-ABL fully human antibody library. As the second step, the screening method described in Experimental Example 4 was used on a library in which mutations were introduced into the CDR sequences of the selected mother antibody, and 17 antibodies were selected.

### Experimental Example 4.1. Experimental Methods and Materials

### Experimental Example 4.1.1. Overview of Phage Display Method

The inventors used the "phage display" method as a method for screening antibodies capable of specifically binding to human CSF3 (hereinafter "anti-CSF3 antibodies").

The phage display method used in this experimental example is a method of screening anti-CSF3 antibodies using a phage library (Naive Antibody Library, Ymax^{®}-ABL, owned by Y-Biologics) in which human scFv antibodies are fused to the phage surface protein (PIII). The scFvs included in the phage library have human antibody sequences derived from human B cells, and the diversity of the scFvs is more than 100 billion.

As a specific method for screening anti-CSF3 antibodies from the phage library, bio panning and ELISA were used. Three rounds of panning using the antigen protein (CSF3) were performed on the phage library to select phages capable of binding to CSF3, and then ELISA was performed on the selected phages to select phages capable of specifically binding to CSF3. Thereafter, the sequence of the scFv fused to the selected phage was converted to an IgG antibody, and additional ELISA was performed on the converted antibodies.

### Experimental Example 4.1.2. Antigen Expression and Purification Method

G-CSF (CSF3) and CSF3-isoform b were used as types of positive antigens for human CSF3 used in bio panning and/or ELISA. Mouse Fc (mFc) and human Fc (hFc) were fused to the C-terminus of CSF3. Information on the G-CSF and CSF3-isoform b is as shown in Table 6. Negative antigens used were ITGA-Fc, hRAGE-Fc, CD58-Fc, and AITR-Fc produced by Y-Biologics.

Cloning of CSF3 was performed by amplification via polymerase chain reaction (PCR) using primers for CSF3 (Table 7). The amplified PCR product was prepared using an expression vector (p297F, p293F/Fc, or p293F/mFc vector) so that His (6x), hFc, or mFc could be fused to the C-terminus of CSF3.

**[Table 6]**

| **Protein Name** | **CLSID** | **Supplier** | **Protein Description** |
|---|---|---|---|
| CSF3-isoform b | CLS0057 | Sino | Positive Ag |
| G-CSF | CLS0074 | GenScript | Positive Ag |

**[Table 7]**

| **Protein Name** | **5'→3' sequence** |
|---|---|
| CSF3-C-6xHis-Forward | TGGT**GGCCACAGCGGCCG**ATGTCCACTCGAcccccctgggccctgcca (SE Q ID NO: 120) |
| CSF3-C-6xHis-Reverse | AAT**TCTCGAGT**CAGTGGTGATGATGGTGATGgggctgggcaaggtggcgta (SEQ ID NO: 121) |
| CSF3-C-Fc-Forward | TGGT**GGCCACAGCGGCCG**ATGTCCACTCGAcccccctgggccctgcca (SE Q ID NO: 122) |
| CSF3-C-Fc- Reverse | A**AGCTAGC**gggctgggcaaggtggcgta (SEQ ID NO: 123) |

| | |
|---|---|
| Bold & underline: restriction enzyme site Italic & underline: His-tagged sequence Lowercase: CSF3-specific sequence | |

The prepared expression vector was transfected into HEK293F cells and cultured with shaking at 125 rpm for 1 day under conditions of 37°C and 8% CO2. After 1 day of culture, Soytone was added at 0.05% of the total media volume, and then cultured with shaking at 125 rpm for 6 days under conditions of 32°C and 5% CO2. On day 7 of culture, the cell culture medium was harvested and centrifuged (4,800 rpm, 30 minutes). Thereafter, the supernatant was filtered through a 0.22 µm Bottle TOP Filter to remove residues. Thereafter, primary purification was performed using protein A beads and Nickel NTA beads, and secondary purification was performed using SEC-FPLC. The purity of the obtained protein was analyzed using SDS-PAGE gels under non-reducing and reducing conditions.

### Experimental Example 4.1.3. Bio Panning Method

Immunotube panning (IM) and/or bead panning methods were used as bio panning methods.

The panning method consists of fixing the prepared antigen (CSF3, or CSF3-C-mFc, etc.) on the surface of a tube or beads, adsorbing a phage (scFv-Phage) solution expressing human scFv antibodies, and then washing and eluting. Through these steps, phage populations expressing antibodies that strongly adsorb to the surface antigen were extracted and selected, amplified, and then the above panning method was repeated again on the amplified phage populations. At this time, a total of 3 rounds of bio panning were consecutively repeated, each conducted under different conditions (varying the type of antigen used, or the strength of washing, etc.).

### Experimental Example 4.1.4. Sequence Analysis Method

Sequence analysis was performed by the Sanger sequencing method. During the PCR process for DNA sequence amplification, when dNTPs are polymerized into the DNA sequence, the hydroxyl group (-OH) at the 3rd carbon of the last nucleotide of the DNA sequence reacts with the phosphate group at the 5th carbon of the new dNTP to form a bond. ddNTP is a nucleotide structure in which oxygen is absent at the 3rd carbon of dNTP, i.e., hydrogen (-H) is bound instead of the hydroxyl group (-OH). At this time, the 4 types of ddNTP (ddATP (adenine), ddGTP (guanine), ddTTP (thymine), and ddCTP (cytosine) for each nucleic acid) were each stained with different fluorescent materials. During the PCR process, when ddNTP was accidentally incorporated instead of dNTP during DNA amplification, since there is no hydroxyl group at the 3rd carbon of the ddNTP, no new phosphate group can be incorporated, and amplification is terminated (chain termination). Since ddNTP incorporation occurs randomly, chain termination occurs at almost every nucleotide position through numerous amplification processes. After the PCR containing ddNTPs was first completed, electrophoresis, which can sort sequences by size in ascending order, was used to arrange the amplified sequences. The fluorescence signals were recognized in the order of the size-sorted sequences, so that the nucleic acid information corresponding to each base sequence in the order of the base sequences was read according to the type of fluorescent material possessed by the ddNTP last incorporated at the end of each sequence.

### Experimental Example 4.1.5. IgG Conversion Method

### 1. Conversion to IgG form

To convert individual monoclonal phages with individual sequences identified through sequence analysis from the scFv form to the IgG form, the nucleotide sequences of the variable regions of the heavy and light chains were cloned into the N293F vector using SfiI/NheI restriction enzyme sites.

### 2. Human antibody production

The cloned N293FH and N293FL vectors were co-transfected into HEK293F cells at a 1:1 ratio. The day 7 culture medium collected was centrifuged at 8,000 rpm for 30 minutes to remove cell debris, and cells and suspended matter were removed by filtration through a bottle top filter with a pore size of 0.22 µm (Millipore, Steritop-GP Filter Unit, Cat. No. SCGPS01RE) for pretreatment.

### 3. Antibody purification

### (1) Protein A chromatography

To perform purification, 4 mL of protein A Sepharose resin slurry (GE Healthcare Life Sciences, Cat. No. 17-1279-04) was added to an empty column (Bio-rad, BR731-1550), and then the resin was packed and washed with 100 mL of DPBS (LB001-02). The pretreated medium was applied to the packed resin and allowed to flow at 1 mL per minute (Bio-Rad, EP-1 Econo pump, U.S.). After washing with 150 mL of DPBS, elution was performed with 10 mL of 0.1 M glycine-HCl (pH 3.3). The eluate was neutralized by adding 10% of 1M Tris-HCl (pH 9.0), and the buffer was exchanged to DPBS using Amicon Ultra-15 (Millipore, UFC901096). After repeating this process approximately 3 times, the sample was concentrated to approximately 1 mL and the concentration was confirmed. Concentration was measured with a NanoDrop instrument.

### (2) SDS-PAGE

To confirm the purity of the purified protein, SDS-PAGE was performed. 3 µg of the purified protein sample was mixed with reducing sample buffer and 5 µL of non-reducing sample buffer and boiled at 100°C for 3 minutes.

A 12% gel was loaded into a running tank (Bio-Rad, 165-8027), the inside of the gel was completely filled with 1x running buffer, and the outside was filled approximately 1/3, and the prepared samples were loaded into the wells using a pipette, being careful not to introduce bubbles. 4 µL of AccuBand Prestained Protein Marker was loaded into the leftmost well. The upper cover of the gel chamber was attached, the power was connected, and running was performed at 200V for 1 hour.

After 1 hour, the gel was separated from the tank and stained on a shaker for 1 hour in staining buffer. Thereafter, the staining solution was discarded and destaining was performed with destaining solution for approximately 1 hour. After destaining was complete, the gel was washed with D.W. and stored using imaging equipment.

### Experimental Example 4.1.6. ELISA Method

The ELISA method was used to confirm whether the selected phages or antibodies converted to IgG specifically bind to human CSF3, or whether they can interfere with the binding of human CSF3 and CSF3R.

The specific method is as follows.

The positive antigen or negative antigen was suspended in PBS at 1 mg/mL, and 100 µL was added to each well of an immunoplate for coating at 4°C for 16 hours. After removing the coating solution from the plate, 200 µL of 4% skim milk (PBST) was added to each well and blocking was performed at room temperature for 1 hour. After discarding the blocking solution, 100 µL of polyphage (or monophage) was added to each well of the plate, and binding was induced for 2 hours at room temperature. 200 µL of PBST was added 3 times for washing. 100 µL/well of 4% skim milk (PBST) containing Anti-M13-HRP (1:8000) was added and incubated at room temperature for 1 hour. The solution containing Anti-M13-HRP (1:8000) was removed and washed 3 times with 200 µL of PBST. 100 µL/well of TMB Peroxidase Substrate was added, and the reaction was stopped when the desired color development was observed by adding 50 µL/well of Stop Solution. Absorbance was measured at OD 450 nm using an ELISA instrument.

### Experimental Example 4.1.7. Method for Preparing Affinity Maturation Library

To increase the specific affinity binding of antibodies with neutralizing activity against human CSF3, the sequence of the variable region of the antibody was partially mutated. The specific methods used were light chain shuffling or CDR-mutation.

The specific method for light chain shuffling is as follows.

To prepare the light chain shuffling vector, 20 µg of light chain shuffling pool library DNA was transferred to a tube, and then 50 µL of 10x buffer and 10 µL of Sfi I (ENZYNOMYCS) were added, and the total volume was adjusted to 500 µL with sterilized distilled water. Thereafter, the reaction was performed in an incubator at 50°C for 3 hours. The light chain shuffling pool library DNA cut with Sfi I was recovered using a QiAquick Gel Extraction Kit (QIAGEN) to obtain the light chain shuffling pool library vector. 10 µg of 12A02 phagemid DNA was transferred to a tube, and then 50 µL of 10x buffer and 10 µL of Sfi I (ENZYNOMYCS) were added, and the total volume was adjusted to 500 µL with sterilized distilled water. Thereafter, the reaction was performed in an incubator at 50°C for 3 hours. Thereafter, electrophoresis was performed on a 1% agarose gel, and only the VH region was recovered using a QiAquick Gel Extraction Kit (QIAGEN). The light chain shuffling pool library DNA vector prepared above and VH were mixed at a molar ratio of 1:3 and ligated at room temperature for 30 minutes. 5 µg of the light chain shuffling library vector prepared above and 403 ng of VH were added to a tube at a molar ratio of 1:1, and then 5 µL of 10x buffer and 2 µL of T4 DNA ligase were added, and the total volume was adjusted to 50 µL with sterilized distilled water. The ligation reaction was induced at room temperature for 30 minutes, and then 1 µL of the ligation mixture was electrophoresed on a 1% agarose gel to confirm the degree of ligation. The ligation mixture was recovered using a QiAquick Gel Extraction Kit (QIAGEN) after removing T4 DNA ligase. A light chain shuffling library was prepared by transformation using 1.5 µg of ligation DNA and 750 µL of electroporation competent cells mixed in a cuvette using a MicroPulser Electroporator (BIO-RAD).

The specific method for CDR-mutation is as follows.

Residues of CDR2 and CDR3 of VH, and CDR1 and CDR3 of VL were subjected to mutagenesis using primers for inducing mutations in CDR residues of CDR2, CDR3 of VH and CDR1, CDR3 of VL by PCR reaction (95°C 5 min, 95°C 30 sec, 52°C 30 sec, 72°C 30 sec, 72°C 5 min, 30 cycles) in a T100 Thermal Cycler. CDR-mutated fragments of CDR2 and CDR3 of VH and CDR1 and CDR3 of VL were electrophoresed on a 1% agarose gel to confirm each PCR fragment, and then the PCR fragments were recovered using a QiAquick Gel Extraction Kit (QIAGEN). To prepare VH with only individual CDRs mutated, VL with only individual CDRs mutated, and VH with CDR2/CDR3 mutations combined with VL with CDR1/CDR3 mutations, 100 ng each of the recovered PCR fragments were used for overlap PCR (95°C 5 min, 95°C 30 sec, 52°C 30 sec, 72°C 30 sec, 72°C 5 min, 25 cycles) to amplify VH and VL fragments. The amplified VH and VL PCR products were recovered using a QiAquick Gel Extraction Kit (QIAGEN). The recovered VH PCR products were digested with Sfi I, and VL PCR products were digested with BstX1. That is, 10 µg of PCR product was transferred to a tube, and then 50 µL of 10x buffer and 10 µL of Sfi I (BstX1) (ENZYNOMYCS) were added, and the total volume was adjusted to 500 µL with sterilized distilled water. Thereafter, the reaction was performed in an incubator at 50°C (37°C) for 3 hours. Thereafter, electrophoresis was performed on a 1% agarose gel, and VH and VL were recovered using a QiAquick Gel Extraction Kit (QIAGEN). The recovered VH was ligated at a 1:1 molar ratio to the Sfi I site of the 12A02 phagemid vector without VH, and the recovered VL was ligated at a 1:1 molar ratio to the BstX1 site of the 12A02 phagemid vector without VL. The ligation mixture was recovered using a QiAquick Gel Extraction Kit (QIAGEN) after removing T4 DNA ligase. Libraries with mixed VH-CDR1, VH-CDR3, VL-CDR1, VL-CDR3, and libraries with mixed VH-CDR2/CDR3 and VL-CDR1/CDR3 were prepared by transformation using 1.5 µg of ligation DNA and 750 µL of electroporation competent cells mixed in a cuvette using a MicroPulser Electroporator (BIO-RAD).

### Experimental Example 4.2. Selection of Mother Antibody

### Experimental Example 4.2.1. Selection of Phage Candidates

Panning was performed on the human antibody phage library with more than 100 billion diversity owned by Y-Biologics. After panning, 6,624 monoclonal colonies remaining were selected.

ELISA was performed on the selected 6,624 monoclonal colonies, and 612 binder colonies (binders capable of specifically binding to CSF3) were selected. Sequence analysis was performed on the 612 colonies, and 102 independent clones excluding duplicate colonies were selected.

ELISA was performed for positive and negative antigens on the selected 102 independent clones, and 38 specific binders with low binding levels to the negative antigen and high binding levels to the positive antigen were selected. That is, the selected 38 specific binders can specifically bind to human CSF3.

### Experimental Example 4.2.2. Selection of Mother Antibody

IgG1 conversion was performed using the scFv sequences of the selected 38 specific binders. As a result, only 26 of the 38 specific binders were converted to the IgG1 form.

ELISA was performed on the 26 antibodies produced through IgG1 conversion to confirm whether they have the ability to inhibit the binding between human CSF3 and CSF3R. As a result, the antibody expected to have the highest neutralizing activity for human CSF3 (the ability to inhibit the binding of CSF3 and CSF3R) was selected as the mother antibody.

### Experimental Example 4.3. Optimization of Anti-CSF3 Antibody (Affinity Maturation)

### Experimental Example 4.3.1. Selection of Phage Candidates

An affinity maturation library was prepared based on the sequence of the mother antibody selected in Experimental Example 4.2. Bio panning was performed on the prepared affinity maturation library. After bio panning, 1,536 monoclonal colonies remaining were selected.

ELISA was performed on the selected 1,536 monoclonal colonies, and 115 binder colonies (binders capable of specifically binding to CSF3) were selected. Sequence analysis was performed on the 115 colonies, and 37 independent clones excluding duplicate colonies were selected.

ELISA was performed for positive and negative antigens on the selected 37 independent clones, and 35 specific binders with low binding levels to the negative antigen and high binding levels to the positive antigen were selected. That is, the selected 35 specific binders can specifically bind to human CSF3.

### Experimental Example 4.3.2. Final Antibody Selection

IgG1 conversion was performed on the top 21 specific binders with high affinity for human CSF3 among the 35 specific binders selected above, and 19 antibodies were prepared.

ELISA was performed on the 19 prepared antibodies to confirm whether they have the ability to inhibit the binding between human CSF3 and CSF3R. As a result, 17 antibodies showing significant inhibitory effects were finally selected as anti-CSF3 antibodies.

Table 8 below lists amino acid sequence information for each region of the mother antibody and the 17 selected anti-CSF3 antibodies.

**[Table 8]**

| | **Antibody Name** | **Heavy chain CDR(CDRH1, CDRH2, CDRH3 order)** | **Light chain CDR(CDRL1, CDRL2, CDRL3 order)** | **Heavy chain FR( FRH1, FRH2, FR H3 order)** | **Light chain FR(FRL1, FRL2, FRL3 order)** | **Full-length VH region, Full-length VL region** | **Full-length h eavy chain, Full-length light chain** |
|---|---|---|---|---|---|---|---|
| 1 | Mother | SEQ ID NO: 1, SEQ ID NO: 2, SEQ ID NO: 3 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 38, SEQ ID NO: 56 | SEQ ID NO: 63, SEQ ID NO: 81 |
| 2 | C33( or Y33) | SEQ ID NO: 1, SEQ ID NO: 2, SEQ ID NO: 4 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 39, SEQ ID NO: 56 | SEQ ID NO: 64, SEQ ID NO: 81 |
| 3 | C34(or Y34) | SEQ ID NO: 1, SEQ ID NO: 2, SEQ ID NO: 5 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 40, SEQ ID NO: 57 | SEQ ID NO: 65, SEQ ID NO: 82 |
| 4 | C35(or Y35) | SEQ ID NO: 1, SEQ ID NO: 2, SEQ ID NO: 6 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 41, SEQ ID NO: 58 | SEQ ID NO: 66, SEQ ID NO: 83 |
| 5 | C36(or Y36) | SEQ ID NO: 1, SEQ ID NO: 2, SEQ ID NO: 7 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 42, SEQ ID NO: 56 | SEQ ID NO: 67, SEQ ID NO: 81 |
| 6 | C38(or Y38) | SEQ ID NO: 1, SEQ ID NO: 2, SEQ ID NO: 8 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 43, SEQ ID NO: 56 | SEQ ID NO: 68, SEQ ID NO: 81 |
| 7 | C39(or Y39) | SEQ ID NO: 1, SEQ ID NO: 2, SEQ ID NO: 9 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 44, SEQ ID NO: 56 | SEQ ID NO: 69, SEQ ID NO: 81 |
| 8 | C40(or Y40) | SEQ ID NO: 1, SEQ ID NO: 2, SEQ ID NO: 10 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 45, SEQ ID NO: 56 | SEQ ID NO: 70, SEQ ID NO: 81 |
| 9 | C41 ( or Y4 1) | SEQ ID NO: 1, SEQ ID NO: 2, SEQ ID NO: 11 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 46, SEQ ID NO: 59 | SEQ ID NO: 71, SEQ ID NO: 84 |
| 10 | C42(or Y42) | SEQ ID NO: 1, SEQ ID NO: 2, SEQ ID NO: 12 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 47, SEQ ID NO: 56 | SEQ ID NO: 72, SEQ ID NO: 81 |
| 11 | C43(or Y43) | SEQ ID NO: 1, SEQ ID NO: 2, SEQ ID NO: 13 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 48, SEQ ID NO: 56 | SEQ ID NO: 63, SEQ ID NO: 81 |
| 12 | C44(o r Y44) | SEQ ID NO: 1, SEQ ID NO: 2, SEQ ID NO: 14 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 49, SEQ ID NO: 56 | SEQ ID NO: 74, SEQ ID NO: 81 |
| 13 | C45(o r Y45) | SEQ ID NO: 1, SEQ ID NO: 2, SEQ ID NO: 15 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 50, SEQ ID NO: 56 | SEQ ID NO: 75, SEQ ID NO: 81 |
| 14 | C46(or Y46) | SEQ ID NO: 1, SEQ ID NO: 2, SEQ ID NO: 16 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 51, SEQ ID NO: 56 | SEQ ID NO: 76, SEQ ID NO: 81 |
| 15 | C47(or Y47) | SEQ ID NO: 1, SEQ ID NO: 2, SEQ ID NO: 17 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 52, SEQ ID NO: 56 | SEQ ID NO: 77, SEQ ID NO: 81 |
| 16 | C48(or Y48) | SEQ ID NO: 1, SEQ ID NO: 2, SEQ ID NO: 18 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 53, SEQ ID NO: 56 | SEQ ID NO: 78, SEQ ID NO: 81 |
| 17 | C49(or Y49) | SEQ ID NO: 1, SEQ ID NO: 2, SEQ ID NO: 19 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 54, SEQ ID NO: 60 | SEQ ID NO: 79, SEQ ID NO: 85 |
| 18 | C50(or Y50) | SEQ ID NO: 1, SEQ ID NO: 2, SEQ ID NO: 20 | SEQ ID NO: 21, YAS(SEQ ID NO: 25, SEQ ID NO: 26 | SEQ ID NO: 27, SEQ ID NO: 29, SEQ ID NO: 30, SEQ ID NO: 32 | SEQ ID NO: 33, SEQ ID NO: 34, SEQ ID NO: 35, SEQ ID NO: 36 | SEQ ID NO: 55, SEQ ID NO: 56 | SEQ ID NO: 80, SEQ ID NO: 81 |

The antibodies listed in Table 8 (mother, C33, C34, C35, C36, C38, C39, C40, C41, C42, C43, C44, C45, C46, C47, C48, C49, and C50) all share in common that the amino acid sequences of the heavy chain constant region (CH) and light chain constant region (CL) correspond to SEQ ID NO: 61 and SEQ ID NO: 62, respectively.

### Experimental Example 5. Analysis of Selected Antibodies

### Experimental Example 5.1. Experimental Methods and Materials

### Experimental Example 5.1.1. Confirmation of Target (CSF3) Binding Affinity of Antibodies

Human CSF3-mFc was coated at 100 ng/well, and candidate antibodies diluted to 1 pmol to 100 nM were treated to confirm the degree of binding by ELISA. After removing the coating solution from the plate, 200 µL of 4% skim milk (PBST) was added to each well and blocking was performed at room temperature for 1 hour. After discarding the blocking solution, 100 µL each of candidate antibodies diluted to 1 pmol to 100 nM was added to each well of the plate, and binding was induced for 2 hours at room temperature. 200 µL of PBST was added 3 times for washing. 100 µL/well of 4% skim milk (PBST) containing Anti-M13-HRP (1:8000) was added and incubated at room temperature for 1 hour. The solution containing Anti-M13-HRP (1:8000) was removed and washed 3 times with 200 µL of PBST. 100 µL/well of TMB Peroxidase Substrate was added, and the reaction was stopped when the desired color development was observed by adding 50 µL/well of Stop Solution. Absorbance was measured at OD 450 nm using an ELISA instrument.

### Experimental Example 5.1.2. Confirmation of Target (CSF3) Neutralization Ability

To confirm whether the candidate antibodies inhibit the binding of human CSF3 and CSF3R, human CSF3R-hFc was coated at 2.5 nM/well, and then human CSF3-mFc (5 nM) and candidate antibodies (0.2 nM to 1000 nM) were treated to confirm the degree of inhibition of binding between human CSF3-mFc and CSF3R-hFc by ELISA.

### Experimental Example 5.1.3. Antibody Purity Analysis Method

The purity of the candidate antibodies was confirmed by the SEC-HPLC method. To analyze the purity of the antibodies, Pump A of HPLC (U3000, Thermo Fisher) was connected to the mobile phase (DPBS/250 mM NaCl/10% ACN) and the system was operated. After exchanging the Pump A line with the mobile phase, a SEC column (SRT-C SEC 300, 7.8×300 mm, 5 µm, 300, SEPAX) was connected and operated at a flow rate of 1 mL/min for 1 hour. Thereafter, 20 µg of sample was injected and run at a flow rate of 1 mL/min for 10 minutes, and the purity of the antibody was analyzed by analyzing the peaks of the antibody at 280 nm.

### Experimental Example 5.1.4. Antibody Thermal Stability Analysis Method

Thermal stability analysis was performed using a Protein Thermal Shift Dye Kit (Catalog No. 4461146, Thermo). The thermal stability of each antibody was assessed using a protein thermal shift (PTS) assay in which fluorescence of the dye is measured upon temperature increase, as the dye binds to the hydrophobic regions exposed when the protein unfolds from its folded state. The PTS assay was performed according to the manufacturer's instructions using the Protein Thermal Shift Dye Kit (Catalog No. 4461146, Thermo). Specifically, 5 µg of each antibody was mixed with protein thermal shift dye and buffer to a total volume of 20 µL, and then ROX signal was monitored from 20°C to 95°C at an increase of 1°C per 60 seconds using RT-PCR (C1000 thermal cycler equipped with a CFX96 optical reaction module, Bio-Rad). The melting temperature (Tm) of each antibody was determined based on the melting curve.

### Experimental Example 5.1.5. Analysis of Pulmonary Fibrosis Inhibitory Efficacy Using Human Alveolar Cells

### (1) Cell Culture Method

Human lung fibroblast cell line (WI38) and lung epithelial cell line (BEAS2B) were purchased from American Type Culture Collection (ATCC, U.S.). Each cell was cultured in DMEM media and RPMI 1640 media, respectively, supplemented with 1% penicillin-streptomycin (Gibco, U.S.) and 10% heat-inactivated fetal bovine serum (Gibco, U.S.; heat-inactivated at 57°C for 30 minutes), in a temperature- and humidity-controlled incubator (Sanyo, Japan) maintained at 5% CO2 and 37°C. Cells were seeded in culture plates at a density of 3×10⁵ cells, treated with bleomycin sulfate (bleomycin sulfate, Sigma) at a concentration of 2 µg/mL, and cells were harvested 48 hours later. Collagen levels were measured by performing a hydroxyproline assay on the harvested cells, protein changes were confirmed by Western blot, and gene changes were confirmed using qPCR.

### (2) Hydroxyproline Assay for Measuring Collagen Levels

Cells treated with bleomycin sulfate (bleomycin sulfate, Sigma) at a concentration of 2 µg/mL were cultured for 48 hours and then harvested. Collagen levels were measured by performing a hydroxyproline assay on the harvested cells. The experimental procedure involved dissolving the harvested cells in 6N HCl, followed by acid hydrolysis at 110°C for 24 hours. Absorbance (OD = 560 nm) was measured using 500 µL each of Oxidant Sol. and Ehrlich's Sol.

### (3) Western Blot Protein Expression Analysis

Western blot was performed to confirm changes in protein expression. Protein was extracted from the harvested cells, and 20 µg of protein was electrophoresed on a 10% SDS-polyacrylamide gel. After protein separation, transfer to a nitrocellulose membrane was performed at 100 V for 1 hour, and the membrane was washed with 1X PBST (2.67 mM potassium chloride, 1.47 mM potassium phosphate monobasic, 137.93 mM sodium chloride, 8.1 mM sodium phosphate dibasic anhydrous, 0.1% Tween 20) and blocked with 5% skim milk (BD, U.S.) for 30 minutes. Primary antibody was diluted 1:1,000 in 1X PBST and incubated at 4°C overnight. The reacted membrane was washed with 1X PBST, then incubated with secondary antibody diluted 1:10,000 in 1X PBST at room temperature for 1 hour. Proteins were detected using ECL solution (PerkinElmer, U.S.) and developed on AGFA film (Agfa, Belgium).

### (4) Real-Time PCR Gene Expression Analysis

Total RNA was extracted from the harvested cells using an RNA minikit (Qiagen, Inc.), and then converted to cDNA using Accupower RT mix reagent (Bioneer Corp., Seoul, Korea). Gene expression changes were confirmed using Real-time PCR Fast Start Essential DNA Green Master (Roche, Indianapolis, IN, USA).

Table 9 below lists the primer sequences used in qPCR analysis.

**[Table 9]**

| **Gene** | **Primer sequence (5'-3') for qPCR** |
|---|---|
| α-SMA | AATCCCATCACCATCTTCCAG (SEQ ID NO: 100) |
| | GCAGTGGCCATCTCATTTTCA (SEQ ID NO: 101) |
| Collagen 1A1 | CCTGGATGCCATCAAAGTCT (SEQ ID NO: 102) |
| | AATCCATCGGTCATGCTCTC (SEQ ID NO: 103) |
| TGF-β | CCCAGCATCTGCAAAGCTC (SEQ ID NO: 108) |
| | GTCAATGTACAGCTGCCGCA (SEQ ID NO: 109) |
| IL-11 | ACAGCTGAGGGACAAATTCC (SEQ ID NO: 110) |
| | AGCACACCTGGGAGCTGTA (SEQ ID NO: 111) |
| GAPDH | AATCCCATCACCATCTTCCAG (SEQ ID NO: 112) |
| | AAATGAGCCCCAGCCTTC (SEQ ID NO: 113) |

### (5) Immunofluorescence Staining

Cells attached to slides were fixed with 4% paraformaldehyde at 4°C overnight. The fixed cells were incubated with PBS containing 1% BSA and 0.5% Triton X-100 at room temperature for 1 hour, then treated with each primary antibody, and reacted with FITC-conjugated secondary antibody and PE-conjugated secondary antibody. Fluorescence images were captured and analyzed using a fluorescence microscope (Olympus, Shinjuku, Tokyo, Japan).

### Experimental Example 5.1.6. Analysis of Pulmonary Fibrosis Inhibitory Efficacy Using Mice

The mice used in the experiment were 7-week-old male C57BL/6 mice weighing approximately 20±2 g. After confirming individual monitoring data and appearance from the supplier, the animals were acclimatized in a quarantine room for approximately 1 week, and only healthy individuals were used in the experiment following observation of general symptoms. Animal care was maintained by auto controller according to the standards of the Seoul Women's University Laboratory Animal Facility, at a temperature of 22±1°C, humidity of 50±10%, and lighting cycle of 12 Light: 12 Dark, and sterilized feed was provided along with sterilized water.

A bleomycin-induced pulmonary fibrosis animal model was used for the animal experiments. Bleomycin Sulfate (B1141000) was purchased from Sigma. Specifically, bleomycin (2 mg/kg) was injected once into the airway of mice to induce pulmonary fibrosis, and starting from day 5, CSF3-4E5 surrogate antibody (2 mg/kg) was intraperitoneally injected 4 times at 2-day intervals, and on day 14 of the experiment, the mice were necropsied and lung tissue was excised.

### Experimental Example 5.2. Confirmation of Effects of Anti-CSF3 Antibody

The names of the antibodies described in the experimental examples and figures are listed in four different ways.

Table 10 below lists the names of the candidate antibodies, with names of the same antibody listed in the same row.

For example, 003A11, CSA1233, Y33, and C33 refer to the same antibody.

**[Table 10]**

| **No.** | | **CSA** | **Y** | **C** | **Remarks** |
|---|---|---|---|---|---|
| 1 | 001a07 | CSA1230 | - | - | Antibody not selected due to low target binding affinity |
| 2 | 003A11 | CSA1233 | Y33 | C33 | Selected antibody |
| 3 | 003H12 | CSA1234 | Y34 | C34 | Selected antibody |
| 4 | 004A08 | CSA1235 | Y35 | C35 | Selected antibody |
| 5 | 004D04 | CSA1236 | Y36 | C36 | Selected antibody |
| 6 | 004D06 | CSA1237 | Y37 | C37 | Antibody not selected due to low target binding affinity |
| 7 | 004G03 | CSA1238 | Y38 | C38 | Selected antibody |
| 8 | 004H01 | CSA1239 | Y39 | C39 | Selected antibody |
| 9 | 005H08 | CSA1240 | Y40 | C40 | Selected antibody |
| 10 | 007A06 | CSA1241 | Y41 | C41 | Selected antibody |
| 11 | 007A12 | CSA1242 | Y42 | C42 | Selected antibody |
| 12 | 007C01 | CSA1243 | Y43 | C43 | Selected antibody |
| 13 | 007E01 | CSA1244 | Y44 | C44 | Selected antibody |
| 14 | 007H05 | CSA1245 | Y45 | C45 | Selected antibody |
| 15 | 008A10 | CSA1246 | Y46 | C46 | Selected antibody |
| 16 | 008H04 | CSA1247 | Y47 | C47 | Selected antibody |
| 17 | 008H07 | CSA1248 | Y48 | C48 | Selected antibody |
| 18 | 008D08 | CSA1249 | Y49 | C49 | Selected antibody |
| 19 | 008F11 | CSA1250 | Y50 | C50 | Selected antibody |
| 20 | Mother | CSA0992 | | | Mother antibody |
| 21 | REF* | | | | |

| | | | | | |
|---|---|---|---|---|---|
| *: REF antibody is an antibody that binds to CSF3, purchased from Abcam (ab9691). | | | | | |

### Experimental Example 5.2.1. Confirmation of Target Binding Affinity

Human CSF3-mFc was coated at 100 ng/well, and candidate antibodies diluted to 1 pmol to 100 nM were treated to confirm the degree of binding by ELISA. The KD values of the candidate antibodies were graphed. The ELISA results and graphs are shown in Figures 55 to 57.

### Experimental Example 5.2.2. Confirmation of Target Neutralization Ability

To confirm whether the binding of human CSF3 and CSF3R is inhibited (target neutralization ability, Neutralization Ability), human CSF3R-hFc was coated at 2.5 nM/well, and then human CSF3-mFc (5 nM) and candidate antibodies (0.2 nM to 1000 nM) were treated to confirm the degree of inhibition of binding between human CSF3-mFc and human CSF3R-hFc by ELISA. The results are shown in Figures 58 to 62.

From the results, the degree of inhibition of binding for each candidate antibody at different concentrations could be confirmed. At this time, the graph prepared at the 25 nM value is shown in Figure 63.

### Experimental Example 5.2.3. Antibody Confirmation

The purity of the candidate antibodies was confirmed by the SEC-HPLC method. The purity of the candidate antibodies was graphed, and the results are shown in Figures 64 to 80. The purity of the candidate antibodies ranged from 87.26 to 96.01. The purity of each antibody is as shown in Table 11 below.

**[Table 11]**

| **No.** | | **CSA** | **Y** | **Purity** |
|---|---|---|---|---|
| 1 | 003A11 | CSA1233 | Y33 | 95.51 |
| 2 | 003H12 | CSA1234 | Y34 | 87.26 |
| 3 | 004A08 | CSA1235 | Y35 | 95.17 |
| 4 | 004D04 | CSA1236 | Y36 | 94.52 |
| 5 | 004D06 | CSA1237 | Y37 | - |
| 6 | 004G03 | CSA1238 | Y38 | 93.68 |
| 7 | 004H01 | CSA1239 | Y39 | 93.68 |
| 8 | 005H08 | CSA1240 | Y40 | 93.29 |
| 9 | 007A06 | CSA1241 | Y41 | 94.6 |
| 10 | 007A12 | CSA1242 | Y42 | 94.84 |
| 11 | 007C01 | CSA1243 | Y43 | 96.46 |
| 12 | 007E01 | CSA1244 | Y44 | 95.35 |
| 13 | 007H05 | CSA1245 | Y45 | 95.25 |
| 14 | 008A10 | CSA1246 | Y46 | 95.39 |
| 15 | 008H04 | CSA1247 | Y47 | 96.01 |
| 16 | 008H07 | CSA1248 | Y48 | 94.51 |
| 17 | 008D08 | CSA1249 | Y49 | 91.65 |
| 18 | 008F11 | CSA1250 | Y50 | 93.09 |

### Experimental Example 5.2.4. Thermal Stability Analysis

Thermal stability was analyzed using a Protein Thermal Shift Dye Kit (Catalog No. 4461146, Thermo). The melting temperatures (Tm) of the candidate antibodies were graphed. The graph is shown in Figure 81. The melting temperatures of the candidate antibodies ranged from 73.8°C to 75.9°C.

### Experimental Example 5.2.5. Hydroxyproline Assay Collagen Level Measurement Results

Cells treated with bleomycin sulfate (bleomycin sulfate, Sigma) at a concentration of 2 µg/mL were cultured for 48 hours, then harvested, and a hydroxyproline assay was performed to measure collagen levels. The results were graphed and shown in Figure 82. As a result of comparing collagen levels by treating the existing commercially available antibody (REF) and each of the 17 candidate antibodies at a concentration of 5 µg/mL, it was confirmed that collagen levels were markedly increased in the bleomycin-treated group, but collagen levels were significantly reduced in the groups treated with the existing commercially available antibody (REF) and candidate antibodies Y34, Y38, Y40, Y41, Y43, Y44, Y45, Y47, Y48, Y49, and Y50, respectively. That is, it was confirmed that the candidate antibodies inhibited collagen synthesis by binding to CSF3 and inhibiting the CSF3 signaling pathway.

### Experimental Example 5.2.6. Gene Expression Analysis Results Using Real-Time PCR

Cells treated with bleomycin at a concentration of 2 µg/mL were cultured for 48 hours, then harvested, and the gene expression of α-SMA (A), Col1A1 (B), TGF-β (C), and IL-11 (D) was compared. The comparison results were graphed and shown in Figures 83 to 86.

As a result of comparing gene expression by treating the existing commercially available antibody (REF) and each of the 17 candidate antibodies at a concentration of 5 µg/mL, it was confirmed that the gene expression of α-SMA, Col1A1, TGF-β, and IL-11 was markedly increased in the bleomycin-treated group, but the gene expression of α-SMA, Col1A1, TGF-β, and IL-11 was significantly reduced in the groups treated with the existing commercially available antibody (REF) and candidate antibodies Y39, Y40, Y41, Y42, Y43, Y45, and Y47, respectively.

### Experimental Example 5.2.7. Confirmation of Pulmonary Fibrosis Marker Protein Expression

Cells treated with bleomycin at a concentration of 2 µg/mL were cultured for 48 hours, then harvested, and the protein expression of pulmonary fibrosis markers α-SMA and Col1A1 was compared.

Specifically, the expression of α-SMA and Col1A1 proteins was measured by Western blot, and the results are shown in Figure 87. Immunofluorescence staining was performed and photographs were taken under a fluorescence microscope to assess α-SMA and Col1A1 protein expression, and the results are shown in Figure 88. Here, IgG in Figures 87 and 88 is a control antibody (human IgG isotype) purchased from Invitrogen (31154).

The existing commercially available antibody (REF) and 4 candidate antibodies Y34, Y40, Y41, and Y47 were each treated and changes in α-SMA and Col1A1 protein expression were compared. As a result, it was confirmed that α-SMA and Col1A1 proteins were significantly reduced in the groups treated with the existing commercially available antibody (REF) and candidate antibodies Y34, Y40, Y41, and Y47, respectively, compared to the bleomycin-treated group. In addition, in fluorescence staining as well, it was confirmed that α-SMA and Col1A1 protein expression was reduced in the groups treated with the existing commercially available antibody (REF) and candidate antibodies Y34, Y40, Y41, and Y47, respectively, compared to the bleomycin group.

### Experimental Example 6. Epitope Analysis

The epitope of the Y41 antibody among the antibodies in Table 10 of Experimental Example 5.2 was analyzed. The epitope analysis process is as follows:
i) the mass of the Y41 antibody was analyzed;
ii) the mass of the antigen, human CSF3 protein, was analyzed;
iii) the mass of the complex formed by the antibody and antigen was analyzed;
iv) it was analyzed whether the enzymes used for epitope analysis can cover the entire sequence of the antigen; and
v) the enzymes were treated on the complex formed by the antibody and antigen, and the epitope was analyzed through the cross-linking positions of the detected peptides.

### Experimental Example 6.1. Experimental Methods and Materials

### Experimental Example 6.1.1. High-Mass MALDI MS Analysis

An AutoflexII MALDI ToF/ToF mass spectrometer (Bruker) equipped with CovalX's HM4 interaction module was used to examine the masses of the antibody, antigen, and the complex of the antibody and antigen.

### Experimental Example 6.1.2. High-Resolution Mass Analysis

nLC chromatography Ultimate 3000-RSLC (Thermo Scientific) and Orbitrap Q-Exactive Plus mass spectrometer (Thermo Scientific) were used for high-resolution analysis of the epitope.

### Experimental Example 6.2. Analysis of Antibody and Antigen

### Experimental Example 6.2.1. High-Mass MALDI MS Analysis

An AutoflexII MALDI ToF/ToF mass spectrometer (Bruker) equipped with CovalX's HM4 interaction module was used to examine the masses of the antibody, antigen, and the complex of the antibody and antigen.

### Experimental Example 6.2.1. Analysis of Antibody

The mass of the Y41 antibody was analyzed by high-mass MALDI mass spectrometry analysis. As a result of the analysis, only one major peak was detected, and the detected peak was MH+ = 149.092 kDa.

In addition, a cross-validation experiment was performed on the Y41 antibody, and as a result, only one major peak was detected, and the detected peak was MH+ = 154.856 kDa. Therefore, it is known that no aggregation was found for the Y41 antibody.

The Y41 antibody subject to analysis is a monoclonal antibody that specifically binds to human CSF3 protein, and the sequence information of the Y41 antibody is as follows.

The sequence of CDRH1 of the Y41 antibody is SEQ ID NO: 1.

The sequence of CDRH2 of the Y41 antibody is SEQ ID NO: 2.

The sequence of CDRH3 of the Y41 antibody is SEQ ID NO: 12.

The sequence of CDRL1 of the Y41 antibody is SEQ ID NO: 21.

The sequence of CDRL2 of the Y41 antibody is YAS (SEQ ID NO: 25).

The sequence of CDRL3 of the Y41 antibody is SEQ ID NO: 26.

The heavy chain sequence of the Y41 antibody is SEQ ID NO: 71.

The light chain sequence of the Y41 antibody is SEQ ID NO: 84.

### Experimental Example 6.2.2. Analysis of Antigen

The mass of the antigen (human CSF3 protein) was analyzed by high-mass MALDI mass spectrometry analysis. As a result of the analysis, only one major peak was detected, and the detected peak was MH+ = 19.349 kDa.

In addition, a cross-validation experiment was performed on the human CSF3 protein, and as a result, three major peaks were detected. The detected peaks were MH+ = 20.082 kDa, MH+ = 40.169 kDa, and MH+ = 59.934 kDa. Therefore, it is known that two non-covalent protein complexes (MH+ = 40.169 kDa and MH+ = 59.934 kDa) were detected for human CSF3 protein.

The antigen subject to analysis is human CSF3 protein, and the sequence of human CSF3 protein (NCBI Reference Sequence: NP_757373.1) is as follows:

MAGPATQSPMKLMALQLLLWHSALWTVQEATPLGPASSLPQSFLLKCLEQVRKIQGDGAALQEKLC
ATYKLCHPEELVLLGHSLGIPWAPLSSCPSQALQLAGCLSQLHSGLFLYQGLLQALEGISPELGPTLDTL
QLDVADFATTIWQQMEELGMAPALQPTQGAMPAFASAFQRRAGGVLVASHLQSFLEVSYRVLRHL
AQP (SEQ ID NO: 86).

### Experimental Example 6.2.3. Analysis of Complex of Antibody and Antigen

The mass of the complex (or conjugate) of the Y41 antibody and human CSF3 protein was analyzed by high-mass MALDI mass spectrometry analysis. Specifically, high-mass MALDI MS analysis was performed on a sample of a mixture of Y41 antibody and human CSF3 protein. As a result of the analysis, two non-covalent protein complexes were detected: MH+ = 169.136 kDa and MH+ = 188.994 kDa. MH+ = 169.136 kDa refers to a non-covalent protein complex composed of one human CSF3 protein and one Y41 protein, and MH+ = 188.994 kDa refers to a non-covalent protein complex composed of two human CSF3 proteins and one Y41 protein.

### Experimental Example 6.3. Epitope Analysis

To analyze the epitope on the antigen of the Y41 antibody at high resolution, the protein complex was incubated with a deuterium-labeled cross-linking agent, and multi-enzyme digestion was performed. After enriching the cross-linked peptides, the samples were analyzed by high-resolution mass spectrometry, and the generated data were analyzed using dedicated software.

The specific analysis method is as follows.

Human CSF3 protein (volume: 10 µL, concentration: 8 µM) and Y41 antibody (volume: 10 µL, concentration: 4 µM) were prepared and mixed to prepare an antigen/antibody mixture (volume: 20 µL, concentration: 4 µM/2 µM). Cross-linking, reduction/alkylation, and proteolysis were performed on the mixture. The 5 enzymes used were Trypsin, Chymotrypsin, ASP-N, Elastase, and Thermolysin. After proteolysis, 1 µL of each peptide solution generated was loaded onto a nano liquid chromatography system for mass spectrometry analysis.

The analysis results are as follows.

Seven cross-linked peptides were detected between human CSF3 protein and Y41 antibody. Table 12 below shows the sequences of the cross-linked peptides, the cross-linking positions, the types of enzymes used, the positions from which the antigen portion of the cross-linked peptides was derived, and the positions from which the antibody portion of the cross-linked peptides was derived. The content described in the peptide sequence in Table 12 means the "antibody sequence - antigen sequence - cross-linking position in the antibody sequence - cross-linking position in the antigen sequence" of the cross-linked peptide. For example, "YASSL (SEQ ID NO: 134) - KCLEQVRKIQGDGAALQEKL (SEQ ID NO: 135) - a3 - b19" means that in the cross-linked peptide, the sequence of the portion derived from the antibody is YASSL (SEQ ID NO: 134), the sequence of the portion derived from the antigen is KCLEQVRKIQGDGAALQEKL (SEQ ID NO: 135), and the 3rd residue of YASSL (SEQ ID NO: 134) (i.e., S) and the 19th residue of KCLEQVRKIQGDGAALQEKL (SEQ ID NO: 135) (i.e., K) are cross-linked.

**[Table 12]**

| **Peptide seq uence** | **Enzyme** | **Sequence p osition in the antibody region ( based on SEQ ID NO: 1 or 84)** | **Sequence p osition in the antigen region (based on SEQ ID NO: 86)** | **Cross-linkin g position in the antibody (based on SEQ ID NO: 1 or 8 4)** | **Cross-linkin g position in the antigen (based on SEQ ID NO: 86)** |
|---|---|---|---|---|---|
| YASSL(SEQ ID NO: 134)-KCLEQVRKI QGDGAALQ EKL(SEQ ID NO: 135)-a3-b19 | Chymotrypsin | 50-54 (light chain) | 46-65 | 52 (light chain) | 64 |
| MGWINPNS GGTIY(SEQ ID NO: 136)-ATYKLCHPEE (SEQ ID NO : 137)-a8-b7 | Thermolysin | 48-60 (heavy chain ) | 67-76 | 55 (heavy chain ) | 73 |
| KAPKLFIYYA (SEQ ID NO : 138)-CHPEELV(SE Q ID NO: 1 39)-a8-b2 | Elastase | 42-51 (light chain) | 72-78 | 49 (light chain) | 73 |
| MGWINPNS GGTIY(SEQ ID NO: 140)-LCHPEELV(S EQ ID NO: 141)-a8-b3 | Thermolysin | 48-60 (heavy chain ) | 71-78 | 55 (heavy chain ) | 73 |
| EWMGWINP NSGGTIY(SEQ ID NO: 1 42)-KLCHPEEL(S EQ ID NO: 143)-a10-b4 | Chymotrypsin | 46-60 (heavy chain) | 70-77 | 55 (heavy chain) | 73 |
| EWMGWINP NSGGTIYAQ KFQGRVTMT R(SEQ ID N O: 144)-ELGPTL(SEQ ID NO: 1 45)-a13-b5 | AspN | 46-72 (heavy chain ) | 128-133 | 58 (heavy chain ) | 132 |
| ATYYCQQGY SSPRTFGQG TK(SEQ ID NO: 146)-LDTLQLD(SE Q ID NO: 1 47)-a9-b3 | Thermolysin | 84-103 (light chain) | 133-139 | 92 (light chain) | 135 |
| ATYYCQQGY SSPRTFGQG TKVE(SEQ ID NO: 148)-LDTLQ(SEQ ID NO: 1 49)-a11-b3 | Thermolysin | 84-105 (light chain) | 133-137 | 94 (light chain) | 135 |
| MGWINPNS GGTIYAQKF (SEQ ID NO : 150)-QPTQGAMP AFASAFQRRAGGVL(SEQ ID NO: 1 51)-a11-b12 | Chymotrypsin | 48-64 (heavy chain ) | 161-182 | 58 (heavy chain ) | 172 |
| ATYYCQQGY SSPRTFGQG TK(SEQ ID NO: 152)-AFQRRAGG( SEQ ID NO: 153)-a10-b4 | Thermolysin | 84-103 (light chain) | 173-180 | 93 (light chain) | 176 |
| ATYYCQQGY SSPRTFGQG TKVE(SEQ ID NO: 154)-AFQRRAGG( SEQ ID NO: 155)-a10-b4 | Thermolysin | 84-105 (light chain) | 173-180 | 93 (light chain) | 176 |
| ASQTISNR(S EQ ID NO: 156)-AFQRRAGG( SEQ ID NO: 157)-a2-b4 | Thermolysin | 25-32 (light chain) | 173-180 | 26 (light chain) | 176 |
| ASGYTFTNY GISWLR(SEQ ID NO: 1 58)-RAGGVLVAS HLQSFLEVSY R(SEQ ID N O: 159)-a4-b1 | Trypsin | 24-38 (heavy chain ) | 177-196 | 27 (heavy chain ) | 177 |
| CQQGYSSPRTFGQGTKVEI K(SEQ ID N O: 160)-QRRAGGVLV ASHL(SEQ I D NO: 161)-a6-b3 | Chymotrypsin | 88-107 (light chain) | 175-187 | 93 (light chain) | 177 |
| ASGYTFTNY GISWLR(SEQ ID NO: 16 2)-RAGGVLV ASHLQSFLE VSYR(SEQ I D NO: 163) -a7-b1 | Trypsin | 24-38 (light chain) | 177-196 | 30 (light chain) | 177 |

From the analysis results, the positions of the amino acid residues at which the antibody interacts with the antigen were found to be the 64th, 73rd, 132nd, 135th, 172nd, 176th, and 177th amino acid residues of human CSF3 protein (SEQ ID NO: 86).

## Claims

1. An anti-CSF3 (Colony-Stimulating Factor 3) antibody for use in treating fibrosis, wherein the tissue in which the fibrosis occurs is any one selected from skin, liver, brain, nervous system, heart, bone marrow, gut, joint, eye, kidney, retroperitoneum, mediastinum, and pancreas.

2. The anti-CSF3 antibody for use in treating fibrosis according to claim 1, wherein the anti-CSF3 antibody is capable of binding to a human CSF3 protein consisting of the amino acid sequence of SEQ ID NO: 86.

3. The anti-CSF3 antibody for use in treating fibrosis according to any one of claims 1 to 2, wherein the anti-CSF3 antibody induces degradation of extracellular matrix accumulated in the tissue in which the fibrosis occurs.

4. The anti-CSF3 antibody for use in treating fibrosis according to claim 3, wherein the extracellular matrix accumulated in the tissue in which the fibrosis occurs is collagen.

5. The anti-CSF3 antibody for use in treating fibrosis according to any one of claims 1 to 4, wherein the anti-CSF3 antibody inactivates myofibroblasts in the tissue in which the fibrosis occurs.

6. The anti-CSF3 antibody for use in treating fibrosis according to claim 5, wherein inactivating myofibroblasts in the tissue in which the fibrosis occurs means at least one selected from the following: (a) degrading extracellular matrix secreted by the myofibroblasts; (b) inhibiting extracellular matrix secretion by the myofibroblasts; and (c) reducing the number of myofibroblasts in the tissue in which the fibrosis occurs.

7. The anti-CSF3 antibody for use in treating fibrosis according to claim 6, wherein (c) occurs by reversion of the myofibroblasts to epithelial cells or fibroblasts.

8. The anti-CSF3 antibody for use in treating fibrosis according to any one of claims 1 to 7, wherein the anti-CSF3 antibody induces the tissue in which the fibrosis occurs to return to normal tissue.

9. The anti-CSF3 antibody for use in treating fibrosis according to any one of claims 1 to 8, wherein the anti-CSF3 antibody inhibits activation of a TGF-β-mediated signaling pathway and a non-TGF-β-mediated signaling pathway.

10. The anti-CSF3 antibody for use in treating fibrosis according to claim 9, wherein activation of the TGF-β-mediated signaling pathway induces at least one of epithelial to mesenchymal transition (EMT), fibroblast-to-myofibroblast transdifferentiation (FMT), and extracellular matrix deposition by transforming growth factor beta (TGF-β).

11. The anti-CSF3 antibody for use in treating fibrosis according to any one of claims 9 to 10, wherein activation of the non-TGF-β-mediated signaling pathway induces at least one of epithelial to mesenchymal transition (EMT), fibroblast-to-myofibroblast transdifferentiation (FMT), and extracellular matrix deposition by interaction between CSF3 and CSF3R.

12. A method of preventing or treating fibrosis, comprising: administering a therapeutically effective amount of the anti-CSF3 antibody of any one of claims 1 to 11 to a subject, wherein the tissue in which the fibrosis occurs is any one selected from skin, liver, brain, nervous system, heart, bone marrow, gut, joint, eye, kidney, retroperitoneum, mediastinum, and pancreas.

13. A pharmaceutical composition for treating fibrosis, comprising: a therapeutically effective amount of the anti-CSF3 antibody of any one of claims 1 to 11; and a pharmaceutically acceptable carrier.

14. The pharmaceutical composition for treating fibrosis according to claim 13, wherein the pharmaceutically acceptable carrier is at least one selected from the following: binders such as lactose, sucrose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol wax; sweeteners; flavoring agents; syrups; liquid carriers such as fatty oils; sterilized aqueous solutions; propylene glycol; polyethylene glycol; injectable esters such as ethyl oleate; suspending agents; emulsifiers; lyophilized preparations; topical preparations; stabilizers; buffers; animal oils; vegetable oils; waxes; paraffin; starch; tragacanth; cellulose derivatives; polyethylene glycol; silicone; bentonite; silica; talc; zinc oxide; and suitable combinations thereof.

15. Use of the anti-CSF3 antibody of any one of claims 1 to 11 for the manufacture of a medicament for treating fibrosis.

16. A method of inhibiting extracellular matrix deposition and epithelial to mesenchymal transition (EMT) in a tissue of a subject, the method comprising: administering an anti-CSF3 (Colony-Stimulating Factor 3) antibody to the subject, wherein the tissue is a tissue at risk of extracellular matrix deposition and EMT, and wherein the anti-CSF3 antibody is capable of inhibiting i) a TGF-β-mediated signaling pathway that induces extracellular matrix deposition and EMT, and ii) a non-TGF-β-mediated signaling pathway that induces extracellular matrix deposition and EMT.

17. The method of inhibiting extracellular matrix deposition and epithelial to mesenchymal transition according to claim 16, wherein inhibition of both the TGF-β-mediated signaling pathway and the non-TGF-β-mediated signaling pathway by the anti-CSF3 antibody results in greater reduction in expression levels of CSF3, TGF-β, α-SMA, COL1A1, and IL-11 compared to inhibition of the TGF-β-mediated signaling pathway alone.

18. The method of inhibiting extracellular matrix deposition and epithelial to mesenchymal transition according to any one of claims 16 to 17, wherein the tissue is any one of skin, brain, nervous system, heart, bone marrow, liver, gut, joint, eye, lung, kidney, retroperitoneum, mediastinum, and pancreas.

19. An anti-CSF3R (Colony-Stimulating Factor 3 Receptor) antibody for use in treating fibrosis, wherein the tissue in which the fibrosis occurs is any one selected from skin, liver, brain, nervous system, heart, bone marrow, gut, joint, eye, kidney, retroperitoneum, mediastinum, and pancreas.

20. The anti-CSF3R antibody for use in treating fibrosis according to claim 19, wherein the anti-CSF3R antibody is capable of binding to a human CSF3R protein consisting of the amino acid sequence of SEQ ID NO: 86.

21. The anti-CSF3R antibody for use in treating fibrosis according to any one of claims 19 to 20, wherein the anti-CSF3R antibody induces degradation of extracellular matrix accumulated in the tissue in which the fibrosis occurs.

22. The anti-CSF3R antibody for use in treating fibrosis according to claim 21, wherein the extracellular matrix accumulated in the tissue in which the fibrosis occurs is collagen.

23. The anti-CSF3R antibody for use in treating fibrosis according to any one of claims 19 to 22, wherein the anti-CSF3R antibody inactivates myofibroblasts in the tissue in which the fibrosis occurs.

24. The anti-CSF3R antibody for use in treating fibrosis according to claim 23, wherein inactivating myofibroblasts in the tissue in which the fibrosis occurs means at least one selected from the following: (a) degrading extracellular matrix secreted by the myofibroblasts; (b) inhibiting extracellular matrix secretion by the myofibroblasts; and (c) reducing the number of myofibroblasts in the tissue in which the fibrosis occurs.

25. The anti-CSF3R antibody for use in treating fibrosis according to claim 24, wherein (c) occurs by reversion of the myofibroblasts to epithelial cells or fibroblasts.

26. The anti-CSF3R antibody for use in treating fibrosis according to any one of claims 19 to 25, wherein the anti-CSF3R antibody induces the tissue in which the fibrosis occurs to return to normal tissue.

27. The anti-CSF3R antibody for use in treating fibrosis according to any one of claims 19 to 26, wherein the anti-CSF3R antibody inhibits activation of a TGF-β-mediated signaling pathway and a non-TGF-β-mediated signaling pathway.

28. The anti-CSF3R antibody for use in treating fibrosis according to claim 19, wherein activation of the TGF-β-mediated signaling pathway induces at least one of epithelial to mesenchymal transition (EMT), fibroblast-to-myofibroblast transdifferentiation (FMT), and extracellular matrix deposition by interaction between transforming growth factor beta (TGF-β) and its receptor.

29. The anti-CSF3R antibody for use in treating fibrosis according to any one of claims 27 to 28, wherein activation of the non-TGF-β-mediated signaling pathway induces at least one of epithelial to mesenchymal transition (EMT), fibroblast-to-myofibroblast transdifferentiation (FMT), and extracellular matrix deposition by interaction between CSF3 and CSF3R.

30. A method of preventing or treating fibrosis, comprising: administering a therapeutically effective amount of the anti-CSF3R antibody of any one of claims 19 to 29 to a subject, wherein the tissue in which the fibrosis occurs is any one selected from skin, liver, brain, nervous system, heart, bone marrow, gut, joint, eye, kidney, retroperitoneum, mediastinum, and pancreas.

31. A pharmaceutical composition for treating fibrosis, comprising: a therapeutically effective amount of the anti-CSF3R antibody of any one of claims 19 to 29; and a pharmaceutically acceptable carrier.

32. The pharmaceutical composition for treating fibrosis according to claim 31, wherein the pharmaceutically acceptable carrier is at least one selected from the following: binders such as lactose, sucrose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol wax; sweeteners; flavoring agents; syrups; liquid carriers such as fatty oils; sterilized aqueous solutions; propylene glycol; polyethylene glycol; injectable esters such as ethyl oleate; suspending agents; emulsifiers; lyophilized preparations; topical preparations; stabilizers; buffers; animal oils; vegetable oils; waxes; paraffin; starch; tragacanth; cellulose derivatives; polyethylene glycol; silicone; bentonite; silica; talc; zinc oxide; and suitable combinations thereof.

33. Use of the anti-CSF3R antibody of any one of claims 19 to 29 for the manufacture of a medicament for treating fibrosis.

34. A method of inhibiting extracellular matrix deposition and epithelial to mesenchymal transition (EMT) in a tissue of a subject, the method comprising: administering an anti-CSF3R (Colony-Stimulating Factor 3 Receptor) antibody to the subject, wherein the tissue is a tissue at risk of extracellular matrix deposition and EMT, and wherein the anti-CSF3R antibody is capable of inhibiting i) a TGF-β-mediated signaling pathway that induces extracellular matrix deposition and EMT, and ii) a non-TGF-β-mediated signaling pathway that induces extracellular matrix deposition and EMT.

35. The method of inhibiting extracellular matrix deposition and epithelial to mesenchymal transition according to claim 34, wherein inhibition of both the TGF-β-mediated signaling pathway and the non-TGF-β-mediated signaling pathway by the anti-CSF3R antibody results in greater reduction in expression levels of CSF3, TGF-β, α-SMA, COL1A1, and IL-11 compared to inhibition of the TGF-β-mediated signaling pathway alone.

36. The method of inhibiting extracellular matrix deposition and epithelial to mesenchymal transition according to any one of claims 34 to 35, wherein the tissue is any one of skin, brain, nervous system, heart, bone marrow, liver, gut, joint, eye, lung, kidney, retroperitoneum, mediastinum, and pancreas.
